# EUROPEAN PATENT APPLICATION

(11) **EP 4 644 375 A1**
(43) Date of publication of application: **05.11.2025**
(21) Application number: 24173078.7
(22) Date of filing: 29.04.2024
(51) Int. Cl.: C07D 215/22, C07D 401/12, C07D 405/12, C07D 409/12, C07D 413/12, A61P 35/00, A61K 31/4709

(54) **SUBSTITUTED HYDROQUINOLINES FOR THE TREATMENT OF GLIOBLASTOMA MULTIFORME**

(71) Applicant: Molius Saglik Teknolojileri Anonim Sirketi, Ümraniye/Istanbul (TR)
(72) Inventor: MARDINOGLU, Adil, 412 60 Göteborg (SE); HANASHALSHAHABY, Essam, Istanbul (TR); ABAD, Nadeem, Istanbul (TR); BUHLAK, Shafeek, Istanbul (TR); AKACHAR, Jihane, Istanbul (TR); SAFFOUR, Sana, Istanbul (TR)
(74) Representative: Kransell & Wennborg KB

(57) **Abstract**

There is provided a compound according to formula (I) 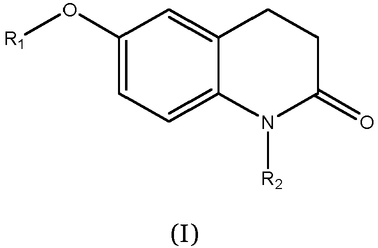 or a pharmaceutically acceptable salt or prodrug thereof.

## Description

### TECHNICAL FIELD

The present disclosure relates to the field of small compounds, in particular small compounds for use in a method of treatment of glioma.

### BACKGROUND

Glioma is a broad term used to describe primary brain tumors classified according to their source cell. These include astrocytomas (astrocytomas, anaplastic astrocytomas, glioblastomas), oligodendrogliomas, ependymomas, and mixed gliomas (Agnihotri S et al., 2013). These are the most common tumors of the central nervous system (CNS), accounting for approximately 80% of all primary malignancies of the brain (Hanif et al., 2017). Glioblastoma multiforme (GBM) is the most malignant and most common form of primary astrocytoma (Mardinoglu A. et al., 2020). It constitutes more than 60% of all brain tumors in adults.

GBM is a disease that exhibits significant heterogeneity when it comes to genetic variations and cell origins. (Alcantara Llaguno and Parada, 2016). As part of GBM carcinogenesis, several of the cell systems undergo significant reprogramming and the metabolic system exhibits some of the most remarkable changes (Agnihotri and Zadeh, 2016).

GBM accounts for 50% of all gliomas in all age groups. Although it can occur at any age, its incidence peaks between 55 and 60 years of age. GBM cells are highly pathogenic and cannot be completely removed because they grow in normal brain tissue. The success rate of the treatments developed in the last decade is very low and the average patient survival time is usually about 14-15 months from diagnosis. In addition, GBM cells have developed resistance to the current multimodal treatment regimen consisting of the alkylating agent temozolomide (TMZ) and radiation. Because many targeted therapies do not effectively cross the blood-brain barrier (BBB), positive results have not been achieved in clinical trials. (Fang, C. et al, 2015). Taken together, these findings warrant the development of new therapeutic approaches to treat GBM.

### SUMMARY

The present inventors realized that there is a need for further development of new therapeutic approaches to treat glioma such as GBM. The present inventors thus hypothesized that by derivatizing cilostazol, a drug known to pass the blood-brain barrier (BBB), novel small compounds with the ability to pass the blood-brain barrier and the potential to reduce proliferation of glioblastoma cells could be obtained.

According to a first aspect, there is provided a compound according to formula (I) 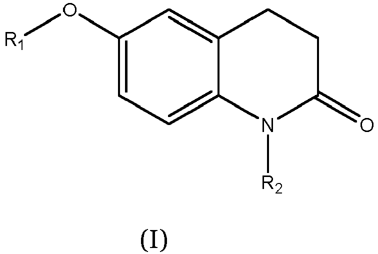 or a pharmaceutically acceptable salt or prodrug thereof;
wherein R1 is selected from optionally branched C3-C7 alkyl, optionally branched C2-C7 alkenyl, optionally substituted benzyl, 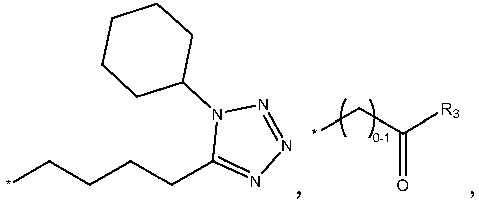 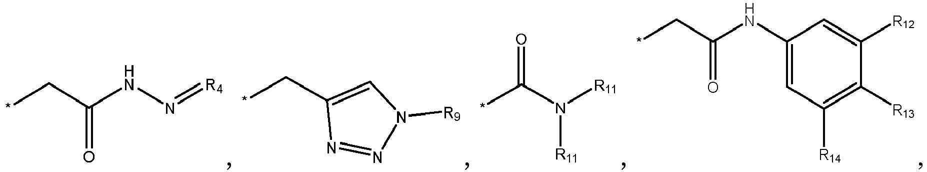 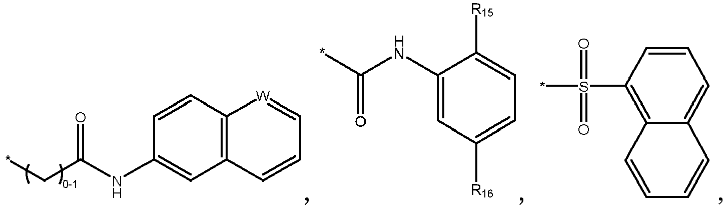 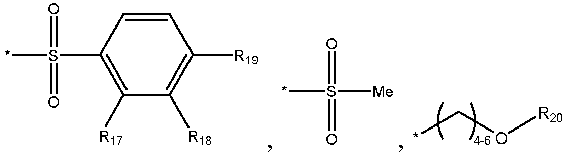 and 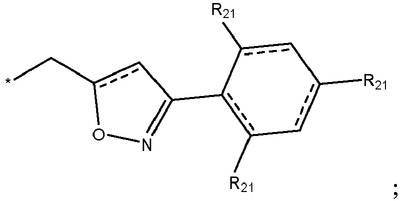
-̅ -̅ -̅ -̅ -̅ is independently a single bond or a double bond;
W is independently selected from carbon and nitrogen;
R3 is selected from optionally substituted naphthalene, 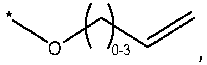 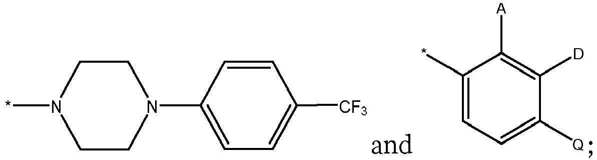
A is selected from -H, -X and -OMe ;
D is selected from -H, -X, -OMe and -CX₃;
Q is selected from -H and -OMe;
X is a halogen;
R4 is selected from 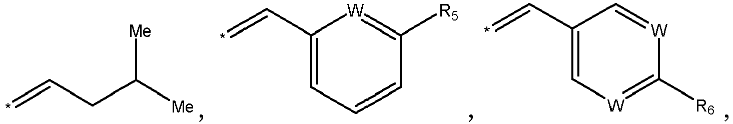 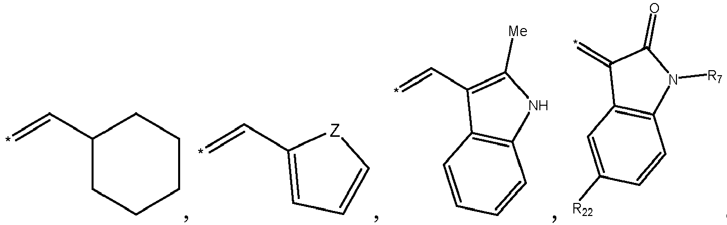 and 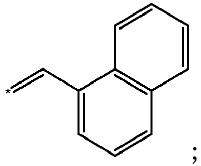
Z is selected from oxygen and sulphur;
R5 is selected from -H and 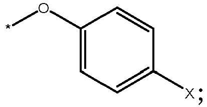
R6 is selected from -H, -X, C3 alkyl, -NO₂ and 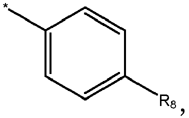
R7 is selected from -H, -Me;
R8 is selected from -H and -X;
R9 is selected from C3-C6 alkyl, 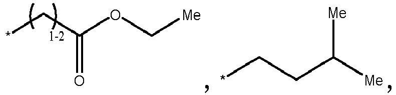 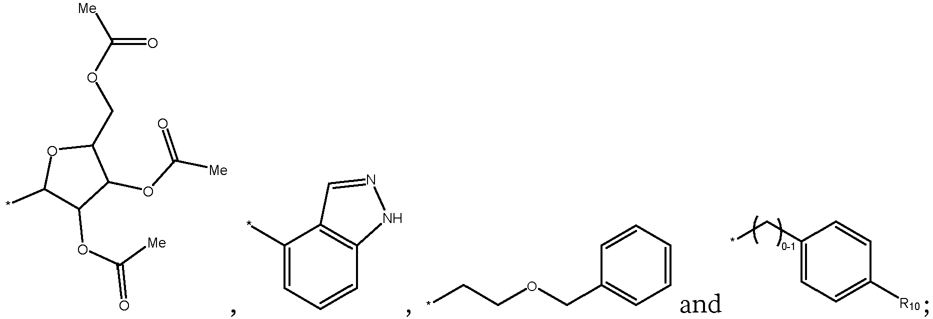
R10 is selected from -X and acetyl;
R11 is selected from a phenyl, C1-C3 alkyl and isopropyl;
R12 is selected from -H and -OMe;
R13 is selected from -OMe, phenyl, acetyl, morpholine and N-methyl piperazine;
R14 is selected from -H and -OMe provided that when R12 is -H, R13 and/or R14 is also -H;
R15 is selected from -H, -X, -Me, -OMe and -CF₃;
R16 is selected from -H and-X;
R17 is selected from -H and-X;
R18 is selected from -H, -X and -OMe;
R19 is selected from -H, -NO₂, -OMe and-X;
R20 is selected from 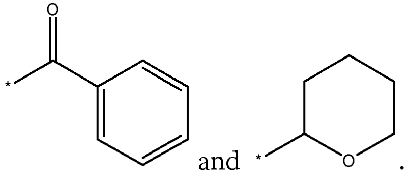
R21 is independently selected from -H and -X;
R22 is selected from -X and -NO₂;
R2 is selected from -H, optionally branched C3-C7 alkyl, C3-C5 alkenyl, 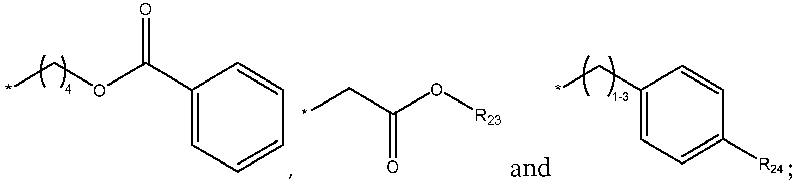
R23 is selected from C1-C4 alkyl and C3-C4 alkenyl; and
R24 is selected from -H,-F, phenyl and C2-C4 alkyl.

Treatment with a compound according to the first aspect has the ability to reduce the proliferation of glioblastoma cells. Furthermore, it has been shown that treating rats with induced GBM with a compound according to the first aspect significantly prolongs their survival and decelerates or reduces tumor growth. A compound of the first aspect is thus well-suited for use in a method of treatment of glioblastomas such as GBM.

According to a second aspect, there is provided a pharmaceutical composition comprising a compound according to the first aspect.

According to a third aspect, there is provided a compound according to the first aspect or a pharmaceutical composition according to the second aspect for use as a medicament.

According to a fourth aspect, there is provided a compound according to the first aspect or a pharmaceutical composition according to the second aspect for use in a method of treatment of glioma.

In an embodiment of the fourth aspect, the compound or the pharmaceutical composition is used in a method of treatment of glioblastoma, such as glioblastoma multiforme.

### BRIEF DESCRIPTION OF THE DRAWINGS

Fig 1 shows the alkylation reactions for synthesizing 6-hydroxy-3,4-dihydroquinolin-2(1H)-one analogues.
Fig 2 shows the acylation reactions for synthesizing 6-hydroxy-3,4-dihydroquinolin-2(1H)-one analogues.
Fig 3 shows the hydrazide formation for synthesizing 6-hydroxy-3,4-dihydroquinolin-2(1H)-one analogues.
Fig 4 shows the synthesis of 2-((2-oxo-1,2,3,4-tetrahydroquinolin-6-yl)oxy) acetohydrazide-hydrazone analogues.
Fig 5 shows the synthesis of triazole analogues of 6-(prop-2-yn-1-yloxy)-3,4-dihydroquinolin-2(1H)-one.
Fig 6 shows the synthesis of oxazole analogues of allyl or propargyl dihydroquinolin-2(1H)-one derivatives.
Fig 7 shows the synthesis of 2-((2-oxo-1,2,3,4-tetrahydroquinolin-6-yl)oxy) carbamate derivatives.
Fig 8 shows the synthesis of 2-((2-oxo-1,2,3,4-tetrahydroquinolin-6-yl)oxy) acetamide derivatives.
Fig 9 shows the synthesis of 2-((2-oxo-1,2,3,4-tetrahydroquinolin-6-yl)oxy) carbamate derivatives from isocyanate.
Fig 10 shows the synthesis of sulfonate derivatives of 6-hydroxy-3,4-dihydroquinolin-2(1H)-one.
Fig 11 shows the timeline diagram of the *in Vivo* study.
Fig 12 shows the IC₅₀ values of compounds TR-G053 and TR-G078 in U87 and IC₅₀ values of TR-G053 and TR-G052 in U138-MG cell lines.
Fig 13 shows the IC₅₀ values of TR-G143, TR-G089 and TR-G075 in U87-MG and IC₅₀ values of TR-G143, TR-G131 and TR-G136 in U138-MG cell lines.
Fig 14 shows the IC₅₀ values of TR-G091, TR-G002 and TR-G045 scale-up compounds in U87 and IC₅₀ values of TR-G091, TR-G002 and TR-G045 scale-up compounds in U138-MG cell lines.
Fig 15 shows the IC₅₀ values of TR-G103, TR-G066 and TR-G141 in U87 and IC₅₀ values of TR-G050, TR-G066 and TR-G027 in U138-MG cell lines.
Fig 16 shows the IC₅₀ values of TR-G138 in U87 and TR-G145 in both U87-MG and U138-MG cell lines.
Fig 17-19 shows the permeability rates of compounds through the blood-brain-barrier mimicking membrane.
Fig 20-22 shows the permeability rates of compounds through the gastrointestinal mimicking membrane.
Fig 23 shows the survival of rats in the *in Vivo* study.
Fig 24 shows the MRI follow-up of tumor progression in the *in Vivo* study after treatment with target compounds and TMZ, respectively.
Fig 25 shows the relative tumor progression in the *in Vivo* study after treatment with target compounds and TMZ, respectively.
Fig 26 shows the MRI of the rat brain with GBM. (a) Axial T2 weighted MRI image on the 14^{th} day after implantation; (b) Axial T2 weighted MRI image on the 28^{th} day after implantation; (c) Coronal T2 weighted MRI image on the 14^{th} day after implantation; (d) Coronal T2 weighted MRI image on the 28^{th} day after implantation.
Fig 27 shows the MRI of the rat brain with GBM treated with TMZ. (a) Axial T2 weighted MRI image on the 14^{th} day after implantation; (b) Axial T2 weighted MRI image after treatment with TMZ (28^{th} day); (c) Coronal T2 weighted MRI image on the 14^{th} day after implantation; (d) Coronal T2 weighted MRI image after treatment with TMZ (28^{th} day).
Fig 28 shows the MRI of the rat brain with GBM treated with TR-G002. (a) Axial T2 weighted MRI image on the 14t^{h} day after implantation; (b) Axial T2 weighted MRI image after treatment with TR-G002 (28^{th} day); (c) Coronal T2 weighted MRI image on the 14^{th} day after implantation; (d) Coronal T2 weighted MRI image after treatment with TR-G002 (28^{th} day).
Fig 29 shows the MRI of the rat brain with GBM treated with TR-G045. (a) Axial T2 weighted MRI image on the 14^{th} day after implantation; (b) Axial T2 weighted MRI image after treatment with TR-G045 (28^{th} day); (c) Coronal T2 weighted MRI image on the 14^{th} day after implantation; (d) Coronal T2 weighted MRI image after treatment with TR-G045 (28^{th} day).
Fig 30 shows the MRI of the rat brain with GBM treated with TR-G053. (a) Axial T2 weighted MRI image on the 14^{th} day after implantation; (b) Axial T2 weighted MRI image after treatment with TR-G053 (28^{th} day); (c) Coronal T2 weighted MRI image on the 14^{th} day after implantation; (d) Coronal T2 weighted MRI image after treatment with TR-G053 (28^{th} day).
Fig 31 shows the MRI of the rat brain with GBM treated with TR-G066. (a) Axial T2 weighted MRI image on the 14^{th} day after implantation; (b) Axial T2 weighted MRI image after treatment with TR-G066 (28^{th} day); (c) Coronal T2 weighted MRI image on the 14^{th} day after implantation; (d) Coronal T2 weighted MRI image after treatment with TR-G066 (28^{th} day).
Fig 32 shows the MRI of the rat brain with GBM treated with TR-G071. (a) Axial T2 weighted MRI image on the 14^{th} day after implantation; (b) Axial T2 weighted MRI image after treatment with TR-G071 (28^{th} day); (c) Coronal T2 weighted MRI image on the 14^{th} day after implantation; (d) Coronal T2 weighted MRI image after treatment with TR-G071 (28^{th} day).
Fig 33 shows the MRI of the rat brain with GBM treated with TR-G091. (a) Axial T2 weighted MRI image on the 14^{th} day after implantation; (b) Axial T2 weighted MRI image after treatment with TR-G091 (28^{th} day); (c) Coronal T2 weighted MRI image on the 14^{th} day after implantation; (d) Coronal T2 weighted MRI image after treatment with TR-G091 (28^{th} day).
Fig 34 shows brain tissues, GBM H&E, 10X Bar:70µm, 40X Bar: 40µm, Nuclear protein Ki67 (Ki67), Cytidine/uridine monophosphate kinase 2 (CMPK2) and Glial fibrillary acidic protein (GFAP) expressions, IHC-P, Bar: 40µm.
Fig 35 shows the histopathological findings observed in brain tissues. (A) Tumor Mass: (****p < 0.0001, T02 vs T66 *p = 0.0402, TMZ vs T66 *p = 0.0465, ns: no significant difference = p > 0.9999), (B) Focus of Necrosis: (****p < 0.0001, ***p = 0.0004), (C) Vascular Proliferation: (****p < 0.0001, *p = 0.0154, ns: no significant difference p = 0.6780), (D) Cellular Proliferation: (****p < 0.0001, **p = 0.0022, ns: no significant difference p = 0.9959).
Fig 36 shows the brain tissues, GBM, NOP10 and H2A.X expressions in neurons, IF, Bar: 50µm.
Fig 37 shows the glioblastoma immunohistochemical and immunofluorescence staining data and statistical analysis findings. (A) Ki67: (****p < 0.0001, ***p = 0.0002, **p = 0.0019, ns: no significant difference = 0.1125), (B) CMPK2: (****p < 0.0001, ***p = 0.0008, ns: no significant difference = 0.7175), (C) GFAP: (****p < 0.0001, *p = 0.0171, ns: no significant difference = 0.3553), (D) NOP10: (****p < 0.0001, **p = 0.0039, ns: no significant difference = 0.2536), (E) H2A.X: (****p < 0.0001, **p = 0.0040, *p = 0.0152, ns: no significant difference = 0.1232)

### DETAILED DESCRIPTION

Glioblastoma multiforme (GBM) is the most malignant and most common form of primary astrocytoma (Mardinoglu A. et al., 2020). It constitutes more than 60% of all brain tumors in adults. It is thus crucial to develop new therapeutic treatments with improved efficacy.

Advances in biotechnology have resulted in the creation of new high-throughput analytical methods that can provide large amounts of data. These approaches often focus on studying the genome, transcriptome, and proteome, resulting in a variety of omics data. Systems biologists interpret omics data to gain biological understanding using statistical analysis, network development, and mathematical modelling (Nielsen and Hohmann, 2017). A new and specific treatment target can be identified and validated with multi-omics data. In target determination, firstly, a preliminary list is created according to the differences in expression level in the patient population in the results of multi-omics data. Candidate targets are evaluated if they can be used as therapeutic targets based on their expression in different tissues, their expression in known cancer cell lines and other literature information that is available. This systems biology approach has been applied to GBM and the CMPK2 protein was found to be a candidate target for the treatment of GBM.

CMPK2, encodes mitochondrial UMP-CMP kinase 2 and may have a function in the synthesis of dUTP and dCTP (UniProt Consortium, T., 2018). In terms of function, CMPK2 appears to be an anomaly. dUTP and dCTP nucleotides are thought to be involved in mitochondrial synthesis (UniProt Consortium, 2018). Since CMPK2 is a critical gene in DNA synthesis, it is required for cell development, which is increased in tumor cells but not in normal brain cells. According to the findings, CMPK2 is involved in the cellular response to a viral infection. Thus, when examining the CMPK2 co-expression cluster and performing enrichment analysis on the 90 included genes, both enriched KEGG pathways found are fully linked to viral infection and cellular defence response. The presence of human cytomegalovirus (HCMV) in brain tumor cells and its possible relevance in the formation and progression of GBM has been previously implicated in GBM research.

In silico computational chemistry methods including molecular docking and ADME (Absorption, Distribution, Metabolism and Elimination) properties of innovative molecules designed by derivatization were analyzed in silico using the Autodock VIna and SwissADME, PreADMET database. The binding sites of the designed compounds in the protein-ligand complex were determined; molecular interactions were described in silico docking, bonding, and interaction analyses.

In order to develop new effective compounds for use in the treatment of glioblastomas, the present inventors derivatized a known drug, Cilostazol, that is known to pass the blood-brain barrier. Cilostazol, an oral anti-platelet drug, is a specific cAMP phosphodiesterase type III inhibitor and has been clinically used to treat thrombosis patients. It has further been shown that Cilostazol can suppress colon cancer cell motility and might be effective as an anti-metastasis drug for cancer patients. Metastasis of cancer cells is initiated by the cellular migration into extracellular matrix and surrounding vessels and drugs that can elevate cAMP levels in cancer cells effectively may be applied to prevent metastasis in cancer patients. (Murata, Kohei, et al. 1999). Furthermore, the big-conductance calcium-activated potassium channel (BK channel) is highly expressed and activated in GBM. Cilostazol is a phosphodiesterase-3 inhibitor and has been shown to reverse CIIR-induced BK channel inactivation. Cilostazol inhibited CIIR-induced cell motility, proliferation, delayed tumor *in vivo* growth, the ability to form tumor spheres and prolonged survival. As such, inactivation of the BK channel can assist GBM in developing radiation resistance (Liu, Chan-Chuan, et al., 2021). Cilostazol has further been shown to reduce intracellular Aβ levels and phosphorylated tau levels in activated neuro2A (N2a) Swe cells, and significantly improved spatial learning and memory in the Aβ-injected mice (Park SH, Kim JH, et al.2011).

According to a first aspect, there is provided a compound according to formula (I) 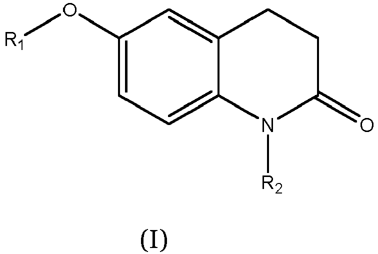 or a pharmaceutically acceptable salt or prodrug thereof;
wherein R1 is selected from optionally branched C3-C7 alkyl, optionally branched C2-C7 alkenyl, optionally substituted benzyl, 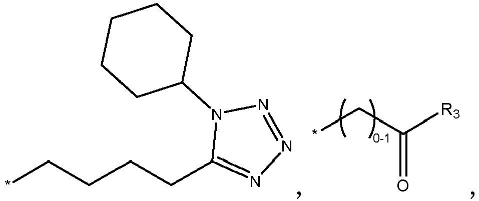 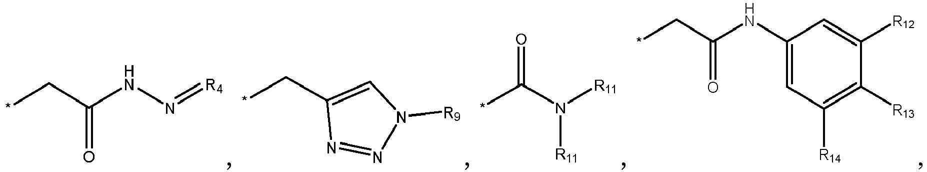 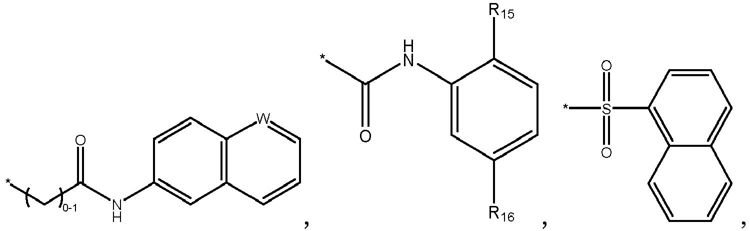 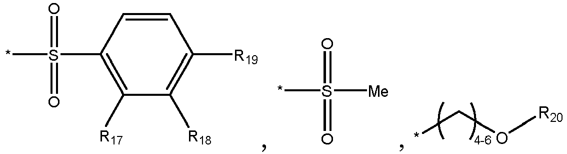 and 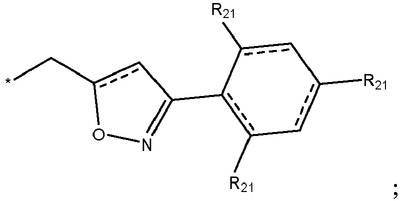
-̅-̅-̅-̅-̅ is independently a single bond or a double bond;
W is independently selected from carbon and nitrogen;
R3 is selected from optionally substituted naphthalene 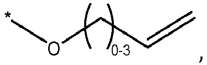 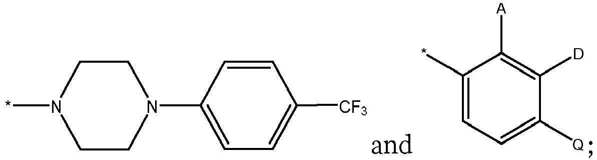
A is selected from -H, -X and -OMe ;
D is selected from -H, -X, -OMe and -CX₃;
Q is selected from -H and -OMe;
X is a halogen;
R4 is selected from 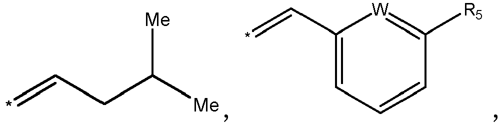 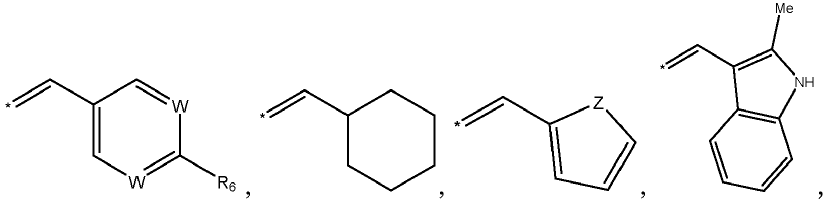 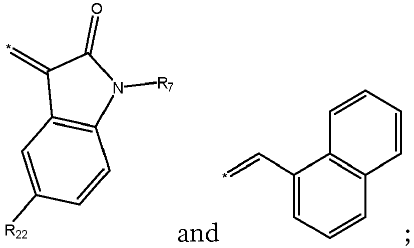
Z is selected from oxygen and sulphur;
R5 is selected from -H and 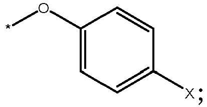
R6 is selected from -H, -X, C3 alkyl, -NO₂ and 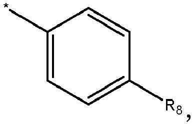
R7 is selected from -H, -Me;
R8 is selected from -H and -X;
R9 is selected from C3-C6 alkyl, 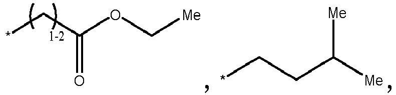 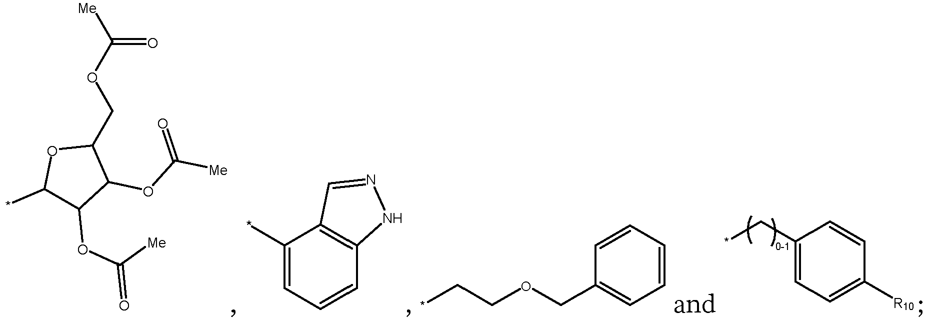
R10 is selected from -X and acetyl;
R11 is selected from a phenyl, C1-C3 alkyl and isopropyl;
R12 is selected from -H and -OMe;
R13 is selected from -OMe, phenyl, acetyl, morpholine and N-methyl piperazine;
R14 is selected from -H and -OMe provided that when R12 is -H, either R13 and/or R14 is also -H;
R15 is selected from -H,-X, -Me, -OMe and -CF₃;
R16 is selected from -H and-X;
R17 is selected from -H and-X;
R18 is selected from -H, -X and -OMe;
R19 is selected from -H, -NO₂, -OMe and-X;
R20 is selected from 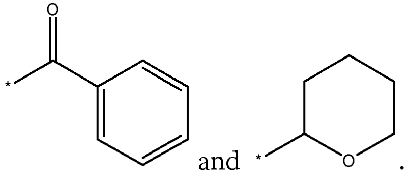
R21 is independently selected from -H and -X;
R22 is selected from -X and -NO₂;
R2 is selected from -H, optionally branched C3-C7 alkyl, C3-C5 alkenyl, 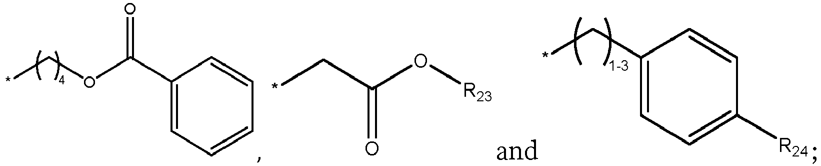
R23 is selected from C1-C4 alkyl and C3-C4 alkenyl; and
R24 is selected from -H,-F, phenyl and C2-C4 alkyl.

Treatment with a compound according to the first aspect is associated with a significant reduction in the proliferation of glioblastoma cells. Furthermore, treatment with a compound according to the first aspect is further associated with a prolonged survival and deceleration or reduction of tumor growth in rats with induced GBM. These compounds are thus particularly well-suited for use in a pharmacological treatment of glioblastoma such as GBM.

Below, various embodiments of the first aspect as well as certain compounds identified in the Examples section are discussed.

In one embodiment, R2 is -H. This embodiment is associated with some of the most potent compounds and treatment with these compounds results in an especially large reduction in the proliferation of glioblastoma cells as well as a prolonged survival and a deceleration in tumor growth in rats with induced GBM. Compounds such as TR-G002, TR-G014, TR-G045, TR-G-053, TR-G066, TR-G078 and TR-G143 are included in this embodiment and are shown to be exceptionally effective since reduced proliferation of glioblastoma cells is obtained *in vitro* together with a prolonged survival and deceleration in tumor growth in rats with induced GBM.

In another embodiment, R2 is selected from 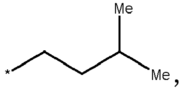 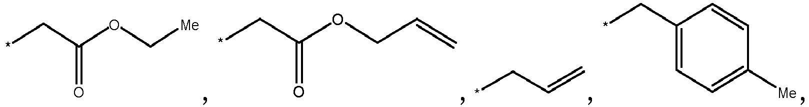 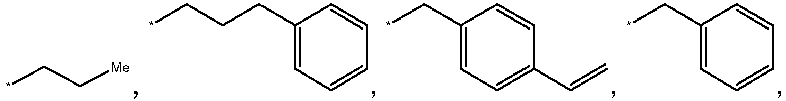 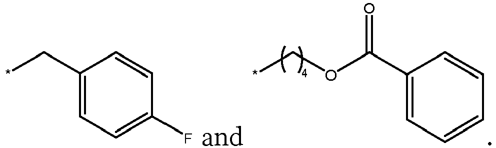 Preferably, R2 is selected from 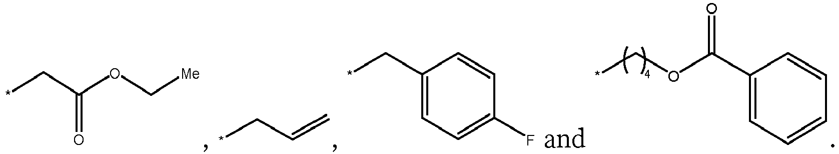 Compounds TR-G013A, TR-G102, TR-G103 and TG-112 are included in this embodiment and treatment with these compounds is associated with an exceptionally low proliferation of glioblastoma cells.

In one embodiment, R1 is 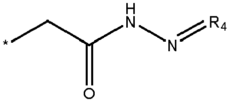 and R4 is preferably selected from 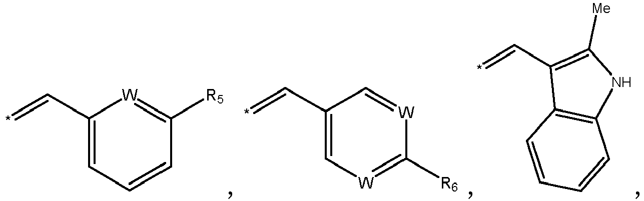 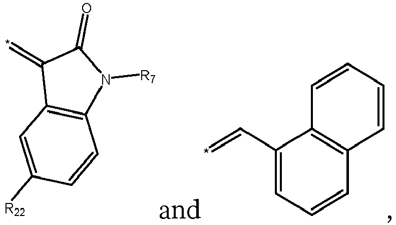 more preferably R4 is 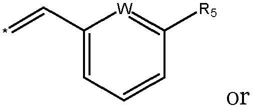 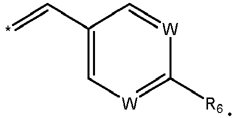 In a preferred embodiment, R4 is 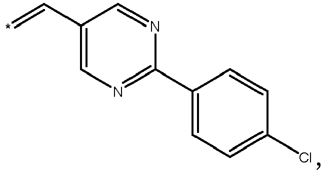 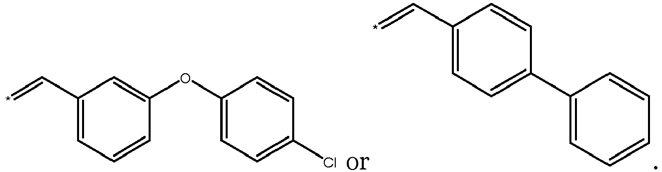 Compounds TR-G003, TR-G025, TR-G-028, TR-G055, TR-G056, TR-G066, TR-G138 and TR-G145 are included in this embodiment. Treatment with these compounds is shown to significantly reduce the proliferation of glioblastoma cell lines and also prolong survival and decelerate the tumor growth in rats with induced GBM. In some cases, the tumor size was significantly reduced after treatment with a compound of this embodiment. As an example, R2 may be -H in this embodiment.

In one embodiment, R1 is a substituted benzyl, preferably R1 is selected from 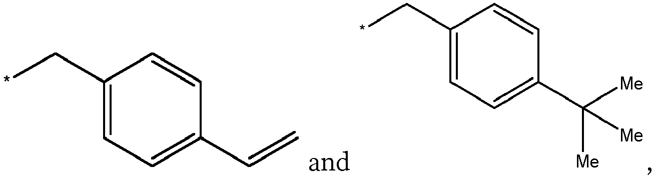 more preferably R1 is 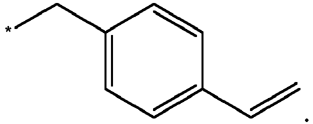 Compounds TR-G053 and TR-G030 are included in this embodiment and treatment with these compounds exhibits a reduction in the proliferation of glioblastoma cells and prolongs the survival of rats with induced GBM. As an example, R2 may be -H in this embodiment.

In one embodiment, both R1 and R2 are 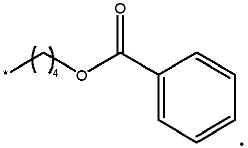 Compound TR-G013A is included in this embodiment and treatment with this compound is associated with a significantly reduced proliferation of glioblastoma cells.

In one embodiment, R1 is a branched C3-C7 alkyl, preferably branched C4-C7 alkyl, more preferably R1 is 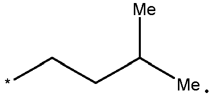 Compound TR-G002 is included in this embodiment and treatment with these compounds results in a reduction in the proliferation of glioblastoma cells as well as a significantly prolonged survival and deceleration in tumor growth in rats. Furthermore, it has been shown that treatment with these compounds can result in a decrease in both vascular and cellular proliferation in rats with induced GBM. As an example, R2 may be -H in this embodiment.

In one embodiment, R1 is 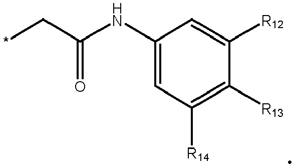 Preferably R1 is 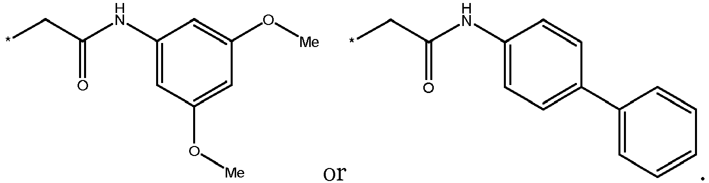 Compounds TR-G091 and TR-G143 are included in this embodiment and treatment with these compounds results in a reduction in proliferation of glioblastoma cells. While treatment with TR-091 did not exhibit a significant reduction in cell proliferation in *vitro,* treatment of rats with induced GBM with this compound resulted in a prolonged survival. As an example, R2 may be -H in this embodiment.

In another embodiment, R1 is 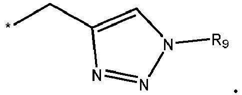 Preferably R1 is selected from 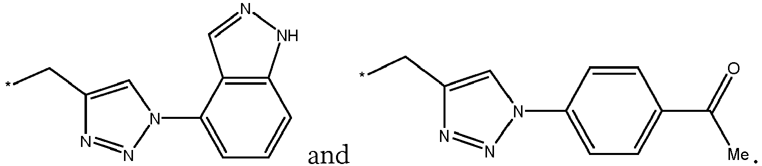 Compounds TR-G078, TR-G082, TR-G093 and TR-G125 are included in this embodiment and treatment with these compounds is associated with a significant reduction in proliferation of glioblastoma cells. As an example, R2 may be -H in this embodiment.

In one embodiment, R1 is selected from 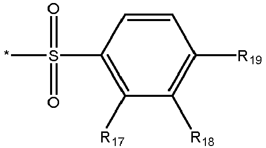 and 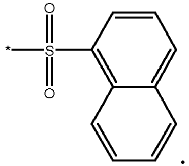 Preferably R1 is selected from 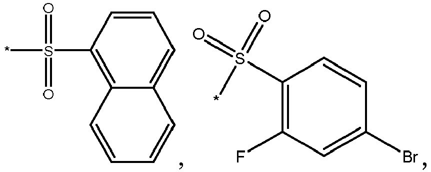 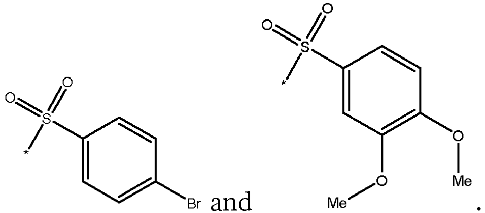 More preferably R1 is 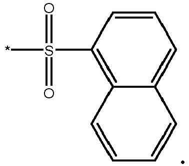 Compounds TR-G085, TR-G063, TR-G048 and TR-G045 are included in this embodiment and treatment with these compounds results in a reduction in the proliferation of glioblastoma cells as well as a prolonged survival and a deceleration in tumor growth in rats with induced GBM. In some cases, the tumor size was significantly reduced after treatment with a compound in this embodiment. As an example, R2 may be -H in this embodiment.

In one embodiment, R1 is 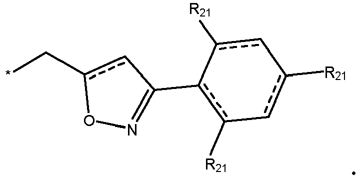 Preferably R1 is selected from 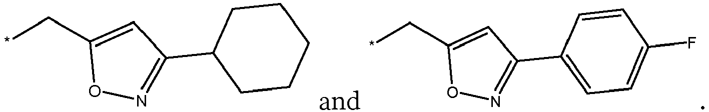 Compounds TR-G092, TR-G116, TR-G142 and TR-G150 are included in this embodiment and treatment with these compounds is shown to reduce the proliferation of glioblastoma cells. As an example, R2 may be -H in this embodiment.

In one embodiment, R1 is 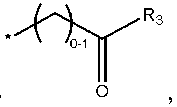 preferably R1 is selected from 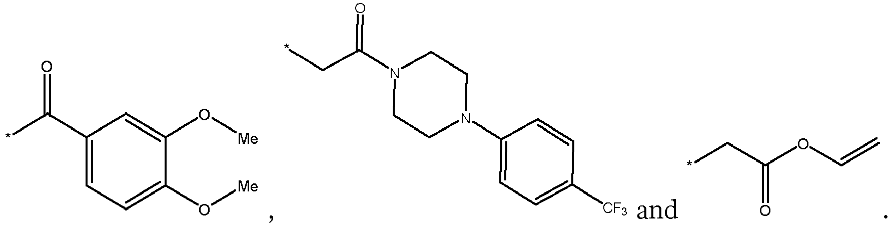 Compounds TR-G087, TR-G071, TR-G008 are included in this embodiment and treatment with these compounds resulted in a reduction in cell proliferation of glioblastoma cells. While treatment with TR-071 did not exhibit a reduction in cell proliferation *in vitro,* treatment with this compound showed a prolonged survival of rats with induced GBM. As an example, R2 may be -H in this embodiment.

In one embodiment, R1 is 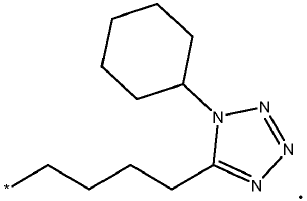 Preferably, in this embodiment R2 is selected from optionally branched C3-C7 alkyl, C3-C5 alkenyl, 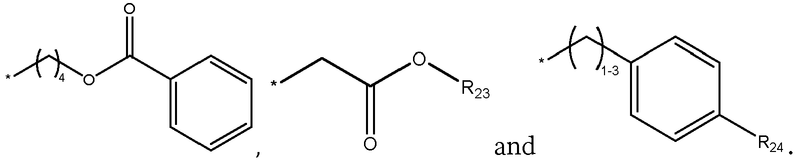 More preferably R2 is selected from 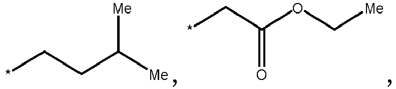 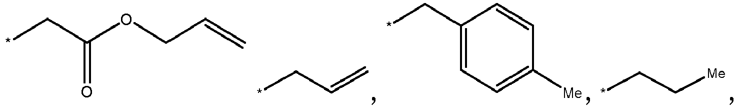 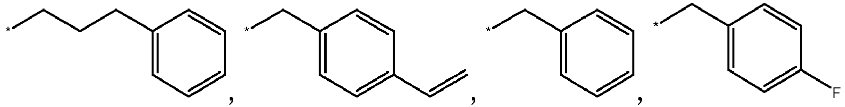 and 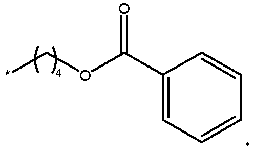 Most preferably R2 is selected from 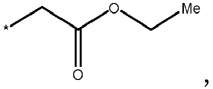 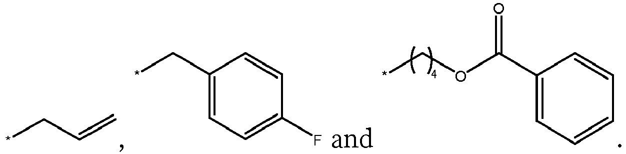 Compounds TR-G102, TR-G103, TR-G105 and TR-G112 are included in this embodiment and treatment with these compounds is shown to exhibit a significant reduction in the proliferation of glioblastoma cells.

The compounds TR-G002, TR-G13A, TR-G014, TR-G045, TR-G053, TR-G066, TR-G078 and TR-G112 are particularly preferred as treatment with these compounds results in a significant reduction in the proliferation of glioblastoma cells as well as a prolonged survival and deceleration in tumor growth in rats induced with GBM. Furthermore, treatment with these compounds, especially TR-G002 and TR-G066, can lead to a decrease in both vascular and cellular proliferation in rats with induced GBM.

According to a second aspect, there is provided a pharmaceutical composition comprising a compound according to the first aspect.

According to a third aspect, there is provided a compound according to the first aspect or a pharmaceutical composition according to the second aspect for use as a medicament.

According to a fourth aspect, there is provided a compound according to the first aspect or a pharmaceutical composition according to the second aspect for use in a method of treatment of glioma. The method may for example comprise oral administration of the compound or pharmaceutical composition.

In an embodiment of the fourth aspect, the compound or the pharmaceutical composition is used in a method of treatment of glioblastoma, such as glioblastoma multiforme (GBM).

### EXAMPLES

### Synthesis of target compounds

To synthesize the target compounds, different synthesis strategies were applied, and the multi-step syntheses were optimized. Optimization was directed towards minimizing the toxicities of the target compounds by using reaction strategies that fall within the green chemistry approach. The use of auxiliaries such as solvents, release agents and catalysts was kept to a minimum. Attention was paid to reducing reagent waste and preventing undesirable side reactions.

UV/Visible Spectroscopy, FTIR, LC-MS-MS, NMR, HPLC and X-Ray crystallography was used to elucidate the structure and impurities of intermediate and target compounds.

The R-groups in the Examples section and the corresponding figures are different from the R-groups in the rest of this specification.

### General Experimental

All reactions were performed with oven-dried glassware and under an inert atmosphere (nitrogen) unless otherwise stated. All reagents and dried solvents were obtained from commercial supplier Sigma-Aldrich and used without further purification. Organic solutions were concentrated under reduced pressure on a Heidolph rotary evaporator.

Reactions were monitored by LCMS/MS (Thermo Fisher TSQ Series, Athena C18-WP,100 Å, 2.1×50 mm, 3 µm) using water: MeOH (0.01 formic acid)) as eluent or by Thin-Layer Chromatography (TLC) using silica gel pre-coated aluminium plates (Kieselgel 60, 254, E. Merck, Germany). The chromatograms were visualized using ultraviolet light (254 and 366 nm) and stained with vanillin dips or aqueous potassium permanganate solution.

¹H NMR spectra were recorded on Avance Bruker 500 MHz spectrometer in CDCl₃ and DMSO-d6. ¹³C NMR spectra were recorded in deuterated solvents on Bruker spectrometer at 126 MHz, with the central peak of the deuterated solvent as the internal standard. The ¹H NMR spectra are reported as δ/ppm downfield from tetramethylsilane (multiplicity, number of protons, coupling constant J/Hz). The ¹³C NMR spectra are reported as δ/ppm. All chemical shifts are reported in parts per million (ppm) relative to the residual solvent peak. The following abbreviations are used to denote signal patterns: (s) singlet, (d) doublet, (t) triplet, (q) quartet, (m) multiplet, and (br) broad unless otherwise noted.

Flash-column chromatography was performed on PuriFlash XS 520 Plus Flash chromatography system (Interchim) with built-in UV-detector, ELSD-detector, and fraction collector with Interchim silica gel columns.

### General procedure A (Alkylation Reactions)

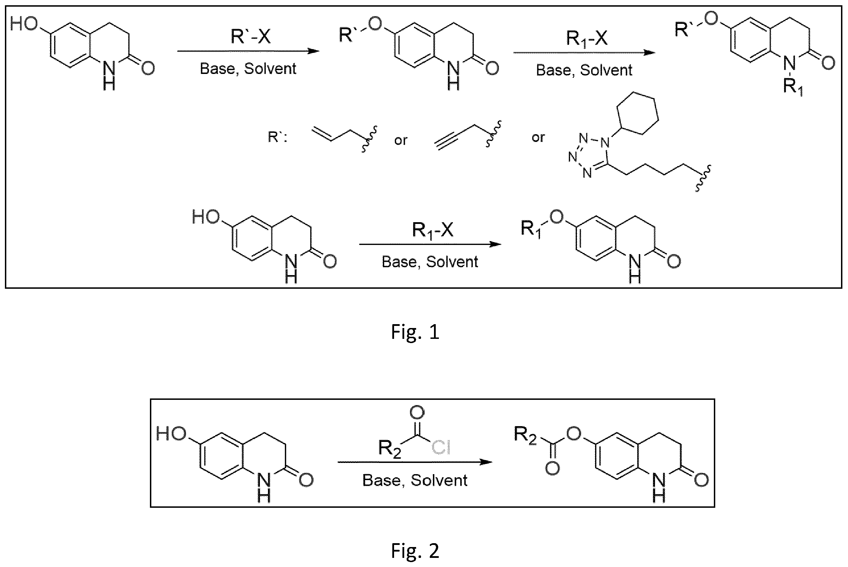

Hydroxyl or amino group-containing compounds (1 eq.) were dissolved in THF and drops of DMF comprising potassium carbonate (K₂CO₃, 1.1-1.5 eq.) or cesium carbonate (Cs₂CO₃, 1.1-1.5 eq.). The mixture was stirred for 30 minutes at ambient temperature. After that, alkyl bromide or alkyl chloride (1-1.2 eq.) was added to the reaction mixture which was monitored with TLC over 12-48 h. Iced water was added to the reaction mixture and then extracted using dichloromethane (DCM) or ethyl acetate. The organic layer was dried over anhydrous magnesium sulfate, evaporated, and purified by flash chromatography to afford the final product, see Fig. 1 and Table 1.

**Table 1 shows target compounds with their reagents for alkylation reactions.**

| CODE | R1 | TARGET COMPOUND |
|---|---|---|
| TR-G001 | 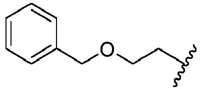 | 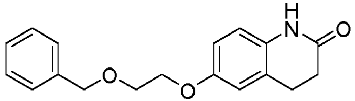 |
| TR-G002 | 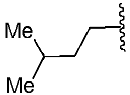 | 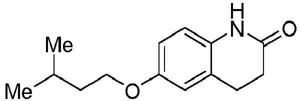 |
| TR-G004 | 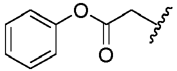 | 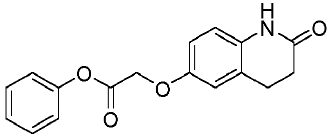 |
| TR-G006 | 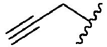 | 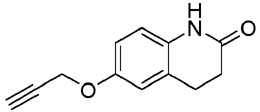 |
| TR-G006A | 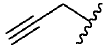 | 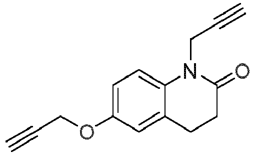 |
| TR-G010 | 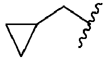 | 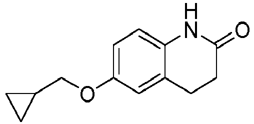 |
| TR-G012 | 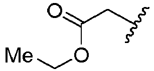 | 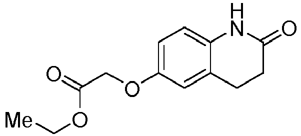 |
| TR-G013 | 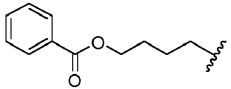 | 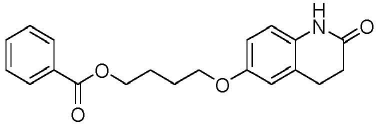 |
| TR-G013A | 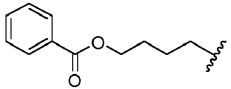 | 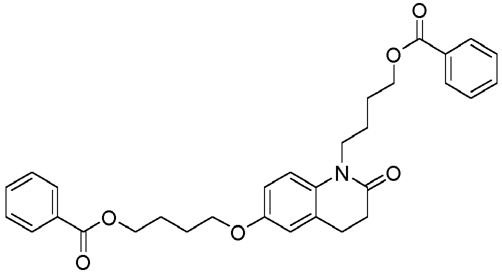 |
| TR-G014 | 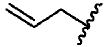 | 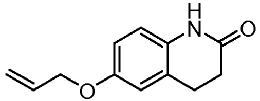 |
| TR-G014A | 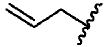 | 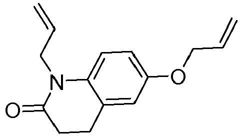 |
| TR-G017 | 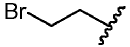 | 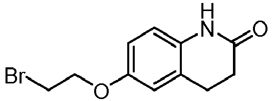 |
| TR-G023 | 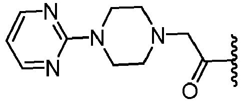 | 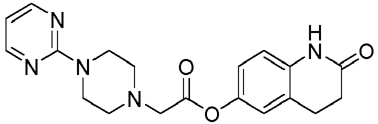 |
| TR-G030 | 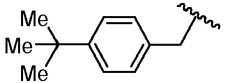 | 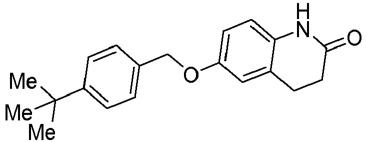 |
| TR-G031 | 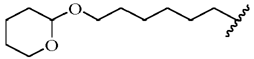 | 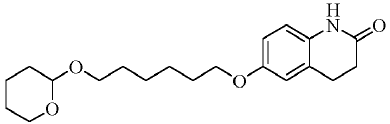 |
| TR-G041 | 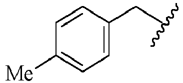 | 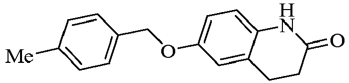 |
| TR-G049 | 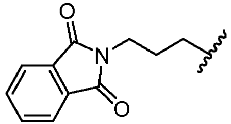 | 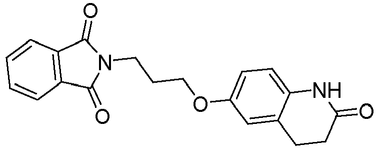 |
| TR-G053 | 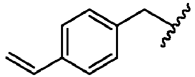 | 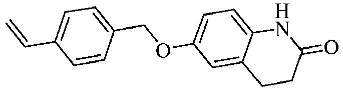 |
| TR-G068 | 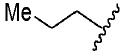 | 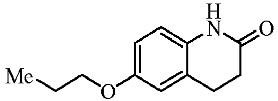 |
| TR-G071 | 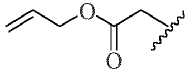 | 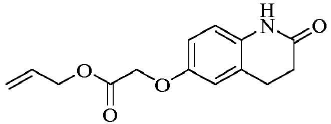 |
| TR-G074 | 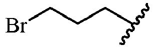 | 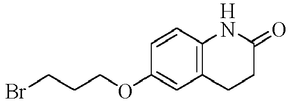 |
| TR-G087 | 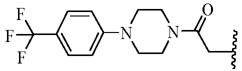 | 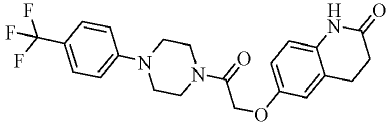 |
| TR-G095 | 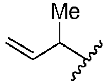 | 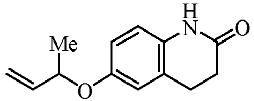 |
| TR-G101 | 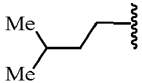 | 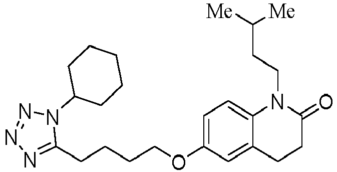 |
| TR-G102 | 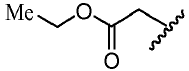 | 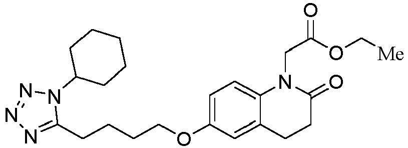 |
| TR-G103 | 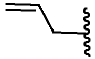 | 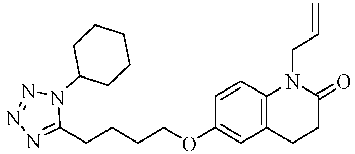 |
| TR-G104 | 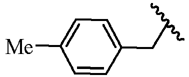 | 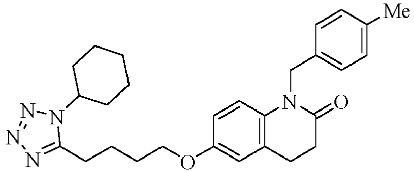 |
| TR-G105 | 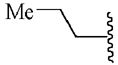 | 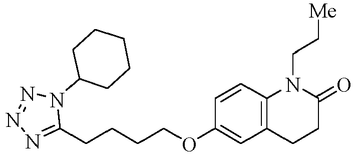 |
| TR-G106 | 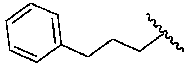 | 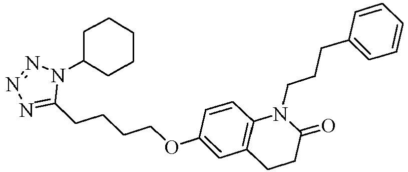 |
| TR-G107 | 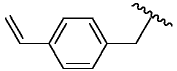 | 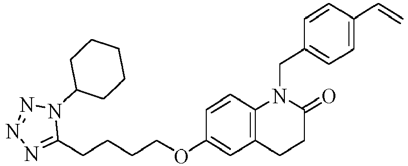 |
| TR-G110 | 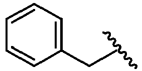 | 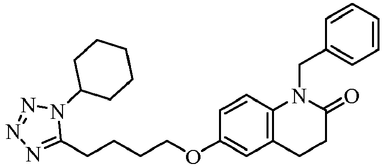 |
| TR-G111 | 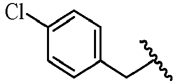 | 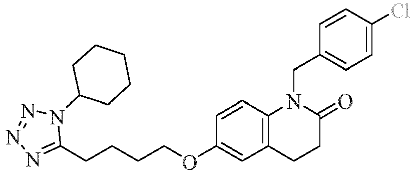 |
| TR-G112 | 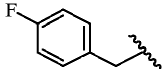 | 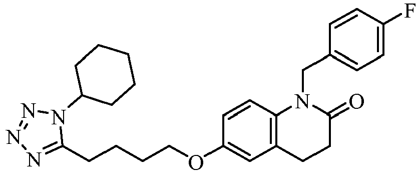 |
| TR-G114 | 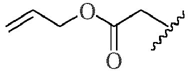 | 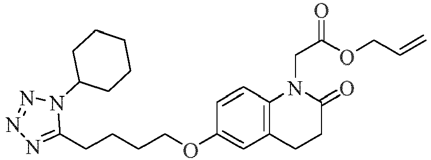 |
| TR-G115 | 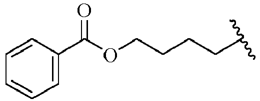 | 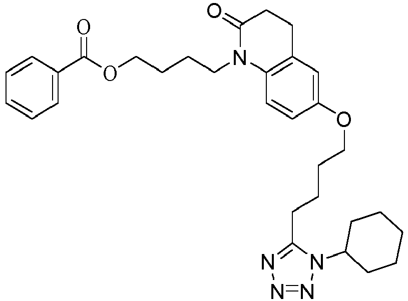 |
| TR-G126 | 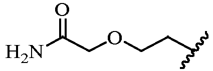 | 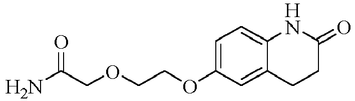 |
| TR-G144 | 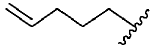 | 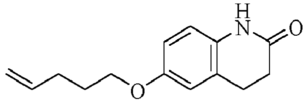 |

### General procedure B (Acylation Reactions)

To a solution of compounds containing hydroxy/amino group (1 eq.) and K₂CO₃ (1.5 eq.) or triethylamine (TEA, 1.5 eq.) in THF, acetone, or DMF, acyl chloride derivatives were added to afford the acylated products. After the completion of reaction, the solvents were evaporated and the residues were washed with water, dried, and purified, see Fig. 2 and Table 2.

**Table 2 shows the target compounds and their reagents for acylation reactions.**

| CODE | R2 | TARGET MOLECULES |
|---|---|---|
| TR-G007 | 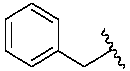 | 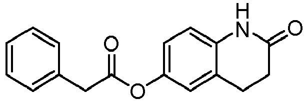 |
| TR-G008 | 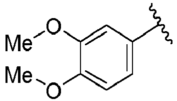 | 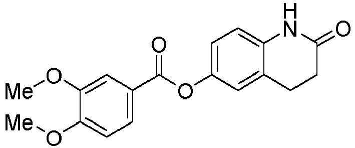 |
| TR-G009 | 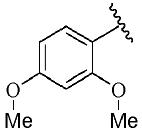 | 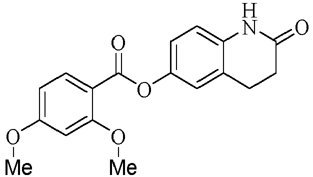 |
| TR-G011 | 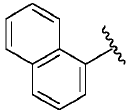 | 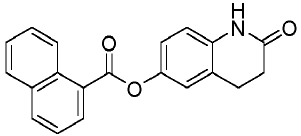 |
| TR-G016 | 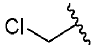 | 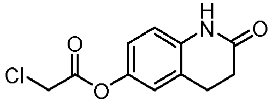 |
| TR-G036 | 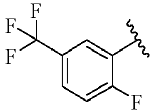 | 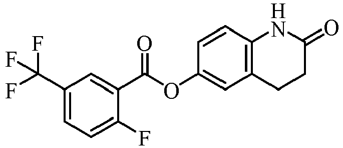 |
| TR-G039 | 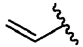 | 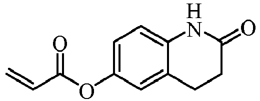 |
| TR-G040 | 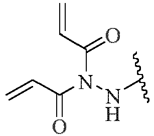 | 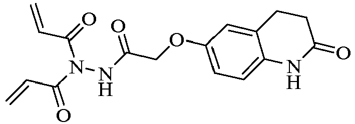 |
| TR-G060 | 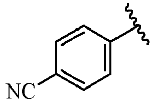 | 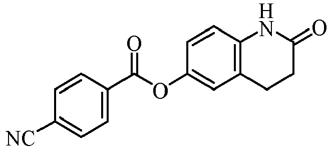 |
| TR-G061 | 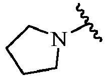 | 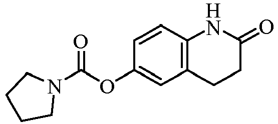 |
| TR-G062 | 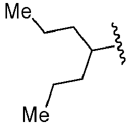 | 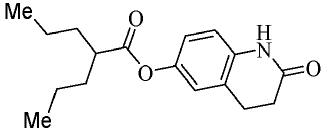 |
| TR-G065 | 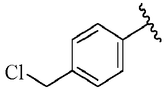 | 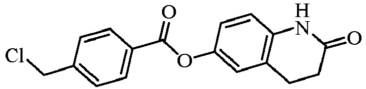 |
| TR-G081 | 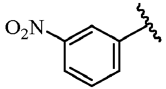 | 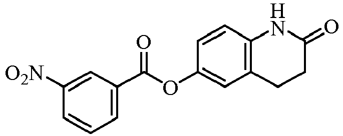 |
| TR-G139 | 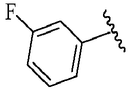 | 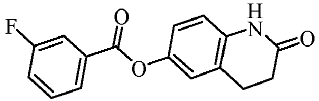 |

### General procedure C (Hydrazide Formation)

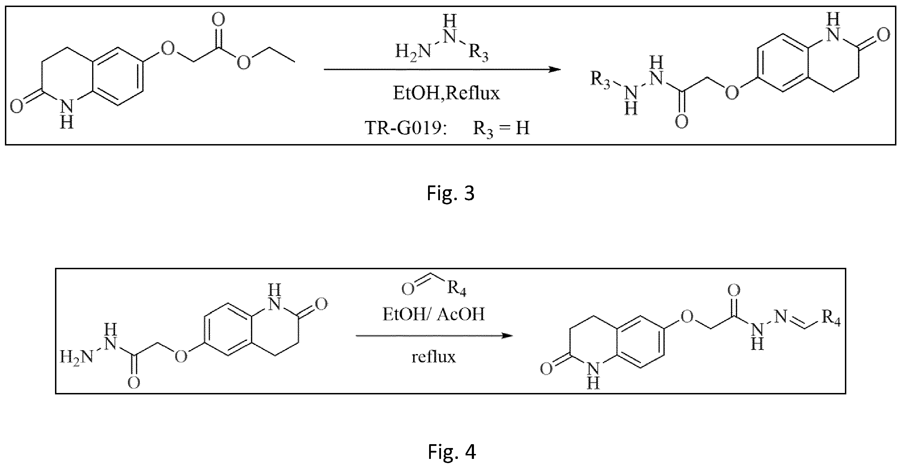

The hydrazide products were obtained by refluxing acetate-containing compounds (1 eq.) and hydrazines (2 eq.) in ethanol. After the completion of the reaction, the product was cooled to room temperature and filtered out of ethanol, see Fig. 3.

### General procedure D - Acetohydrazide-hydrazones (Schiff-base reactions)

The hydrazones were accomplished by reacting hydrazides and aldehydes/ketones in ethanol and drops of acetic acid at reflux temperature for 4-12 hours. After the reaction was cooled, the product was filtered and purified by column chromatography, see Fig. 4 and Table 3.

**Table 3 shows target compounds with their reagents for acetohyrazide-hydrazone reactions.**

| CODE | R4 | TARGET MOLECULES |
|---|---|---|
| TR-G003 | 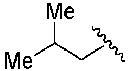 | 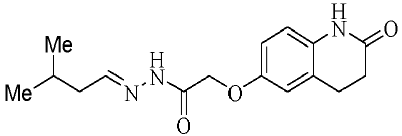 |
| TR-G021 | 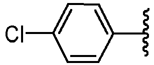 | 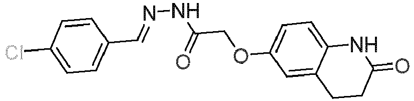 |
| TR-G022 | 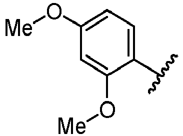 | 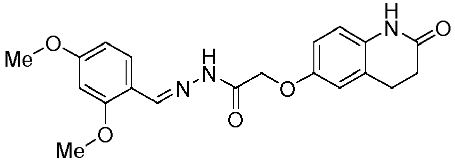 |
| TR-G024 | 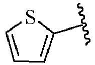 | 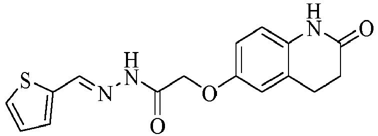 |
| TR-G025 | 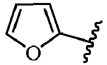 | 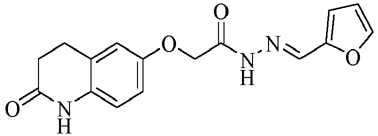 |
| TR-G026 | 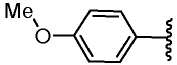 | 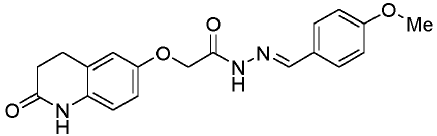 |
| TR-G027 | 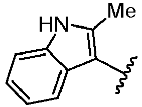 | 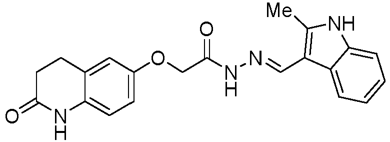 |
| TR-G028 | 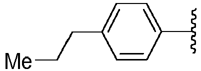 | 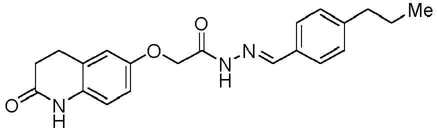 |
| TR-G029 | 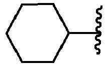 | 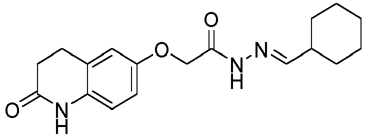 |
| TR-G035 | 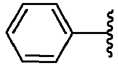 | 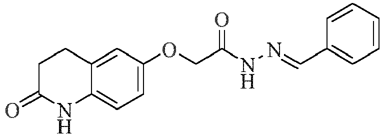 |
| TR-G038 | 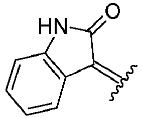 | 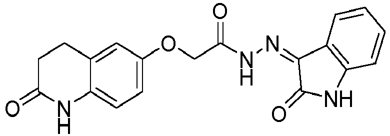 |
| TR-G047 | 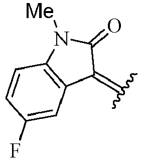 | 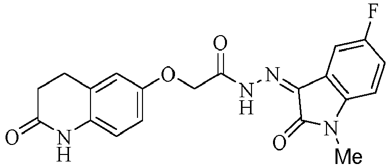 |
| TR-G050 | 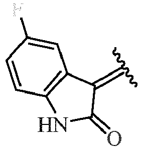 | 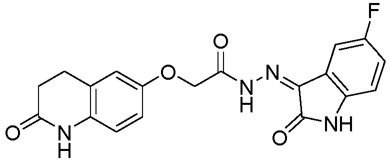 |
| TR-G055 | 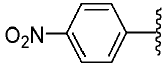 | 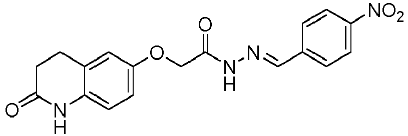 |
| TR-G056 | 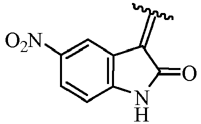 | 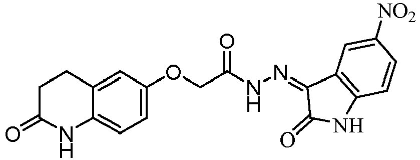 |
| TR-G059 | 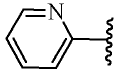 | 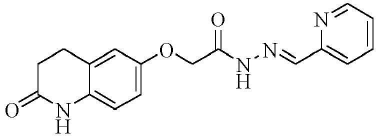 |
| TR-G066 | 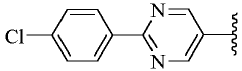 | 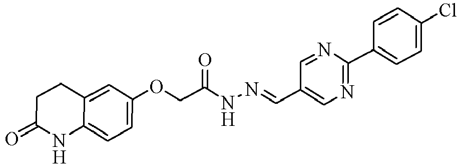 |
| TR-G076 | 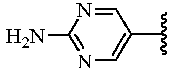 | 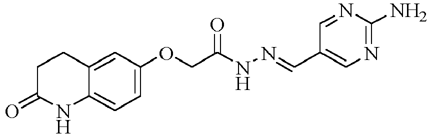 |
| TR-G128 | 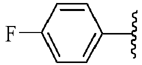 | 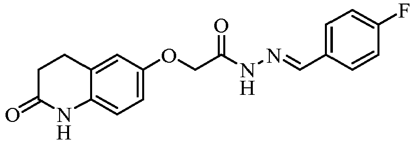 |
| TR-G138 | 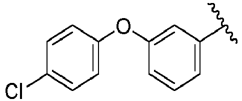 | 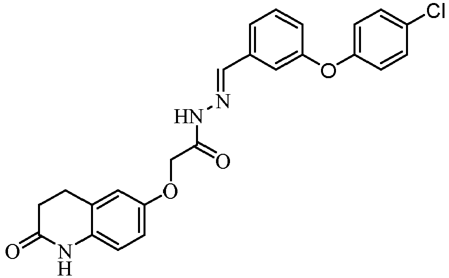 |
| TR-G141 | 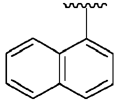 | 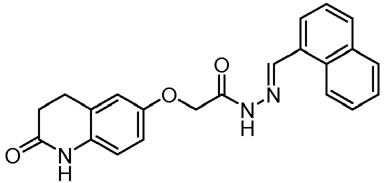 |
| TR-G145 | 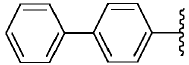 | 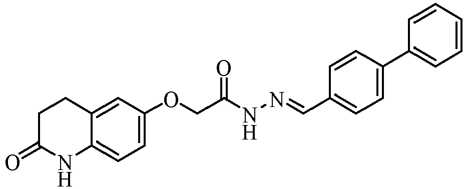 |
| TR-G146 | 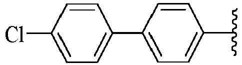 | 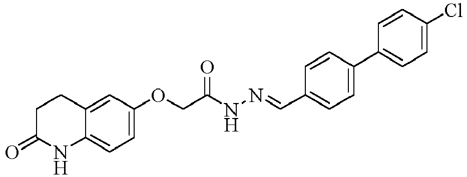 |

### General procedure E - Cycloaddition Reactions (Triazole Preparation)

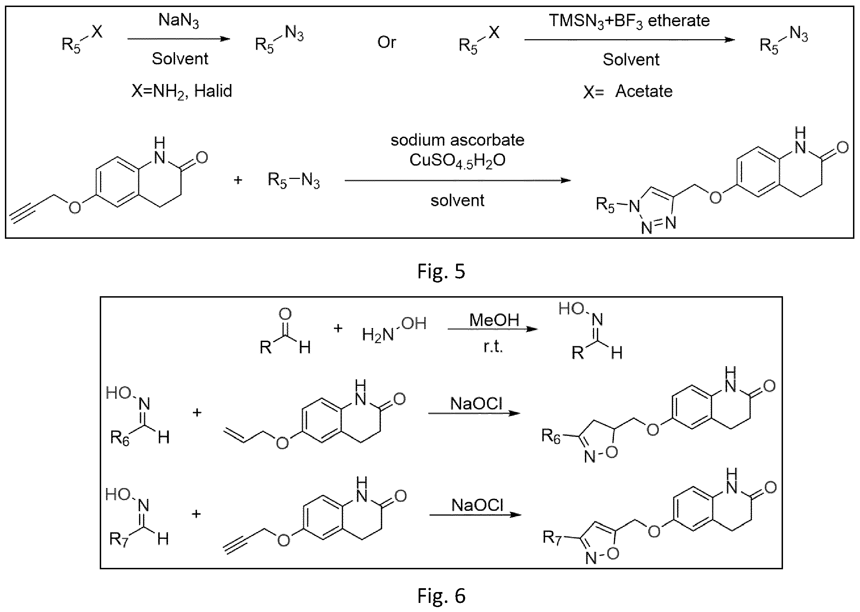

The azides were prepared starting from alkyl bromide, amines and acetate derivative (1 eq.) which were reacted with azide salts (2 eq.) for 2 hours in appropriate solvents, see Fig. 5 and Table 4.

**Table 4 shows the target compounds with their reagents for triazoles formation.**

| CODE | R5 | TARGET MOLECULES |
|---|---|---|
| TR-G033 | 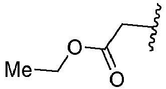 | 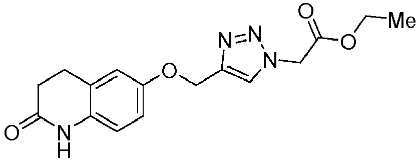 |
| TR-G052 | 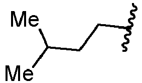 | 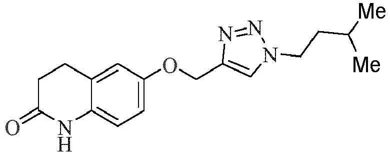 |
| TR-G058 | 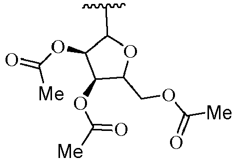 | 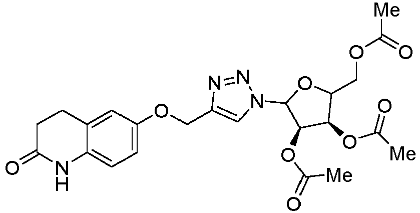 |
| TR-G078 | 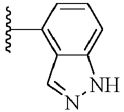 | 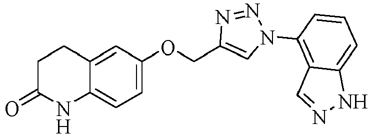 |
| TR-G079 | 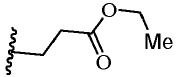 | 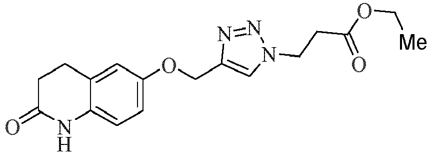 |
| TR-G080 | 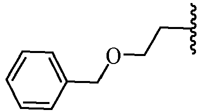 | 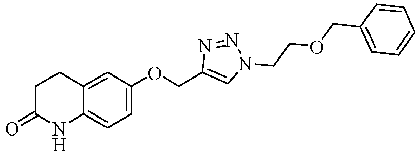 |
| TR-G082 | 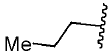 | 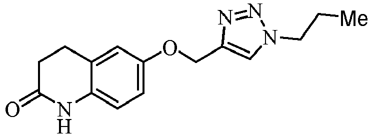 |
| TR-G086 | 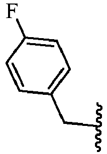 | 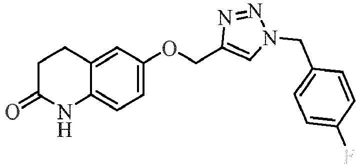 |
| TR-G093 | 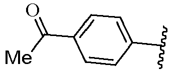 | 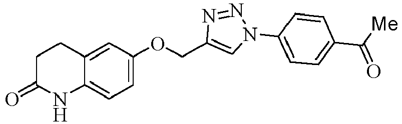 |
| TR-G117 | 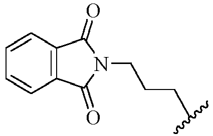 | 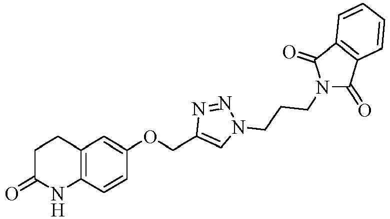 |
| TR-G125 | 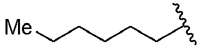 | 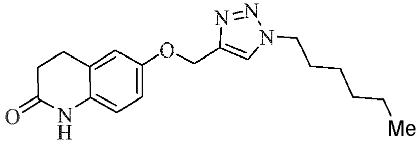 |
| TR-G147 | 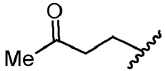 | 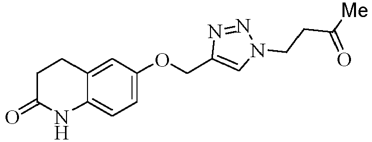 |

### General procedure F - Cycloaddition Reactions (Oxazole Formation)

Firstly, TR-G014 was prepared according to **procedure A**. After that, to prepare TR-G051, TR-G070, and TR-G150, a solution of 4-fluorobenzaldehyde, 4-chlorobenzaldehyde or 2-chloro-6-fluorobenzaldehyde respectively (1 eq.), hydroxylamine hydrochloride (2 eq.) and NaHCO₃ in ethanol were refluxed for 3h to prepare the corresponding oxime. The reaction was monitored by TLC until completion of reaction. After that, the product was filtered, washed and dried. In the cyclization step, TR-G014 (1 eq.) with the corresponding oxime were dissolved in chloroform then NaOCl (12-16 grade) (2 eq.) were added slowly to the mixture in ice bath at 0 °C for 8 h. Water was then added and the product was extracted with chloroform (3× 30 mL). The organic layer was dried over anhydrous magnesium phosphate and the solvent was evaporated under vacuum. The crude product was purified using flash chromatography.

For TR-G084, TR-G092, TR-G116 and TR-G142; TR-G006 was firstly prepared according to **procedure A**. After that, the corresponding aldehyde (1 eq.), hydroxylamine hydrochloride (2 eq.) and NaHCO₃ in ethanol were refluxed for 3h to prepare the corresponding oxime. The reaction was monitored by TLC until completion of reaction. After that, the product was filtered, washed and dried. In the cyclization step, TR-G006 (1 eq.) with the corresponding oxime were dissolved in chloroform and then NaOCl (12-16 grade) (2 eq.) was added slowly to the mixture in an ice bath at 0 °C for 8 h. Water was then added, and the product was extracted with chloroform (3× 30 mL). The organic layer was dried over anhydrous magnesium phosphate and the solvent was evaporated under vacuum. The crude product was purified using flash chromatography.

The cycloaddition reaction for the oxazole formation can be seen in Fig. 6 and Table 5.

**Table 5 shows the target compounds with their reagents for oxazole analogues.**

| CODE | R6 | TARGET COMPOUNDS |
|---|---|---|
| TR-G051 | 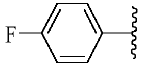 | 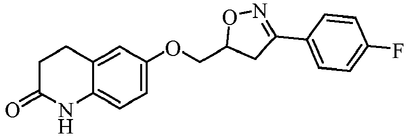 |
| TR-G070 | 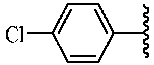 | 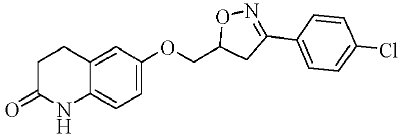 |
| TR-G150 | 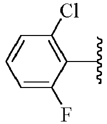 | 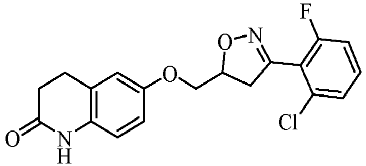 |

| CODE | R7 | TARGET COMPOUNDS |
|---|---|---|
| TR-G084 | 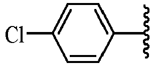 | 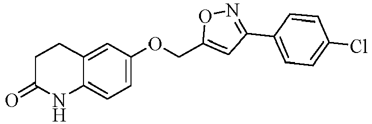 |
| TR-G092 | 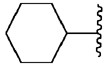 | 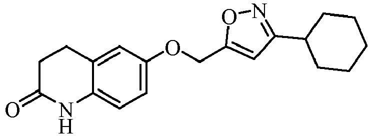 |
| TR-G116 | 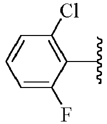 | 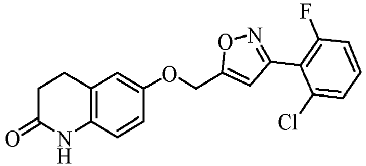 |
| TR-G142 | 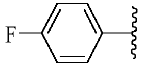 | 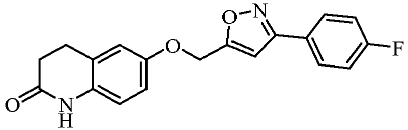 |

### Carbamate Formation Reaction (from carbamoyl chlorides)

The starting material 6-hydroxy-3,4-dihydroquinolin-2(1H)-one was reacted with carbmoyl chlorides (1,2 eq.) under basic conditions (K₂CO₃) (1.3 eq.) at room temperature for 8-16 h.

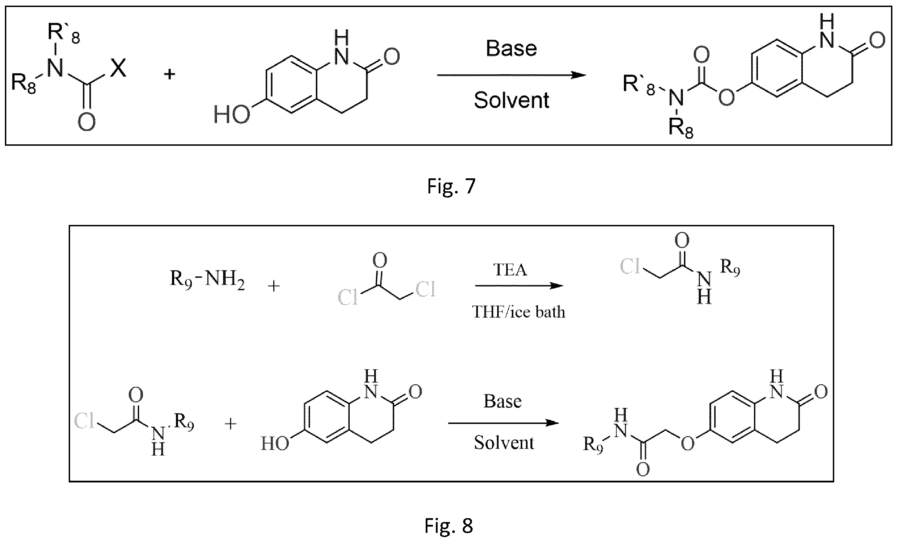

See Fig. 7 and Table 6 for the target compounds.

**Table 6 shows target molecules with their reagents for carbamate derivatives.**

| CODE | R8 | R'8 | TARGET COMPOUNDS |
|---|---|---|---|
| TR-G005 | -methyl | -methyl | 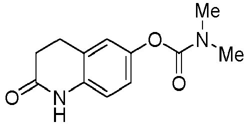 |
| TR-G020 | -phenyl | -phenyl | 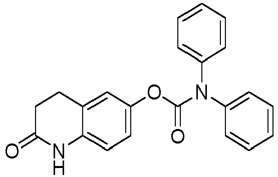 |
| TR-G037 | -Me | -ethyl | 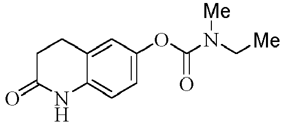 |
| TR-G072 | -methyl | -phenyl | 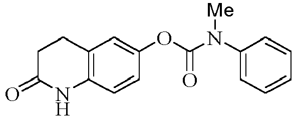 |
| TR-G129 | -isopropyl | -isopropyl | 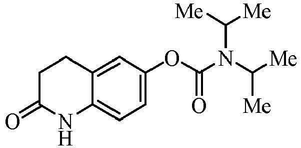 |

### Acetamide Formation Reaction

In the first step, amines have been reacted with chloroacetyl chloride in the presence of a base according to procedure B. In the second step the acetylated amine reacted with 6-hydroxy-3,4-dihydroquinolin-2(1H)-one under basic conditions according to procedure A, see Fig. 8 and Table 7.

**Table 7 shows the target compounds with their reagents for acetamide derivatives**

| CODE | R9 | TARGET MOLECULES |
|---|---|---|
| TR-G043 | 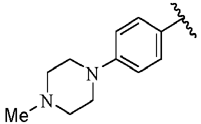 | 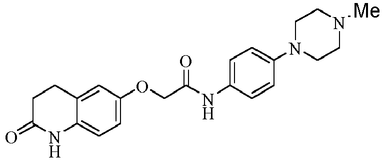 |
| TR-G054 | 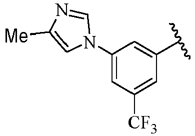 | 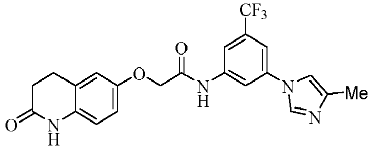 |
| TR-G064 | 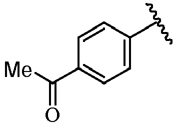 | 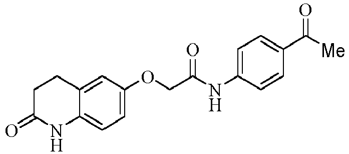 |
| TR-G075 | 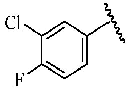 | 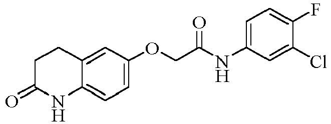 |
| TR-G077 | 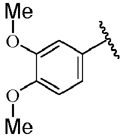 | 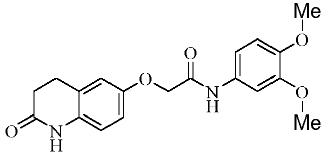 |
| TR-G089 | 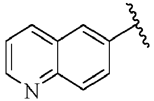 | 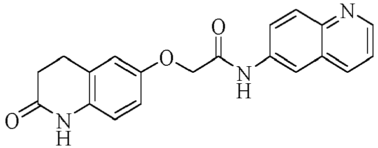 |
| TR-G091 | 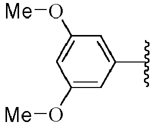 | 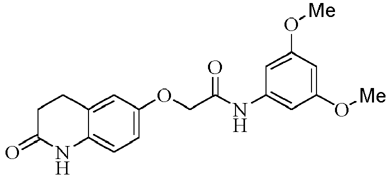 |
| TR-G094 | 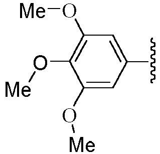 | 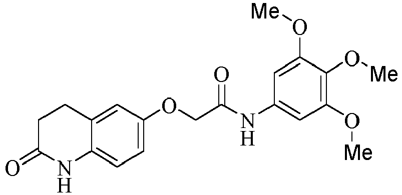 |
| TR-G120 | 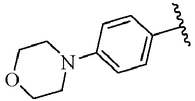 | 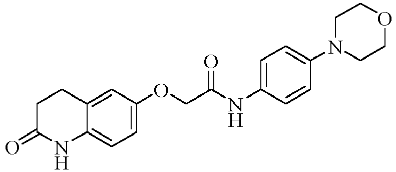 |
| TR-G121 | 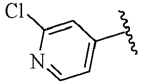 | 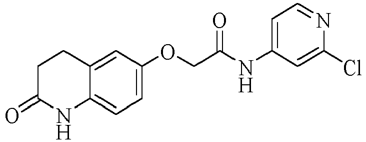 |
| TR-G122 | 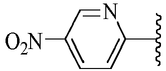 | 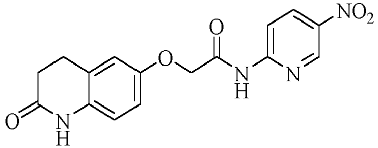 |
| TR-G123 | 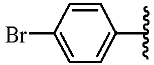 | 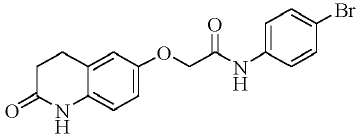 |
| TR-G143 | 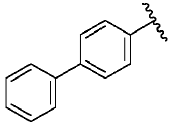 | 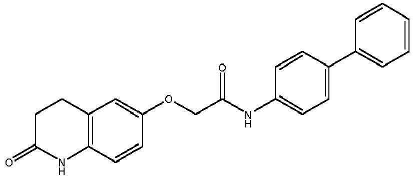 |

### Carbamate Formation Reaction (From Isocyanate Derivatives)

In two round bottom flask, 6-hydroxy-3,4-dihydroquinolin-2(1H)-one (1 eq.) was stirred with TEA (1.3 eq.) for 1h in THF and drops of DMF, then the desire isocyanate (1.3 eq.) was added to the reaction mixture and stirred for 2-4 h at room temperature. After that, the product was precipitated in ice water, filtered and air-dried. Finally, the product was purified using flash chromatography.

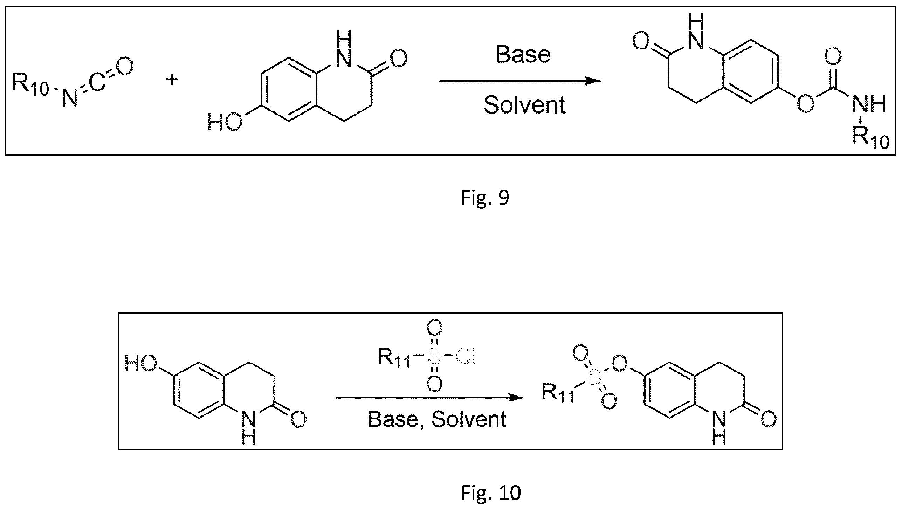

See Fig. 9 and Table 8 for the target compounds.

**Table 8 shows the target molecules with their reagents for carbamate derivatives.**

| CODE | R10 | TARGET MOLECULES |
|---|---|---|
| TR-G096 | 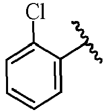 | 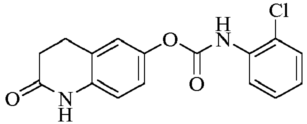 |
| TR-G097 | 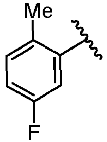 | 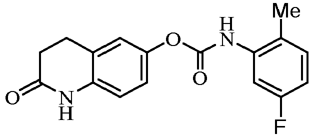 |
| TR-G131 | 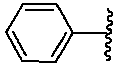 | 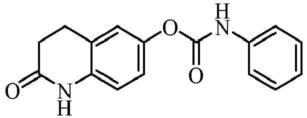 |
| TR-G132 | 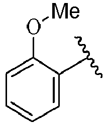 | 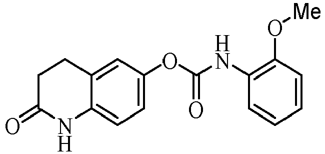 |
| TR-G133 | 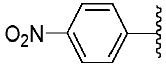 | 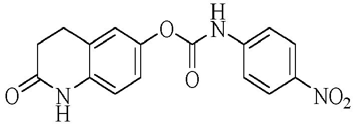 |
| TR-G134 | 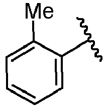 | 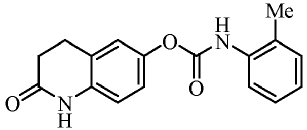 |
| TR-G135 | 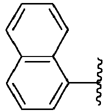 | 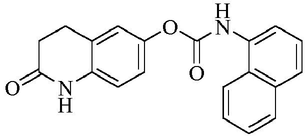 |
| TR-G136 | 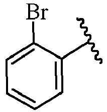 | 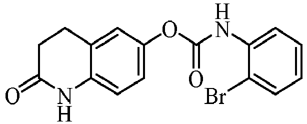 |
| TR-G137 | 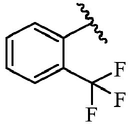 | 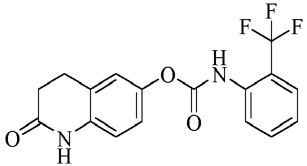 |
| TR-G140 | 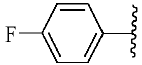 | 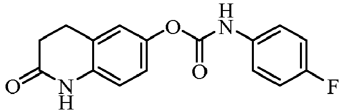 |

### General procedure H for sulfonyl chloride reactions (Sulfonate Ester)

6-hydroxy-3,4-dihydroquinolin-2(1H)-one was dissolved in tetrahydrofuran (THF), and a small amount of dimethylformamide (DMF) was added, along with either K₂CO₃, TEA or Cs₂CO₃ at a ratio of 1.1-1.5 equivalents. The mixture was stirred for approximately 30 minutes at room temperature. Following this, alkyl sulfonyl chloride (at a 1-1.2 molar ratio) was introduced into the reaction mixture, and the progress was monitored over 12-48 hours using TLC. The reaction mixture was subsequently treated with cold water and then subjected to extraction using either DCM or ethyl acetate. The organic layer underwent drying through anhydrous magnesium sulfate, followed by evaporation. The resultant material was further refined through flash chromatography to yield the ultimate product, see Fig. 10 and Table 9.

**Table 9 shows the target compounds with their reagents for sulfonate derivatives of 6-hydroxy-3,4-dihydroquinolin-2(1H)-one.**

| CODE | R11 | TARGET MOLECULES |
|---|---|---|
| TR-G042 | 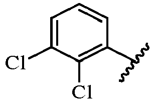 | 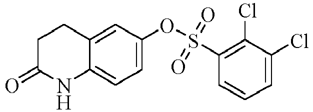 |
| TR-G045 | 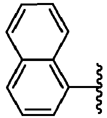 | 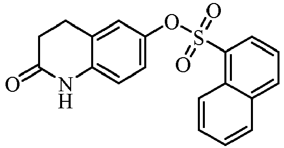 |
| TR-G046 | 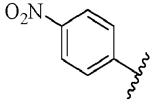 | 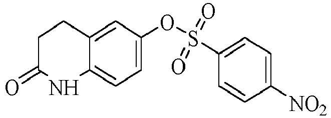 |
| TR-G048 | 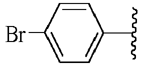 | 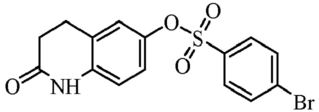 |
| TR-G057 | 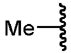 | 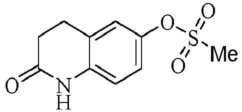 |
| TR-G063 | 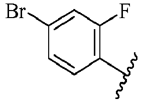 | 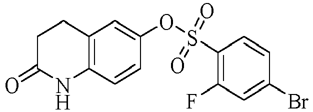 |
| TR-G069 | 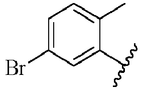 | 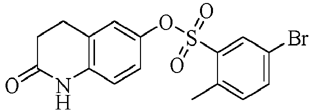 |
| TR-G073 | 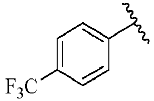 | 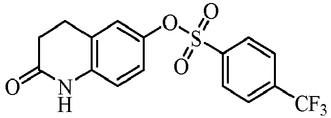 |
| TR-G085 | 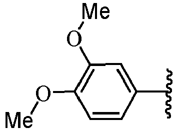 | 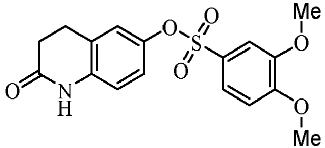 |

### Synthesis of 6-(2-(benzyloxy)ethoxy)-3,4-dihydroquinolin-2(1H)-one (TR-G001)

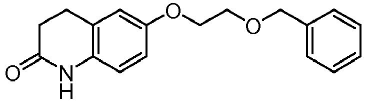

Following procedure A, 250 mg of 6-hydroxy-3,4-dihydroquinolin-2(1H)-one (1.53 mmol.) was stirred with 317 mg of the base K₂CO₃ (2.3 mmol.) for 30 min in THF and drops of DMF, then 0.26 ml of ((2-chloroethoxy)methyl)benzene (1.6 mmol.) was added to the reaction mixture at room temperature. After that workup with brine and extraction by ethyl acetate, column chromatography (Hexane:EtOAc) (60:40) was done to obtain 313.72 mg of TR-G001 (86%) as yellowish powder. LCMS/MS: m/z 297.14 [M+H]⁺.

1H NMR (500 MHz, DMSO-d6) δ 9.91 (s, 1H. N-H), 7.37-6.72 (m, 8Harom), 4.55 (S, 2H.), 4.06-4.08 (t, J = 7.2 Hz, 2H.), 3.72-3.74 (t, J = 7.2 Hz, 2H.), 2.80-2.83 (t, J = 7.2 Hz, 2H. CH2-CH2-C=O), 2.38 -2.40 (t, 2H J = 7.2 Hz, 2H. CH2-C=O). ¹³C NMR (126 MHz, DMSO) δ 170.20, 154.10, 138.80, 132.29, 128.71, 127.98, 127.90, 125.29 116.21, 114-55, 113-53, 72.52, 68.77, 67.83, 40.15, 30.82, 25.56.

### Synthesis of 6-(isopentyloxy)-3,4-dihydroquinolin-2(1H)-one (TR-G002)

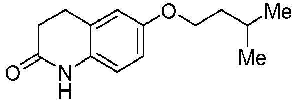

Following procedure A, a mixture of 6-hydroxy-3,4-dihydroquinolin-2(1H)-one (1.23 mmol, 200 mg) and K₂CO₃ (1.84 mmol, 254.09 mg) was stirred for 1 h in THF and drops of DMF in round bottom flask. After that, 1-bromo-3-methylbutane (1.47 mmol, 222.16 mg) was added to the solution at room temperature. The mixture was allowed to stir and the reaction was controlled by TLC. The product was precipitated in iced water. The desire product was filtered and purified using column chromatography (Hexane:EtOAc) (60:40), 210 mg (73%) of the desire product was obtained as white crystals. LCMS/MS: m/z 234.11 [M+H]⁺

¹H NMR (500 MHz, DMSO-*d*₆) δ 9.90 (s, 1H), 6.79 - 6.6 (m, 3Hₐᵣₒₘ), 3.90 (t, J = 6.6 Hz, 2H), 2.81 (t, J = 7.5 Hz, 2H), 2.39 (d, J = 15.1 Hz, 2H), 1.80 - 1.70 (m, 1H), 1.56 (q, J = 6.7 Hz, 2H), 0.91 (d, J = 6.7 Hz, 6H). ¹³C NMR (126 MHz, DMSO) δ 169.72, 153.86, 131.64, 124.80, 115.75, 113.95, 112.91, 66.07, 37-56, 30.39, 25.12, 24.59, 22.44.

### Synthesis of (E)-N'-(3-methylbutylidene)-2-((2-oxo-1,2,3,4-tetrahydroquinolin-6-yl)oxy)acetohydrazide (TR-G003)

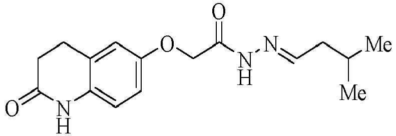

According to procedure A, C and D starting from 6-hydroxy-3,4-dihydroquinolin-2(1H)-one Firstly, 300 mg of 6-hydroxy-3,4-dihydroquinolin-2(1H)-one (1.84 mmol) was stirred with 381 mg of the base K₂CO₃ (2.76 mmol) for 15 min in THF and drops of DMF, then 244 µL of ethyl 2-bromoacetate (2.21 mmol) was added slowly to the reaction mixture. After the completing of reaction, the solvent was evaporated and the residue was washed with water, filtered and air-dried to obtain ethyl 2-((2-oxo-1,2,3,4-tetrahydroquinolin-6-yl)oxy)acetate (TR-G012) which was further refluxed with 241 µL hydrazine hydrate 99% (4.81 mmol) for 5 hours then it was cooled to room temperature, filtered out of ethanol and dried to obtain 2-((2-oxo-1,2,3,4-tetrahydroquinolin-6-yl)oxy)acetohydrazide TR-G019. Later, 300 mg of TR-G019 (1.28 mmol) was refluxed with 168 µL of isovaleraldehyde (1.53 mmol) in ethanol and drops of acetic acid for 5 hours. After that the precipitate was filtered out of ethanol to obtain the final product as white powder with a yield of 85%. LCMS/MS: m/z 304.11 [M⁺H]⁺

¹H NMR (500 MHz, DMSO-d6) δ 1H NMR (500 MHz, DMSO-d6) δ 11.08* (s, 1H. C-NH-N=), 9.93 (*9.95, s, 1H. C-NH-C), 7.63-6.72 m, 3Harom), 6.67 - 6.64 (m, 1H. N=CH-), 4.87 (s, 1H. O-CH2-C=O), 4.48 (s, 1H. O-CH2-C=O), 2.84-2.80 (m, 2H), 2.43 - 2.36 (m, 2H), 2.12-2.06 (m, 2H), 1.84-1.78 (m, 1H), 0.91 (d, J = 2.9 Hz, 3H), 0.90(d, J = 2.9 Hz, 3H). (* refer to the rotameric peak). ¹³C NMR (126 MHz, DMSO) δ 169.80, 163.86, 151.89, 147.77, 132.43, 131.99, 124.75, 114.41, 113.28, 66.90, 40.53, 30.32, 26.14, 25.12, 22.29, 22.26.

### Synthesis of phenyl 2-((2-oxo-1,2,3,4-tetrahydroquinolin-6-yl)oxy)acetate (TR-G004)

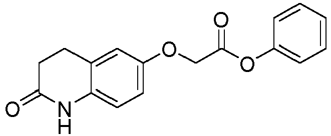

Following procedure A, 150 mg of 6-hydroxy-3,4-dihydroquinolin-2(1H)-one (0.919 mmol) was stirred with 190.6 mg of the base K₂CO₃ (1.38 mmol) for 3h. in THF and drops of DMF, then 0.296 gr of phenyl 2-bromoacetate (1.38 mmol) was added to the reaction at room temperature. After workup and purification by flash chromatography (Hexane:EtOAc) (50:50) 220 mg (80.5%) of TR-G004 as yellowish white powder. LCMS/MS: m/z 298.31 [M+H]⁺

1H NMR (500 MHz, DMSO-d6) δ 10.14 (s, 1H), 7.36 - 6.84 (m, 8Harom), 5.05 (s, 2H), 2.87 (t, J = 7.5 Hz, 2H), 2.4-2.42 (m, 2H). ¹³C NMR (126 MHz, DMSO) δ 170.03 (O=C), 168.02 (O=C-O), 157.48, 144.34, 136.33, 129.59, 124.82, 121.35, 120.91, 120.11, 115.55, 114.55, 64.56 (O=C-CH2-O), 30.01, 24.68.

### Synthesis of 2-oxo-1,2,3,4-tetrahydroquinolin-6-yl dimethylcarbamate (TR-G005)

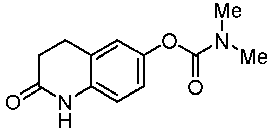

Following procedure B, 200 mg of 6-hydroxy-3,4-dihydroquinolin-2(1H)-one (1.23 mmol) was stirred with 254 mg of the base K₂CO₃ (1.84 mmol) for 30 min in EtOH, then 0.17 mL of dimethylcarbamic chloride (1.84 mmol) was added, after workup and column chromatography (Hexane:EtOAc) (60:40) then afford 260 mg (72%) of TR-G005 as white powder. LCMS/MS: m/z 234.42 [M+H]⁺

1H NMR (500 MHz, DMSO-d6) δ 10.08 (s, 1H. N-H), 6.95-6.78 (m, 3Harom.), 3.01 (S, 3H. N-CH3-), 2.89 (S, 3H. N-CH3-), 2.84 (t, 2H. J = 7 Hz. O=C-CH2-CH2-), 2.42 (m, 2H. J = 7 Hz. O=C-CH2-). ¹³C NMR (126 MHz, DMSO) δ 170.00 (O=C), 154.32 (O=C-O), 145.84, 135.45, 124.38, 121.31, 120.36, 115.24, 36.29 (-CH3), 36.05 (-CH3), 30.12, 24.71.

### Synthesis of 6-(prop-2-yn-1-yloxy)-3,4-dihydroquinolin-2(1H)-one (TR-G006)

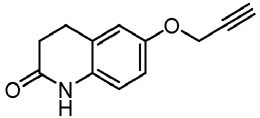

According to procedure A, 6-hydroxy-3,4-dihydroquinolin-2(1H)-one (1.53 mmol, 250 mg) was dissolved in N,N-Dimethylacetamide in ice bath. NaH (2.6 mmol, 62.5 mg) was added, and the mixture was allowed to stir for 2 h. After that, propargyl bromide (3.83 mmol, 0.3 mL) was added to the mixture at 0°C for 6h., after workup and column chromatography (Hexane:EtOAc) (50:50), 206 mg (67%) of TR-G006 was obtained as off-white crystals.

According to this procedure two products (TR-G006 and TR-G006A) can be obtained. However, when DMSO was used as a solvent TR-G006 was obtained as one product and in higher yield. LCMS/MS: m/z 202.28 [M+H]⁺

1H NMR (500 MHz, DMSO-d6) δ 9.93 (s, 1H. N-H), 6.83-6.78 (m, 3Harom.), 4.71 (S, 2H. O-CH2-), 3.52 (S, 1H. C≡C-H), 2.84-2.81(m, 2H. O=C-CH2-CH2-), 2.42-2.39 (m, 2H. O=C-CH2-). 13C NMR (126 MHz, DMSO) δ 169.80, 152.34, 132.43, 124.83, 115.70, 114.54, 113-50, 79.50, 78.01, 55.72, 30.30, 25.10.

### Synthesis of 1-(prop-2-yn-1-yl)-6-(prop-2-yn-1-yloxy)-3,4-dihydroquinolin-2(1H)-one (TR-G006A)

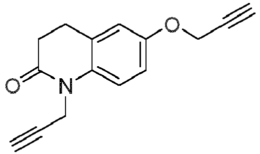

According to procedure A, 6-hydroxy-3,4-dihydroquinolin-2(1H)-one (1.53 mmol, 250 mg) was dissolved in N,N-Dimethylacetamide in ice bath. NaH (2.6 mmol, 62.5 mg) was added, and the mixture was allowed to stir for 2 h. After that, propargyl bromide (3.83 mmol, 0.3 mL) was added to the mixture at 0°C for 7 h., after workup and column chromatography (Hexane:EtOAc) (50:50), 68 mg (19%) of TR-G006A was obtained as off-white crystals. LCMS/MS: m/z 240.21 [M+H]⁺

¹H N1MR (500 MHz, DMSO-d6) δ 7.14-6.90 (d, J = 9.1 Hz, 3H), 4.77 (d, J = 2.3 Hz, 2H. -O-CH2-), 4.65 (d, J = 2.3 Hz, 2H. N-CH2-), 3.55 (t, J = 2.3 Hz, 1H. =C-H), 3.16 (t, J = 2.3 Hz, 1H. =C-H), 2.86-2.80 (m, 2H. CH2CH2-C=O), 2.55 (m, 2H. CH2-C=O). ¹³C NMR (126 MHz, DMSO) δ 168.54, 152.80, 132.71, 127.67, 115.99, 114.82, 112.99, 79-76, 79.38, 78.20, 73.96, 55.64, 31-03, 30.98, 24.70.

### Synthesis of 2-oxo-1,2,3,4-tetrahydroquinolin-6-yl 2-phenylacetate (TR-G007)

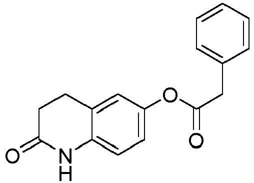

Following procedure B, 150 mg of 6-hydroxy-3,4-dihydroquinolin-2(1H)-one (0.920 mmol) was stirred with 190 mg of the base K₂CO₃ (1.38 mmol) for 2 h. in THF and drops of DMF, then 219.5 mg 2-phenylacetyl bromide (1.1 mmol) was added to the reaction at room temperature. After workup 226 mg (87.4%) of TR-G007 was obtained as white powder. LCMS/MS: m/z 282.11 [M+H]⁺

1H NMR (500 MHz, DMSO-d6) δ 9.92 (s, 1H. N-H), 8.02-6.76 (m, 8Harom), 5.49 (S, 2H), 2.83 (t, J = 7.5 Hz, 2H. CH2CH2-C=O), 2.41-2.38 (m, 2H. CH2-C=O). ¹³C NMR (126 MHz, DMSO) δ 195.20 (NH-C=O), 170.22 (C=O), 153.58, 134.90, 134.21, 132.55, 129.28, 128.32, 125.25, 116.14, 114.60 (2C), 113.65 (2C), 70.91, 30.79, 25.53.

### Synthesis of 2-oxo-1,2,3,4-tetrahydroquinolin-6-yl 3,4-dimethoxybenzoate (TR-G008)

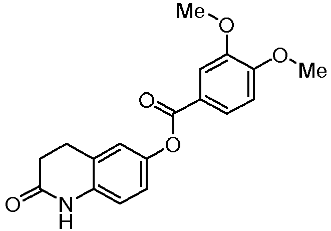

Following procedure B, 250 mg of 6-hydroxy-3,4-dihydroquinolin-2(1H)-one (1.5 mmol) was stirred with 317 mg of the base K₂CO₃ (2.3 mmol) for 30 min in THF and drops of DMF, then 306 mg of 3,4-dimethoxybenzoyl chloride (1.84 mmol) was added to the reaction at room temperature. After workup 405.00 mg (81%) of TR-G008 as white powder. LCMS/MS: m/z 328.31 [M+H]⁺

1H NMR (500 MHz, DMSO-d6) δ 10.17 (s, 1H. N-H), 7.76-6.90 (m, 6Harom.), 3.87 (S, 3H.), 3.84 (S, 3H.), 2.91-2.82 (t, J = 5 Hz, 2H - O=C-CH2-CH2), 2.48-2.45 (t, J = 5 Hz, 2H. O=C-CH2). ¹³C NMR (126 MHz, DMSO) δ 170.51, 165.03, 153.96, 149.01, 145.64, 136.52, 125.18, 124.42, 121.70, 121.41, 120.85, 115.95, 112.43, 111.71, 56.26, 56.06, 30.54, 25.17.

### Synthesis of 2-oxo-1,2,3,4-tetrahydroquinolin-6-yl 2,4-dimethoxybenzoate (TR-G009)

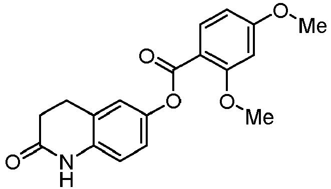

Following procedure B, 200 mg of 6-hydroxy-3,4-dihydroquinolin-2(1H)-one (1.23 mmol) was stirred with 254 mg of the base K₂CO₃ (1,84 mmol) for 30 min in THF and drops of DMF, then 295 mg of 2,4-dimethoxybenzoyl chloride (1.47 mmol) was added to the reaction at room temperature. After workup 320 mg (79.8%) of TR-G009 was obtained as yellowish white powder. LCMS/MS: m/z 328.42 [M+H]⁺

¹H NMR (500 MHz, DMSO-d6) δ 10.15 (s, 1H. N-H), 7.76-6.88 (m, 8Harom), 3.87 (S, 3H), 3.83 (S, 3H), 2.89 (t, J = 7.6 Hz, 2H. CH2CH2-C=O), 2.46 (t, J = 7.6 Hz, 2H. CH2-C=O). ¹³C NMR (126 MHz, DMSO-d6) δ 170.51 (NH-C=O), 165.03 (C=O), 153.97, 149.01, 145.63, 136.52, 125.19, 124.43, 121.71, 121.41, 120.86, 115.94, 112.44, 111.74, 56.28, 56.08, 30.55, 25.17.

### Synthesis of 6-(cyclopropylmethoxy)-3,4-dihydroquinolin-2(1H)-one (TR-G010)

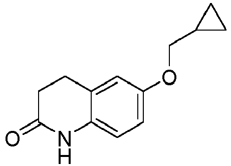

According to procedure A, 6-hydroxy-3,4-dihydroquinolin-2(1H)-one (1.23 mmol, 200 mg) was dissolved in THF and drops of DMF. K₂CO₃ (1.84 mmol, 254 mg) was added, and the mixture was allowed to stir for 2 h. After that, (chloromethyl)cyclopropane (1.59 mmol, 0.147 mL) was added to the mixture at room temperature. After workup and column chromatography (Hexane:EtOAc) (50:50), 251 mg of TR-G010 (94.25%) was obtained as light yellow crystals. LCMS/MS: m/z 217.98 [M+H]⁺

¹H NMR (500 MHz, DMSO-d6) δ 9.90 (s, 1H. N-H), 6.76-6.69 (m, 3Harom), 3.73 (d, J = 5 Hz, 2H), 2.81 (t, J = 5 Hz, 2H. CH2CH2-C=O), 2.40-2.37 (t, 2H. J = 5Hz .CH2-C=O), 1.21-1.17 (m, 1H. CH), 0.56-0.53 (m, 2H. CH2), 0.30-0.27 (m, 2H. CH2). ¹³C NMR (126 MHz, DMSO) δ 170.19 (NH-C=O), 154.27, 132.06, 125.24, 116.19, 114-43, 113-47, 72.70, 30.84, 25.57, 10.70, 3.52 (2C).

### Synthesis of 2-oxo-1,2,3,4-tetrahydroquinolin-6-yl 1-naphthoate (TR-G011)

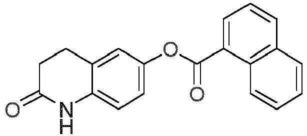

Following procedure B, 300 mg of 6-hydroxy-3,4-dihydroquinolin-2(1H)-one (1.23 mmol) was stirred with 266 mg of the base TEA (1.84 mmol) for 30 min in THF and drops of DMF, then 222 ml of 1-naphthoyl chloride (1.5 mmol) was added, . After workup and column chromatography (Hexane:EtOAc) (70:30) 313 mg (81%) of TR-G011 was obtained as white powder. LCMS/MS: m/z 318.33 [M+H]⁺

1H NMR (500 MHz, DMSO-d6) δ 10.18 (s, 1H. N-H), 8.8-6.92 (m, 10Harom.), 2.92 - 2.89 (m, 2H. O=C-CH2-), 2.48 -2.45 (m, 2H. O=C-CH2-CH2-). ¹³C NMR (126 MHz, DMSO-d6) δ 170.10, 165.63, 145.09, 136.28, 134.20, 133.49, 130.89, 130.62, 128.90, 128.23, 126.60, 125.63, 125.00, 124.85, 121.38, 120.51, 115.59, 30.11, 24.75.

### Synthesis of ethyl 2-((2-oxo-1,2,3,4-tetrahydroquinolin-6-yl)oxy)acetate (TR-G012)

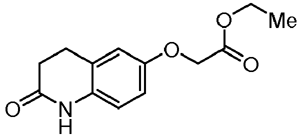

Following procedure A, 6-hydroxy-3,4-dihydroquinolin-2(1H)-one 250 mg (1.23 mmol) was stirred with 317.61 mg of the base K₂CO₃ (2.3 mmol) for 1 h. in THF and drops of DMF. Then 0.204 mL (1.51 g/cm³) of ethyl 2-bromoacetate (1.5 mmol) was added, after workup and column chromatography (Hexane:EtOAc) (50:50) then afford 313 mg (81%) of TR-G012 as white crystals. LCMS/MS: m/z 249.96 [M+H]⁺

1H NMR (500 MHz, DMSO-d6) δ 9.93 (s, 1H. N-H), 6.79-6.70 (m, 3Harom), 4.68 (s, 2H. O=C-CH2-O), 4.15 (q, J = 7.1 Hz, 2H. O-CH2-CH3), 2.82 (t, J = 7.5 Hz, 2H. O=C-CH2-CH2-), 2.39 (m, 2H. 2H. O=C-CH2-), 1.20 (t, J = 7.1 Hz, 3H. CH3). ¹³C NMR (126 MHz, DMSO) δ 172.07, 169.05, 153.81, 131.93, 125.13, 116.47, 114-93, 113-41, 65.97, 61.44, 30.54, 25.66, 14.24.

### Synthesis of 4-((2-oxo-1,2,3,4-tetrahydroquinolin-6-yl)oxy)butyl benzoate (TR-G013)

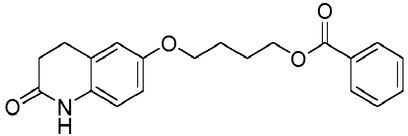

Following procedure A, 6-hydroxy-3,4-dihydroquinolin-2(1H)-one 200 mg (1.23 mmol) was stirred with 254.09 mg of the base K₂CO₃ (1.84 mmol) for 2 h. in THF and drops of DMF, then 0.204 mL (1.51 g/cm³) of 4-chlorobutyl benzoate (1.5 mmol, 0.296 mL, d:1,143 g/cm³) was added, . After workup and column chromatography (Hexane:EtOAc) (50:50) then afford 382 mg (91%) of TR-G013 as white powder. LCMS/MS: m/z 339.97 [M+H]⁺

¹H NMR (500 MHz, DMSO-d6) δ 9.92 (s, 1H. N-H), 7.99-6.71 (m, 8H. Harom.), 4.34 (t, J = 6.0 Hz, 2H. O=C-O-CH2-), 3.97 (t, J = 5.9 Hz, 2H. -O-CH2-), 2.81 (t, J = 7.5 Hz, 2H. O=C-CH2-CH2-), 2.42 - 2.36 (m, 2H. O=C-CH2-), 1.86 (m, 4H. -O-CH2-CH2-CH2-CH2-O-). ¹³C NMR (126 MHz, DMSO) δ 170.19, 166.18, 154.18, 133.72, 132.18, 130.26, 129.55 (2C), 129.19 (2C), 125.27, 116.21, 114.47, 113.44, 67.70, 64.89, 30.83, 25.92, 25.57, 25.48.

### Synthesis of 4-(6-(4-(benzoyloxy)butoxy)-2-oxo-3,4-dihydroquinolin-1(2H)-yl)butyl benzoate (TR-G013A)

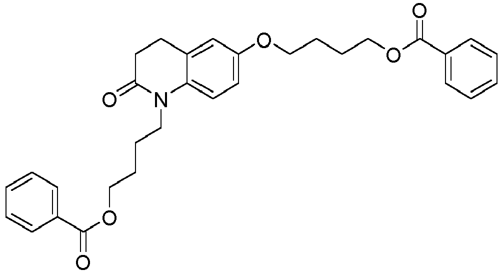

According to procedure A, 6-hydroxy-3,4-dihydroquinolin-2(1H)-one (1.23 mmol, 200 mg) was dissolved in N,N-Dimethylacetamide and put it in ice bath. NaH (1.84 mmol, 44.12 mg) was added, and the mixture was allowed to stir for 1 h. After that, 4-chlorobutyl benzoate (1.84 mmol, 0.342 mL) was added to the mixture at 0°C for 8 h, after workup and column chromatography (Hexane:EtOAc) (50:50), 173.00 mg (27 %) of TR-G013A was obtained as off-white oily product. LCMS/MS: m/z 516.26 [M+H]⁺

### Synthesis of 6-(allyloxy)-3,4-dihydroquinolin-2(1H)-one (TR-G014)

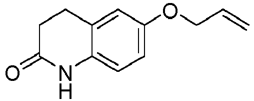

According to procedure A, 6-hydroxy-3,4-dihydroquinolin-2(1H)-one (1.53 mmol, 250 mg) was dissolved in N,N-Dimethylacetamide in ice bath. NaH (2.6 mmol, 62.5 mg) was added, and the mixture was allowed to stir for 2 h. After that, allyl bromide (3.83 mmol, 0.331 mL) was added to the mixture at 0°C for 8 h., after workup and column chromatography (Hexane:EtOAc) (50:50), 201 mg (65%) of TR-G014 was obtained as off-white crystals. LCMS/MS: m/z 204.22 [M+H]⁺

Note: According to this, two products (TR-G014 and TR-G014A) could be obtained. However, when DMSO was used as a solvent TR-G014 was obtained as one product and in higher yield.

¹H NMR (500 MHz, Chloroform-d) δ 9.92 (s, 1H. N-H), 6.8-6.72 (m, 3H. Hₐᵣₒₘ.), 6.06-5.98 (m, 1H. CH=CH₂), 5.39-5.22 (m, 2H. C=CH₂), 4.50-4.48 (t, *J* = 5.2, 1.0 Hz, 2H. O-CH₂-), 2.84-2.81 (m, 2H. O=C-CH₂-), 2.41-2.38 (m, 2H. O=C-CH₂-CH₂-). ¹³C NMR (126 MHz, Chloroform) δ 169.75, 153.34, 133.96, 131.87, 124.83, 117.19, 115.74, 114.23, 113.18, 68.44, 30.36, 25.11.

### Synthesis of 1-allyl-6-(allyloxy)-3,4-dihydroquinolin-2(1H)-one (TR-Go14A)

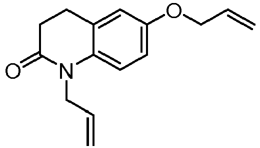

According to procedure A, 6-hydroxy-3,4-dihydroquinolin-2(1H)-one (1.53 mmol, 250 mg) was dissolved in N,N-Dimethylacetamide and put it in ice bath. NaH (2.6 mmol, 62.5 mg) was added, and the mixture was allowed to stir for 2 h. After that, allyl bromide (3.83 mmol, 0.331 mL) was added to the mixture at 0°C for 8 h., after workup and column chromatography (Hexane:EtOAc) (50:50), 80 mg (22 %) of TR-G014A was obtained as off-white crystals. LCMS/MS: m/z 244.21 [M+H]⁺

*1*H NMR (500 MHz, DMSO-d6) δ 6.94-6.76 (m, 3Harom), 6.06-5.98 (m, 1H. -O-CH2-CH=), 5.87-5.77 (m, 1H. -N-CH2-CH=), 5.40-5.23 (m, 2H. -CH=CH2), 5.24 (m, 2H. -CH=CH2), 4.52 (dt, J = 5.1, 1.4 Hz, 2H. -O-CH2-), 4.49-4.43 (m, 2H. >N-CH2-), 2.87-2.80 (m, 2H. O=C-CH2-CH2-), 2.55 (dd, J = 8.4, 6.3 Hz, 2H. O=C-CH2-). *13*C NMR (126 MHz, DMSO) δ 169.08, 154.00, 134.30, 133.77, 133.45, 128.11, 117.72, 116.59, 116.18, 114.84, 113.14, 68.83, 44.30, 31.62, 25.44.

### Synthesis of 2-oxo-1,2,3,4-tetrahydroquinolin-6-yl 2-chloroacetate (TR-G016)

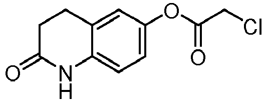

Following procedure B, 250 mg of 6-hydroxy-3,4-dihydroquinolin-2(1H)-one (1.5 mmol) was stirred with 317 mg of the base K₂CO₃ (2.3 mmol) for 30 min in THF and drops of DMF, then 0.146 mL of 2-chloroacetyl chloride (1.84 mmol) was added to the reaction in ice bath. After workup 295 mg (80%) of TR-G016 as yellowish white powder. LCMS/MS: m/z 240.36 [M+H]⁺

¹H NMR (500 MHz, DMSO-d6) δ 10.15 (s, 1H. N-H), 6.94 (m, 3 Harom), 4.66 (s, 2H. -CH2-Cl), 2.87 (t, J = 7.6 Hz, 2H. O=C-CH2-CH2-), 2.47 - 2.42 (m, 2H. O=C-CH2-). ¹³C NMR (126 MHz, DMSO) δ 170.05, 166.66, 144.63, 136.43, 124.87, 120.77, 119.95, 115.57, 41.30, 30.00, 24.67.

### Synthesis of 6-(2-bromoethoxy)-3,4-dihydroquinolin-2(1H)-one (TR-G017)

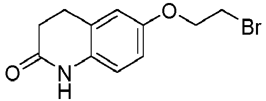

According to procedure A, 6-hydroxy-3,4-dihydroquinolin-2(1H)-one (1.23 mmol, 200 mg) was dissolved in THF and drops of DMF. K₂CO₃ (1.84 mmol, 254 mg) was added and the mixture was allowed to stir for 2 h. After that, 1,2-dibromoethane (1.59 mmol, 0.137 mL, d: 2.18 g/ml) was added to the mixture at room temperature. After workup and column chromatography (Hexane:EtOAc:MeOH) (49:50:1), 251 mg of TR-G017 (94.25%) was obtained as light yellow crystals.

1H NMR (500 MHz, DMSO-d6) δ 9.93 (s, 1H. N-H), 6.82-6.74 (m, 3Harom), 4.26-4.23 (t, 2H. J = 5Hz .CH2), 3.277 (t, 2H. J = 5Hz .CH2), 2.83 (t, J = 5 Hz, 2H. CH2CH2-C=O), 2.40 (t, J = 5 Hz, 2H. CH2C=O). ¹³C NMR (126 MHz, DMSO) δ 169.14 (NH-C=O), 152.38, 131.61, 124.33, 115.17, 113.73, 112.68, 67.45, 30.99, 29.68, 24.42.

### Synthesis of 2-((2-oxo-1,2,3,4-tetrahydroquinolin-6-yl)oxy)acetohydrazide (TR-G019)

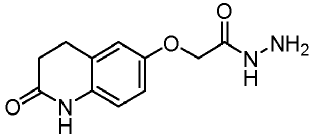

According to procedure C, 300 mg of TR-G012 (1.2 mmol) was heated with hydrazine hydrate 99% (3.6 mmol) in ethanol at 70 °C. After workup, the product was obtained as white powder with a yield of 90%.

1H NMR (500 MHz, DMSO-d6) δ 9.92 (s, 1H. N-H), 9.27 (s, 1H. O=C-N-H), 6.81-6.73 (m, 3Harom), 4.40 (s, 1H. O-CH2-C=O), 4.31 (s, 2H. NH2), 2.83-2.80 (m, 2H. O=C-CH2-), 2.41-2.38 (m, 2H. O=C-CH2-CH2). ¹³C NMR (126 MHz, DMSO) δ 170.22, 167.19, 153.44, 132.75, 125.22, 116.13, 114.76, 113-72, 67.16, 30.77, 25.56.

### Synthesis of 2-oxo-1,2,3,4-tetrahydroquinolin-6-yl diphenylcarbamate (TR-G020)

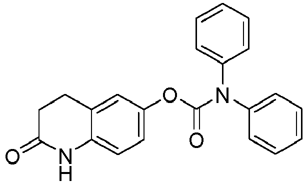

Following procedure B, 250 mg of 6-hydroxy-3,4-dihydroquinolin-2(1H)-one (1.5 mmol) was stirred with 317.6 mg of the base K₂CO₃ (2.3 mmol) for 30 min in DMSO, then 0.462 mg of diphenylcarbamic chloride (1.99 mmol) was added slowly to the reaction at room temperature. The product precipitated in ice water the final product filtered and recrystallized by EtOH and obtained 525 mg (96.1%) of TR-G020 as white powder. LCMS/MS: m/z 259.11 [M+H]⁺

1H NMR (500 MHz, Chloroform-d) δ 10.11 (s, 1H. N-H), 7.42-6.82 (m, 13H. Harom.), 2.87 - 2.84 (m, 2H. O=C-CH2-), 2.44 - 2.41 (m, 2H. O=C-CH2-CH2-). ¹³C NMR (126 MHz, Chloroform) δ 170.01, 152.80, 145.34, 142.26, 135.86, 129.16, 127.18, 126.66, 124.58, 121.17, 120.25, 115.31, 30.06, 24.66.

### Synthesis of N'-(4-chlorobenzylidene)-2-((2-oxo-1,2,3,4-tetrahydroquinolin-6-yl)oxy)acetohydrazide (TR-G021)

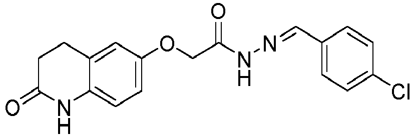

150 mg of TR-G019 (0.8 mmol) was heated with 134.45 mg of 4-chlorobenzaldehyde (0.956 mmol) in ethanol under reflux with presents of AcOH (2 drops). The desire molecule was precipitated, when the reaction finish (controlled by TLC) the solvent was evaporated and obtained compound was washed by ethanol to obtain 210 mg (92%) as white powder. LCMS/MS: m/z 357.91 [M+H]⁺

1H NMR (500 MHz, DMSO-d6) δ11.62 (*11.59, s, 1H. C-NH-N=), 9.96 (*9.92, s, 1H. C-NH-C), 8.33 (_{*}7.99, s, 1H. N=CH-) 7.74-6.72 (m, 7Harom), 5.07 (s, 1H. O-CH2-C=O), 4.60 (s, 1H. O-CH2-C=O), 2.86-2.81 (m, 2H. CH2CH2-C=O), 2.42 - 2.38 (m, 2H. CH2C=O). (* refer to the rotameric peak). ¹³C NMR (126 MHz, DMSO) δ 170.23, 164.97, 153.86, 147.04, 142.89, 134.81, 133.39, 132.46, 129.32 (2C), 129.04, 125.21, 116.13, 114.59, 113.52, 65.50, 30.81, 25.55.

### Synthesis of N'-(2,4-dimethoxybenzylidene)-2-((2-oxo-1,2,3,4-tetrahydroquinolin-6-yl)oxy)acetohydrazide (TR-G022)

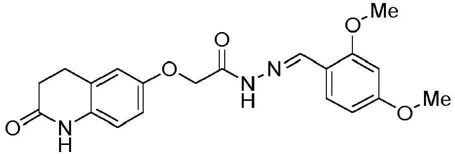

Starting from 6-hydroxy-3,4-dihydroquinolin-2(1H)-one, TR-G022 was prepared through 3 steps following procedures A, C and D respectively. 200 mg of TR-G019 (0.8 mmol) was refluxed with 0.16 ml of 2,4-dimethoxybenzaldehyde (1 mmol) in ethanol and drops of acetic acid. After filtration obtained pure white powder TR-G022 product. LCMS/MS: m/z 383.97 [M+H]⁺

¹H NMR (500 MHz, DMSO-d6) δ11.39 (*11.36, s, 1H. C-NH-N=), 9.95 (*9.91, s, 1H. C-NH-C), 8.58 (*8.25, s, 1H. N=CH-) 7.77-6.59 (m, 6Harom), 5.02 (s, 1H. O-CH2-C=O), 4.55 (s, 1H. O-CH2-C=O), 3.85 (s, 3H. -O-CH3), 3.81 (s, 3H. -O-CH3), 2.86-2.81 (m, 2H. CH2CH2-C=O), 2.43 - 2.38 (m, 2H. CH2C=O). (* refer to the rotameric peak). ¹³C NMR (126 MHz, DMSO) δ 170.22, 164.39, 162.74, 159.45, 153.93,143.91 (-N=CH-), 139.95, 132.41, 127.16, 125.20, 116.13, 114.56, 113.51, 106.91, 98.56, 65.54, 56.23 (-O-CH3), 55.90 (-O-CH3), 30.81, 25.55.

### Synthesis of 2-oxo-1,2,3,4-tetrahydroquinolin-6-yl 2-(4-(pyrimidin-2-yl)piperazin-1-yl)acetate (TR-G023)

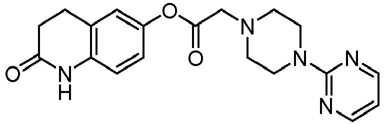

Compound TR-G023 was synthesized according to procedure B and A respectively. In the first step, compound TR-G016 was synthesized according to procedure B as previously mentioned. After that 200 µL of 2-(piperazin-1-yl)pyrimidine was (1.52 mmol, d=1.15 g/ml) was stirred with TEA (2.28 mmol, 0.330 ml, d=0.7 g/ml) for 1 h in THF, then 544 mg of TR-G016 (2.28 mmol) was added to the reaction mixture and stirred for 10h in THF. The reaction controlled by TLC and when it finished the solvent was evaporated and the product was washed with cold water filtered, dried and purified by column chromatography (Hexane:EtOAc:MeOH) (49:50:1), 291 mg of TR-G023 (86.16%) was obtained as light-yellow crystals. LCMS/MS: m/z 368.21 [M+H]+

¹H NMR (500 MHz, DMSO-d6) δ 9.95 (s, 1H. N-H), 8.40-8.39 (m, 2Harom), 6.82 -6.66 (m, 4Harom) 4.79 (s, 2H. CH2). 3.80 -3.55 (m, 8H. CH2 Piperazine), 2.83 (t, J = 5 Hz, 2H.CH2CH2-C=O), 2.40 (t, J=5 Hz, 2H. CH2C=O). ¹³C NMR (126 MHz, DMSO) δ 170.20 (NH-C=O), 166.68, 161.54, 158.46, 153.63, 132.59, 125.21, 116.14, 114.69, 113.63, 110.95, 66.86, 44-40 (2C), 43.84, 43.51, 41.47, 30.80, 25.56.

### Synthesis 2-((2-oxo-1,2,3,4-tetrahydroquinolin-6-yl)oxy)-N'-(thiophen-2-ylmethylene)acetohydrazide (TR-G024)

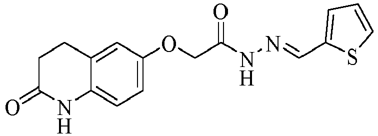

161 mg of TR-G019 (0.684 mmol) was heated with 134.45 mg of thiophene-2-carbaldehyde (0.821 mmol) in ethanol under reflux with presents of AcOH (3 drops). The desire molecule was precipitated, when the reaction finish (controlled by TLC) the solvent was evaporated and obtained compound was washed by ethanol to obtain 200 mg of TR-G024 (88.7%) as white powder. LCMS/MS: m/z 329.97 [M+H]⁺

¹H NMR (500 MHz, Chloroform-d) δ11.54 (*11.48, s, 1H. C-NH-N=), 9.95 (*9.92^{,} s, 1H. C-NH-C), 8.55 (*8.18, s, 1H. N=CH-) 7.67-6.70 (m, 6Harom), 4.97 (s, 1H. O-CH2-C=O), 4.57 (s, 1H. O-CH2-C=O), 2.85-2.80 (m, 2H), 2.42 - 2.38 (m, 2H). (* refer to the rotameric peak). ¹³C NMR (126 MHz, Chloroform) δ 169.78, 164.26, 152.88, 143.06, 138.90, 132.48, 131.15, 129.10, 127.88, 124.87, 115.75, 114.39, 113.33, 66.98, 30.30, 25.10.

### Synthesis (E)-N'-(furan-2-ylmethylene)-2-((2-oxo-1,2,3,4-tetrahydroquinolin-6-yl)oxy)acetohydrazide (TR-G025)

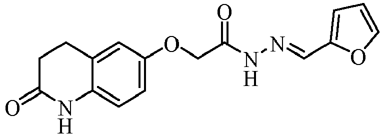

Starting from 6-hydroxy-3,4-dihydroquinolin-2(1H)-one, TR-G025 was prepared through 3 steps following procedures A, C and D respectively. 210 mg of TR-G019 (0.892 mmol) was refluxed with 0.89 ml of furan-2-carbaldehyde (1.07 mmol, d=1.16 g/ml) in ethanol and drops of acetic acid. After filtration obtained pure white powder TR-G025 product. LCMS/MS: m/z 314.04 [M+H]⁺

¹H NMR (500 MHz, DMSO-d) δ 11.50 (*11.47, s, 1H. C-NH-N=), 9.95 (*9.91, s, 1H. C-NH-C), 8.24 (*7.88, s, 1H. N=CH-) 7.84-6.62 (m, 6Harom), 4.98 (s, 1H. O-CH2-C=O), 4.58 (s, 1H. O-CH2-C=O), 2.85-2.82 (m, 2H. O=C-CH2-CH2-), 2.42-2.38 (m, 2H. O=C-CH2-CH2-). (*refer to the rotameric peak). ¹³C NMR (126 MHz, DMSO) δ 169.79, 164.37, 153.37, 148.97, 145.05, 137.72, 133.91, 132.02, 124.78, 115.69, 113.79, 113.06, 112.16, 64.88, 30.35, 25.10.

### Synthesis (E)-N'-(4-methoxybenzylidene)-2-((2-oxo-1,2,3,4-tetrahydroquinolin-6-yl)oxy)acetohydrazide (TR-G026)

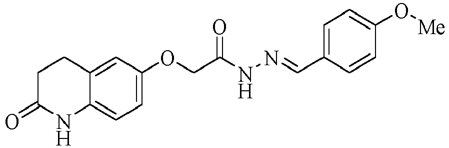

190 mg of TR-G019 (0.807 mmol) was heated with 0.118 ml of 4-methoxybenzaldehyde (0.97 mmol, d=1.12 g/ml) in ethanol under reflux with presents of AcOH (3 drops). The desire molecule was precipitated. When the reaction finish (controlled by TLC). After workup, TR-G026 was obtain 217 mg (76%) as yellowish white powder. LCMS/MS: m/z 354.22 [M+H]⁺

¹H NMR (500 MHz, DMSO-d) δ 11.44 (*11.39, s, 1H. C-NH-N=), 9.96 (*9.92, s, 1H. C-NH-C), 8.28 (*7.95, s, 1H. N=CH-) 7.65-6.72 (m, 7Harom), 5.05 (s, 1H. O-CH2-C=O), 4.58 (s, 1H. O-CH2-C=O), 3.80 (s, 3H. -O-CH3) 2.86-2.81 (m, 2H. O=C-CH2-CH2-), 2.43-2.38 (m, 2H. O=C-CH2-CH2-). (* refer to the rotameric peak). ¹³C NMR (126 MHz, DMSO) δ 169.78 (O=C<), 164.12 (O=C-NH=N-), 160.69, 153.46, 143.61 (N=CH-), 131.98, 128.51, 126.57, 124.76, 115.69, 114.34, 114.28, 114.11, 113.30, 113.05, 65.04 (O-CH2-C=O), 55.31 (-CH3), 30.36, 25.10.

### Synthesis of (E)-N'-((2-methyl-1H-indol-3-yl)methylene)-2-((2-oxo-1,2,3,4-tetrahydroquinolin-6-yl)oxy)acetohydrazide (TR-G027)

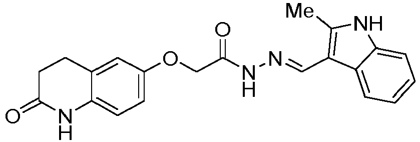

Starting from 2-((2-oxo-1,2,3,4-tetrahydroquinolin-6-yl)oxy)acetohydrazide (TR-G019) was prepared through 3 steps following procedures A, C and D respectively. 200 mg of TR-G019 (0.8 mmol) was refluxed with 162.41mg of 2-Methylindole-3-carboxaldehyde (1 mmol) in ethanol and drops of acetic acid. After workup and recrystallization by EtOH obtained 110 mg (34.5%) of TR-G027 as white powder. LCMS/MS: m/z 377.12 [M+H]⁺

¹H NMR (500 MHz, DMSO-d6) δ 13.69 (s, 1H. N-H), 12.45 (O=C-NH-N), 11.25 (s, 1H. N=CH-), 9.97 (s, 1H. C-NH-), 7.58-6.81 (m, 7 Harom), 4.78 (s, 2H O-CH2-), 2.91-2.78 (m, 2H. O=C-CH2-CH2-), 2.50 (s, 3H. -CH3), 2.41 (t, J = 6.9 Hz, 2H. O=C-CH2-). 13C NMR (126 MHz, DMSO) δ 169.80 (O=C), 162.54 (O=C-NH-N=), 152.19, 142.67, 135.57, 131.99, 132.95, 125.06, 122.70, 121.07, 119.71, 115.84 (2C), 114.48, 113.47, 111.22 (2C), 67.45 (O-CH2-C=O), 30.26, 25.21, 25.06 (-CH3).

### Synthesis of (E)-2-((2-oxo-1,2,3,4-tetrahydroquinolin-6-yl)oxy)-N'-(4-propylbenzylidene)acetohydrazide (TR-G028)

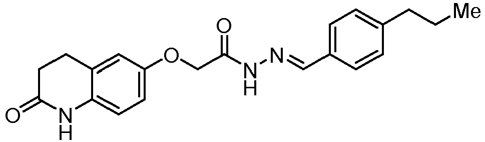

Starting from 6-hydroxy-3,4-dihydroquinolin-2(1H)-one, TR-G028 was prepared through 3 steps following procedures A, C and D respectively. 210 mg of TR-G019 (0.893 mmol) was refluxed with 0.158 ml of 4-propylbenzaldehyde (1.07 mmol, d=1.005 g/ml) in ethanol and drops of acetic acid. After filtration obtained pure white powder TR-G028 product (285 mg, 87.3%). LCMS/MS: m/z 366.20 [M+H]+

¹H NMR (500 MHz, DMSO-d6) δ11.50 (*11.45, s, 1H. C-NH-N=), 9.96 (*9.92, s, 1H. C-NH-C), 8.31 (*7.97, s, 1H. N=CH-) 7.61-6.73 (m, 7Harom), 5.05 (s, 1H. O-CH2-C=O), 4.58 (s, 1H. O-CH2-C=O), 2.85-2.81 (m, 2H. O=C-CH2-), 2.57 (t, J = 7.5 Hz, 2H. O=C-CH2-CH2-), 2.43 - 2.36 (m, 2H. =C-CH2-CH2), 1.62-1.56 (m, 2H. -CH2-CH3), 0.88 (t, J = 8.6 Hz, 3H. CH3). (* refer to the rotameric peak). ¹³C NMR (126 MHz, DMSO) δ 169·76, 164.27, 153·44, 147·96, 144·63, 143·81, 132.00, 131.56, 128.80, 127.12, 126.90, 124.75, 115.68, 114.12, 113.05, 65.05, 37.10, 30.36, 25.09, 23.92, 13.59.

### Synthesis of (E)-N'-(cyclohexylmethylene)-2-((2-oxo-1,2,3,4-tetrahydroquinolin-6-yl)oxy)acetohydrazide (TR-G029)

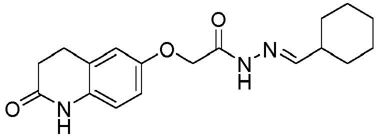

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. After that following procedure D,180 mg of TR-G019 (0.765 mmol) was allowed to stir under refluxed with 0.112 ml of cyclohexanecarbaldehyde (0.918 mmol) in ethanol and 3 drops of acetic acid. After workup by extracting with DCM and water obtained 200 mg (79.3%) of TR-G029 as white powder. LCMS/MS: m/z 329.99 [M+H]+

¹H NMR (500 MHz, DMSO-d6) δ11.08 (*11.01, s, 1H. C-NH-N=), 9.94 (*9.90, s, 1H. C-NH-C), 7.52 (*7.21, s, 1H. N=CH-) 6.82-6.65 (m, 3Harom), 4.86 (s, 1H. O-CH2-C=O), 4.47 (s, 1H. O-CH2-C=O), 2.84-2.80 (m, 2H. O=C-CH2-), 2.41-2.37 (m, 2H. O=C-CH2-CH2-), 2.21- 2.15 (m, 1H. =CH-CH<), 1.76-1.60 (m, 5HCycloHexane), 1.31-1.13 (m, 5HCycloHexane). (* refer to the rotameric peak). ¹³C NMR (126 MHz, DMSO) δ 169.75, 168.62, 163.85, 155.78, 153.40, 151.61, 131.96, 124.73, 115.66, 114.04, 112.99, 64.89, 30.34, 30.29, 29.60, 29.53, 25.50, 25.08, 24.96, 24.95.

### Synthesis of 6-((4-(tert-butyl)benzyl)oxy)-3,4-dihydroquinolin-2(1H)-one (TR-G030)

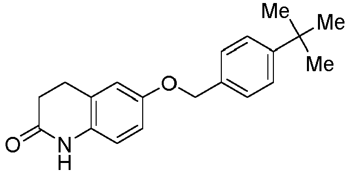

Following procedure A, 250 mg of 6-hydroxy-3,4-dihydroquinolin-2(1H)-one (1.5 mmol) was stirred with 317.61 mg of the base K₂CO₃ (2.3 mmol) for 1 h. in. in THF and drops of DMF, then 0.16 ml of 1-(tert-butyl)-4-(chloromethyl)benzene (1.99 mmol) was added slowly to the reaction at room temperature. The reaction was screened by TLC and after workup by ice water the final product was filtered and obtained 380 mg (80%) of TR-G030 as white powder. LCMS/MS: m/z 310.33 [M+H]+

¹H NMR (500 MHz, DMSO-d6) δ 9.91 (s, 1H. N-H), 7.40-6.76 (m, 7 Harom), 4.98 (s, 2H. O-CH2-), 2.82 (t, J = 7.5 Hz, 2H. O=C-CH2-), 2.39 (m, 2H. O=C-CH2-CH2-), 1.27 (s, 9H. CH3). ¹³C NMR (126 MHz, DMSO) δ 169.73, 153.57, 150.19, 134.27, 131.88, 127.52 (2C), 125.13 (2C), 124.86, 115.73, 114.34, 113.18, 69.22, 34.28, 31.14 (3C), 30.35, 25.11.

### Synthesis of 6-((6-((tetrahydro-2H-pyran-2-yl)oxy)hexyl)oxy)-3,4-dihydroquinolin-2(1H)-one (TR-G031)

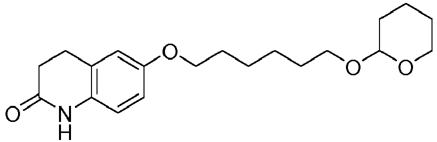

Following procedure A, 250 mg of 6-hydroxy-3,4-dihydroquinolin-2(1H)-one (1.5 mmol) was stirred with 317.61 mg of the base K₂CO₃ (2.3 mmol) for 1 h. in THF and drops of DMF, then 394 µL of 2-(6-chlorohexyloxy)tetrahydro-2H-pyran (1.84 mmol) was added slowly to the reaction at room temperature. The reaction was screened by TLC and after workup by ice water the final product was filtered and obtained 320 mg (60%) of TR-G031. LCMS/MS: m/z 348.22 [M+H]⁺

¹H NMR (500 MHz, DMSO-d6) δ 9.90 (s, 1H. N-H), 6.75-6.68 (m, 3Harom), 4.52 (t, J = 3.4 Hz, 1H. CHpyran) 3.87 (t, 2H. J = 5 Hz O-CH2). 3.74 -3.70 (m, 2H. O-CH2pyran), 3.40 (t, 2H. J = 5 Hz CH2-O), 2.81 (t, J = 5 Hz, 2H. CH2CH2-C=O), 2.38 (t, J = 5 Hz, 2H. CH2C=O), 1.73-1.56 (m, 6H. CH2pyran), 1.45 -1.36 (m, 8H. -CH2 Alphatic). ¹³C NMR (126 MHz, DMSO) δ 170.16 (NH-C=O), 154.31, 132.08, 125.24, 116.19, 114.40, 113-34, 98.37, 68.03, 67.00, 61.71, 30.84, 30.80, 29.66, (29.20 2C), 26.01, 25.85, 25.57, 25.54.

### Synthesis of Ethyl 2-(4-(((2-oxo-1,2,3,4-tetrahydroquinolin-6-yl)oxy)methyl)-1H-1,2,3-triazol-1-yl)acetate (TR-G033)

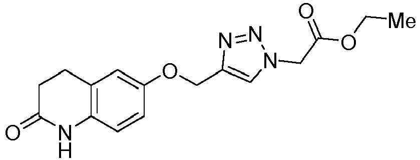

Compound TR-G033 has been prepared according to procedure A and E. In the first step, ethyl 2-azidoacetate was prepared starting from 500 µL of ethyl bromoacetate (4.94 mmol) which were refluxed with 641 mg of sodium azide salt (9.87 mmol) for 2 hours in ethanol/ water (2:1). After that, the mixture was cooled to room temperature and filtered. Secondly, 6-(prop-2-yn-1-yloxy)-3,4-dihydroquinolin-2(1H)-one TR-G006 was prepared according to procedure A as mentioned before. In the last step, a mixture of -(prop-2-yn-1-yloxy)-3,4-dihydroquinolin-2(1H)-one TR-G006 (250 mg, 1.24 mmol), 29.74 mg of copper sulfate (0.186 mmol) and 12 mg of sodium ascorbate (0.062 mmol) in EtOH was added to the ethyl 2-azidoacetate solution and stirred for 8 h. After that, the ethanol was evaporated. The residue was dissolved in water and the mixture was extracted with DCM (3 × 50 ml), dried over anhydrous magnesium sulfate MgSO4 and the solvent was evaporated under vacuum. Finally, the product was purified using flash chromatography (Hexane:EtOAc:TEA) (40:60:1%) to obtain 230 mg (52%) of compound TR-G033 as crystals. LCMS/MS: m/z 331.42 [M+H]⁺

¹H NMR (500 MHz, DMSO-d6) δ 9.90 (s, 1H. N-H), 8.20 (s, 1HTraizole), 6.90-6.76 (m, 3Harom), 5.40 (S, 2H), 5.10 (S, 2H), 4.18 (q, J = 7.1 Hz, 2H CH2-CH3), 2.83 (t, J = 7.5 Hz, 2H. CH2CH2-C=O) 2.40 (m, 2H. CH2-C=O) 1.22 (t, J = 7.1 Hz, 3H. CH3). ₁₃C NMR (126 MHz, DMSO) δ 170.21 (NH-C=O), 167.69 (C=O), 153.62 (Ctraizole), 143.35, 132.54, 126.34, 125.33, 116.19, 114.84, 113-72, 61.97, 61.74, 50.84, 30.79, 25.57, 14.44.

### Synthesis of (E)-N'-benzylidene-2-((2-oxo-1,2,3,4-tetrahydroquinolin-6-yl)oxy)acetohydrazide (TR-G035)

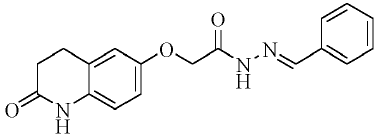

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. After that following procedure D, 200 mg of TR-G019 (0.850 mmol) was refluxed with 95 µL of benzaldehyde (0.935 mmol) were refluxed in ethanol and drops of acetic acid for 6 h. After completing the reaction, the precipitate was filtered out of the hot ethanol, washed with hot ethanol and dried to obtain 220 mg (80%) of the final product as white powder. LCMS/MS: m/z 324.11 [M+H]⁺

¹H NMR (500 MHz, DMSO-d6) δ 11.56 (*11.53, s, 1H. C-NH-N=), 9.96 (*9.92, s, 1H. C-NH-C), 8.34 (*8.01, s, 1H. N=CH-) 7.70-6.74 (m, 8Harom), 5.07 (s, 1H. O-CH2-C=O), 4.60 (s, 1H. O-CH2-C=O), 2.86-2.81 (m, 2H. O=C-CH2-CH2-), 2.43-2.38 (m, 2H. O=C-CH2-CH2-). (* refer to the rotameric peak). ¹³C NMR (126 MHz, DMSO) δ 170.26 (O=C<), 164.89 (O=C-NH=N-), 153.88, 148.39, 144.21 (N=CH-), 134.41, 132.44, 130.41, 129.30, 127.37 (2C), 125.23, 116.15, 114.59, 113.51, 65.51, 30.80, 25.54.

### Synthesis of 2-oxo-1,2,3,4-tetrahydroquinolin-6-yl 2fluoro-5-(trifluoromethyl)benzoate (TR-G036)

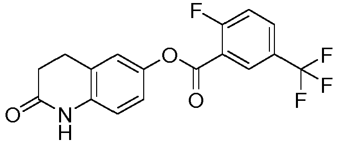

Following procedure B, 200 mg of 6-hydroxy-3,4-dihydroquinolin-2(1H)-one (1.23 mmol) was stirred with 254 mg of the base K₂CO₃ (1.84 mmol) for 30 min in THF and drops of DMF, then 222 µL of 2-fluoro-5-(trifluoromethyl)benzoyl chloride (1.47 mmol) was slowly added to the reaction mixture and stirred for 16 h. After completing the reaction, the solvent was evaporated and the residue washed with distilled water, filtered and air-dried and then recrystallized in ethanol to obtain 370 mg of the product. LCMS/MS: m/z 354.21 [M+H]+

### Synthesis of 2-oxo-1,2,3,4-tetrahydroquinolin-6-yl ethyl(methyl)carbamate (TR-G037)

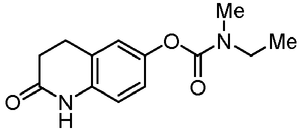

Following procedure B, 200 mg of 6-hydroxy-3,4-dihydroquinolin-2(1H)-one (1.23 mmol) was stirred with 254 mg of the base K₂CO₃ (1.84 mmol) for 30 min in THF and drops of DMF, then 163 µL of ethyl(methyl)carbamic chloride (1.47 mmol) was slowly added to the reaction mixture and stirred for 12 h. After completing the reaction, the solvent was evaporated and the residue washed with distilled water, filtered and air-dried and then recrystallized in ethanol to obtain 210 mg (69%) of the product as crystals. LCMS/MS: m/z 249.23 [M+H]⁺

### Synthesis of (Z)-2-((2-oxo-1,2,3,4-tetrahydroquinolin-6-yl)oxy)-N'-(2-oxoindolin-3-ylidene)acetohydrazide (TR-G038)

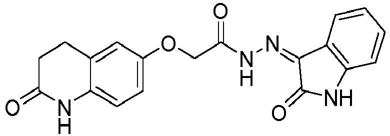

Starting from hydroxy-3,4-dihydroquinolin-2(1H)-one, TR-G038 was prepared through three steps following procedure A,C and D. In the first and second steps compounds TR-G012 and TR-G019 were obtained as previously mentioned. In the third step, 250 mg of 2-((2-oxo-1,2,3,4-tetrahydroquinolin-6-yl)oxy)acetohydrazide TR-G019 (1.06 mmol) was refluxed with 156 mg of isatin (1.06 mmol) in ethanol and 3 drops of acetic acid for 5 hours. After completing the reaction, the product was filtered out the hot ethanol to obtain 335 mg of the final product as yellow powder with a yield of 86%. LCMS/MS: m/z 363.₃₄ [M-H]-[00256] ₁H-NMR (500 MHz, DMSO) δ 13.69 (s, ¹H .O=C-NH-), 11.25 (s, ¹H .C=N-NH-), 9.97 (s, ¹H .N=N=CH-), 7.58-6.81 (m, 6Harom.), 4.78 (s, 2H. O-CH2-), 2.89-2.84 (m, 2H. O=C-CH2-), 2.41 (t, J = 3 Hz, 2H. O=CH2-CH2-). ₁₃C-NMR (126 MHz, DMSO) δ 169.80, 162.54, 152.19, 142.67, 135.57, 131.99, 125.06, 122.70, 121.07, 119.71,115.84 (2C), 114.48, 113.47, 111.22 (2C), 67.45, 30.26, 25.06

### Synthesis of 2-oxo-1,2,3,4-tetrahydroquinolin-6-yl acrylate (TR-G039)

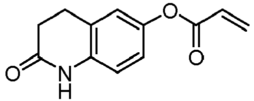

Following procedure B, 200 mg of 6-hydroxy-3,4-dihydroquinolin-2(1H)-one (1.23 mmol) was stirred with 265 µL of the base TEA (1.84 mmol) for 30 min DMF, then 119 µL of acroyl chloride (1.47 mmol) was slowly added to the reaction mixture and stirred for 6 h. After completing the reaction, and the final product was obtained by extraction in DCM /water system (25ml ×3), dried over anhydrous magnesium sulfate MgSO4 and the solvent was evaporated under vacuum. Finally, the product was purified using flash chromatography (Hexane:EtOAc:TEA) (40:60:1%) to obtain 201 mg (75%) of compound TR-G039 as light white powder. LCMS/MS: m/z 218.21 [M+H]+

₁H NMR (500 MHz, DMSO-d6) δ 10.14 (s, 1H. N-H), 7.01-6.86 (m, 3Harom), 6.53 -6.41 (m, 2H. CH2=C) 6.39-6.11 (m, 1H. CH=CH2), 2.87 (t, J = 5 Hz, 2H. CH2CH2-C=O), 2.45 (t, J = 5 Hz, 2H. CH2C=O). ₁₃C NMR (126 MHz, DMSO) δ 170.50 (NH-C=O), 164.89, 145.14, 136.62, 133.87, 128.17, 125.20, 121.46, 120.61, 115.95, 30.50, 25.13.

### Synthesis of N-acryloyl-N'-(2-((2-oxo-1,2,3,4-tetrahydroquinolin-6-yl)oxy)acetyl)acrylohydrazide (TR-G040)

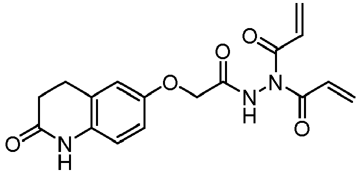

TR-G040 have synthesized according to procedure A and B. In the first and second steps compounds TR-G012 and TR-G019 have been synthesized respectively. After that, 200 mg of TR-G019 (0.85 mmol) was stirred with 368 µL of the base TEA in DMF for 15 min then 171 µL of acroyl chloride (2.55 mmol) was slowly added to the reaction mixture and stirred for 6 h. After completing the reaction, and the final product was obtained by extraction in DCM /water system (25ml X 3), dried over anhydrous magnesium sulfate MgSO4 and the solvent was evaporated under vacuum. Finally, the product was purified using flash chromatography (Hexane:EtOAc:TEA) (40:60:1%) to obtain 198 mg (67%) of compound TR-G040 as white powder. LCMS/MS: m/z 342.21 [M+H]+

### Synthesis of 6-((4-methylbenzyl)oxy)-3,4-dihydroquinolin-2(1H)-one (TR-G041)

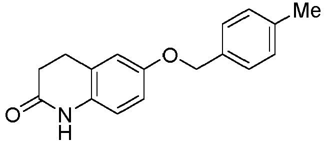

Following procedure A, 250 mg of 6-hydroxy-3,4-dihydroquinolin-2(1H)-one (1.5 mmol) was stirred with 600 mg of the base Cs₂CO₃ (1.84 mmol) for 2 h. in THF and drops of DMF, then 295 mg of 1-(bromomethyl)-4-methylbenzene (1.59 mmol) was added slowly to the reaction at room temperature. The reaction was tested until finished by TLC and after workup and column chromatography (Hexan:EtOAc) (60:40), the final product was filtered and obtained 209 mg (64%) of TR-G041 as milky white crystals. LCMS/MS: m/z 268.44 [M+H]⁺

¹H NMR (500 MHz, DMSO-d6) δ 9.91 (s, 1H. N-H), 7.31-6.74 (m, 7 Harom), 4.97 (s, 2H O-CH2-), 2.81 (t, J = 7.5 Hz, 2H. O=C-CH2-CH2-), 2.39 (m, 2H. O=C-CH2-), 2.29 (s, 3H. - CH3). ¹³C NMR (126 MHz, DMSO) δ 169.74 (>C=O), 153.53, 136.95, 134.23, 131.89, 128.94 (2C), 127.71 (2C), 124.83, 115.72, 114.40, 113.28, 69.35, 30.35, 25.11, 20.78.

### Synthesis of 2-oxo-1,2,3,4-tetrahydroquinolin-6-yl 2,3-dichlorobenzenesulfonate (TR-G042)

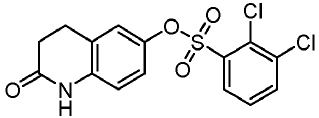

Following procedure A, 250 mg of 6-hydroxy-3,4-dihydroquinolin-2(1H)-one (1.5 mmol) was stirred with 317.6 mg of the base K₂CO₃ (2.3 mmol) for 30 min in THF and drops of DMF, then 2,3-dichlorobenzenesulfonyl chloride (1.5 mmol) was added slowly to the reaction at room temperature. The product precipitated in ice water the final product filtered and recrystallized by EtOH and obtained 483.00 mg (84.7%) of TR-G042 as white powder. LCMS/MS: m/z 373.11 [M+H]⁺

¹H NMR (500 MHz, DMSO-d6) δ 10.19 (s, 1H. N-H), 8.11-7.57 (m, 3Harom), 7.03-6.78 (m, 3Harom), 2.84 (t, J = 7.6 Hz, 2H. O=C-CH2-CH2-), 2.41 (m, 2H. O=C-CH2-). ¹³C NMR (126 MHz, DMSO) δ 169.98 (>C=O), 142.98 (C-O-S), 137.81, 136.82, 135.07, 134.49, 131.09, 130.02, 129.03, 125.50, 121.30, 120.22, 115.92, 29.73, 24.49.

### Synthesis of N-(4-(4-methylpiperazin-1-yl)phenyl)-2-((2-oxo-1,2,3,4-tetrahydroquinolin-6-yl)oxy)acetamide (TR-G043)

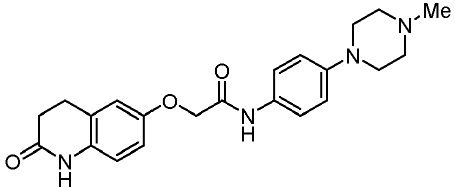

Following procedure B, the intermediate 2-chloro- N-(4-(4-methylpiperazin-1-yl)phenylacetamide was prepared by dissolving 300 mg of 4-(4-methylpiperazin-1-yl )aniline (1.57 mmol) with 340 µLTEA (2.35 mmol) in THF, then 1 52µL of chloroacetyl chloride (1.88 mmol) was added, drop wisely to the reaction mixture in ice bath (0-5 °C). After completing the reaction, the solvent was evaporated under vacuum, and the residue was washed with distilled water, filtered, and dried. The second step was according to procedure A, 200 mg of 6-hydroxy-3,4-dihydroquinolin-2(1H)-one (1.23 mmol) was stirred with 254 mg of the base K2CO3 (1.84 mmol) for 15 min in DMF after that 328 mg of 2-chloro- N-(4-(4-methylpiperazin-1-yl)phenylacetamide (1.23 mmol) was added to the reaction mixture and stirred for 24h. After that, the product was precipitated in ice water and brine, filtered and air-dried. The product was recrystallized in ethanol to obtain 385 mg (79%) as brown powder. LCMS/MS: m/z 395.41 [M+H]+

### Synthesis of 2-oxo-1,2,3,4-tetrahydroquinolin-6-yl naphthalene-1-sulfonate (TR-G045)

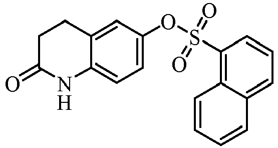

Following procedure H, In round bottom flask, 250 mg of 6-hydroxy-3,4-dihydroquinolin-2(1H)-one (1.53 mmol) was stirred with 317.6 mg of the base K₂CO₃ (2.3 mmol) for 1 hours min in THF and drops of DMF, then 416.74 mg of 1-naphthalenesulfonyl chloride (1.84 mmol) was added to the reaction at room temperature. The product was precipitated in iced water, washed with distilled water, filtered, dried under vacuum, and then recrystallized by EtOH to obtain 462.00 mg (85%) of TR-G045 as off-white powder. LCMS/MS: m/z 354.21 [M+H]+

¹H NMR (500 MHz, DMSO-d6) δ 10.14 (s, 1H. N-H), 10.14 (s, 1H), 8.66 - 7.63 (m, 7Hₐᵣₒₘ. (Naph).), 6.85 - 6.50 (m, 3Hₐᵣₒₘ), 2.75 (t, J = 7.6 Hz, 2H. O=C-CH₂-CH₂-), 2.37 - 2.34 (m, 2H. O=C-CH₂-). ¹³C NMR (126 MHz, DMSO) δ 169.94 (>C=O), 143.29 (C-O-S), 137.51, 136.31, 133.76, 131.32, 129.80, 129.44, 129.32, 127.65, 127.59, 125.21, 124.61, 124.15, 121.30, 120.10, 115.69, 29.74, 24.52.

### Synthesis of 2-oxo-1,2,3,4-tetrahydroquinolin-6-yl 4-nitrobenzenesulfonate (TR-G046)

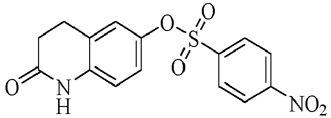

Following procedure H, 250 mg of 6-hydroxy-3,4-dihydroquinolin-2(1H)-one (1.5 mmol) was stirred with 317.6 mg of the base K₂CO₃ (2.3 mmol) for 30 min in THF and drops of DMF, then 332.43 mg of 4-nitrobenzenesulfonyl chloride (1.5 mmol) was added to the reaction at room temperature. The product was precipitated in iced water, washed with distilled water, filtered, dried under vacuum and then recrystallized by EtOH to obtain 462.00 mg (87%) of the product TR-G046 as white powder. LCMS/MS: m/z 348.95 [M+H]⁺

¹H NMR (500 MHz, DMSO-*d*₆) δ 10.20 (s, 1H. N-H), 8.46-8.12 (m, 4Hₐᵣₒₘ. O₂N-Ph-SO₃), 7.02-6.73 (m, 3Hₐᵣₒₘ.), 2.83 (t, *J* = 7.6 Hz, 2H. O=C-CH₂-), 2.41 (m, 2H. O=C-CH₂-CH₂-). ¹³C NMR (126 MHz, DMSO) δ 170.01 (>C=O), 151.02 (C-NO₂), 143.03 (C-O-S), 139.58 (C-SO₃), 137.80, 130.01(2C.), 125.45, 125.00 (2C.), 121.58, 120.58, 115.80, 29.74, 24.48.

### Synthesis of (Z)-N'-(5fluoro-1-methyl-2-oxoindolin-3-ylidene)-2-((2-oxo-1,2,3,4-tetrahydroquinolin-6-yl)oxy)acetohydrazide (TR-G047)

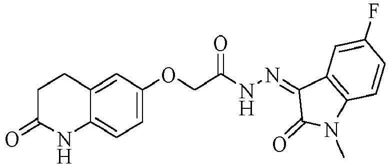

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. Later, 5-fluoro-1-methyl-2-indolin-2,3-dione was prepared according to procedure A by reacting 5-fluoruisatin with methyl iodide under basic condition of K2CO3 at room temperature. After that following procedure D, 200 mg of TR-G019 (0.850 mmol) was refluxed with 152 mg of 5-fluoro-1-methyl-2-indolin-2,3-dione (0.850 mmol) were refluxed in ethanol and drops of acetic acid for 10 h. After completing the reaction, the precipitate was filtered out of the hot ethanol, washed with hot ethanol, and dried to obtain 275 mg (81%) of the final product as white powder. LCMS/MS: m/z 397.12 [M+H]+

¹H-NMR (500 MHz, DMSO-d6) δ 13.74 (s, ¹H .O=C-NH-), 11.35 (s, ¹H .C=N-NH-), 7.51-6.78 (m, 6Hₐᵣₒₘ.), 4.80 (s, 2H. O-CH₂-), 3.38 (s, 3H. -CH₃), 2.89-2.86 (m, 2H. O=C-CH₂-), 2.48 (t, *J* = 3 Hz, 2H. O=CH₂-CH₂-). ¹³C-NMR (126 MHz, DMSO) δ 170.24, 163.10, 159.74, 157.85, 139.35, 125.46, 121.46, 121.39, 118.64, 116.28, 114.88, 113.89, 112.81, 112.74, 108.72, 108.50, 56.49, 30.72, 27.48, 25.51.

### Synthesis of 2-oxo-1,2,3,4-tetrahydroquinolin-6-yl 4-bromobenzenesulfonate (TR-G048)

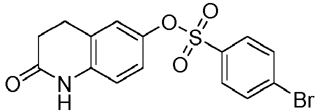

Following procedure H, 250 mg of 6-hydroxy-3,4-dihydroquinolin-2(1H)-one (1.5 mmol) was stirred with 317.6 mg of the base K2CO3 (2.3 mmol) for 30 min in THF and drops of DMF, then 391 mg of 4-bromobenzenesulfonyl chloride (1.5 mmol) was added to the reaction at room temperature and stirred for 18h. The product was precipitated in iced water, washed with distilled water, filtered, dried under vacuum, and then recrystallized by EtOH to obtain 470 mg (80%) of the product TR-G048 as crystals. LCMS/MS: m/z 383.8 [M+H]⁺

¹H NMR (500 MHz, DMSO-*d*₆) δ 10.20 (s, 1H. N-H), 8.04-6.76 (m, 7Hₐᵣₒₘ), 2.82 (t, J = 5 Hz, 2H. CH₂CH₂-C=O) 2.41 (t, J = 5 Hz, 2H. CH₂-C=O). ¹³C NMR (126 MHz, DMSO) δ 170.45 (NH-C=O), 143.55, 138.32, 138.13, 136.68, 132.38, 130.76, 127.80, 125.76, 122.98, 122.06, 121.09, 116.20, 30.25, 25.00.

### Synthesis of 2-(3-((2-oxo-1,2,3,4-tetrahydroquinolin-6-yl)oxy)propyl)isoindoline-1,3-dione (TR-G049)

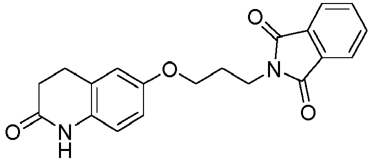

Following procedure A, 250 mg of 6-hydroxy-3,4-dihydroquinolin-2(1H)-one (1.5 mmol) was stirred with 317.6 mg of the base K2CO3 (2.3 mmol) for 30 min in THF and drops of DMF, then 402 mg of N-(3-bromopropyl) phthalimide (1.5 mmol) was added to the reaction at room temperature. After that, the product was precipitated in ice water, filtered, and dried. Then purified using flash chromatography (Hexane:EtOAc:TEA) (40:60:1%) to obtain 430 mg (81%) as light-yellow powder. LCMS/MS: m/z 350.2 [M+H]+

¹H NMR (500 MHz, DMSO-*d*₆) δ 9.88 (s, 1H. N-H), 7.88-7.80 (m, 4H_{Arom. Isoindoline}), 6.71-6.56 (m, 3Hₐᵣₒₘ.), 3.94 (t, *J* = 5.8 Hz, 2H. -CH₂-O-), 3.74 (t, *J* = 6.7 Hz, 2H. -CH₂-N-), 2.76 (t, *J* = 7.5 Hz, 2H. O=C-CH₂-), 2.37 (m, 2H. O=C-CH₂-CH₂-), 2.02 (m, 2H. -O-CH₂-CH₂-). ¹³C NMR (126 MHz, DMSO) δ 169.71, 167.97, 153.54, 134.30 (2C), 134.26 (2C), 131.76 (2C), 124.71, 122.99 (2C), 115.65, 113.99, 112.78, 65.79, 35.14, 30.32, 27.57, 25.08.

### Synthesis of (Z)-N'-(5-fluoro-2-oxoindolin-3-ylidene)-2-((2-oxo-1,2,3,4-tetrahydroquinolin-6-yl)oxy)acetohydrazide (TR-G050)

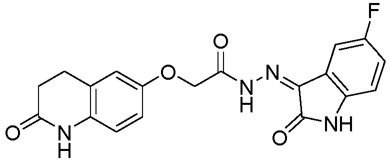

Starting from hydroxy-3,4-dihydroquinolin-2(1H)-one, TR-G050 was prepared through three steps following procedure A, C and D. In the first and second steps compounds TR-G012 and TR-G019 were obtained as previously mentioned. In the third step, 250 mg of 2-((2-oxo-1,2,3,4-tetrahydroquinolin-6-yl)oxy)acetohydrazide TR-G019 (1.06 mmol) was refluxed with 175 mg of 5-flouroisatin (1.06 mmol) in ethanol and 3 drops of acetic acid for 9hours. After completing the reaction, the product was filtered out the hot ethanol to obtain 321 mg of the final product as yellow powder with a yield of 79%. LCMS/MS: m/z 381.10 [M-H]-

¹H-NMR (500 MHz, DMSO-d6) δ 13.70 (s, ¹H .O=C-NH-), 11.28 (s, 1H .C=N-NH-), 9.97 (s, 1H .N=N=CH-), 7.40-6.81 (m, 6Hₐᵣₒₘ.), 4.80 (s, 2H. O-CH₂-), 2.87-2.84 (m, 2H. O=C-CH₂-), 2.41 (t, *J* = 3 Hz, 2H. O=CH₂-CH₂-). ¹³C-NMR (126 MHz, DMSO) δ 170.24, 163.10, 159.74, 157.85, 139.35, 125.46, 121.46, 121.39, 118.64, 116.28, 114.88, 113.89, 112.81, 112.74, 108.72, 108.50, 56.49, 30.72, 25.51.

### Synthesis of 6-((3-(4-fluorophenyl)-4,5-dihydroisoxazol-5-yl)methoxy)-3,4-dihydroquinolin-2(1H)-one (TR-G051)

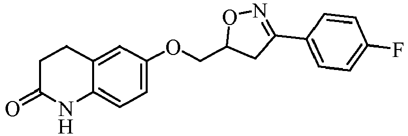

Compound TR-G051 has been prepared according to procedure A and F. Firstly, TR-G014 was prepared according to procedure A as previously mentioned. After that, a solution of 700 mg of 4-fluorobenzaldehyde (5.64 mmol), 784 mg of hydroxylamine hydrochloride (11.28 mmol) and 948 mg of NaHCO₃ in ethanol were refluxed for 3h to prepare the corresponding oxime. The reaction was monitored by TLC until completing reaction. After that, the product was filtered, washed and dried. In cyclization step, 250 mg of TR-G014 (1.23 mmol) and 205 mg of 4-flourobenzaldehyde oxime (1.48 mmol) were dissolved in chloroform then catalytic amount of calcium hypochlorite was added to the mixture in ice bath 0 °C for 8 h, then water was added, and the product was extracted with chloroform (3× 30 mL). Organic layer was dried over anhydrous magnesium sulfate and the solvent was evaporated under vacuum. The crude was purified using flash chromatography (Hexane:EtOAc) (60:40) to afford 210 mg (50%) of the final product. LCMS/MS: m/z 341.43 [M+H]⁺.

¹H NMR (500 MHz, DMSO-*d*₆) δ 9.41 (s, 1H .N-H), 7.60-6.88 (m, 7Hₐᵣₒₘ), 5.18-5.12 (qui,H oxazol), 4,17 -4.07 (m, 2H .CH₂), 3,55 -3.22 (m, 2H .CH_{2oxazol}), 2.91-2.89 (t, 2H. J = 5Hz .CH₂CH₂-C=O) 2.47-2.445 (m, 2H. J = 5Hz .CH₂C=O).¹³C NMR (126 MHz, DMSO) δ (170.48 NH-C=O), 161.54, 159.32, 153.98, 151.14, 133.78, 132.86, 129.34, 128.44, 126.46, 119.81, 118.08, 115.62, 114.22, 113.67, 79.33, 69.78, 30.84, 26.09.

### Synthesis of 6-((1-isopentyl-1H-1,2,3-triazol-4-yl)methoxy)-3,4-dihydroquinolin-2(1H)-one (TR-G052)

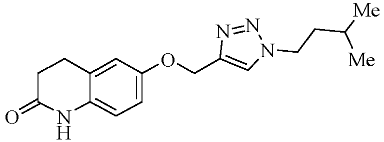

Compound TR-G052 has been prepared according to procedure A and E. In the first step, 1-azido-3-methylbutne was prepared starting from 500 µL of 1-bromo-3-methylbutane (4.17 mmol) which were refluxed with 542 mg of sodium azide salt (8.34 mmol) for 2 hours in ethanol/ water (2:1). After that, the mixture was cooled to room temperature and filtered. Secondly, 6-(prop-2-yn-1-yloxy)-3,4-dihydroquinolin-2(1H)-one TR-G006 was prepared according to procedure A as mentioned before. In the last step, a mixture of -(prop-2-yn-1-yloxy)-3,4-dihydroquinolin-2(1H)-one TR-G006 (250 mg, 1.24 mmol), 29.74 mg of copper sulfate (0.186 mmol) and 12 mg of sodium ascorbate (0.062 mmol) in EtOH was added to the 1-azido-3-methylbutne solution and stirred for 12 h. After that, the ethanol was evaporated. Residue was dissolved in water and the mixture was extracted with DCM (3 × 50 ml), dried over anhydrous magnesium sulfate MgSO₄ and the solvent was evaporated under vacuum. Finally, the product was purified using flash chromatography (Hexane:EtOAc:TEA) (40:60:1%) to obtain 218 mg (52%) of compound TR-G052 as crystals. LCMS/MS: m/z 315.61 [M+H]+

¹H NMR (500 MHz, DMSO-*d*₆) δ 9.92 (s, 1H. N-H), 8.21 (s, 1H_{Traizole}), 6.88-6.76 (m, 3Hₐᵣₒₘ), 5.05 (S, 2H), 4.37(t, *J* = 5 Hz. 2H. N-CH₂), 2.82 (t, *J* = 5 Hz, 2H. CH₂CH₂-C=O) 2.40 (t, 2H. *J* = 5 Hz. CH₂-C=O) 1.73-1.69 (m, 2H. -CH₂₋), 1.48-1.42 (m, 1H. >CH-), 0.90 (m, 3H. -CH₃), 0.90 (s, 3H. -CH₃). ¹³C NMR (126 MHz, DMSO) δ 170.20 (NH-C=O), 153.64 (Ctraizole), 143.22, 132.51, 125.30, 124.71, 116.18, 114.85, 113.77, 61.93, 48.20, 38.97, 30.80, 25.56, 25.37, 22.49 (2C).

### Synthesis of 6-((4-vinylbenzyl)oxy)-3,4-dihydroquinolin-2(1H)-one (TR-G053)

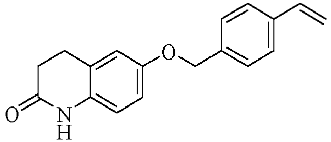

Following procedure A, 250 mg of 6-hydroxy-3,4-dihydroquinolin-2(1H)-one (1.53 mmol) was stirred with 749 mg Cs₂CO₃ (2.3 mmol) for 30 min in THF and drops of DMF, then 274 µL of 4-Vinylbenzyl chloride (1.84 mmol) was added slowly to the reaction at room temperature. After that, the product was precipitated in ice water, filtered and dried. Then purified using flash chromatography (Hexane:EtOAc:TEA) (40:60:1%) to obtain 360 mg (85%) as crystals. LCMS/MS: m/z 280.01 [M+H]⁺

¹H NMR (500 MHz, DMSO-*d*₆) δ 9.92 (s, 1H. N-H), 7.50-7.37 (m, 4H_{arom.}), 6.87-6.76 (m, 3Hₐᵣₒₘ), 6.75 - 6.69 (m, 1H. CH₂=CH-), 5.85-5.82 (m, 1H. CH₂=CH-), 5.27-5.25 (m, 1H. CH₂=CH-), 5.02 (s, 2H. -O-CH₂-), 2.82 (t, J = 7.5 Hz, 2H. O=C-CH₂-CH₂-), 2.42 - 2.36 (m, 2H. O=C-CH₂-). ¹³C NMR (126 MHz, DMSO) δ 170.20, 153.91, 137.42, 137.05, 136.76 (2C), 132.43, 128.32 (2C), 126.62 (2C), 125.32, 116.20, 114.89, 113.77, 69.64, 30.80, 25.57.

### Synthesis of N-(3-(4-methyl-1H-imidazol-1-yl)-5-(trifluoromethyl)phenyl)-2-((2-oxo-1,2,3,4-tetrahydroquinolin-6-yl)oxy)acetamide (TR-G054)

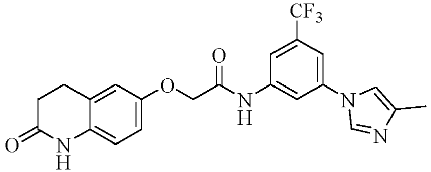

Following procedure B, the intermediate 2-chloro- N-(3-(4-methyl-1H-imidazol-1-yl)-5-(trifluoromethyl)phenylacetamide was prepared by dissolving 400 mg of 3-(4-methyl-1H-imidazol-1-yl)aniline (1.66 mmol) with 360 µL TEA (2.49 mmol) in THF, then 160 µL of chloroacetyl chloride (1.99 mmol) was added, drop wisely to the reaction mixture in ice bath (0-5 °C). After completing the reaction, the solvent was evaporated under vacuum, and the residue was washed with distilled water, filtered and dried. The second step was according to procedure A, 200 mg of 6-hydroxy-3,4-dihydroquinolin-2(1H)-one (1.23 mmol) was stirred with 254 mg of the base K2CO3 (1.48 mmol) for 15 min in DMF after that 389 mg of 2-chloro- N-(3-(4-methyl-1H-imidazol-1-yl)-5-(trifluoromethyl)phenylacetamide (1.23 mmol) was added to the reaction mixture and stirred for 24h. After that, the product was precipitated in ice water and brine, filtered and air-dried. Then purified using flash chromatography (Hexane:EtOAc:TEA) (40:60:1%) to obtain 420 mg (77%) as white powder. LCMS/MS: m/z 445.04 [M+H]

¹H NMR (500 MHz, DMSO-*d*₆) δ 10.00(s, 1H. N-H), 10.00 (s, 1H. N-H), 8.24-8.12 (m, 3Hₐᵣₒₘ), 7.71 (S, 1H_{Imidazol}), 7.46 (S, 1H_{Imidazol}), 6.91-6.81 (m, 3Hₐᵣₒₘ), 4.76 (s, 2H), 2.84 (t, J = 7.6 Hz, 2H. O=C-CH₂-CH₂-), 2.41 (t, J = 7.6 Hz, 2H. CH₂-C=O), 2.17 (S, 3H). ¹³C NMR (126 MHz, DMSO) δ (170.25, NH-C=O), (168.27, C=O), 153.34, 141.21, 139.34, 138.36, 135.41, 132.89, 131.47, 131.21, 125.34, 116.23, 114.91, 114.69, 114.60, 113.99, 113.66, 112.00, 67.78, 30.75, 25.52, 14.01.

### Synthesis of (E)-N'-(4-nitrobenzylidene)-2-((2-oxo-1,2,3,4-tetrahydroquinolin-6-yl)oxy)acetohydrazide (TR-G055)

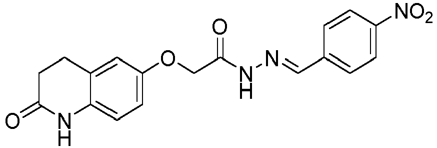

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. After that following procedure D, 200 mg of TR-G019 (0.850 mmol) was refluxed with 151 mg of 4-nitrobenzaldehyde (0.850 mmol) were refluxed in ethanol and drops of acetic acid for 6 h. After completing the reaction, the precipitate was filtered out of the hot ethanol, washed with hot ethanol and dried to obtain 271 mg (86%) of the final product as white powder. LCMS/MS: m/z 367.21 [M-H]⁻

¹H NMR (500 MHz, DMSO-*d*₆) δ 11.86 (*11.84, s, 1H. C-NH-N=), 9.97 (*9.93, s, 1H. C-NH-C), 8.45 (*8.25, s, 1H. N=CH-) 8.30-8.25 (m, 2Hₐᵣₒₘ), 7.98-6.77 (m, 5Hₐᵣₒₘ), 5.12 (s, 1H. O-CH₂-C=O), 4.65 (s, 1H. O-CH₂-C=O), 2.85-2.82 (m, 2H. O=C-CH₂-CH₂-), 2.42-2.39 (m, 2H. O=C-CH₂-CH₂-). (* refer to the rotameric peak). ¹³C NMR (126 MHz, DMSO) δ 170.23 (O=C<), 165.38 (O=C-NH=N-), 153.81, 148.17, 145.89, 141.80, 140.72, 132.51, 128.32 (2C), 124.43 (2C), 116.14, 114.61, 113.52, 65.52 (O-CH₂-C=O), 30.80, 25.54.

### Synthesis of (Z)-N'-(5-nitro-2-oxoindolin-3-ylidene)-2-((2-oxo-1,2,3,4-tetrahydroquinolin-6-yl)oxy)acetohydrazide (TR-G056)

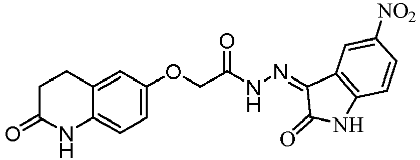

Starting from hydroxy-3,4-dihydroquinolin-2(1H)-one, TR-G056 was prepared through three steps following procedure A, C and D. In the first and second steps compounds TR-G012 and TR-G019 were obtained as previously mentioned. In the third step, 250 mg of 2-((2-oxo-1,2,3,4-tetrahydroquinolin-6-yl)oxy)acetohydrazide TR-G019 (1.06 mmol) was refluxed with 204 mg of 5-nitroisatin (1.06 mmol) in ethanol and 3 drops of acetic acid for 9 hours. After completing the reaction, the product was filtered out the hot ethanol to obtain 387 mg of the final product as yellow powder with a yield of 86%. LCMS/MS: m/z 408.26 [M-H]-

¹H-NMR (500 MHz, DMSO-d6) δ 13.40 (s, 1H .O=C-NH-), 11.30 (s, 1H .C=N-NH-), 10.02 (s, 1H. O=C-NH_{Indol}), 7.43-6.84 (m, 6Hₐᵣₒₘ.), 4.81 (s, 2H. O-CH₂-), 2.88-2.86 (m, 2H. O=C-CH₂-), 2.42 (t, J = 3 Hz, 2H. O=CH₂-CH₂-). ¹³C-NMR (126 MHz, DMSO) δ 172.61, 163.14, 160.03, 158.03, 140.39, 126.49, 121.46, 121.41, 118.68, 117.23, 117.88, 115.89, 114.81, 113.74, 109.72, 109.50, 57.49, 29.72, 26.51.

### Synthesis of 2-oxo-1,2,3,4-tetrahydroquinolin-6-yl methanesulfonate (TR-G057)

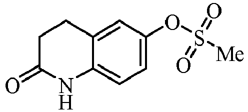

Following procedure H, 250 mg of 6-hydroxy-3,4-dihydroquinolin-2(1H)-one (1.5 mmol) was stirred with 317.6 mg of the base K2CO3 (2.3 mmol) for 30 min in in THF and drops of DMF, then 145 µL of methanesulfonyl chloride (1.84 mmol) was added slowly to the reaction at room temperature. After completing the reaction, the final product was obtained by extraction in DCM /water system three times. The product was purified using flash chromatography (Hexane:EtOAc) (50:50) to obtain 320 mg (86%) of the product as white powder. LCMS/MS: m/z 242.21 [M+H]+

¹H NMR (500 MHz, DMSO-d₆) δ 10.21 (s, 1H. N-H), 7.21 - 6.88 (m, 3Hₐᵣₒₘ.), 3.34 (s, 3H. -CH₃), 2.90 (t, J = 7.6 Hz, 2H. O=C-CH₂-), 2.46 (m, 2H. O=C-CH₂-CH₂-). ¹³C NMR (126 MHz, DMSO) δ 170.08 (O=C<), 143.50, 137.44, 125.31, 121.66, 120.96, 115.84, 37.15 (-CH₃), 29.88, 24.64.

### Synthesis of (3R,4R)-2-(acetoxymethyl)-5-(4-(((2-oxo-1,2,3,4-tetrahydroquinolin-6-yl)oxy)methyl)-1H-1,2,3-triazol-1-yl)tetrahydrofuran-3,4-diyl diacetate (TR-G058)

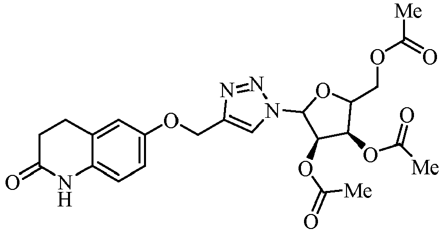

Starting from D-ribofuranose, tetraacetate the intermediate (3R,4R)-2-(acetoxymethyl)-5-azidotetrahydrofuran-3,4-diyl diacetate have been synthesized by reacting 700 mg of D-ribofuranose, tetraacetate (2.20 mmol), 506 mg of trimethylsilyl azide (1.40 mmol) and 62 mg of boron trifluoride etherate (0.439 mmol) in DCM for 1 hour at room temperature. After that. Water was added to the reaction mixture and the product was extracted in DCM/water system and the organic phase was dried over anhydrous magnesium sulfate (3× 30mL). In the next step, a mixture of -(prop-2-yn-1-yloxy)-3,4-dihydroquinolin-2(1H)-one TR-G006 (250 mg, 1.24 mmol), 29.74 mg of copper sulfate (0.186 mmol) and 12 mg of sodium ascorbate (0.062 mmol) in EtOH was added to 3R,4R)-2-(acetoxymethyl)-5-azidotetrahydrofuran-3,4-diyl diacetate solution and stirred for 12 h. After that, water was added, and the mixture was extracted with DCM (3 × 50 ml), the organic phase dried over anhydrous magnesium sulfate MgSO₄ and the solvent was evaporated under vacuum to obtain (TR-G058) as liquid. LCMS/MS: m/z 503.51 [M+H]⁺.

¹H NMR (500 MHz, Chloroform-d) 88.09 (s, 1H. N-H), 7.81 (s, 1H_{Triazol}), 6.83-6.68 (m, 3Hₐᵣₒₘ.), 6.16 (d, *J* = 4 Hz, 1H), 5.86-5.81 (m, 1H), 5.61 (m, 1H), 5.18 (s, 2H. -O-CH₂-C_{Triazol}), 4.50-4.47 (m, 1H. -O-CH₂-CH_{THF}), 4.43-4.20 (m, 2H. -O-CH₂-C_{THF}), 2.93 (t, *J* = 7.5 Hz, 2H), 2.62-2.59 (m, 2H), 2.127 (s, 3H. -CH₃), 2.126 (s, 3H. - CH₃), 2.06 (s, 3H. -CH₃). ¹³C NMR (126 MHz, CDCl₃) δ171.28, 170.50, 169.60, 169.44, 154.20, 144.79, 131.51, 125.36, 122.04, 116.26, 114.95, 113.66, 90.29, 81.04, 74.54, 70.75, 62.92, 62.48, 30.69, 25.83, 20.82, 20.64, 20.59.

### Synthesis of (E)-2-((2-oxo-1,2,3,4-tetrahydroquinolin-6-yl)oxy)-N'-(pyridin-2-ylmethylene)acetohydrazide (TR-G059)

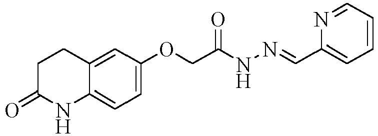

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. After that following procedure D, 200 mg of TR-G019 (0.850 mmol) was refluxed with 88 µL of 2-pyridinecarboxaldehyde (0.935 mmol) were refluxed in ethanol and drops of acetic acid for 6 h. After completing the reaction, the precipitate was filtered out of the hot ethanol, washed with hot ethanol, and dried to obtain 220 mg (80%) of the final product as beige powder. LCMS/MS: m/z 325.2 [M+H]⁺.

¹H-NMR (500 MHz, DMSO-*d*₆) δ 11.84 (s, 1H. C-NH-N=), 9.97 (*9.93, s, 1H. C-NH-C), 8.59 (*8.37, s, 1H. N=CH-) 8.05-6.74 (m, 7Hₐᵣₒₘ), 5.09 (s, 1H. O-CH₂-C=O), 4.64 (s, 1H. O-CH₂-C=O), 2.84-2.81 (m, 2H. O=C-CH₂-CH₂-), 2.41-2.38 (m, 2H. O=C-CH₂-CH₂-). (* refer to the rotameric peak). ¹³C-NMR (126 MHz, DMSO) δ 169.91, 153.44, 152.88, 149.52, 144.25, 136.88, 132.07, 124.85, 124.40, 119.91, 115.77, 114.46, 114.20, 113.13, 65.08, 30.34, 25.13.

### Synthesis of 2-oxo-1,2,3,4-tetrahydroquinolin-6-yl 4-cyanobenzoate (TR-G060)

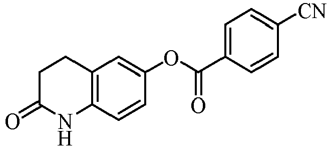

Following procedure B, 250 mg of 6-hydroxy-3,4-dihydroquinolin-2(1H)-one (1.5 mmol) was stirred with 317.6 mg of the base K₂CO₃ (2.3 mmol) for 2 h. in in THF and drops of DMF, then 329.78 mg of 4-cyanobenzoyl chloride (1.99 mmol) was added slowly to the reaction at room temperature. The product precipitated in ice water the final product filtered and recrystallized by EtOH and obtained 410 mg (91.6%) of TR-G060 as white powder. LCMS/MS: m/z 263.22 [M+H]+

¹H NMR (500 MHz, DMSO-*d*₆) δ 10.18 (s, 1H), 8.25-8.06 (m, 4Hₐᵣₒₘ), 7.15-6.90 (m, 3H_{arom.}), 2.89 (t, *J* = 7.5 Hz, 2H. O=C-CH₂-), 2.49-2.43 (m, 2H. O=C-CH₂-CH₂-). ¹³C NMR (126 MHz, DMSO) δ 170.05 (O=C), 163.67 (Ph-C=O), 144.83, 136.43, 133.02, 132.96 (2C), 130.35 (2C), 124.82, 121.04, 120.21, 118.01, 115.98, 115.54, 30.04, 24.72.

### Synthesis of 2-oxo-1,2,3,4-tetrahydroquinolin-6-yl pyrrolidine-1-carboxylate (TR-G061)

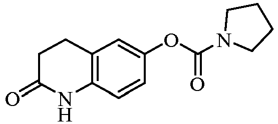

Following procedure B, 250 mg of 6-hydroxy-3,4-dihydroquinolin-2(1H)-one (1.5 mmol) was stirred with 317.6 mg of the base K₂CO₃ (2.3 mmol) for 30 min in in THF and drops of DMF, then 200 µL of 1-pyrrolidinecarbonyl chloride (1.8 mmol) was added slowly to the reaction at room temperature. After completing the reaction, the product was extracted in DCM three times from water to obtain 280 mg of TR-G061 as crystal. LCMS/MS: m/z 261.07 [M+H]+

¹H NMR (500 MHz, DMSO-*d*₆) δ 10.08 (s, 1H. N-H), 6.95 - 6.78 (m, 3Hₐᵣₒₘ), 3.46 (t, *J* = 6.6 Hz, 2H. N-CH₂), 3.31 (t, *J* = 6.6 Hz, 2H. N-CH₂-CH₂-), 2.85 (t, *J* = 7.5 Hz, 2H. O=C-CH₂-), 2.46 - 2.40 (m, 2H. O=C-CH₂-CH₂-), 1.91-1.81 (m, 4H. -CH₂-CH₂- _{Pyrolidine}). ¹³C NMR (126 MHz, DMSO) δ 170.02 (N-C=O), 152.55 (O-C=O), 145.73 (O-C=C), 135.41, 124.38, 121.36, 120.40, 115.25, 46.24, 45.99, 30.14, 25.35, 24.72, 24.48.

### Synthesis of 2-oxo-1,2,3,4-tetrahydroquinolin-6-yl 2-propylpentanoate (TR-G062)

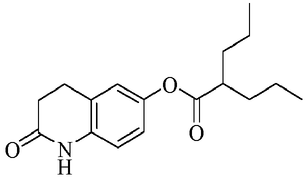

Following procedure B, 250 mg of 6-hydroxy-3,4-dihydroquinolin-2(1H)-one (1.5 mmol) was stirred with 317.6 mg of the base K₂CO₃ (2.3 mmol) for 30 min in in THF and drops of DMF, then 322 µL of 2-propylvaleryl chloride (1.7 mmol) was added slowly to the reaction in ice bath 0-5 °C and stirred for 6 h. After that, THF was evaporated, the residue was dissolved in water and the product was extracted in DCM (3 × 50 ml), dried over anhydrous magnesium sulfate MgSO₄ and the solvent was evaporated under vacuum to obtain 342 mg of TR-G062 (77%) as crystal. LCMS/MS: m/z 290.20 [M+H]⁺

¹H NMR (500 MHz, DMSO-*d*₆) δ 10.12 (s, 1H. N-H), 6.95-6.84 (m, 3Hₐᵣₒₘ), 2.89 - 2.86 (t, 2H. J = 5Hz. CH₂-CH₂-C=O), 2.45-2.42 (t, 2H. J = 5 Hz .CH₂-C=O), 1.65-1.51 (m, 1H. O=C-CH<), 1.47-1.39 (m, 4H- CH₂-CH₂-), 1.37-1.34 (m, 4H. - CH₂-CH₂-), 0.93-0.91 (t, J = 5Hz, 3H. -CH₃), 0.87-0.84 (t, J = 5Hz, 3H. CH₃). ¹³C NMR (126 MHz, DMSO) δ (177.50 NH-C=O), (175.00 C=O), 170.47, 145.31, 136.51, 125.24, 121.41, 120.58, 115.98, 44-92, 44.87, 34.62, 30.51, 25.09, 20.60, 14.36 (2C).

### Synthesis of 2-oxo-1,2,3,4-tetrahydroquinolin-6-yl 4-bromo-2-fluorobenzenesulfonate (TR-G063)

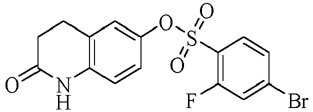

Following procedure H, 200 mg of 6-hydroxy-3,4-dihydroquinolin-2(1H)-one (1.23 mmol) was stirred with 203.2 mg of the base K₂CO₃ (1.47 mmol) for 2 h. in THF and drops of DMF, then 4-bromo-2-fluorobenzenesulfonyl chloride (1.47 mmol) was added slowly to the reaction at room temperature. The product precipitated in ice water the final product filtered and recrystallized by EtOH and obtained 483.0 mg (84.7%) of TR-G063 as white powder. LCMS/MS: m/z 261.07 [M+H]+

¹H NMR (500 MHz, DMSO-*d*₆) δ 10.20 (s, 1H. N-H), 8.07-7.64 (m, 3H), 7.03-6.79 (m, 3H), 2.84 (t, J = 7.6 Hz, 2H. O=C-CH₂-CH₂-), 2.43-2.40 (m, 2H. O=C-CH₂-). ¹³C NMR (126 MHz, DMSO) δ 170.00 (O=C), 159.31(F-C), 142.97, 137.84, 132.29, 130.62, 128.88, 125.47, 121.81, 121.70, 121.35, 120.29, 115.88, 29.74, 24.48.

### Synthesis of N-(4-acetylphenyl)-2-((2-oxo-1,2,3,4-tetrahydroquinolin-6-yl)oxy)acetamide (TR-G064)

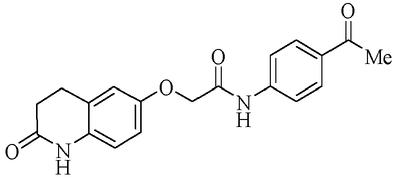

Following procedure B, the intermediate N-(4-acetylphenyl)-2-chloroacetamide was prepared by dissolving 350 mg 4'-aminoacetophenone (2.59 mmol) with 561 µLTEA (3.88 mmol) in THF, then 250 µL of chloroacetyl chloride (3.11 mmol) was added, drop wisely to the reaction mixture in ice bath (0-5 °C). After completing the reaction, the solvent was evaporated under vacuum, and the residue was washed with distilled water, filtered and dried. The second step was according to procedure A, 200 mg of 6-hydroxy-3,4-dihydroquinolin-2(1H)-one (1.23 mmol) was stirred with 254 mg of the base K₂CO₃ (1.84 mmol) for 15 min in DMF after that 259 mg of N-(4-acetylphenyl)-2-chloroacetamide (1.23 mmol) was added to the reaction mixture and stirred for 24h. After that, the product was precipitated in ice water and brine, filtered, air-dried then purified using flash chromatography (Hexane:EtOAc:TEA) (40:60:1%) to obtain 259 mg (71%) as crystals. LCMS/MS: m/z 339.42 [M+H]⁺

¹H NMR (500 MHz, DMSO-*d*₆) δ 10.37(s, 1H. N-H), 9.95 (s, 1H.N-H), 7.95-6.77 (m, 7Hₐᵣₒₘ), 4.67 (s, 2H), 2.85 -2.82 (t, J = 5Hz, 2H. CH₂-CH₂-C=O), 2.53 (s, 3H), 2.42-2.39 (t, J = 5 Hz, 2H. CH₂-C=O). ¹³C NMR (126 MHz, DMSO) δ (197.03 NH-C=O), (170.23 NH-C=O), (167.81 C=O), 153.37, 143.20, 132.90 (2C), 132.51 (2C), 129.90, 125.34, 119.34, 116.18, 114.89, 113.69, 68.02, 30.76, 26.94, 25.54.

### Synthesis of 2-oxo-1,2,3,4-tetrahydroquinolin-6-yl 4-(chloromethyl)benzoate (TR-G065)

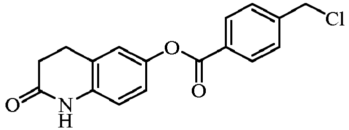

Following procedure B, 250 mg of 6-hydroxy-3,4-dihydroquinolin-2(1H)-one (1.5 mmol) was stirred with 317.6 mg of the base K₂CO₃ (2.3 mmol) for 30 min in in THF and drops of DMF, then 289 mg of 4-(chloromethyl)benzoyl chloride (1.5 mmol) was added to the reaction at room temperature and stirred for 10h. After that, THF was evaporated, the residue was dissolved in water and the product was extracted in ethyl acetate (3 × 50 ml), dried over anhydrous magnesium sulfate MgSO4 and the solvent was evaporated under vacuum to obtain 380 mg (78%) of TR-G065 as brown powder. LCMS/MS: m/z 316.01 [M+H]+

¹H NMR (500 MHz, DMSO-*d*₆) δ 10.17 (s, 1H. N-H), 8.12-6.90 (m, 7Hₐᵣₒₘ), 4.88 (S, 2H), 2.89 (t, J = 5Hz, 2H. CH₂CH₂-C=O), 2.48-2.45 (m, 2H. CH₂-C=O). ¹³C NMR (126 MHz, DMSO) δ (170.51, NH-C=O), (164.91, C=O), 145.47, 144.01, 136.70, 130.56,129.72 (2C), 129.22 (2C), 125.24, 121.63, 120.77, 115-99, 45.63, 30.53, 25.17.

### Synthesis of (E)-N'-((2-(4-chlorophenyl)pyrimidin-5-yl)methylene)-2-((2-oxo-1,2,3,4-tetrahydroquinolin-6-yl)oxy)acetohydrazide (TR-G066)

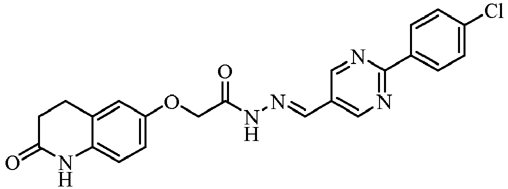

Starting from 6-hydroxy-3,4-dihydroquinolin-2(1H)-one, TR-G066 was prepared through 3 steps following procedures A, C and D respectively. Starting from 6-hydroxy-3,4-dihydroquinolin-2(1H)-one. Firstly, 300 mg of 6-hydroxy-3,4-dihydroquinolin-2(1H)-one (1.84 mmol) was stirred with 381 mg K₂CO₃ (2.76 mmol) for 15 min in THF and drops of DMF, then 244 µL of ethyl 2-bromoacetate (2.21 mmol, d= 1.51 g/mL) was added slowly to the reaction mixture. After the completing of reaction, the solvent was evaporated and the residue was washed with water, filtered and air-dried to obtain ethyl 2-((2-oxo-1,2,3,4-tetrahydroquinolin-6-yl)oxy)acetate (TR-G012) which was further refluxed with 156 µL hydrazine hydrate 99% (4.81 mmol) for 5 hours then it was cooled to room temperature, filtered out of ethanol and dried to obtain 2-((2-oxo-1,2,3,4-tetrahydroquinolin-6-yl)oxy)acetohydrazide. Later, 200 mg of 2-((2-oxo-1,2,3,4-tetrahydroquinolin-6-yl)oxy)acetohydrazide (TR-G019) (0.850 mmol) was refluxed with 278.83 mg of 2-(4-chlorophenyl)pyrimidine-5-carbaldehyde (1.28 mmol) in ethanol and drops of acetic acid for 6 hours. After filtration, the product was obtained as pure white powder. LCMS/MS: m/z 436.81 [M+H]+

¹H NMR (500 MHz, DMSO-*d*₆) δ 11.96 (*11.88, s, 1H. C-NH-N=), 9.96 (*9.92, s, 1H. C-NH-C), 9.20-9.13 (m, 2Hpyrimidine), 8.42-8.39 (m, 2Harom), 8.07 (*8.04, s, 1H. N=CH-) 7.60-6.74 (m, 5Hₐᵣₒₘ), 5.07 (s, 1H. O-CH₂-C=O), 4.60 (s, 1H. O-CH₂-C=O), 2.86-2.81 (m, 2H. O=C-CH₂-CH₂-), 2.43-2.38 (m, 2H. O=C-CH₂-CH₂-). (* refer to the rotameric peak). ¹³C NMR (126 MHz, DMSO) δ 169.77, 169.58, 162.18, 155.66, 153.38, 152.87, 142.62, 138.08, 136.12, 135.52, 132.51, 132.02, 129.56, 128.96, 126.30, 124.76, 115.68, 114.15, 113.11, 65.10, 30.36, 25.10.

### Synthesis of 6-propoxy-3,4-dihydroquinolin-2(1H)-one (TR-G068)

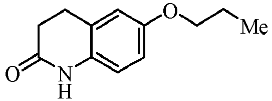

Following procedure A, 250 mg of 6-hydroxy-3,4-dihydroquinolin-2(1H)-one (1.5 mmol) was stirred with 317.6 mg of the base K₂CO₃ (2.3 mmol) for 30 min in THF and drops of DMF, then 167 µL of 1-bromopropane (1.84 mmol) was added, slowly to the reaction mixture at room temperature. After completing the reaction, the solvent was evaporated and the product was washed with distilled water, filtered, and dried. The purification was done using flash chromatography (Hexan:EtOAc)(60:40) to obtain 240 mg of the final product as crystals. LCMS/MS: m/z 206.02 [M+H]⁺

¹H NMR (500 MHz, DMSO-*d*₆) δ 9.90 (s, 1H. N-H), 6.77-6.69 (m, 3Hₐᵣₒₘ), 3.86 -4.83 (t, J=5 Hz. 2H. O-CH2), 2.84-2.81 (m, 2H. CH₂-), 2.41 -2.38 (t, 2H. J =5 Hz, 2H. CH₂-CH₂-C=O), 1.72 -1.65 (m, 2H. -CH₂-) 0.97-0.974(t, J = 5 Hz. 3H.). 13C NMR (126 MHz, DMSO) δ (170.24, NH-C=O), 154.31, 132.06, 125.29, 116.22, 114.44, 113.38, 69.63, 30.82, 25.55, 22.57, 10.88.

### Synthesis of 2-oxo-1,2,3,4-tetrahydroquinolin-6-yl 5-bromo-2-methylbenzenesulfonate (TR-G069)

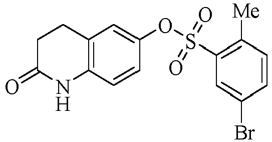

Following procedure H, 200 mg of 6-hydroxy-3,4-dihydroquinolin-2(1H)-one (1.23 mmol) was stirred with 254 mg of the base K₂CO₃ (1.84 mmol) for 30 min in THF and drops of DMF, then 0.17 mL of 5-bromo-2-methylbenzenesulfonyl chloride (1.84 mmol) was added, after workup and column chromatography (Hexane:EtOAc) (60:40) then afford 260 mg (72%) of TR-G069 as white powder. LCMS/MS: m/z 367.44 [M+H]⁺

¹H NMR (500 MHz, DMSO-*d*₆) δ 10.18 (s, 1H. N-H), 7.92-7.55 (m, 3Harom), 6.96-6.76 (m, 3H), 2.82 (t, *J* = 7.6 Hz, 2H. O=C-CH₂-CH₂-), 2.64 (s, 3H. -CH₃), 2.42 (d, *J* = 7.9 Hz, 2H. O=C-CH₂-). ¹³C NMR (126 MHz, DMSO) δ 169.97 (O=C), 142.96 (-S-O-C), 137.68 (S-C), 137.57, 135.21, 134.95, 131·71, 125.37, 121.40, 120.32, 118.90, 115.79 (Br-C), 29.76, 24.51, 19.41 (CH₃).

### Synthesis of 6-((3-(4-chlorophenyl)-4,5-dihydroisoxazol-5-yl)methoxy)-3,4-dihydroquinolin-2(1H)-one (TR-G070)

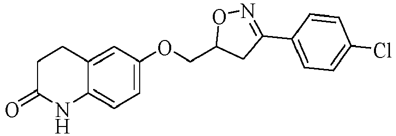

Compound TR-G070 has been prepared according to procedure A and F. Firstly, TR-G014 was prepared according to procedure A as previously mentioned. After that, a solution of 700 mg of 4-chlorobenzaldehyde (4.98 mmol), 692 mg of hydroxylamine hydrochloride (9.96 mmol) and 836 mg of NaHCO₃ in ethanol were refluxed for 3h to prepare the corresponding oxime. The reaction was monitored by TLC until the completion of reaction after that the product was filtered, washed and dried. In cyclization step, 250 mg of TR-G014 (1.23 mmol) and 231 mg of 4-chlorobenzaldehyde oxime (1.48 mmol) were dissolved in chloroform then catalytic amount of calcium hypochlorite was added slowly to the mixture in ice bath 0 °C for 8 h, then water was added, and the product was extracted with chloroform (3× 30 mL). Organic layer was dried over anhydrous magnesium sulfate and the solvent was evaporated under vacuum. The crude was purified using combiflash chromatography Hexane:EtOAc) (60:40) to afford 220 mg (50%) of the final product. LCMS/MS: m/z 357.04 [M+H]⁺.

¹H NMR (500 MHz, DMSO-*d*₆) δ 9.93 (s, 1H. N-H), 7.71-6.74 (m, 7Hₐᵣₒₘ), 5.01-5.02 (qui,Hoxazol), 4,08 -4.02 (m, 2H .CH₂), 3,59 -3.29 (m, 2H, CH_{2Oxazol}), 2.81 (t, *J* = 7.5 Hz, 2H. CH₂CH₂-C=O) 2.40-2.37 (m, 2H. CH₂C=O).

¹³C NMR (126 MHz, DMSO) δ (170.21 NH-C=O), 156.25, 153.88, 135.10, 132.58, 129.37, 128.81, 128.67, 125.34, 116.21, 114.70 (2C), 113.64 (2C), 79.66, 69.73, 36.91, 30.78, 25.51.

### Synthesis of Allyl 2-((2-oxo-1,2,3,4-tetrahydroquinolin-6-yl)oxy)acetate (TR-G071)

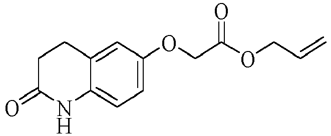

Following procedure A, 250 mg of 6-hydroxy-3,4-dihydroquinolin-2(1H)-one (1.5 mmol) was stirred with 317.6 mg of the base K₂CO₃ (2.3 mmol) for 3 hours in THF and drops of DMF, then 214 µL (247.39 mg, d=1.16 g/mL) of allyl chloroacetate (1.84 mmol) was added slowly to the reaction at room temperature. After that, the product was precipitated in ice water, filtered, and dried. Then purified using flash chromatography (Hexane:EtOAc:TEA) (40:60:1%) to obtain 316 mg (79%) as crystals. LCMS/MS: m/z 262.02 [M+H]+

¹H NMR (500 MHz, DMSO-*d*₆) δ 9.94 (s, 1H. N-H), 6.82 - 6.71 (m, 3Harom), 5.92 (m, 1H), 5.34-5.22 (m, 2H. C=CH₂), 4.76 (s, 2H. O-CH₂-C=O), 4.65 (d, *J* = 5.4 Hz, 2H. O-CH₂-CH=), 2.82 (t, *J* = 7.5 Hz, 2H. O=C-CH₂-), 2.43 - 2.36 (m, 2H. O=C-CH₂-CH₂-). ¹³C NMR (126 MHz, DMSO) δ 169.78 (O=C-N), 168.61 (O=C-O-), 152.74 (C=C-O), 132.40, 132.28, 124.87, 118.09, 115.71, 114.21, 113.06, 64.92(O-C-C=O), 64.85 (O=C-O-C-C), 30.29, 25.05.

### Synthesis of 2-oxo-1,2,3,4-tetrahydroquinolin-6-yl methyl(phenyl)carbamate (TR-G072)

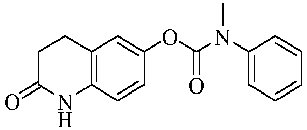

Following procedure B, 250 mg of 6-hydroxy-3,4-dihydroquinolin-2(1H)-one (1.5 mmol) was stirred with 317.6 mg of the base K₂CO₃ (2.3 mmol) for 30 min in in THF and drops of DMF, then 260 mg of N-methyl-N-phenylcarbamoyl chloride (1.5 mmol) was added to the reaction at room temperature and stirred for 12h. After that, THF was evaporated, the residue was dissolved in water and the product was extracted in ethyl acetate (3 × 50 ml), dried over anhydrous magnesium sulfate MgSO4 and the solvent was evaporated under vacuum to obtain 320 mg (70%) of TR-G072 as white powder. LCMS/MS: m/z 297.03 [M+H]+

¹H NMR (500 MHz, DMSO-*d*₆) δ 10.10(s, 1H. N-H), 7.45-6.81 (m, 8Hₐᵣₒₘ), 3.18 (s, 3H), 2.86 -2.83 (t, J = 5Hz, 2H. CH₂-CH₂-C=O), 2.44-2.41 (t, J = 5 Hz, 2H. CH₂-C=O). ¹³C NMR (126 MHz, DMSO) δ (170.51, NH-C=O), (153.88, C=O), 146.06, 143.30, 136.10, 129.41, 126.36, 124.97, 121.67 (2C), 120.74 (2C), 115.74, 49.06, 40.08, 30.53, 25.12.

### Synthesis of 2-oxo-1,2,3,4-tetrahydroquinolin-6-yl 4-(trifluoromethyl)benzenesulfonate (TR-G073)

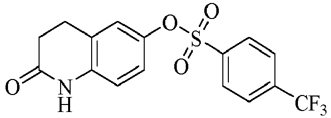

Following procedure H, 200 mg of 6-hydroxy-3,4-dihydroquinolin-2(1H)-one (1.23 mmol) was stirred with 254 mg of the base K₂CO₃ (1.84 mmol) for 1h. in acetone, then 299.81 mg of 4-(trifluoromethyl)benzene sulfonyl chloride (1.23 mmol) was added, after workup and column chromatography (Hexane:EtOAc) (60:40) then afford 405 mg (89%) of TR-G073 as white powder. LCMS/MS: m/z 372.39 [M+H]+

¹H NMR (500 MHz, DMSO-*d*₆) δ 10.19 (s, 1H. N-H), 8.10-8.05 (m, 4Hₐᵣₒₘ), 6.99-6.74 (m, 3Hₐᵣₒₘ), 2.82 (t, J = 7.6 Hz, 2H), 2.44-2.39 (m, 2H). ¹³C NMR (126 MHz, DMSO) δ 170.00 (>C=O), 143.08 (C-O-S), 138.31, 137.73, 134.37, 134·11, 129.35, 127.01, 125.38, 124.27, 122.09, 121.59, 120.57, 115.77, 29.75, 24.50.

### Synthesis of 6-(3-bromopropoxy)-3,4-dihydroquinolin-2(1H)-one (TR-G074)

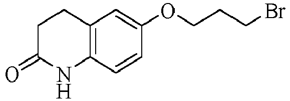

Following procedure B, 250 mg of 6-hydroxy-3,4-dihydroquinolin-2(1H)-one (1.5 mmol) was stirred with 317.6 mg of the base K₂CO₃ (2.3 mmol) for 30 min in in THF and drops of DMF, then 187 µL of 1,3-dibromopropane (1.84 mmol) was added to the reaction at room temperature and stirred for 10h. After that, THF was evaporated, the residue was dissolved in water and the product was extracted in ethyl acetate (3 × 50 ml), dried over anhydrous magnesium sulfate MgSO4 and the solvent was evaporated under vacuum to obtain 341 mg (70%) of TR-G074 as white powder. LCMS/MS: m/z 285.08 [M+H]+

### Synthesis of N-(3-chloro-4-fluorophenyl)-2-((2-oxo-1,2,3,4-tetrahydroquinolin-6-yl)oxy)acetamide (TR-G075)

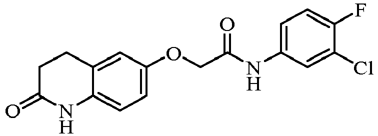

Following procedure B, the intermediate 2-chloro- N-(3-chloro-4-fluorophenyl)acetamide was prepared by dissolving 300 mg of 3-chloro-4-fluoroaniline (2.06 mmol) with 447 PLTEA (3.09 mmol) in THF, then 200 µL of chloroacetyl chloride (2.47 mmol) was added, drop wisely to the reaction mixture in ice bath (0-5 °C). After completing the reaction, the solvent was evaporated under vacuum, and the residue was washed with distilled water, filtered and dried. The second step was according to procedure A, 200 mg of 6-hydroxy-3,4-dihydroquinolin-2(1H)-one (1.23 mmol) was stirred with 254 mg of the base K₂CO₃ (1.84 mmol) for 15 min in DMF after that 272 mg of 2-chloro- N-(3-chloro-4-fluorophenyl)-acetamide (1.23 mmol) was added to the reaction mixture and stirred for 24h. After that, the product was precipitated in ice water and brine, filtered and air-dried then purified using column chromatography (Hexane:EtOAc) (60:40) to afford 385 mg (84%) of the product as white powder. LCMS/MS: m/z 347.11 [M-H]-

¹H NMR (500 MHz, DMSO-*d*₆) δ 10.21 (s, 1H. N-H), 9.93 (s, 1H. N-H), 7.93-6.75 (m, 6Hₐᵣₒₘ), 4.61(S, 2H), 2.82 (t, J = 7.5 Hz, 2H. CH₂CH₂-C=O), 2.40-2.37 (t, 2H. J = 7.5Hz .CH₂-C=O). ¹³C NMR (126 MHz, DMSO) δ (170.23, NH-C=O), (167.57, C=O), 154.77, 153.31, 136.08, 132.95, 125.34, 121.69, 120.55, 119.46, 117.47, 116.19, 114.95, 113.75, 68.02, 30.75, 25.54.

### Synthesis of (E)-N'-((2-aminopyrimidin-5-yl)methylene)-2-((2-oxo-1,2,3,4-tetrahydroquinolin-6-yl)oxy)acetohydrazide (TR-G076)

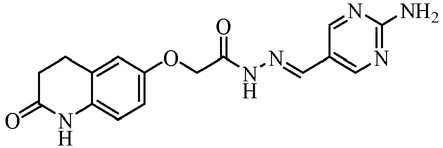

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. After that following procedure D, 200 mg of TR-G019 (0.850 mmol) was refluxed with 105 mg of 2-pyridinecarboxaldehyde (0.850 mmol) were refluxed in ethanol and drops of acetic acid for 6 h. After completing the reaction, the precipitate was filtered out of the hot ethanol, washed with hot ethanol and dried to obtain 231 mg (79%) of the final product as white powder. LCMS/MS: m/z 339.21 [M-H]-

1H-NMR (500 MHz, DMSO-d) δ 11.42 (*11,40, s, 1H. C-NH-N=), 9.95 (*9.90, s, 1H. C-NH-C), 8.55 (s, 1H. -NH₂), 8.51 (s, 1H. -NH₂), 8.16 (*7.82, s, 1H. N=CH-), 7.17-6.71 (m, 5Hₐᵣₒₘ), 5.02 (s, 1H. O-CH₂-C=O), 4.55 (s, 1H. O-CH₂-C=O), 2.84-2.81 (m, 2H. O=C-CH₂-CH₂-), 2.42-2.37 (m, 2H. O=C-CH₂-CH₂-). (*refer to the rotameric peak). ¹³C-NMR (126 MHz, DMSO) δ 169.79, 163.70, 156.98, 153.46, 144.36, 139.97, 132.44, 131.94, 124.87, 116.91, 115.67, 114.11, 113.08, 65.05, 30.31, 25.10.

### Synthesis of N-(3,4-dimethoxyphenyl)-2-((2-oxo-1,2,3,4-tetrahydroquinolin-6-yl)oxy)acetamide (TR-G077)

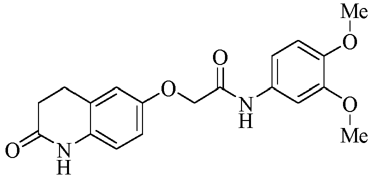

Following procedure B, the intermediate 2-chloro- N-(3,4-dimethoxyphenyl)acetamide was prepared by dissolving 300 mg of 3,4-dimethoxyaniline (1.96 mmol) with 424 µLTEA (2.94 mmol) in THF, then 190 µL of chloroacetyl chloride (2.35 mmol) was added, drop wisely to the reaction mixture in ice bath (0-5 °C). After completing the reaction, the solvent was evaporated under vacuum, and the residue was washed with distilled water, filtered, and dried. The second step was according to procedure A, 20₀ mg of 6-hydroxy-3,4-dihydroquinolin-2(1H)-one (1.23 mmol) was stirred with 254 mg of the base K2CO3 (1.84 mmol) for 15 min in DMF after that 282 mg of 2-chloro- N-(3,4-dimethoxyphenyl)acetamide (1.23 mmol) was added to the reaction mixture and stirred for 24h. After that, the product was precipitated in ice water and brine, filtered and air-dried then purified using column chromatography (Hexane:EtOAc) (60:40) to afford 359 mg (82%) of the product as beige powder. LCMS/MS: m/z 355.17 [M-H]-

¹H NMR (500 MHz, DMSO-*d*₆) δ 9.98 (s, 1H. N-H), 9.85 (s, 1H. N-H), 7.34-6.77 (m, 6Hₐᵣₒₘ), 4.58 (s, 2H), 3.72 (s, 6H. -CH₃), 2.86 -2.83 (t, 2H. J = 5 Hz. CH₂-CH₂-C=O J), 2.42-2.39 (t, 2H. J = 5 Hz. CH₂-C=O). ¹³C NMR (126 MHz, DMSO) δ (170.23 NH-C=O), (166.69 C=O), 153.42, 148.93, 145.57, 132.85, 132.36, 125.31, 116.17, 114-93, 113-72, 112.37, 112.14, 105-34, 68.10, (56.15 -CH3), (55.84 -CH3), 30.77, 25.56.

### Synthesis of 6-((1-(1H-indazol-4-yl)-1H-1,2,3-triazol-4-yl)methoxy)-3,4-dihydroquinolin-2(1H)-one (TR-G078)

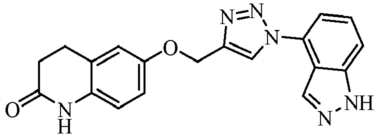

Compound TR-G078 has been prepared according to procedure A and E. In the first step, 4-azido-1H-indazole was prepared starting from 700 mg of 1H-indazol-4-amine (5.2 mmol) which was dissolved in 5 mL of HCl (2.5 mL of HCl 37% + 2.5 mL H₂O) at 0 °C and 535 mg of NaNO₃ (7.77 mmol) dissolved in 12.5 mL of H₂O was added dropwise to the reaction and stirred for 4 h at 0 °C. The mixture was extracted with EtOAc (3× 50mL) and dried over anhydrous magnesium sulfate.

Secondly, 6-(prop-2-yn-1-yloxy)-3,4-dihydroquinolin-2(1H)-one (TR-G006) was prepared according to procedure A as mentioned before. In the last step, a mixture of 6-(prop-2-yn-1-yloxy)-3,4-dihydroquinolin-2(1H)-one (TR-G006) (250 mg, 1.24 mmol), 29.74 mg of copper sulfate (0.186 mmol) and 12 mg of sodium ascorbate (0.062 mmol) in DMF/water (3:1) was added to 4-azido-1H-indazole and stirred for 24 h. After that, the mixture was extracted with DCM (3×50 ml), the organic layer was dried over anhydrous MgSO₄ and the solvent was evaporated under vacuum. Finally, the product was purified using flash chromatography (Hexane:EtOAc:TEA) (40:60:1%) to obtain 320 mg (65%) of compound TR-G078. LCMS/MS: m/z 361.21 [M+H]⁺.

¹H NMR (500 MHz, DMSO-*d*₆) δ 13.41 (s, 1H. N-H), 9.96 (s, 1H. N-H), 8.91 (s, 1H_{Traizole}), 8.28-6.80 (m, 7Hₐᵣₒₘ), 5.18 (S, 2H), 2.87-2.84 (t, 2H. J = 5 Hz CH₂CH₂-C=O) 2.42 -2.43 (t, 2H. J = 7.5 Hz. CH₂-C=O).

¹³C NMR (126 MHz, DMSO) δ (170.24 C=O), (153.64 C_{traizole}), 144.30, 139.72, 134.95, 132.62, 130.68, 125.38, 123.64, 123.09, 120.20, 116.24, 114.86, 113-79, 112.90, 111.96, 61.84, 30.80, 25.57.

### Synthesis of ethyl 3-(4-(((2-oxo-1,2,3,4-tetrahydroquinolin-6-yl)oxy)methyl)-1H-1,2,3-triazol-1-yl)propanoate (TR-G079)

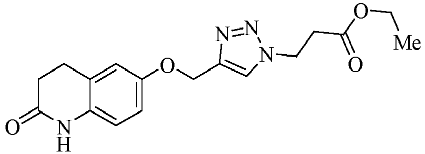

Compound TR-G079 has been prepared according to procedure A and E. In the first step, ethyl 3-azidopropanoate was prepared starting from ethyl 3-chloropropanoate (1 eq.) which were refluxed with sodium azide salt (2 eq.) for 12 hours in ethanol/ water (2:1). After that, the mixture was cooled to room temperature and filtered. Secondly, 6-(prop-2-yn-1-yloxy)-3,4-dihydroquinolin-2(1H)-one TR-G006 was prepared according to procedure A as mentioned before. In the last step, a mixture of 6-(prop-2-yn-1-yloxy)-3,4-dihydroquinolin-2(1H)-one TR-G006 (250 mg, 1.24 mmol), 29.74 mg of copper sulfate (0.186 mmol) and 12 mg of sodium ascorbate (0.062 mmol) in EtOH was added to the ethyl 3-azidopropanoate solution and stirred for 6 h. After that, the ethanol was evaporated. The residue was dissolved in water and the mixture was extracted with DCM (3 × 50 ml), dried over anhydrous magnesium sulfate MgSO₄ and the solvent was evaporated under vacuum. Finally, the product was purified using flash chromatography (Hexane:EtOAc:TEA) (40:60:1%) to obtain 350 mg (81%) of compound TR-G079 as crystals. LCMS/MS: m/z 345.12 [M+H]⁺.

¹H NMR (500 MHz, DMSO-*d*₆) δ 9.91 (s, ¹H .N-H), 8.19 (s, 1H_{Traizole}), 6.88-6.77 (m, 3Hₐᵣₒₘ), 5.06 (S, 2H), 4.62- 4.59 (t, 2H. CH₂ J = 7.5 Hz.), 4.07- 4.03 (q, 2H. CH₃**CH₂**), 2.99- 2.597 (t, 2H. **CH₂** J = 7.5 Hz.), 2.85-2.82 (t, 2H. J = 5 Hz C**H₂**CH₂-C=O) 2.42 -2.39 (t, 2H. J = 5 Hz. C**H₂**-C=O) 1.16-1.13(t, 3H J = 5 Hz. C**H₃**).

**¹³C NMR** (126 MHz, DMSO) δ (170.74 NH-C=O), (170.24 C=O), (153.65 C_{Traizole}), 143.20, 132.51, 125.32, 125.10, 116.20, 114.79, 113.69, 61.81, 60.79, 45.72, 34.40, 30.79, 25.56, 14.42.

### Synthesis of 6-((1-(2-(benzyloxy)ethyl)-1H-1,2,3-triazol-4-yl)methoxy)-3,4-dihydroquinolin-2(1H)-one (TR-G080)

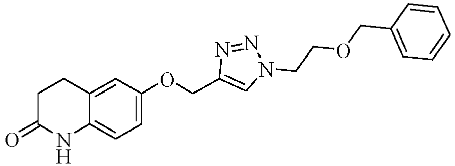

Compound TR-G080 has been prepared according to procedure A and E. In the first step, ((2-azidoethoxy)methyl)benzene was prepared starting from 700 µL of ((2-chloroethoxy)methyl)benzene (4.64 mmol) which were refluxed with 602 mg of sodium azide salt (9.27 mmol) for 2 hours in ethanol/ water (2:1). After that, the mixture was cooled to room temperature and filtered. Secondly, 6-(prop-2-yn-1-yloxy)-3,4-dihydroquinolin-2(1H)-one TR-G006 was prepared according to procedure A as mentioned before. In the last step, a mixture of TR-G006 (250 mg, 1.24 mmol), 29.74 mg of copper sulfate (0.186 mmol) and 12 mg of sodium ascorbate (0.062 mmol) in EtOH was added to the ((2-azidoethoxy)methyl)benzene solution and stirred for 12 h. After that, the ethanol was evaporated. The residue was dissolved in water and the mixture was extracted with ethyl acetate (3 × 50 ml), dried over anhydrous magnesium sulfate MgSO₄ and the solvent was evaporated under vacuum. Finally, the product was purified using flash chromatography (Hexane:EtOAc:TEA) (40:60:1%) to obtain 261 mg (52%) of compound TR-G080 as beige powder. LCMS/MS: m/z 379.32 [M+H]+.

¹H NMR (500 MHz, DMSO-*d*₆) δ 9.98 (s, ¹H .N-H), 8.18 (s, 1H_{Traizole}), 7.31-6.78 (m, 8Hₐᵣₒₘ), 5.09 (S, 2H), 4.60- 4.58 (t, 2H. CH₂ J = 7.5 Hz), 4.47 (S, 2H), 3.84- 3.82 (t, 2H. CH₂ J = 7.5 Hz.), 2.85-2.81 (t, 2H. J = 5 Hz CH₂CH₂-C=O) 2.41 - 2.36 (m, 2H. CH₂-C=O).

¹³C NMR (126 MHz, DMSO) δ (170.24 NH-C=O), 153.68, 152.82, 143.22, 138.37, 132.50, 130.72, 128.71, 127.89, 125.30, 116.21, (114.80 2C), (113.71 2C), 72.20, 68.45, 61.84, 49.93, 30.79, 25.56.

### Synthesis of 2-oxo-1,2,3,4-tetrahydroquinolin-6-yl 3-nitrobenzoate (TR-G081)

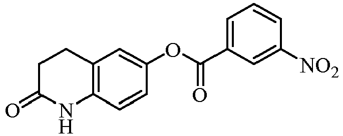

Following procedure B, 250 mg of 6-hydroxy-3,4-dihydroquinolin-2(1H)-one (1.5 mmol) was stirred with 317.6 mg of the base K2CO3 (2.3 mmol) for 30 min in in THF and drops of DMF, then 284 mg of 3-nitrobenzoyl chloride was added to the reaction at room temperature and stirred for 9h. After that, THF was evaporated, the residue was dissolved in water and the product was extracted in DCM (3 × 50 ml), dried over anhydrous magnesium sulfate MgSO₄ and the solvent was evaporated under vacuum to obtain 365 mg (76%) of TR-G081 as white powder. LCMS/MS: m/z 313.00 [M+H]⁺.

¹H NMR (500 MHz, DMSO-*d*₆) δ 10.19 (s, 1H. N-H), 8.76-6.91 (m, 7Hₐᵣₒₘ), 2.92 -2.89 (t, J = 5 Hz, 2H. CH₂-CH₂-C=O), 2.49-2.46 (t, J = 5 Hz, 2H. CH₂-C=O). ¹³C NMR (126 MHz, DMSO) δ (170.52 NH-C=O), (163.67 C=O), 148.45, 145.25, 136.95, 136.22, 131.40, 131.13, 128.80, 125.31, 124.57, 121.53, 120.70, 116.02, 30.50, 25.18.

### Synthesis of 6-((1-propyl-1H-1,2,3-triazol-4-yl)methoxy)-3,4-dihydroquinolin-2(1H)-one (TR-G082)

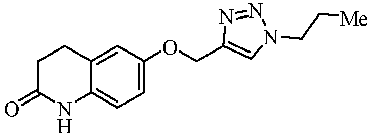

Compound TR-G082 has been prepared according to procedure A and E. In the first step, 1-azidopropane was prepared starting from 200 µL of 1-brompropane (3.17 mmol) which were refluxed with 412 mg of sodium azide salt (6.34 mmol) for 2 hours in ethanol/ water (2:1). After that, the mixture was cooled to room temperature and filtered. Secondly, 6-(prop-2-yn-1-yloxy)-3,4-dihydroquinolin-2(1H)-one TR-G006 was prepared according to procedure A as mentioned before. In the last step, a mixture of -(prop-2-yn-1-yloxy)-3,4-dihydroquinolin-2(1H)-one TR-G006 (250 mg, 1.24 mmol), 29.74 mg of copper sulfate (0.186 mmol) and 12 mg of sodium ascorbate (0.062 mmol) in EtOH was added to the azidopropane solution and stirred for 6 h. After that, the ethanol was evaporated. the residue was dissolved in water and the mixture was extracted with ethyl acetate (3 × 50 ml), dried over anhydrous magnesium sulfate MgSO₄ and the solvent was evaporated under vacuum. Finally, the product was purified using flash chromatography (Hexane:EtOAc:TEA) (40:60:1%) to obtain 210 mg (59%) of compound TR-G082 as light yellow powder. LCMS/MS: m/z 287.10 [M+H]⁺

¹H NMR (500 MHz, DMSO-*d*₆) δ 9.94 (s, 1H. N-H), 8.20 (s, 1H_{Traizole}), 6.77-6.88 (m, 3Hₐᵣₒₘ), 5.07 (S, 2H), 4.31-4.34 (t, J = 5 Hz, 2H. N-CH₂), 2.85-2.82 (t, 2H. J = 5 Hz CH₂CH₂-C=O) 2.42 -2.39 (t, 2H. J = 5 Hz. CH₂-C=O) 1.92-1.81 (m, 2H.), 0.85-0.82 (t, 3H J = 5 Hz. CH₃). ¹³C NMR (126 MHz, DMSO) δ (170.22 NH-C=O), (153.67, C_{traizole}), 143.22, 132.51, 125.31, 124.74, 116.19, 114.84, 113-74, 61.93, 51.39, 30.79, 25.56, 23.63, 11.25.

### Synthesis of 6-((3-(4-chlorophenyl)isoxazol-5-yl)methxy)-3,4-dihydroquinolin-2(1H)-one (TR-G084)

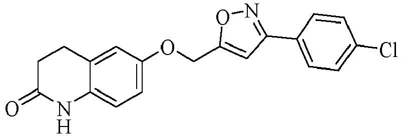

Compound TR-G084 has been prepared according to procedure A and F. Firstly, TR-G006 was prepared according to procedure A as previously mentioned. After that, a solution of 700 mg of 4-chlorobenzaldehyde (4.98 mmol), 692 mg of hydroxylamine hydrochloride (9.96 mmol) and 836 mg of NaHCO₃ in ethanol were refluxed for 3h to prepare the corresponding oxime. The reaction was monitored by TLC until the reaction complete after that the product was filtered, washed, and dried. In cyclization step, 250 mg of TR-G006 (1.24 mmol) and 231 mg of 4-chlorobenzaldehyde oxime (1.49 mmol) were dissolved in chloroform then catalytic amount of calcium hypochlorite was added to the mixture in ice bath 0 °C for 8 h, then water was added, and the product was extracted with chloroform (3× 30 mL). Organic layer was dried over anhydrous magnesium sulfate and the solvent was evaporated under vacuum. The crude was purified using flash chromatography (Hexane:EtOAc) (60:40) to afford 231 mg (52%) of the final product. LCMS/MS: m/z 355.81 [M+H]⁺

¹H NMR (500 MHz, DMSO-*d*₆) δ 9.90 (s, 1H. N-H), 7.89-7.09 (m, 7Hₐᵣₒₘ), 6.99 (s, 1H_{Oxazole}), 5.33 (S, 2H), 2.92 -2.89 (t, J = 5Hz, 2H. CH₂-CH₂-C=O), 2.48-2.45 (t, J = 5 Hz. 2H. CH₂-C=O) ¹³C NMR (126 MHz, DMSO) δ (170.49 NH-C=O), 168.81, 162.41, 153-14, 130.84, 129.84, 129.63, 128.71, 128.50, 127.12, 119.82, 114.58 (2C), 114.06 (2C), (102.87_{oxazole}), 61.44, 30.79, 26.07.

### Synthesis of 2-oxo-1,2,3,4-tetrahydroquinolin-6-yl 3,4-dimethoxybenzenesulfonate (TR-G085)

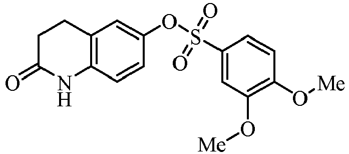

Following procedure H, 200 mg of 6-hydroxy-3,4-dihydroquinolin-2(1H)-one (1.23 mmol) was stirred with 254 mg of the base K2CO3 (1.84 mmol) for 1h. in acetone, then 291 mg of 3,4-dimethoxybenzenesulfonyl chloride (1.23 mmol) was added. After workup, column chromatography (Hexane:EtOAc) (60:40) was done to afford 380 mg (85%) of TR-G085 as beige powder. LCMS/MS: m/z 364.41 [M+H]⁺

¹H NMR (500 MHz, DMSO-*d*₆) δ 10.17 (s, 1H. N-H), 7.38 - 6.71 (m, 6Hₐᵣₒₘ), 3.86 (s, 3H. O-CH₃), 3.81 (s, 3H. O-CH₃), 2.82 (t, *J* = 7.6 Hz, 2H. O=C-CH₂-), 2.43 - 2.39 (m, 2H. O=C-CH₂-CH₂-). ¹³C NMR (126 MHz, DMSO) δ 170.02 (>C=O), 153.81, 148.96, 143.51, 137.35, 125.37, 125.09, 122.76, 121.68, 120.73, 115.63, 111.31, 110.12, 56.04, 55.98, 29.85, 24.57.

### Synthesis of 6-((1-(4-fluorobenzyl)-1H-1,2,3-triazol-4-yl)methoxy)-3,4-dihydroquinolin-2(1H)-one (TR-G086)

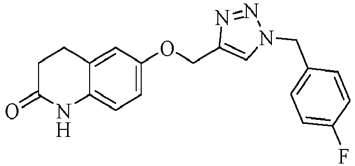

Compound TR-G086 has been prepared according to procedure A and E. In the first step, 1-(azidomethyl)-4-fluorobenzene was prepared starting from 700 µL of 4-fluorbenzyl chloride (6.49 mmol) which were refluxed with 843 mg of sodium azide salt (12.98 mmol) for 2 hours in ethanol/ water (2:1). After that, the mixture was cooled to room temperature and filtered. Secondly, 6-(prop-2-yn-1-yloxy)-3,4-dihydroquinolin-2(1H)-one TR-G006 was prepared according to procedure A as mentioned before. In the last step, a mixture of 6-(prop-2-yn-1-yloxy)-3,4-dihydroquinolin-2(1H)-one TR-G006 (250 mg, 1.24 mmol), 29.74 mg of copper sulfate (0.186 mmol) and 12 mg of sodium ascorbate (0.062 mmol) in EtOH was added to the 1-(azidomethyl)-4-fluorobenzene solution and stirred for 6 h. After that, the ethanol was evaporated. The residue was dissolved in water and the mixture was extracted with ethyl acetate (3 × 50 ml), dried over anhydrous magnesium sulfate MgSO₄ and the solvent was evaporated under vacuum. Finally, the product was purified using flash chromatography (Hexane:EtOAc:TEA) (40:60:1%) to obtain 230 mg (68%) of compound TR-G086 as yellow powder. LCMS/MS: m/z 353.19 [M+H]+.

¹H NMR (500 MHz, DMSO-*d*₆) δ 9.92 (s, 1H. N-H), 8.25 (s, 1H_{Traizole}), 7.4 - 6.75 (m, 7Hₐᵣₒₘ), 5.60 (S, 2H), 5.06 (S, 2H), 2.83-2.80 (t, 2H. J = 7.5 Hz CH₂CH₂-C=O) 2.41 -2-38(m, 2H. CH₂-C=O).

¹³C NMR (126 MHz, DMSO) δ (206.97 C-F), (170.20 NH-C=O), 153.61, 143.67, 132.54, 130.79, 130.72, 124.94, 116.18, 115.98, (114.84 2C), (113.77 2C), 61.85, 52.47, 31.17, 30.79, 25.55.

### Synthesis of 6-(2-oxo-2-(4-(4-(trifluoromethyl)phenyl)piperazin-1-yl)ethoxy)-3,4-dihydroquinolin-2(1H)-one (TR-G087)

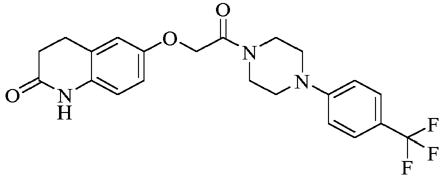

Following procedure B, the intermediate 2-chloro-1-(4-(4-trifluoromethyl)phenyl)piprazin-1-yl)ethan-1-one was prepared by dissolving 350 mg of 1-(4-(trifluoromethyl)phenyl)piprazine (1.52 mmol) with 329 µL TEA (2.28 mmol) in THF, then 147 µL of chloroacetyl chloride (1.82 mmol) was added, drop wisely to the reaction mixture in ice bath (0-5 °C). After the reaction complete, the solvent was evaporated under vacuum, and the residue was washed with distilled water, filtered and dried. The second step was according to procedure A, 200 mg of 6-hydroxy-3,4-dihydroquinolin-2(1H)-one (1.23 mmol) was stirred with 254 mg of the base K2CO3 (1.84 mmol) for 15 min in DMF after that 272 mg of 2-chloro-1-(4-(4-trifluoromethyl)phenyl)piprazin-1-yl)ethan-1-one (1.23 mmol) was added to the reaction mixture and stirred for 24h. After that, the product was precipitated in ice water and brine, filtered and air-dried then purified using column chromatography (Hexane:EtOAc) (60:40) to afford 410 mg (77%) of the product as white powder. LCMS/MS: m/z 434.2 [M+H]⁺

¹H NMR (500 MHz, DMSO-*d*₆) δ 90.93 (s, 1H. N-H), 7.54-6.75 (m, 7Hₐᵣₒₘ), 4.79 (S, 2H.), 3.62 (m, 4H_{piprazin}), 3.37-3.30 (m, 4H_{piprazin}), 2.84-2.81 (t, J = 5Hz, 2H. CH₂-CH₂-C=O), 2.41-2.38 (t, J = 5Hz, 2H. CH₂-C=O). ¹³C NMR (126 MHz, DMSO) δ (170.20, NH-C=O), (166.60, C=O), 153.62, 153.38, 132.60, 126.67, 125.22, 116.11,114.87 (2C), 114.69 (2C), 113.62, 66.84, 47.54, 47.22, 44.15 (2C), 41.23 (2C), 30.79, 25.56.

### Synthesis of 2-((2-oxo-1,23,4-tetrahydroquinolin-6-yl)oxy)-N-(quinolin-6-yl)acetamide (TR-G089)

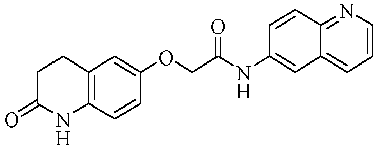

Following procedure B, the intermediate 2-chloro-N-6-quinolinylacetamide was prepared by dissolving 350 mg of 6-aminoquinoline (2.42 mmol) with 520 µLTEA (3.64 mmol) in THF, then 134 µL of chloroacetyl chloride (2.90 mmol) was added, drop wisely to the reaction mixture in ice bath (0-5 °C). After the reaction complete, the solvent was evaporated under vacuum, and the residue was washed with distilled water, filtered, and dried. The second step was according to procedure A, 250 mg of 6-hydroxy-3,4-dihydroquinolin-2(1H)-one (1.5 mmol) was stirred with 317.6 mg of the base K2CO3 (2.3 mmol) for 15 min in DMF after that, 330 mg of the intermediate 2-chloro-N-6-quinolinylacetamide (1.5 mmol) was added, . After TLC control, the product was added, onto iced water and precipitated in brine water then filtered and dried. The product was purified using flash chromatography (Hexan:EtOAc) (50:50) to obtain 400 mg (76%) as brownish powder. LCMS/MS: m/z 348.1 [M+H]⁺

¹H NMR (500 MHz, DMSO-*d*₆) δ 10.39 (s, 1H. N-H), 9.96 (s, 1H. N-H), 6.81-8.81 (m, 9Hₐᵣₒₘ), 4.71 (S, 2H), 2.87-2.84 (t, J = 5 Hz, 2H. CH₂-CH₂-C=O), 2.43-2.40 (t, J = 5 Hz, 2H. CH₂-C=O). ¹³C NMR (126 MHz, DMSO) δ (170.24, NH-C=O), (167.69, -O-CH2-C=O), 153.44, 149.71, 145.30, 136.76, 136.02, 132.89, 129.96, 128.67, 125.35, 124.12 (2C), 122.28, 116.20, 114.94, 113.74, 68.11, 30.77, 25.56.

### Synthesis of N-(3,5-dimethoxyphenyl)-2-((2-oxo-1,2,3,4-tetrahydroquinolin-6-yl)oxy)acetamide (TR-G091)

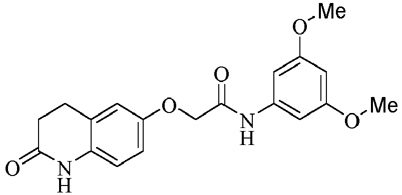

Following procedure B, the intermediate 2-chloro- N-(3,5-dimethoxyphenyl)acetamide was prepared by dissolving 300 mg of 3,5-dimethoxyaniline (1.96 mmol) with 424 µL TEA (2.94 mmol) in THF, then 190 µL of chloroacetyl chloride (2.35 mmol) was added drop wisely to the reaction mixture in ice bath (0-5 °C). After the reaction was completed, the solvent was evaporated under vacuum, and the residue was washed with distilled water, filtered, and dried. The second step was performed according to procedure A, 200 mg of 6-hydroxy-3,4-dihydroquinolin-2(1H)-one (1.23 mmol) was stirred with 254 mg of the base K2CO3 (1.84 mmol) for 2 hours in DMF. After that, 338 mg of 2-chloro- N-(3,5-dimethoxyphenyl)acetamide (1.47 mmol) was added to the reaction mixture and stirred for 24 h. After that, the product was precipitated in ice water and brine, filtered and air-dried then purified using column chromatography (Hexane:EtOAc) (60:40) to afford 332 mg (76%) of the product as beige powder. LCMS/MS: m/z 357.63 [M+H]⁺

¹H NMR (500 MHz, DMSO-*d*₆) δ 9.95 (s, 2H. N-H), 6.92-6.24 (m, 6Hₐᵣₒₘ), 4.60 (s, 2H), 3.71 (s, 6H), 2.85 -2.82 (t, J = 5 Hz, 2H. CH₂-CH₂-C=O), 2.41(t, J = 5 Hz, 2H. CH₂-C=O) ¹³C NMR (126 MHz, DMSO) δ 170.23 (NH-C=O), 167.23 (C=O), 160.91, 153.39, 140.50, 132.86, 125.32, 116.17, 114.89, 113.68, 98.39 (2C), (96.09 2C), 68.03, 55-57, 30 (2C).76, 25.55.

### Synthesis of 6-((3-cyclohexylisoxazol-5-yl)methoxy)-3,4-dihydroquinolin-2(1H)-one (TR-G092)

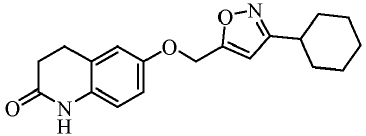

Compound TR-G092 has been prepared according to procedure A and F. Firstly, TR-G006 was prepared according to procedure A as previously mentioned. After that, a solution of 700 µL of cyclohexancarbaldehyde (5.74 mmol), 789 mg of hydroxylamine hydrochloride (11.48 mmol) and 964 mg of NaHCO₃ (11.48 mmol) in ethanol were refluxed for 3h to prepare the corresponding oxime. The reaction was monitored by TLC until the reaction complete after that the product was filtered, washed, and dried. In cyclization step, 250 mg of TR-G006 (1.23 mmol) and 185 mg of the oxime (1.48 mmol) were dissolved in chloroform then catalytic amount of calcium hypochlorite was added to the mixture in ice bath 0 °C for 8 h, then water was added, and the product was extracted with chloroform (3× 30 mL). The organic layer was dried over anhydrous magnesium sulfate and the solvent was evaporated under vacuum. The crude was purified using combi flash chromatography (Hexane:EtOAc) (60:40) to afford 241 mg (59%) of the final product. LCMS/MS: m/z 329.41 [M+H]⁺.

¹H NMR (500 MHz, DMSO-*d*₆) δ 9.40 (s, ¹H .N-H), 7.04-6.94 (m, 3Hₐᵣₒₘ), 6.55 (s,H _{oxazol}), 5,19 (s, 2H .CH₂), 2.91-2.88 (t, 2H. J = 5Hz .CH₂CH₂-C=O), 2.73-2.68 (qui, H_{cycohexan}). 2.47-2.44 (m, 2H. J = 5Hz .CH₂C=O)- ), 1.88-1.23 (m, 10H_{cyclohexan}).

¹³C NMR (126 MHz, DMSO) δ (170.48 NH-C=O), 168.34, 167.24, 153.21, 129.72, 128.44, 119.79, 114.49, 113.90, 103.23, 61.37, (35.56 2C), (31.81 2C), 30.80, 30.28, 26.06, 25.82.

### Synthesis of 6-((1-(4-acetylphenyl)-1H-1,2,3-triazol-4-yl)methoxy)-3,4-dihydroquinolin-2(1H)-one (TR-G093)

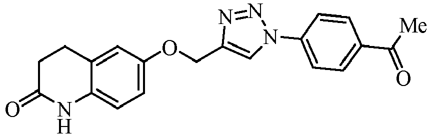

Compound TR-G093 has been prepared according to procedure A and E. In the first step, 1-(4-azidophenyl)ethane-1-one was prepared starting from 700 mg of 1-(4-aminophenyl)ethan-1-one (5.18 mmol) which wa dissolved in 5 mL of HCl (2.5 mL of HCl 37% + 2.5 mL H2O) at 0 °C and 535 mg of NaNO3 (7.77 mmol) dissolved in 12.5 mL of H2O was added, dropwise to the reaction and stirred for 4 h at 0 °C. The mixture was extracted with EtOAc (3× 50mL) and dried over anhydrous magnesium sulfate.

Secondly, 6-(prop-2-yn-1-yloxy)-3,4-dihydroquinolin-2(1H)-one TR-G006 was prepared according to procedure A as mentioned before. In the last step, a mixture of 6-(prop-2-yn-1-yloxy)-3,4-dihydroquinolin-2(1H)-one TR-G006 (250 mg, 1.24 mmol), 29.74 mg of copper sulfate (0.186 mmol) and 12 mg of sodium ascorbate (0.062 mmol) in DMF/water (3:1) was added, to the 1-(4-azidophenyl)ethane-1-one and stirred for 24 h. After that, the mixture was extracted with DCM (3 × 50 ml), the organic layer was dried over anhydrous magnesium sulfate MgSO₄ and the solvent was evaporated under vacuum. Finally, the product was purified using flash chromatography (Hexane:EtOAc:TEA) (40:60:1%) to obtain 320 mg (66%) of compound TR-G093 as crystals. LCMS/MS: m/z 363.45 [M+H]⁺

¹H NMR (500 MHz, DMSO-*d*₆) δ 9.90 (s, 1H. N-H), 9.08 (s, 1H_{Traizole}), 8.19-6.79 (m, 7Hₐᵣₒₘ), 5.19 (S, 2H), 2.87 -2.84 (t, J = 5 Hz, 2H. CH₂-CH₂-C=O), 2.65 (s, 3H), 2.50-2.40 (t, J = 5 Hz, 2H. CH₂-C=O). ¹³C NMR (126 MHz, DMSO) δ (197.42 O=C-CH₃), (170.22 NH-C=O), (153.56_{traizol}), 144.92, 139.99, 136.88, 132.69, 130.56 (2C), 125.39 (2C), 123.36, 120.28, 116.24, 114.88, 113.81, 61.78, 30.78, 27.34, 25.56.

### Synthesis of 2-((2-oxo-1,2,3,4-tetrahydroquinolin-6-yl)oxy)-N-(3,4,5-trimethoxyphenyl)acetamide (TR-G094)

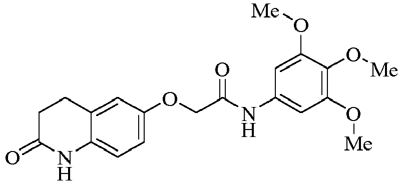

Following procedure B, the intermediate 2-chloro-N-3,4,5-trimethoxyphenylacetamide was prepared by dissolving 350 mg of 3,4,5-trimethoxyaniline (1.91 mmol) with 414 µLTEA (2.87 mmol) in THF, then 184 µL of chloroacetyl chloride (2.29 mmol) was added, drop wisely to the reaction mixture in ice bath (0-5 °C). After completing the reaction, the solvent was evaporated under vacuum, and the residue was washed with distilled water, filtered, and dried. The second step was according to procedure A, 250 mg of 6-hydroxy-3,4-dihydroquinolin-2(1H)-one (1.53 mmol) was stirred with 317.6 mg of the base K₂CO₃ (2.3 mmol) for 15 min in DMF after that 397 mg of 2-chloro-N-3,4,5-trimethoxyphenylacetamide (1.53 mmol) was added to the reaction mixture and stirred for 24h. After that, the product was precipitated in ice water and brine, filtered and air-dried. The product was purified using flash chromatography (Hexane:EtOAc) (60:40) to obtain 480 mg (81%) as white powder. LCMS/MS: m/z 387.42 [M+H]⁺

¹H NMR (500 MHz, DMSO-*d*₆) δ 10.23 (s, 1H. N-H), 9.98 (s, 1H. N-H), 7.12-6.81 (m, 5Hₐᵣₒₘ), 4.63 (S, 2H. O-CH₂-C=O), 3.73 (S, 6H), 3.62 (S, 3H), 2.85-2.82 (t, J = 5Hz, 2H.CH₂CH₂-C=O), 2.42 -2.39 (m, 2H. J = 5Hz, 2H.CH₂-C=O) ¹³C NMR (126 MHz, DMSO) δ (170.22, NH-C=O), (167.03, NH-C=O), 153.43, 153.09, 135.123, 134.03, 132.82, 125.31, 116.20 (2C), 114.88 (2C), 113.64 (2C), 97.82, 67.95, 60.56, 56.17, 30.77, 25.55.

### Synthesis of 6-(but-3-en-2-yloxy)-3,4-dihydroquinolin-2(1H)-one (TR-G095)

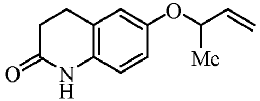

Following procedure A, 250 mg of 6-hydroxy-3,4-dihydroquinolin-2(1H)-one (1.5 mmol) was stirred with 317.6 mg of the base K₂CO₃ (2.3 mmol) for 30 min in THF and drops of DMF, then 185 µL of 3-chloro-1-butene (1.8 mmol) was added, slowly to the reaction at room temperature. After that, the product was precipitated in ice water, filtered, and dried. Then purified using flash chromatography (Hexane:EtOAc:TEA) (40:60:1%) to obtain 281 mg (84%). LCMS/MS: m/z 274.21 [M+H]⁺

### Synthesis of 2-oxo-1,2,3,4-tetrahydroquinolin-6-yl (2-chlorophenyl)carbamate (TR-G096)

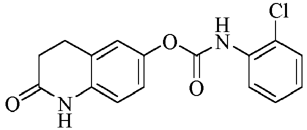

250 mg of 6-hydroxy-3,4-dihydroquinolin-2(1H)-one (1.5 mmol) was stirred with 265 µL of the TEA (1.84 mmol) for 1 h in THF and drops of DMF, then 214 µL of 2-chlorophenyl isocyanate (1.84 mmol) was added, to the reaction mixture and stirred for 2h at room temperature. After that, the product was precipitated in ice, filtered and air-dried. Finally, the product was purified using flash chromatography (Hexane:EtOAc) (40:60) to obtain 360 mg (74%) of the final product. LCMS/MS: m/z 317.44 [M+H]⁺

### Synthesis of 2-oxo-1,2,3,4-tetrahydroquinolin-6-yl (5-fluoro-2-methylphenyl)carbamate (TR-G097)

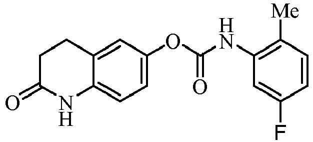

250 mg of 6-hydroxy-3,4-dihydroquinolin-2(1H)-one (1.53 mmol) was stirred with 265 µL of the TEA (1.84 mmol) for 1 h in THF and drops of DMF, then 240 µL of 2-fluoro-5-methylphenyl isocyanate (1.84 mmol) was added, to the reaction mixture and stirred for 2h at room temperature. After that, the product was precipitated in ice, filtered and air-dried. Finally, the product was purified using flash chromatography (Hexane:EtOAc:TEA) (40:60:1%) to obtain 360 mg (75%) of the final product. LCMS/MS: m/z 315.66 [M+H]⁺

¹H NMR (500 MHz, CDCL₃-*d*₆) δ 8.74 (s, H. N-H), 7.92 (s, H. N-H), 7.18-6.96 (m, 6Hₐᵣₒₘ), 2.99-2.96 (t, J = 5 Hz, 2H. CH₂-CH₂-C=O), 2.66-2.63 (m, 2H. CH₂-C=O), 2.31 (s, 3H .CH₃). ¹³C NMR (126 MHz, CDCl₃) δ (170.67 NH-C=O), (150.56 C=O), 144.75, 134.03, 133.45, 133.42, 124.25, 123.79, 123.25, 120.35, 119.57, 115.01, 113.56, 113.40, 29.29, 24.37, 20.22

### Synthesis of 6-(4-(1-cyclohexyl-1H-tetrazol-5-yl)butoxy)-1-isopentyl-3,4-dihydroquinolin-2(1H)-one (TR-G101)

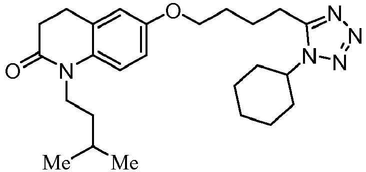

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs2CO3 (1.01 mmol) for 1 h in THF and drops of DMF, then 98 µL of 1-bromo-3-methylbutane (0.812 mmol) was added, to the reaction mixture and stirred for 12h at room temperature. After that, the solvent was evaporated and the product was extracted in DCM/water system, the organic layer was dried over anhydrous magnesium sulfate MgSO4 and the solvent was evaporated under vacuum. Finally, the product was purified using flash chromatography (Hexane:EtOAc:TEA) (40:60:1%) to obtain 198 mg (67%) of the final product. LCMS/MS: m/z 440.29 [M+H]⁺

¹H NMR (500 MHz, DMSO-de) δ 7.00-6.80 (m, 3Hₐᵣₒₘ), 4.43-3.38 (qui, 2H cyclohexan), 4.01-3.98 (t, 2H.. J = 5Hz. N-CH₂), 3.87-3.84 (t, 2H._{.} J = 5Hz. CH₂-O), 2,99-2.96 (t, 2H. J = 5Hz CH₂-N-C-CH₂), 2.78 (t, 2H. J = 5Hz. CH₂-CH₂-C=O), 2.48-2.45(t, 2H. J=5Hz. CH₂-C=O), 1.97-1.87 (m, 4H_{Cyclohexan}), 1.84-1.78(m, 4H Cyclohexan), 1.76-1.56(m, 2H_{Cyclohexan}), 1.55-1.42(m, 2H. CH₂), 1.39-1.36 (m, 2H, CH₂). 1.36-1.26 (m, 2H. CH₂), 1.24-1.06 (m, 1H. >CH-), 0.91 (S, 3H. -CH₃) 0.90 (S, 3H. -CH₃). ¹³C NMR (126 MHz, DMSO) δ (169.00, C=O), 154.58, 154.30, 132.95, 128.52, 116.23, 114.80 (2C), 113.12 (2C), 67.54, 56.51, 36.10, 32.93, 31.87, 28.46, 26.05, 25.49 (2C), 25.06, 25.03, 23.74, 22.92, 22.25 (2C).

### Synthesis of ethyl 2-(6-(4-(1-cyclohexyl-1H-tetrazol-5-yl)butoxy)-2-oxo-3,4-dihydroquinolin-1(2H)-yl)acetate (TR-G102)

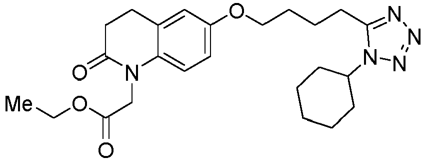

Following procedure A, 250 mg of 6-(3-(1-cyclohexyl-1H-tetrazol-5-yl)propoxy)-3,4-dihydroquinolin-2(1H)-one (0.703 mmol) was stirred with 145.81 mg of the base Cs₂CO₃ (1.06 mmol) for 2 h. in THF and drops of DMF, then 129.29 mg of ethyl 2-chloroacetate (1.06 mmol) was added, slowly to the reaction at room temperature. The reaction was tested until finished by TLC and after workup and flash chromatography (Hexan:EtOAc:MeOH) (50:50:3), the final product was filtered and obtained 279 mg (87%) of TR-G102 as milky white crystals. LCMS/MS: m/z 456.2 [M+H]⁺

¹H NMR (500 MHz, DMSO-*d*₆) δ 6.86 - 6.77 (m, 3H_{arom.}), 4.61 (s, 2H. >N-CH₂-C=O), 4.45 - 4.38 (m, 1H. >N-CH<), 4.13 (q, *J* = 7.1 Hz, 2H. -O-CH₂-CH₃), 4.00 (t, *J* = 6.1 Hz, 2H. O-CH₂-CH₂-), 2.99 (t, *J* = 7.5 Hz, 2H. -CH₂-CH₂-C=O), 2.88 - 2.82 (m, 2H. N=CH-CH₂-CH₂), 2.57 - 2.51 (m, 2H. CH₂-CH₂-C=O), 1.98-1.95 (m, 2H. - CH₂-CH₂-CH₂-O-), 1.92 - 1.73 (m, 8H. 6H_{Cyclohexane}, O-CH₂-CH₂), 1.70-1.65 (m, 1H_{Cyclohexane}), 1.48-1.38 (m, 2H_{Cyclohexane}), 1.31 - 1.23 (m, 1H_{Cyclohexane}), 1.20 (t, *J =* 7.1 Hz, 3H. -CH₃). ¹³C NMR (126 MHz, DMSO) δ 169.36 (>N-C=O), 168.69 (O-C=O), 154.18 (C_{Tetrazole}), 154.08 (O-Carom)., 132.96, 127.58, 115.77, 114.22, 112.53, 67.15, 60.76, 56.05, 44.09, 32.46 (2C_{Cyclohexane}), 30.94, 28.01, 24.86, 24.60 (2C_{Cyclohexane}), 24.57, 23.27, 21.82, 14.01 (-CH₃).

### Synthesis of 1-allyl-6-(4-(1-cyclohexyl-1H-tetrazol-5-yl)butoxy)-3,4-dihydroquinolin-2(1H)-one (TR-G103)

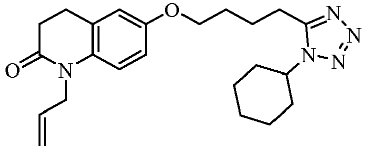

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 70 µL of allyl bromide (0.812 mmol) was added, to the reaction mixture and stirred for 8h at room temperature. After that, the solvent was evaporated and the product was extracted in DCM/water system, the organic layer was dried over anhydrous magnesium sulfate MgSO4 and the solvent was evaporated under vacuum. Finally, the product was purified using flash chromatography (Hexane:EtOAc) (40:60) to obtain 195 mg (70%) of the final product. LCMS/MS: m/z 410.68 [M+H]⁺.

### Synthesis of 6-(4-(1-cyclohexyl-1H-tetrazol-5-yl)butoxy)-1-(4-methylbenzyl)-3,4-dihydroquinolin-2(1H)-one (TR-G104)

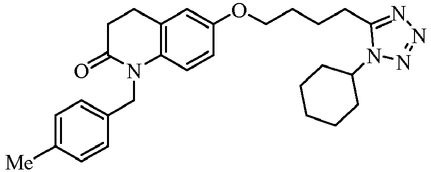

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 108 µL of 4-methylbenzyl chloride (0.812 mmol) was added, to the reaction mixture and stirred for 10h at room temperature. After that, the product was precipitated in water. Filtered and air-dried. Finally, the product was purified using flash chromatography (Hexane:EtOAc) (40:60) to obtain 210 mg (66%) of the final product as beige powder. LCMS/MS: m/z 474.87 [M+H]⁺

### Synthesis of 6-(4-(1-cyclohexyl-1H-tetrazol-5-yl)butoxy)-1-propyl-3,4-dihydroquinolin-2(1H)-one (TR-G105)

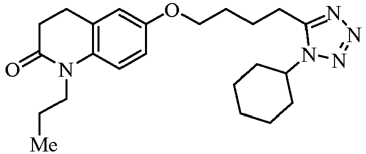

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 74 µL of 1-bromopropane (0.812 mmol) was added, to the reaction mixture and stirred for 14h at room temperature. After that, the solvent was evaporated and the product was extracted in DCM/water system, the organic layer was dried over anhydrous magnesium sulfate MgSO4 and the solvent was evaporated under vacuum. Finally, the product was purified using flash chromatography (Hexane:EtOAc:TEA) (40:60:1%) to obtain 210 mg (75%) of the final product as light yellow powder. LCMS/MS: m/z 412.82 [M+H]⁺

¹H NMR (500 MHz, DMSO-*d*₆) δ 7.03-6.79 (m, 3Hₐᵣₒₘ), 4.43-3.38 (m, 2H _{Cyclohexan}), 4.01-3.98 (t, *J* = 5Hz. 2H. N-CH₂), 3.82-3.79 (t, *J* = 5Hz, 2H. CH₂-O), 2.98 (t, *J* = 5Hz, 2H. CH₂-N-C-CH₂), 2.81-2.78 (t, 2H. *J* = 5Hz. CH₂-CH₂-C=O), 2.50-2.47 (t, J=5Hz, 2H.CH₂-C=O), 2.02-1.89 (m, 2H Cyclohexan), 1.87-1.76 (m, 4H Cyclohexan), 1.75-1.66 (m, 4H Cyclohexan), 1.41-1.24(m, 4H, CH₂CH₂), 0.86-0.83 (t, *J =* 5Hz. 3H. CH₃). ¹³C NMR (126 MHz, DMSO) δ 169.14 (C=O), 154.57, 154.31, 133.02, 128.42, 116.37, 114.75, 113.11(2C), 67.55, 56.52, 42.90, 32.93 (2C), 31.85, 28.47, 25.51, 25.06 (2C), 23.74, 22.26, 20.44, 11.46.

### Synthesis of 6-(4-(1-cyclohexyl-1H-tetrazol-5-yl)butoxy)-1-(3-phenylpropyl)-3,4-dihydroquinolin-2(1H)-one (TR-G106)

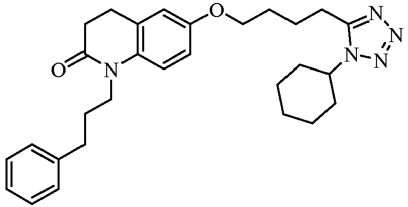

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 124 µL of (3-broopropyl)benzene (0.812 mmol) was added, to the reaction mixture and stirred for 16h at room temperature. After that, the solvent was evaporated and the product was extracted in DCM/water system, the organic layer was dried over anhydrous magnesium sulfate MgSO4 and the solvent was evaporated under vacuum. Finally, the product was purified using flash chromatography (Hexane:EtOAc:TEA) (40:60:1%) to obtain 276 mg (83%) of the final product as beige powder. LCMS/MS: m/z 488.81 [M+H]⁺.

¹H NMR (500 MHz, DMSO-*d*₆) δ 7.27-6.71 (m, 8Hₐᵣₒₘ), 4.43-3.37 (qui, 2H _{cyclohexan}), 4.01-3.98 (t, 2H. J = 5Hz._{.}N-CH₂), 3.89-3.86 (t, 2H._{.} J = 5Hz. CH₂-O), 2,99-2.96 (t, 2H. J = 5Hz CH₂-N-C-CH₂), 2.80-2.77 (t, 2H. J = 5Hz. CH₂-CH₂-C=O), 2.63-2.59 (t, 2H. J = 5Hz CH₂-Ph), 2.48-2.46(t, 2H. J=5Hz CH₂-C=O), 2.09-1.95 (m, 2H _{cyclohexan}), 1.92-1.86 (m, 4H _{cyclohexan}), 1.83-1.80 (m, 4H _{cyclohexan}), 1.76-1.65(m, 2H. CH₂), 1.47-1.41 (m, 2H. CH₂). 1.30-1.21 (m, 2H. CH₂). ¹³C NMR (126 MHz, DMSO) δ 169.17 (C=O), 154.57, 154.32, 141·91, 132.97, 128.66, 128.49, 126.26, 116.22, 114.78 (2C), 113.04 (2C), 67.54, 56.52, 41.21, 32.92, 32.83, 31.84, 28.97, 28.46, 25.46 (2C), 25.05 (2C), 23.74, 22.26.

### Synthesis of 6-(4-(1-cyclohexyl-1H-tetrazol-5-yl)butoxy)-1-(4-vinylbenzyl)-3,4-dihydroquinolin-2(1H)-one (TR-G107)

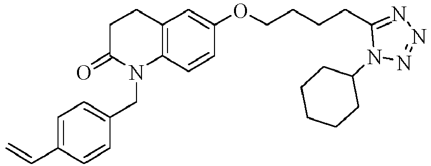

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 115 µL of 4-vinylbenzyl chloride (0.812 mmol) was added, to the reaction mixture and stirred for 15h at room temperature. After that, the product was precipitated in water. Filtered and air-dried. Finally, the product was purified using flash chromatography (Hexane:EtOAc) (40:60) to obtain 264 mg (80%) of the final product. LCMS/MS: m/z 486.77 [M+H]⁺.

### Synthesis of 1-benzyl-6-(4-(1-cyclohexyl-1H-tetrazol-5-yl)butoxy)-3,4-dihydroquinolin-2(1H)-one (TR-G110)

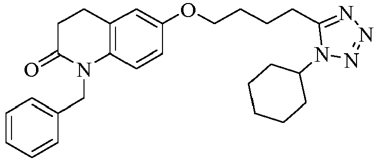

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 94 µL of benzyl chloride (0.812 mmol) was added, to the reaction mixture and stirred for 10h at room temperature. After that, the product was precipitated in water. Filtered and air-dried. Finally, the product was purified using flash chromatography (Hexane:EtOAc) (40:60) to obtain 240 mg (77%) of the final product as brown powder. LCMS/MS: m/z 460.32 [M+H]⁺.

¹H NMR (500 MHz, DMSO-*d*₆) δ 7.24-6.53 (m, 8Hₐᵣₒₘ), 5.08(S, 2H), 4.07-4.01 (qui, H _{cyclohexan}), 3.89-3.86 (t, 2H. J = 6Hz. CH₂-O), 2.88-2.85 (t, 2H. J = 7.6 - CH₂), 2.84-2.81 (t, 2H. J = 7.6Hz .CH₂-CH₂-C=O), 2.71-2.68 (m, 2H.CH₂-C=O), 1.95-1.78(m, 10H cyclohexan), 1.33-1.18 (m, 4H, CH_{2. aliphatic}).

¹³C NMR (126 MHz, Chloroform) δ (169.07 C=O), 153.43, 152.46, 136.01, 132.43, 127.67, 126.89, 126.00, 125.34, 115.49, 113.40, 111.42, 66.33, 56.56, 45.16, 31.86 (2C), 30.82 (2C), 27.47, 24.81, 24.28, 23.76 (2C), 22.96 (2C), 21.97.

### Synthesis of 1-(4-chlorobenzyl)-6-(4-(1-cyclohexyl-1H-tetrazol-5-yl)butoxy)-3,4-dihydroquinolin-2(1H)-one (TR-G111)

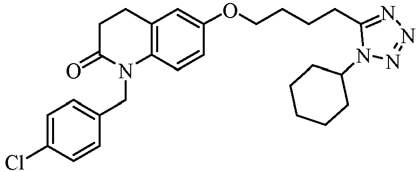

Following procedure A, 400 mg of 6-(3-(1-cyclohexyl-1H-tetrazol-5-yl)propoxy)-3,4-dihydroquinolin-2(1H)-one (1.08 mmol) was stirred with 529 mg of Cs₂CO₃ (1.62 mmol) for 2h in THF and drops of DMF, then 174 mg of 4-chlorobenzyl chloride (1.08 mmol) was added, to the reaction mixture and stirred for 24h. After completing the reaction, the solvent was evaporated and the crude product was washed with ice water, filtered, and dried. The final product was purified using flash chromatography (Hexane:EtOAc:) (50:50) to obtain 420 mg (78%) as white powder. LCMS/MS: m/z 495.31 [M+H]⁺.

### Synthesis of 6-(4-(1-cyclohexyl-1H-tetrazol-5-yl)butoxy)-1-(4-fluorobenzyl)-3,4-dihydroquinolin-2(1H)-one (TR-G112)

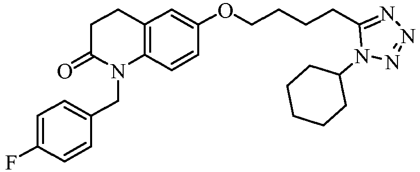

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 94 µL of 4-flourobenzyl chloride (0.812 mmol) was added, to the reaction mixture and stirred for 12h at room temperature. After that, the product was precipitated in water. Filtered and air-dried. Finally, the product was purified using flash chromatography (Hexane:EtOAc) (40:60) to obtain 258 mg (79%) of the final product as beige powder. LCMS/MS: m/z 478.66 [M+H]⁺.

¹H NMR (500 MHz, DMSO-*d*₆) δ 7.27-6.68 (m, 7Hₐᵣₒₘ),5.10 (S, 2H), 4.41-3.36 (qui, 2H _{cyclohexan}), 3.97-3.94 (t, 2H. J = 5Hz. CH₂-O), 2.95 (t, J = 5Hz, 2H. CH₂), 2,90 (t, J = 5Hz, 2H. CH₂-CH₂-C=O), 2.64 (t, 2H. J = 5Hz. CH₂-C=O), 2.09-1.85 (m, 4H _{cyclohexan}), 1.83-1.79(m, 4H _{Cyclohexan}), 1.77-1.67(m, 2H _{Cyclohexan}), 1.65-1.39 (m, 2H, CH₂), 1.28-1.22 (m, 2H. CH₂). ¹³C NMR (126 MHz, DMSO) δ 169.71 (C=O), 162.52, 160.59, 154-50, 134.03, 132.95, 128.84, 128.37, 116.69, 115.81, 115.64 (2C), 114.77 (2C), 112.90, 67.52, 56.50, 44.24, 32.91, 31.67, 28.42, 25.39, 25.04 (2C), 23.71 (2C), 22.23.

### Synthesis of Allyl 6-(4-(1-cyclohexyl-1H-tetrazol-5-yl)butoxy)-2-oxo-3,4-dihydroquinoline-1(2H)-carboxylate (TR-G114)

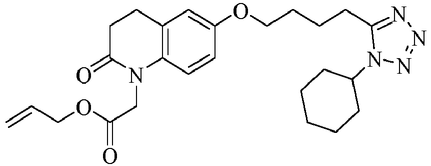

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 84 µL of allyl chloroacetate (0.812 mmol) was added to the reaction mixture and stirred for 13h at room temperature. After that, the product was precipitated in water. Filtered and air-dried. Finally, the product was purified using flash chromatography (Hexane:EtOAc) (40:60) to obtain 238 mg (77%) of the final product. LCMS/MS: m/z 454.72[M+H]⁺.

### Synthesis of 4-(6-(4-(1-cyclohexyl-1H-tetrazol-5-yl)butoxy)-2-oxo-3,4-dihydroquinolin-1(2H)-yl)butyl benzoate (TR-G115)

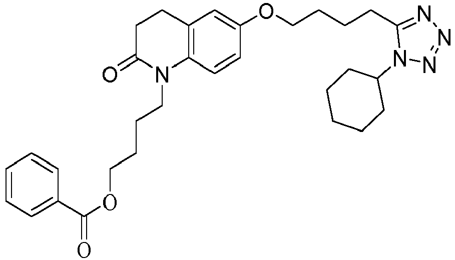

Following procedure A, 250 mg of 6-(3-(1-cyclohexyl-1H-tetrazol-5-yl)propoxy)-3,4-dihydroquinolin-2(1H)-one (0.676 mmol) was stirred with 330 mg of the base Cs₂CO₃ (1.01 mmol) for 1 h in THF and drops of DMF, then 152 µL of 4-chlorobutyl benzoate (0.812 mmol) was added to the reaction mixture and stirred for 18h at room temperature. After that, the product was precipitated in water. Filtered and air-dried. Finally, the product was purified using flash chromatography (Hexane:EtOAc) (40:60) to obtain 245 mg (66%) of the final product. LCMS/MS: m/z 446.90 [M+H]⁺.

### Synthesis of 6-((3-(2-chloro-6-fluorophenyl)isoxazol-5-yl)methoxy)-3,4-dihydroquinolin-2(1H)-one (TR-G116)

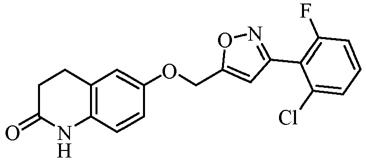

Compound TR-G116 has been prepared according to procedure A and F. Firstly, TR-G006 was prepared according to procedure A as previously mentioned. After that, a solution of 700 mg of 2-chloro-6-fluorobenzaldehyde (4.14 mmol), 614 mg of hydroxylamine hydrochloride (8.83 mmol) and 742 mg of NaHCO₃ in ethanol were refluxed for 3h to prepare the corresponding oxime. The reaction was monitored by TLC until compliting reaction. Then the product was filtered, washed and dried. In cyclization step, 250 mg of TR-G006 (1.24 mmol) and 259 mg of 2-chloro-6-fluorobenzaldehyde oxime (1.49 mmol) were dissolved in chloroform then catalytic amount of calcium hypochlorite was added to the mixture in ice bath 0 °C for 8 h, then water was added, and the product was extracted with chloroform (3× 30 mL). The organic layer was dried over anhydrous magnesium sulfate and the solvent was evaporated under vacuum. The crude was purified using combiflash chromatography (Hexane:EtOAc) (60:40) to afford 256 mg (55%) of the final product. LCMS/MS: m/z 373.90 [M+H]+.

¹H NMR (500 MHz, DMSO-*d*₆) δ 9.44 (s, 1H. N-H), 7.93-6.97 (m, 6Hₐᵣₒₘ), 7.09 (s, 1H_{Oxazole}), 5.33 (S, 2H), 2.92 -2.89 (t, 2H. J = 7.5Hz. CH₂-CH₂-C=O), 2.48-2.45 (m, 2H. CH₂-C=O).

¹³C NMR (126 MHz, DMSO) δ (170.49, NH-C=O), 169.11, 161.51, 153.11, 135.53, 129.86, 129.73, 128.93, 128.50, 127.58, 119.82, 114.58 (2C), 114.06 (2C), (102.87_{oxazole}), 61.42, 30-79, 26.06.

### Synthesis of 2-(3-(4-(((2-oxo-1,2,3,4-tetrahydroquinolin-6-yl)oxy)methyl)-1H-1,2,3-triazol-1-yl)propyl)isoindoline-1,3-dione (TR-G117⁾

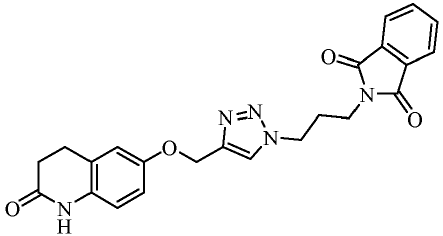

Compound TR-G117 has been prepared according to procedure A and E. In the first step, 2-(3-azidopropyl)isoindoline-1,3-dione was prepared starting from 700 mg of 2-(3-chloropropyl)isoindoline-1,3-dione (4.10 mmol) which were refluxed with 534 mg of sodium azide salt (8.20 mmol) for 2 hours in ethanol/ water (2:1). After that, the mixture was cooled to room temperature and filtered. Secondly, 6-(prop-2-yn-1-yloxy)-3,4-dihydroquinolin-2(1H)-one TR-G006 was prepared according to procedure A as mentioned before. In the last step, a mixture of 6-(prop-2-yn-1-yloxy)-3,4-dihydroquinolin-2(1H)-one TR-G006 (250 mg, 1.24 mmol), 29.74 mg of copper sulfate (0.186 mmol) and 12 mg of sodium ascorbate (0.062 mmol) in EtOH was added to the 2-(3-azidopropyl)isoindoline-1,3-dione solution and stirred for 18 h. After that, the ethanol was evaporated. The residue was dissolved in water and the mixture was extracted with ethyl acetate (3 × 50 ml), dried over anhydrous magnesium sulfate MgSO4 and the solvent was evaporated under vacuum. Finally, the product was purified using flash chromatography (Hexane:EtOAc:TEA) (40:60:1%) to obtain 259 mg (45%) of compound TR-G117. LCMS/MS: m/z 432.10 [M+H]⁺.

¹H NMR (500 MHz, DMSO-*d*₆) δ 9.90 (s, 1H. N-H), 8.20 (s, 1H_{Traizole}), 6.86-6.76 (m, 7Hₐᵣₒₘ), 5.04 (S, 2H), 4.45- 4.42 (t, 2H. CH₂ J = 7.5 Hz.), 3.64- 3.61 (t, 2H. CH₂ J = 7.5 Hz.), 2.85-2.82 (t, 2H. J = 5 Hz CH₂CH₂-C=O) 2.41 -2.38 (t, 2H. J = 5 Hz. CH₂-C=O) 2.21-2.16(m, 2H. CH₂).¹³C NMR (126 MHz, DMSO) δ (170.21 NH-C=O), (168.42 2C=O), 153.66, 143.17, 134.76, 132.52, 132.21, 125.31, (124.94, 2C), (123.45 2C), (116.20 2C), 114.83, 113.74, 61.92, 47.72, 35.37, 30.79, 29.16, 25.56.

### Synthesis of N-(4-morpholinophenyl)-2-((2-oxo-1,2,3,4-tetrahydroquinolin-6-yl)oxy)acetamide (TR-G120)

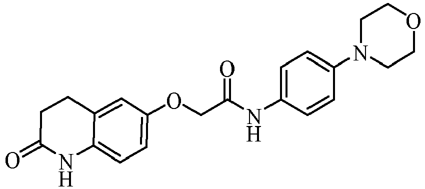

Following procedure B, the intermediate 2-chloro-N-(-(4-morpholinophenyl)acetamide was prepared by dissolving 300 mg of 4-morpholinoaniline (1.68 mmol) with 365 µLTEA (2.52 mmol) in THF, then 163 µL of chloroacetyl chloride (2.02 mmol) was added drop wisely to the reaction mixture in ice bath (0-5 °C). After completing the reaction, the solvent was evaporated under vacuum, and the residue was washed with distilled water, filtered and dried. The second step was according to procedure A, 200 mg of 6-hydroxy-3,4-dihydroquinolin-2(1H)-one (1.23 mmol) was stirred with 254 mg of the base K₂CO₃ (1.84 mmol) for 15 min in DMF after that 312 mg of 2-chloro- N-(-(4-morpholinophenyl)acetamide (1.23 mmol) was added to the reaction mixture and stirred for 24h. After that, the product was precipitated in ice water and brine, filtered and air-dried then purified using column chromatography (Hexane:EtOAc) (60:40) to afford 400 mg (85%) of the product as brown powder. LCMS/MS: m/z 382.11 [M+H]⁺.

¹H NMR (500 MHz, DMSO-*d*₆) δ 90.95(s, 1H. N-H), 90.82 (s, 1H. N-H), 7.51-6.77(m, 7Hₐᵣₒₘ), 4.58 (S, 2H.), 3.73-3.71 (t, J = 5Hz .4Hₘₒᵣₚₕₗᵢₙₑ), 3.05-3.03 (t, J = 5Hz. 4H_{Morphline}), 2.85-2.82 (t, J = 7.6 Hz, 2H. CH₂-CH₂-C=O), 2.42-2.39 (t, J = 7.6 Hz, 2H. CH₂-C=O). ¹³C NMR (126 MHz, DMSO) δ (170.24 NH-C=O), (166.51 -C=O), 153-45, 148.00, 132.82, 130-99, 125-30, 121-36, 116.17 (2C), 115.77 (2C), 114.91, 113.72, 68.11, 66.56 (2C), 49.30 (2C), 30.77, 25.56.

### Synthesis of N-(2-chloropyridin-4-yl)-2-((2-oxo-1,2,3,4-tetrahydroquinolin-6-6-yl)oxy)Acetamide (TR-G₁₂₁)

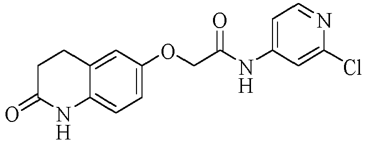

Following procedure B, the intermediate 2-chloro- N-(2-chloropyridin-4-yl)acetamide was prepared by dissolving 300 mg of 2-chloropyridin-4-amine (2.33 mmol) with 506 µLTEA (2.5 mmol) in THF, then 266 µL of chloroacetyl chloride (2.80 mmol) was added drop wisely to the reaction mixture in ice bath (0-5 °C). After completing the reaction, the solvent was evaporated under vacuum, and the residue was washed with distilled water, filtered and dried. The second step was according to procedure A, 200 mg of 6-hydroxy-3,4-dihydroquinolin-2(1H)-one (1.23 mmol) was stirred with 254 mg of the base K₂CO₃ (1.84 mmol) for 15 min in DMF after that 251 mg of 2-chloro- N-(2-chloropyridin-4-yl)acetamide (1.23 mmol) was added to the reaction mixture and stirred for 24h. After that, the product was precipitated in ice water and brine, filtered and air-dried then purified using column chromatography (Hexane:EtOAc) (60:40) to afford 325 mg (80%) of the product as beige powder. LCMS/MS: m/z 373.90 [M+H]+.

### Synthesis of N-(5-nitropyridin-2-yl)-2-((2-oxo-1,2,3,4-tetrahydroquinolin-6-yl)oxy)acetamide (TR-G122)

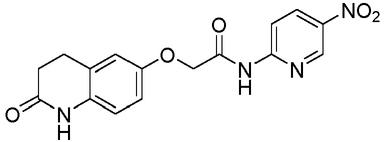

Following procedure B, the intermediate 2-chloro-N-(5-nitropyridin-2-yl)acetamide was prepared by dissolving 320 mg of 5-nitropyridin-2-amine (2.30 mmol) with 481 µL TEA (3.45 mmol, d=0.726 g/mL) in THF, then 220 µL of chloroacetyl chloride (2.76 mmol, d=1.42 g/mL) was added drop wisely to the reaction mixture in ice bath (0-5 °C). After the reaction complete, the solvent was evaporated under vacuum, and the residue was washed with distilled water, filtered, and dried. The second step was according to procedure A, 250 mg of 6-hydroxy-3,4-dihydroquinolin-2(1H)-one (1.35 mmol) was stirred with 280 mg of the base K₂CO₃ (2.02 mmol) for 3 hours in DMF. After that, 348 mg of 2-chloro-N-(5-nitropyridin-2-yl)acetamide (1.62 mmol) was added to the reaction mixture and stirred for 24 h. After that, the product was precipitated in ice water and brine, filtered and air-dried then purified using column chromatography (Hexane:EtOAc) (50:50) to afford 390 mg (78%) of the product as white powder. LCMS/MS: m/z 343.22 [M+H]+.

¹H NMR (500 MHz, DMSO-*d*₆) δ 9.47 (s, 1H), 9.16-8.39 (m, 3H_{pyridine}), 7.39-6.78 (m, 3Hₐᵣₒₘ), 4.77 (s, 2H), 2.93-2.87 (m, 2H), 2.65-2.50 (m, 2H). ¹³C NMR (125 MHz, DMSO) δ 170.23, 170.01, 155.29, 151.59, 146.09, 138.29, 134.41, 134.36, 124.67, 119.38, 115.51, 115.29, 112.87, 66.90, 31.01, 27.81.

### Synthesis of 2-((2-oxo-1,2,3,4-tetrahydroquinolin-6-yl)oxy)-N-4-bromophenyl)acetamide (TR-G123)

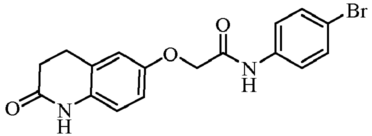

Following procedure B, the intermediate 2-chloro-N-(4-bromophenyl)acetamide was prepared by dissolving 300 mg of 4-bromoaniline (1.74 mmol) with 378 µLTEA (2.62 mmol) in THF, then 169 µL of chloroacetyl chloride (2.09 mmol) was added drop wisely to the reaction mixture in ice bath (0-5 °C). After completing the reaction, the solvent was evaporated under vacuum, and the residue was washed with distilled water, filtered, and dried. The second step was according to procedure A, 200 mg of 6-hydroxy-3,4-dihydroquinolin-2(1H)-one (1.23 mmol) was stirred with 254 mg of the base K₂CO₃ (1.84 mmol) for 15 min in DMF after that 305 mg of 2-chloro-N-(4-bromophenyl)acetamide (1.23 mmol) was added to the reaction mixture and stirred for 24h. After that, the product was precipitated in ice water and brine, filtered and air-dried then purified using column chromatography (Hexane:EtOAc) (60:40) to afford 377 mg (81%) of the product. LCMS/MS: m/z 375.01 [M+H]⁺.

### Synthesis of 6-((1-hexyl-1H-1,2,3-triazol-4-yl)methoxy)-3,4-dihydroquinolin-2(1H)-one (TR-G125)

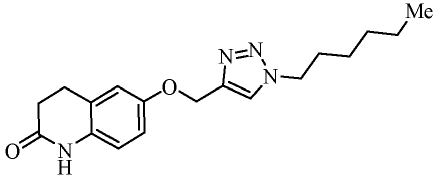

Compound TR-G125 has been prepared according to procedure A and E. In the first step, 1-azidohexane was prepared starting from 500 µL of 1-bromhexane (3.57 mmol) which were refluxed with 464 mg of sodium azide salt (7.15 mmol) for 2 hours in ethanol/ water (2:1). After that, the mixture was cooled to room temperature and filtered. Secondly, 200 mg of 6-(prop-2-yn-1-yloxy)-3,4-dihydroquinolin-2(1H)-one TR-G006 was prepared according to procedure A as mentioned before. In the last step, a mixture of -(prop-2-yn-1-yloxy)-3,4-dihydroquinolin-2(1H)-one TR-G006 (200 mg, 1 mmol), 23.79 mg of copper sulfate (0.149 mmol) and 8 mg of sodium ascorbate (0.049 mmol) in EtOH was added to the azidohexane solution and stirred for 24 h. After that, the ethanol was evaporated. The residue was dissolved in water and the mixture was extracted with ethyl acetate (3 × 50 ml), dried over anhydrous magnesium sulfate MgSO₄ and the solvent was evaporated under vacuum. Finally, the product was purified using flash chromatography (Hexane:EtOAc:TEA) (40:60:1%) to obtain 310 mg (68%) of compound TR-G125 as brown powder. LCMS/MS: m/z 329.41 [M+H]⁺.

¹H NMR (500 MHz, DMSO-d₆) δ 9.93 (s, 1H. N-H), 8.19 (s, 1H_{Traizole}), 6.88-6.76 (m, 3Hₐᵣₒₘ), 5.06 (S, 2H), 4.36-4.33 (t, 2H. J = 5Hz. N-CH2), 2.85-2.82 (t, 2H J = 5Hz.CH₂- CH₂-C=O) 2.42 -2.39 (t, 2H. J = 5Hz. CH₂-C=O), 1.83-1.77 (q, 2H), 1.25-1.19 (m, 6H.), 0.85-0.82 (t, 3H. J = 5 Hz. CH₃). ¹³C NMR (126 MHz, DMSO) δ (170.22, NH-C=O), (153.64, C_{traizole}), 143.21, 132.50, 125.30, 124.72, 116.19, 114.84, 113-74, 61.90, 49-81, 31.03, 30.79, 30-11, 25.94, 25.56, 22.39, 14-30.

### Synthesis of 2-(2-((2-oxo-1,2,3,4-tetrahydroquinolin-6-yl)oxy)ethoxy)acetamide (TR-G126)

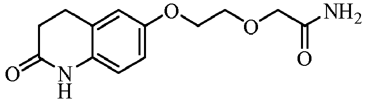

Following procedure A, 250 mg of 6-hydroxy-3,4-dihydroquinolin-2(1H)-one (1.5 mmol) was stirred with 317.6 mg of the base K₂CO₃ (2.3 mmol) for 30 min in THF and drops of DMF, then 214 µL of 2-(2-chloroethoxy)acetamide (1.84 mmol) was added slowly to the reaction mixture at room temperature and stirred for 18 h. After completing the reaction, the product was precipitated in water, filtered and air-dried. After that, the purification was carried out using flash chromatography (Hexane:EtOAc) (40:60) to obtain 318 mg as beige powder. LCMS/MS: m/z 265.27 [M+H]+.

¹H NMR (500 MHz, DMSO-d₆) δ 9.97 (s, 1H. N-H), 7.30 (s, 1H. N-H), 7.18 (s, 1H. N-H) 6.80-6.73 (m, 3Hₐᵣₒₘ), 4.08-4.087 (t, 2H J =5 Hz. O-CH₂), 3.88 (S, 2H-CH₂-C=O) 3.77 -3.75 (t, 2H. J = 5Hz. CH₂-O), 2.84-2.81 (t, 2H. J =5 Hz CH₂CH₂-C=O), 2.41 -2.38 (t, 2H. J = 5Hz. CH₂-C=O). ¹³C NMR (126 MHz, DMSO) δ (172.03, NH-C=O), (170.23, C=O-NH₂), 153.98, 132.35, 125.33, 116.23, 114.52, 113.48, 70.34, 69.91, 67.65, 30.80, 25.55.

### Synthesis of (E)-N'-(4-fluorobenzylidene)-2-((2-oxo-1,2,3,4-tetrahydroquinolin-6-yl)oxy)acetohydrazide (TR-G128)

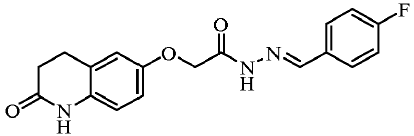

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. After that following procedure D, 200 mg of TR-G019 (0.850 mmol) was refluxed with 100 µL of 4-flourobenzaldehyde (0.935 mmol) were refluxed in ethanol and drops of acetic acid for 6 h. After completing the reaction, the precipitate was filtered out of the hot ethanol, washed with hot ethanol and dried to obtain 250 mg (86%) of the final product as white powder. LCMS/MS: m/z 342.22 [M+H]⁺.

¹H NMR (500 MHz, DMSO-d6) δ 11.58 (*11.54, s, 1H. C-NH-N=), 9.97 (*9.93, s, 1H. C-NH-C), 8.35 (*8.01, s, 1H. N=CH-) 7.79-6.73 (m, 7Hₐᵣₒₘ), 5.08 (s, 1H. O-CH₂-C=O), 4.61 (s, 1H. O-CH₂-C=O), 2.87-2.82 (m, 2H. O=C-CH₂-CH₂-), 2.43-2.39 (m, 2H. O=C-CH₂-CH₂-). (* refer to the rotameric peak). ¹³C NMR (126 MHz, DMSO) δ 169.82, 164.47, 153.45, 146.84, 142.61, 132.49, 132.02, 129.37, 129.10, 124.79, 115-95, 115-78, 114.41, 114.15, 113.08, 65.07, 30.38, 25.12.

### Synthesis of 2-oxo-1,2,3,4-tetrahydroquinolin-6-yl diisopropylcarbamate (TR-G129)

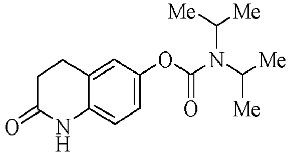

Following procedure B, 250 mg of 6-hydroxy-3,4-dihydroquinolin-2(1H)-one (1.5 mmol) was stirred with 317.6 mg of the base K₂CO₃ (2.3 mmol) for 30 min in in THF and drops of DMF, then 250 mg of diisopropylcarbamic chloride(1.5 mmol) was added to the reaction and stirred for 16h at room temperature. After that, THF was evaporated, the residue was dissolved in water and the product was extracted in DCM (3 × 50 ml), dried over anhydrous magnesium sulfate MgSO₄ and the solvent was evaporated under vacuum to obtain 320 mg (71%) of TR-G129. LCMS/MS: m/z 291.41 [M+H]⁺.

### Synthesis of 2-oxo-1,2,3,4-tetrahydroquinolin-6-yl phenylcarbamate (TR-G131)

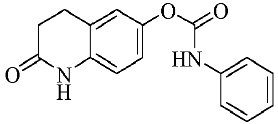

250 mg of 6-hydroxy-3,4-dihydroquinolin-2(1H)-one (1.5 mmol) was stirred with 265 µL of the TEA (1.84 mmol) for 1 h in THF and drops of DMF, then 201 µL of phenyl isocyanate (1.84 mmol) was added to the reaction mixture and stirred for 2h at room temperature. After that, the product was precipitated in ice, filtered, and air-dried. Finally, the product was purified using flash chromatography (Hexane:EtOAc) (40:60) to obtain 350 mg (81%) of the final product. LCMS/MS: m/z 283.11 [M+H]+.

¹H NMR (500 MHz, DMSO-d₆) δ 10.18 (s, 1H. N-H), 10.13 (s, 1H. N-H), 7.52-6.87(m, 8Hₐᵣₒₘ), 2.90 -2.87 (t, J = 5 Hz, 2H .CH₂-CH₂-C=O) 2.47-2.4₄ (t, J =5 Hz, 2H. CH₂-C=O) ¹³C NMR (126 MHz, DMSO) δ (170.22 NH-C=O), (152.49 NH-C=O), 145.46, 139.13, 136.24, 129.33, 125.10, 123.36, 121.83, 120.94, 118.82, 115.90, 114.31, 113.88, 30.56, 25.18.

### Synthesis of 2-oxo-1,2,3,4-tetrahydroquinolin-6-yl (2-methoxyphenyl)carbamate (TR-G132)

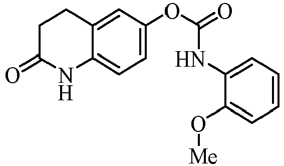

250 mg of 6-hydroxy-3,4-dihydroquinolin-2(1H)-one (1.5 mmol) was stirred with 265 µL of the TEA (1.84 mmol) for 1 h in THF and drops of DMF, then 244 µL of 2-methoxyphenyl isocyanate (1.84 mmol) was added to the reaction mixture and stirred for 2h at room temperature. After that, the product was precipitated in ice, filtered and air-dried. Finally, the product was purified using flash chromatography (Hexane:EtOAc) (40:60) to obtain 371 mg (77%) of the final product. LCMS/MS: m/z 313.33 [M+H]⁺.

¹H NMR (500 MHz, DMSO-d₆) δ 10.11 (s, 1H. N-H), 9.90 (s, 1H. N-H), 7.12-6.57(m, 8Hₐᵣₒₘ), 2.90 -2.86 (t, J=7.2Hz, 2H. -CH₂-CH₂-C=O), 2.46-2.47(m, 2H. - CH₂-C=O), 2.09 (S, 3H). ¹³C NMR (126 MHz, DMSO) δ (170.50 NH-C=O), (170.10 NH-C=O), 152.81, 145.68, 136.13, 127.00, 125.02, 121.74, 120.77, 116.27, 115.84, 115.00, 113.88, 111.88, 56.12, 49.06, 25.17.

### Synthesis of 2-oxo-1,2,3,4-tetrahydroquinolin-6-yl (4-nitrophenyl)carbamate (TR-G133)

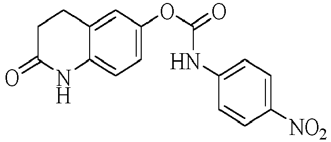

250 mg of 6-hydroxy-3,4-dihydroquinolin-2(1H)-one (1.5 mmol) was stirred with 265 µL of the TEA (1.84 mmol) for 1h in THF and drops of DMF, then 2011µL of 4-nitrophenyl isocyanate (1.84 mmol) was added to the reaction mixture and stirred for 2h at room temperature. After that, the product was precipitated in ice, filtered, and air-dried. Finally, the product was purified using flash chromatography (Hexane:EtOAc) (40:60) to obtain 402 mg (80%) of the final product. LCMS/MS: m/z 328.44 [M+H]⁺.

### Synthesis of 2-oxo-1,2,3,4-tetrahydroquinolin-6-yl-o-tolylcarbamate (TR-G134)

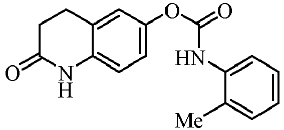

250 mg of 6-hydroxy-3,4-dihydroquinolin-2(1H)-one (1.5 mmol) was stirred with 265 µL of the TEA (1.84 mmol) for 1 h in THF and drops of DMF, then 228 µL of o-tolyl isocyanate (1.84 mmol) was added to the reaction mixture and stirred for 2h at room temperature. After that, the product was precipitated in ice, filtered and air-dried. Finally, the product was purified using flash chromatography (Hexane:EtOAc) (40:60) to obtain 375 mg (82%) of the final product. LCMS/MS: m/z 297.44 [M+H]⁺.

### Synthesis of 2-oxo-1,2,3,4-tetrahydroquinolin-6-yl naphthalene-1-ylcarbamate (TR-G135)

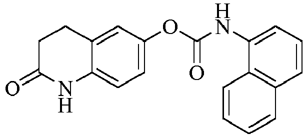

250 mg of 6-hydroxy-3,4-dihydroquinolin-2(1H)-one (1.5 mmol) was stirred with 265 µL of the TEA (1.84 mmol) for 1h in THF and drops of DMF, then 263 µL of 1-naphthyl isocyanate (1.84 mmol) was added to the reaction mixture and stirred for 2h at room temperature. After that, the product was precipitated in ice, filtered, and air-dried. Finally, the product was purified using flash chromatography (Hexane:EtOAc) (40:60) to obtain 368 mg (72%) of the final product. LCMS/MS: m/z 333.53 [M+H]⁺.

### Synthesis of 2-oxo-1,2,3,4-tetrahydroquinolin-6-yl (2-bromophenyl)carbamate (TR-G136)

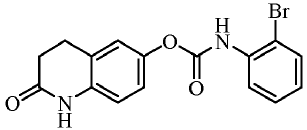

250 mg of 6-hydroxy-3,4-dihydroquinolin-2(1H)-one (1.5 mmol) was stirred with 265 µL of the TEA (1.84 mmol) for 1 h in THF and drops of DMF, then 228 µL of 2-bromophenyl isocyanate (1.84 mmol) was added to the reaction mixture and stirred for 2h at room temperature. After that, the product was precipitated in ice, filtered, and air-dried. Finally, the product was purified using flash chromatography (Hexane:EtOAc) (40:60) to obtain 415 mg (75%) of the final product. LCMS/MS: m/z 362.44 [M+H]⁺.

### Synthesis of 2-oxo-1,2,3,4-tetrahydroquinolin-6-yl (2-(trifluoromethyl)phenyl)carbamate (TR-G137)

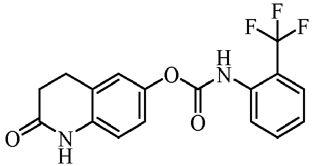

250 mg of 6-hydroxy-3,4-dihydroquinolin-2(1H)-one (1.5 mmol) was stirred with 265 µL of the TEA (1.84 mmol) for 1 h in THF and drops of DMF, then 277 µL of 2-(trifluoromethyl)phenyl isocyanate (1.84 mmol) was added to the reaction mixture and stirred for 2h at room temperature. After that, the product was precipitated in ice, filtered, and air-dried. Finally, the product was purified using flash chromatography (Hexane:EtOAc) (40:60) to obtain 391 mg (73%) of the final product. LCMS/MS: m/z 351.33 [M+H]⁺.

### Synthesis of (E)-N'-(3-(4-chlorophenoxy)benzylidene)-2-((2-oxo-1,2,3,4-tetrahydroquinolin-6-yl)oxy)acetohydrazide (TR-G138)

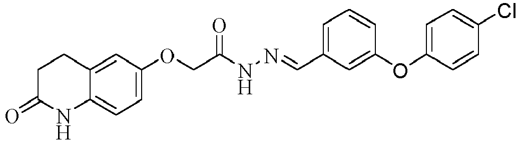

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. After that following procedure D, 200 mg of TR-G019 (0.850 mmol) was refluxed with 198 mg of 3-(4-chlorophenoxy)benzaldehyde (0.850 mmol) were refluxed in ethanol and drops of acetic acid for 6 h. After completing the reaction, the precipitate was filtered out of the hot ethanol, washed with hot ethanol and dried to obtain 320 mg (83%) of the final product as white powder. LCMS/MS: m/z 450.92 [M+H]⁺.

1H-NMR (500 MHz, DMSO-*d*) δ 11.61 (*11,56, s, 1H. C-NH-N=), 9.96 (*9.92, s, 1H. C-NH-C), 8.32 (*7.98, s, 1H. N=CH-) 7.49-6.70 (m, 11Hₐᵣₒₘ), 5.03 (s, 1H. O-CH₂-C=O), 4.59 (s, 1H. O-CH₂-C=O), 2.83-2.80 (m, 2H. O=C-CH₂-CH₂-), 2.41-2.37 (m, 2H. O=C-CH₂-CH₂-). (*refer to the rotameric peak). ¹³C-NMR (126 MHz, DMSO) δ 169.77, 164.52, 156.57, 155.44, 153.39, 152.88, 147.08, 142.87, 136.19, 132.47, 132.00, 130.71, 129.94, 127.39, 124.74, 122.72, 120.28, 116.74, 115.74, 114.13, 113.03, 65.03, 30.36, 25.10.

### Synthesis of 2-oxo-1,2,3,4-tetrahydroquinolin-6-yl 3-flurobenzoate (TR-G139)

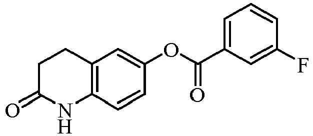

Following procedure B, 250 mg of 6-hydroxy-3,4-dihydroquinolin-2(1H)-one (1.5 mmol) was stirred with 317.6 mg of the base K₂CO₃ (2.3 mmol) for 30 min in in THF and drops of DMF, then 224 µL of 3-fluorobenzoyl chloride was added slowly to the reaction at room temperature and stirred for 7h. After that, THF was evaporated, the residue was dissolved in water and the product was extracted in DCM (3 × 50 ml), dried over anhydrous magnesium sulfate MgSO₄ and the solvent was evaporated under vacuum to obtain 332 mg of TR-G139 (76%) as white powder. LCMS/MS: m/z 286.33 [M+H]⁺.

### Synthesis of 2-oxo-1,2,3,4-tetrahydroquinolin-6-yl (4-fluorophenyl)carbamate (TR-G140)

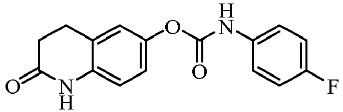

250 mg of 6-hydroxy-3,4-dihydroquinolin-2(1H)-one (1.5 mmol) was stirred with 265 µL of the TEA (1.84 mmol) for 1 h in THF and drops of DMF, then 210 µL of 4-fluorophenyl isocyanate (1.84 mmol) was added to the reaction mixture and stirred for 2h at room temperature. After that, the product was precipitated in ice, filtered and air-dried. Finally, the product was purified using flash chromatography (Hexane:EtOAc) (40:60) to obtain 378 mg (82%) of the final product. LCMS/MS: m/z 301.11 [M+H]⁺.

¹H NMR (500 MHz, Chloroform-*d*) δ 8.17 (s, 1H. -O-CO-NH-), 8.10 (s, 1H. O=C-NH), 7.21-6.77 (m, 7H), 3.01-2.96 (m, 2H. -CH₂-CH₂-C=O), 2.65 (m, 2H. -CH₂-C=O). ¹³C NMR (126 MHz, CDCl₃) δ 171.40, 151.68 (F-Cₚₕ), 145.88, 135.19, 125.04, 124.92, 124.89, 121.63 (2C), 121.59, 120.77 (2C), 116.04 (2C), 30.47, 25.58.

### Synthesis of (E)-N'-(naphthalen-1-ylmethylene)-2-((2-oxo-1,2,3,4-tetrahydroquinolin-6-yl)oxy)acetohydrazide (TR-G141)

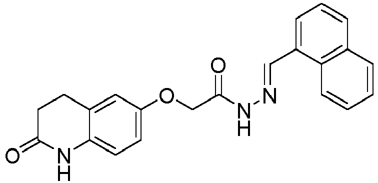

Starting from 6-hydroxy-3,4-dihydroquinolin-2(1H)-one, compound 2-((2-oxo-1,2,3,4-tetrahydroquinolin-6-yl)oxy)acetohydrazide (TR-G019) was prepared through couple of steps following procedures A and C respectively. After that following procedure D, 200 mg of TR-G019 (0.850 mmol) was refluxed with 125 µL of 1-naphthaldehyde (0.935 mmol) were refluxed in ethanol and drops of acetic acid for 6 h. After completing the reaction, the precipitate was filtered out of the hot ethanol, washed with hot ethanol, and dried to obtain 231 mg (72%) of the final product as white powder. LCMS/MS: m/z 374.28 [M+H]⁺.

¹H-NMR (500 MHz, DMSO-*d*) δ 11.63 (*11,59, s, 1H. C-NH-N=), 9.97 (*9.93, s, 1H. C-NH-C), 9.02 (*8.68, s, 1H. N=CH-) 8.81-6.75 (m, 10Hₐᵣₒₘ), 5.15 (s, 1H. O-CH₂-C=O), 4.66 (s, 1H. O-CH₂-C=O), 2.87-2.82 (m, 2H. O=C-CH₂-CH₂-), 2.43-2.38 (m, 2H. O=C-CH₂-CH₂-). (*refer to the rotameric peak). ¹³C-NMR (126 MHz, DMSO) δ 169.78, 164.46, 153.44, 147.88, 143.50, 133.50, 132.02, 130.43, 130.05, 129.22, 128.85, 127.39, 126.30, 125.56, 124.78, 123.75, 115.72, 114.49, 113.07, 65.24, 54.94, 30.31, 25.11.

### Synthesis of 6-((3-(4-fluorophenyl)isoxazol-5-yl)methoxy)-3,4-dihydroquinolin-2(1H)-one (TR-G142)

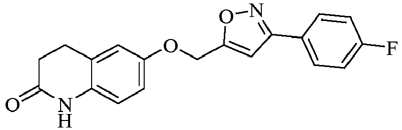

Compound TR-G142 has been prepared according to procedure A and F. Firstly, TR-G006 was prepared according to procedure A as previously mentioned. After that, a solution of 700 mg of 4-fluorobenzaldehyde (5.64 mmol), 784 mg of hydroxylamine hydrochloride (11.28 mmol) and 948 mg of NaHCO₃ in ethanol were refluxed for 3h to prepare the corresponding oxime. The reaction was monitored by TLC until the completion of reaction. After that, the product was filtered, washed, and dried. In cyclization step, 250 mg of TR-G006 (1.24 mmol) and 229 mg of 4-flourobenzaldehyde oxime (1.49 mmol) were dissolved in chloroform then 184 µL of Calcium hypochlorite (12-16 grade) (2.48 mmol) were added slowly to the mixture in ice bath 0 °C for 8 h, then water was added, and the product was extracted with chloroform (3× 30 mL). The organic layer was dried over anhydrous magnesium sulfate and the solvent was evaporated under vacuum. The crude was purified using combiflash chromatography (Hexane:EtOAc) (60:40) to afford 196 mg (46%) of the final product. LCMS/MS: m/z 339.29 [M+H]⁺.

¹H NMR (500 MHz, CDCL3-*d*) δ 7.72 (s, 1H .N-H), 7.62-6.69 (m, 7Hₐᵣₒₘ), 6.54 (s, 1H_{Oxazole}), 5.09 (S, 2H),2.92 -2.89 (t, 2H. J = 6.7Hz. CH₂-CH₂-C=O), 2.58-2.55 (m, 2H. CH₂-C=O).

¹³C NMR (126 MHz, CDCl₃) δ (168.96 NH-C=O), 166.96, 160.63, 152.20, 127.79, 127.73, 125.57, 123.76, 118.98, 115.20 (2C), 115.02(2C), 112.93, 112.90, 100.41, 60.91, 29.36, 25.12.

### Synthesis of N((1,1'-biphenyl)-4-yl)-2-((2-oxo-1,2,3,4-tetrahydroquinolin-6-yl)oxy)acetamide (TR-G143)

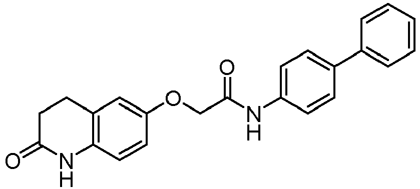

Following procedure B, the intermediate N-((1,1'-biphenyl)-4-yl)-2-chloroacetamide was prepared by dissolving 350 mg of (1,1'-biphenyl)-4-amine (2.07 mmol) with 448 µLTEA (3.10 mmol) in THF, then 200 µL of chloroacetyl chloride (2.48 mmol) was added drop wisely to the reaction mixture in ice bath (0-5 °C). After completing the reaction, the solvent was evaporated under vacuum, and the residue was washed with distilled water, filtered, and dried. The second step was according to procedure A, 200 mg of 6-hydroxy-3,4-dihydroquinolin-2(1H)-one (1.23 mmol) was stirred with 254 mg of the base K₂CO₃ (1.84 mmol) for 15 min in DMF after that 282 mg of N-((1,1'-biphenyl)-4-yl)-2-chloroacetamide (1.23 mmol) was added to the reaction mixture and stirred for 24h. After that, the product was precipitated in ice water and brine, filtered and air-dried then purified using column chromatography (Hexane:EtOAc) (60:40) to afford 397 mg (83%) of the product. LCMS/MS: m/z 373.92 [M+H]⁺.

### Synthesis of 6-(pent-4-en-i-yloxy)-3,4-dihydroquinolin-2(1H)-one (TR-G144)

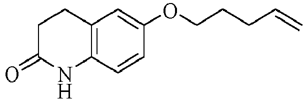

Following procedure A, a mixture of 6-hydroxy-3,4-dihydroquinolin-2(1H)-one (1.23 mmol, 200 mg) and K₂CO₃ (1.84 mmol, 254.09 mg) was stirred for 30 min in THF and drops of DMF. After that, 5-bromopent-1-ene (1.47 mmol, 219.20 mg) 0.174 ml was added to the solution at room temperature. After workup and column chromatography (Hexane:EtOAc) (50:50), 219 mg of TR-G144 (77%) was obtained as off-white crystals. LCMS/MS: m/z 232.44 [M+H]⁺.

¹H NMR (500 MHz, CDCL3-*d*) δ 8.82 (s, H .N-H), 6.74-6.69 (m, 3Hₐᵣₒₘ), 5.89-5.81 (m, H .C=H), 5.08-4.99 (m, 2H .CH₂=C), 3.94 -3.91 (t, 2H. J = 6.4 Hz. O-CH₂), 2.94 -2.91 (t, 2H. J = 6.6Hz. CH₂-CH₂-C=O), 2.62-2.59 (m, 2H. CH₂-C=O), 2.25 -2.21 (q, 2H. J = 7 Hz. CH=CH₂-CH₂), 1.89 -1.83 (m, 2H. CH₂-CH₂-CH₂).

¹³C NMR (126 MHz, CDCl₃) δ (170.62 NH-C=O), 154.01, 136.75, 129.69, 123.93, 115.19, 114.18, 113.45, 112.10, 66.56, 29.61, 29.06, 27.42, 24.67.

### Synthesis of (E)-N'-([1,1'-biphenyl}-4-ylmethylene)-2-((2-oxo-1,2,3,4-tetrahydroquinolin-6-yl)oxy)acetohydrazide (TR-G145)

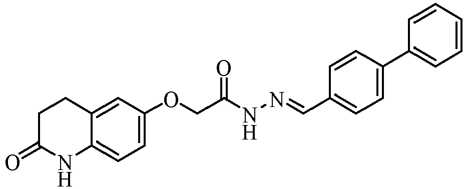

According to procedure A, C and D starting from 6-hydroxy-3,4-dihydroquinolin-2(1H)-one. Firstly, 300 mg of 6-hydroxy-3,4-dihydroquinolin-2(1H)-one (1.84 mmol) was stirred with 381 mg of the base K₂CO₃ (2.76 mmol) for 15 min in THF and drops of DMF, then 244 µL of ethyl 2-bromoacetate (2.21 mmol, d= 1.51 g/mL) was added slowly to the reaction mixture. After the completing of reaction, the solvent was evaporated and the residue was washed with water, filtered and air-dried to obtain ethyl 2-((2-oxo-1,2,3,4-tetrahydroquinolin-6-yl)oxy)acetate (TR-G012) which was further refluxed with 241 µL hydrazine hydrate 99% (4.81 mmol) for 5 hours then it was cooled to room temperature, filtered out of ethanol and dried to obtain 2-((2-oxo-1,2,3,4-tetrahydroquinolin-6-yl)oxy)acetohydrazide (TR-G019). Later, 210 mg of (TR-G019) (0.892 mmol) was refluxed with 211.47 mg of [1,1'-biphenyl]-4-carbaldehyde (1.16 mmol) in ethanol and drops of acetic acid for 6 hours. After that the precipitate was filtered out of ethanol to obtain 283 mg of the final product as white powder with a yield of 79%. LCMS/MS: m/z 400.59 [M+H]⁺.

¹H-NMR (500 MHz, DMSO-d) δ 11.59 (s, 1H. C-NH-N=), 9.97 (*9.93, s, 1H. C-NH-C), 8.39 (*8.05, s, 1H. N=CH-) 7.79-6.76 (m, 12Hₐᵣₒₘ), 5.10 (s, 1H. O-CH₂-C=O), 4.61 (s, 1H. O-CH₂-C=O), 2.84-2.81 (m, 2H. O=C-CH₂-CH₂-), 2.42-2.38 (m, 2H. O=C-CH₂-CH₂-). (* refer to the rotameric peak). ¹³C-NMR (126 MHz, DMSO) δ 169.79 (O=C<), 164.43 (O=C-NH=N-), 153.45, 147.49, 143.35, 141-42, 139.32, 133.09, 132.48,132.02,129.04 (2C), 127.75, 127.06, 126.68 (2C), 124.78, 115.77, 115.71, 114.15, 113.08, 65.09, 30.31, 25.1₁.

### Synthesis of (E)-N'-((4'-chloro-[1,1'-biphenyl)-4-yl)methylene)-2-((2-oxo-1,2,3,4-tetrahydroquinolin-6-yl)oxy)acetohydrazide (TR-G146)

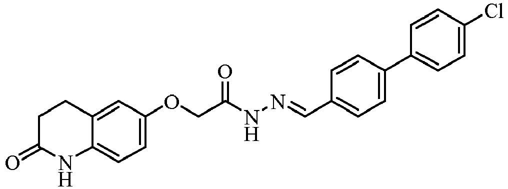

Starting from 6-hydroxy-3,4-dihydroquinolin-2(1H)-one, TR-G146 was prepared through 3 steps following procedures A, C and D respectively. 200 mg of TR-G019 (0.850 mmol) was refluxed with 221 mg of 4'-chloro-[1,1'-biphenyl]-4-carbaldehyde (1.02 mmol) in ethanol and drops of acetic acid. After filtration, the product was obtained as pure off-white powder with yield of 84% (310 mg). LCMS/MS: m/z 434.97 [M+H]⁺.

¹H-NMR (500 MHz, DMSO-d) δ 11.61 (s, 1H. C-NH-N=), 9.96 (*9.92, s, 1H. C-NH-C), 8.38 (*8.04, s, 1H. N=CH-) 7.79-6.73 (m, 11Hₐᵣₒₘ), 5.09 (s, 1H. O-CH₂-C=O), 4.61 (s, 1H. O-CH₂-C=O), 2.86-2.81 (m, 2H. O=C-CH₂-CH₂-), 2.42-2.38 (m, 2H. O=C-CH₂-CH₂-). (* refer to the rotameric peak). ¹³C-NMR (126 MHz, DMSO) δ 169.77 (O=C<), 164.45 (O=C-NH=N-), 153.43, 147.33, 143.20, 140.00, 138.11, 133.43, 132-75, 132-47, 132.01, 128.98, 128-45, 128-44 (2C), 127.58, 126.96, 124.76, 115.69, 114.14, 113.07, 65.08 (O-CH2-C=O), 30.36, 25.10.

### Synthesis of 6-((1-(3-oxobutyl)-1H-1,2,3-triazol-4-yl)methoxy)-3,4-dihydroquinolin-2(1H)-one (TR-G147)

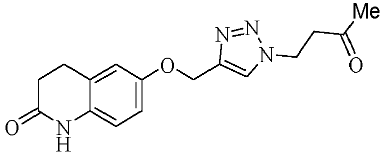

Compound TR-G147 has been prepared according to procedure A and E. In the first step, 4-azidobutan-2-one was prepared starting from 600 µL of 4-chlorobutan-2-one (5.63 mmol) which were refluxed with 732 mg of sodium azide salt (11.26mmol) for 2 hours in ethanol/ water (2:1). After that, the mixture was cooled to room temperature and filtered. Secondly, 200 mg of 6-(prop-2-yn-1-yloxy)-3,4-dihydroquinolin-2(1H)-one TR-G006 was prepared according to procedure A as mentioned before. In the last step, a mixture of -(prop-2-yn-1-yloxy)-3,4-dihydroquinolin-2(1H)-one TR-G006 (200 mg, 1 mmol), 23.79 mg of copper sulfate (0.149 mmol) and 8 mg of sodium ascorbate (0.049 mmol) in EtOH was added to the azidohexane solution and stirred for 12 h. After that, the ethanol was evaporated. The residue was dissolved in water and the mixture was extracted with ethyl acetate (3 × 50 ml), dried over anhydrous magnesium sulfate MgSO₄ and the solvent was evaporated under vacuum. Finally, the product was purified using flash chromatography (Hexane:EtOAc:TEA) (40:60:1%) to obtain 240 mg (76%) of compound TR-G147 as brown powder. LCMS/MS: m/z 315.51 [M+H]⁺.

¹H NMR (500 MHz, DMSO-*d*₆) δ 9.93 (s, 1H. N-H), 8.15 (s, 1H_{Traizole}), 6.88-6.76 (m, 3Hₐᵣₒₘ), 5.04 (S, 2H), 4.54-4.151 (t, 2H. J = 7.5 Hz. C=OCH₂CH₂), 3.15-3.12 (t, 2H. J = 7.5 Hz. C=OCH₂CH₂), 2.85-2.82 (t, 2H. J = 5 Hz CH₂CH₂-C=O) 2.42 -2.39 (t, 2H. J = 5 Hz. CH₂-C=O) 2.12(S, 3H. CH₃).

13C NMR (126 MHz, DMSO) δ (206.29 C=O), (170.25 NH-C=O), (153.66 C_{traizole}), 143-14, 132.49, 125.33, 125.08, 116.20, 114.79, 113-70, 61.82, 44.78, 42.72, 30.78, 30.22, 25.55.

### Synthesis of 6-((3-(2-chloro-6-fluorophenyl)-4,5-dihydroisoxazol-5-yl)methoxy)-3,4-dihydroquinolm-2(1H)-one (TR-G150)

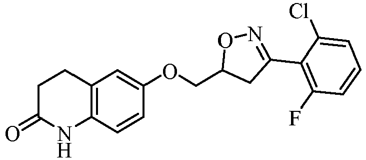

Compound TR-G150 has been prepared according to procedure A and F. Firstly, TR-G014 was prepared according to procedure A as previously mentioned. After that, a solution of 700 mg of 2-chloro-6-fluorobenzaldehyde (4.41mmol), 583 mg of hydroxylamine hydrochloride (17.66 mmol) and 800 mg of NaHCO₃ in ethanol were refluxed for 3h to prepare the corresponding oxime. The reaction was monitored by TLC until the completion of reaction after that the product was filtered, washed and dried. In cyclization step, 300 mg of TR-G014 (2.21 mmol) and 384 mg of 2-chloro-6-fluorobenzaldehyde oxime (2.21mmol) were dissolved in chloroform then catalytic amount of calcium hypochlorite was added to the mixture in ice bath 0 °C for 8 h, then water was added, and the product was extracted with chloroform (3× 30 mL). The organic layer was dried over anhydrous magnesium sulfate and the solvent was evaporated under vacuum. The crude was purified using flash chromatography (Hexane:EtOAc) (60:40) to afford 360 mg (65%) of the final product as yellow powder. LCMS/MS: m/z 375.32 [M+H]+.

¹H NMR (500 MHz, DMSO-*d*₆) δ 9.61 (s, 1H, N-H), 7.50-6.69 (m, 6H_{arom.}), 5.01 (tt, J = 6.8, 5.5 Hz, 1H. CH_{Oxazol}), 4.22-4.17 (m, 2H), 3.62 (m, 1H. CH_{2Oxazol}), 3.23 (m, 1H. CH_{2Oxazol}), 2.95-2.85 (m, 2H), 2.67-2.53 (m, 2H). ¹³C NMR (125 MHz, DMSO-d₆) δ 170.26, (159.04, 157.02 : Coupled with fluorine), 153.16, 149.52, 133.95, (130.76, 130.70 : Coupled with fluorine), (130.47, 130.41: Coupled with fluorine), 125.14, 125.12 : Coupled with fluorine), 124.44, (119.74, 119.58 : Coupled with fluorine), 119.48,115.72, 115.48, (114.46, 114.30 : Coupled with fluorine), 77.81, 67.92, (37.87, 37.84 : Coupled with fluorine), 30.95, 27.47.

### Methods

### In Vitro study

### Cell Proliferation Analysis

U87-MG and U138-MG human glioma cell, were incubated with 10% fetal bovine serum (FBS) (Gibco, 10500-064), 1% L-glutamine (Gibco, 25030-024), in a humidified incubator with 5% CO₂ at 37 °C. The cells were contained in DMEM High Glucose (Gibco, 41966-029) supplemented with 1% penicillin-streptomycin (P/S) (Gibco 15140-122) solution. The cytotoxic effects of the target compounds on U87-MG and U138-MG cell lines were assessed using the MTT (Thiazolyl Blue Tetrazolium Bromide) method. 7×103 cells were seeded into a 96-well plate in 100 µl DMEM High Glucose 10% FBS and 1% P/S. 24 hours after cell incubation, each target compound was added at a dose of 10 µM and the drug treatment was carried out for two days. After the incubation period, 10 µL of MTT solution was added to each well (5 mg/ml in PBS). After 2 hours, MTT solutions were removed from the wells. Then, 100 µl DMSO was added, and mixed to dissolve the formazan crystals. Cell proliferation was analysed by measuring the absorbance of dissolved formazan at a wavelength of 570 nm.

IC₅₀ values were determined using the same method as above wherein the cells were treated with the target compounds at different doses.

### Permeability study

PAMPA assay (BioAssay System, PMBBB-096, USA) has been used for assessing the permeability rates of the target compounds through the BBB mimicking membrane and the gastrointestinal mimicking membrane. Permeability assay and analyses were performed according to the manufacturer's instructions. Briefly, high permeability and low permeability standards, drugs (500 µM) and equilibrium standards (200 µM) were prepared. After the loading of lipid solutions into the pores, all drug solutions and the permeability controls were put onto the lipid membrane in donor wells. The permeability assay was performed for ₁8 hours at 37 °C incubator. All samples were collected from acceptor wells after the incubation period was finished and the absorbance values were measured with equilibrium standards for normalization.

### In Vivo study

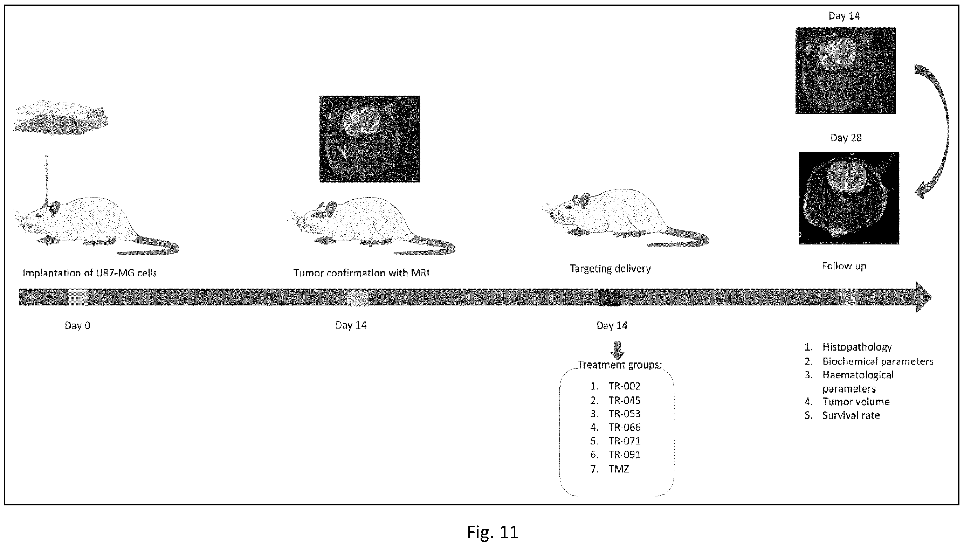

An *in Vivo* study was performed to assess the therapeutic potential of TR-G002, TR-G045, TR-G053, TR-G066, TR-G071 and TR-G091 in the GBM xenograft rat model when administered orally by gavage for five consecutive days. 152 female rats of the Sprague Dawley strain were allocated to the study. Six groups of sixteen females were given dose of 30 mg/kg of the respective target compounds. Temozolomide (TMZ, a known drug for treatment of GBM) was used as a positive control, and sixteen females were given 30 mg/kg of TMZ. The study design can be seen in Fig. 11 and Table 10.

**Table 10 shows the study design with details on groups and dosing.**

| Group No. | Compound | Number of Animals | Application Dose (mg/kg) |
|---|---|---|---|
| 1 | Healthy control | 8 F | 0 |
| 2 | GBM | 32 F | 30 |
| 3 | TMZ | 16 F | 30 |
| 4 | TR-G02 | 16 F | 30 |
| 5 | TR-G45 | 16 F | 30 |
| 6 | TR-G53 | 16 F | 30 |
| 7 | TR-G66 | 16 F | 30 |
| 8 | TR-G71 | 16 F | 30 |
| 9 | TR-G91 | 16 F | 30 |

This study has been approved by Ethics Committee of Laboratory Animals (HADYEK), Atatürk University (E-75296309-050.01.04-2300011862; 10.01.2023; Decision Number: 2023/01/02).

All efforts were made to decrease suffering and possible stress for the animals and performed in accordance with the Directive 2010/63/EU of the European Parliament and of the Council of 22 September 2010 on the protection of animals used for scientific purposes.

The method of blind research was applied at all stages.

The animals were included in the study if their tumor implantation was successfully defined by a tumor size greater than 80 mm³ with MRI and the animals were having alterations in motor functions. The animals were excluded if they were unable to continue their life functions (agony).

*Animals, animal care and husbandry*

| | |
|---|---|
| Species | Rat |
| Strain | RjHan: SD |
| Supplier | ATADEM, Erzurum, Turkey |
| Age | 5 weeks on arrival, approximately 8 weeks at start of dosing |
| Number and sex | 152 females |
| Body weight at start of dosing | 255-310 g |
| Animal identification | Each animal was uniquely identified within the study by an animal reference number and ear notching. |
| Acclimatisation | At least 14 days before start of dosing. |
| Housing, water and diet | Animals were group-housed under standard conditions. Animal had free access to water from the site drinking water supply and standard rodent diet supplied by Bayramoglu Feed Factory, Erzurum, Turkey. |
| Environmental conditions, temperature and humidity | Animal rooms were air-conditioned. The air was changed about 15 times per hour via a centrally placed air intake and peripheral ventilator. Target values for temperature and humidity were 20-26°C and 40-70%, respectively. |
| Light cycle | 12 hours light; 12 hours dark. |
| *Dosing* | |
| Route of administration | Oral gavage with soft tube. |
| Frequency and duration of administration | Once daily for 5 consecutive days. |
| Time of dosing | In the morning |

### Cell culture

U87-MG human glioma cells (American Type Culture Collection, Manassas, VA) were grown in DMEM medium (Thermo Fisher Scientific, Waltham, MA) containing 10% fetal bovine serum and 1% P/S. All cells were grown at 37 °C in a 5% CO₂ incubator.

### In vivo GBM rat model

Female Sprague Dawley rats (age: 6-8 weeks; weight: 255-310 g; ATADEM, Erzurum, Turkey) were utilized in the study. Rats underwent anaesthesia with 5% isoflurane, maintained at 2% isoflurane (1 L/min oxygen), and were positioned in a stereotactic frame for cell implantation (Stoelting Co., IL). The fur on the superior side of the head was removed to expose the skin, and the bregma was marked after incision. A burr hole was drilled 1-mm anterior and 3-mm lateral to the right bregma. A microsyringe (Hamilton 1700 series) containing 5×104 U87-MG cells suspended in 5 µL of medium at 37 °C was advanced to a depth of 4 mm into the brain. The cells were injected at a rate of 3 µL/ min. Bone wax was used to fill the burr hole to prevent cell suspension reflux. The incision was sutured, and 1 mL of Meloxicam (5 mg/mL; Metacam, Boehringer Ingelheim Vetmedica, Ingelheim, Germany) was administered via a subcutaneous injection.

Fourteen days after inoculation, tumor development in rats was visualized and confirmed through magnetic resonance imaging (MRI). Rats confirmed to have developed tumors were then distributed into six different groups for treatment. Rats were treated with the test compounds (target compounds and TMZ) for 5 days. 9 days after the final treatment dose, the rats underwent another MRI scan and blood and brain samples were taken under anaesthesia.

A check of animal health and welfare status was done at least once on working days and weekend days.

### Magnetic resonance imaging (MRI)

The anaesthetic was administered via an intraperitoneal route on animals fixed in the supine position with parallel hind limbs. MRI was performed using a 3 Tesla clinical scanner (Magnetom Skyra; Siemens Healthineers, Erlangen, Germany). Rats were placed in the prone position. T2-SPACE (sampling perfection with application-optimized contrast using different flip angle Evolution) sequence was obtained to view the brain in coronal and transverse planes. (TR: 6000 ms, TE: 100 ms, voxel size: 0.5 × 0.5 × 1 mm, 34 slices, slice thickness: 2 mm, slice spacing: 0.6 mm). The volume measurements of brain tumors were performed manually. One radiologist calculated the tumour volume - manually measuring tumour length (L), width (W), and height (H) - and then calculated the tumour volume using the formula 0.5236 × L × W × H (Stuppner S et al., 2020).

### Blood sampling

| | |
|---|---|
| Animals that were bled | All animals. |
| Blood sampling time | Blood for scheduled hematology and plasma chemistry were taken at necropsy. |
| Blood sample volume and anticoagulant | Plasma chemistry: 0.6 mL (lithium heparin) per animal. Haematology: 0.5 mL (K₂ EDTA) per animal. |
| Sample analyses | Blood and plasma were analysed using BS-200 Automatic Biochemistry Analyzer (Mindary Co., Ltd.) and automated hematology system analyzer (Archem, BM240, Istanbul, Turkey) |
| Hematology sample procedures | Blood was analyzed within 60 minutes of sampling. On the morning of analysis, the automated hematology system analysis (Archem, BM240, Istanbul, Turkey) was checked against a reference sample provided by the manufacturer, and a control sample analysis cycle was performed. For |

| | |
|---|---|
| | each study sample, Animal ID, Sample number was manually entered on the analyzer. |
| | The analyzer was set to automatic print-out and each individual sample print-out was collected and saved in the study folder. |
| Plasma chemistry sample procedures | Blood samples were kept in room temperature (approx. 20 °C) after collection and mixing. The blood samples were analysed as whole blood within 60 minutes of collection. BS-200 Automatic Biochemistry Analyzer (Mindary Co., Ltd.) was calibrated with calibration samples with known plasma levels of each parameter before start of analysis, occasionally during the analysis period of study samples and after completed analyses. For each study sample, Animal ID, Sample number was manually entered on the analyzer. The analyzer was set to automatic print-out and each individual sample print-out was collected and saved in the study folder. |

The parameters that were analysed can be seen in Table 11.

**Table 11 shows the haematology and plasma chemistry parameters that were analysed in the in Vivo study.**

| Haematology | | Plasma chemistry | |
|---|---|---|---|
| Red Blood cells | RBC | Aspartate aminotransferase | AST |
| White blood cells | WBC | Alanine aminotransferase | ALT |
| Hemoglobin | HGB | Alkaline phosphatase | ALP |
| Mean Red cell hemoglobin | MCH | Lactate dehydrogenase | LDH |
| Hematocrit | HCT | Triglyceride | TRIGLY |
| Mean Red cell hemoglobin concentration | MCHC | Total cholesterol | TC |
| Mean Red cell volume | MCV | HDL C | HDL C |
| Platelets | PLT | LDL C | LDL C |
| - | - | Glucose | GLU |
| - | - | Creatinine | CREA |
| - | - | Uric acid | UA |

### Histopathological Examinations

Brain tissue samples were fixed in a 10% buffered formaldehyde solution. After fixation, the tissues were processed through graded alcohol and xylene series and embedded in paraffin blocks. Sections of 5 µm thickness were taken from the paraffin blocks at intervals of 50-100 µm. Hematoxylin and eosin staining was performed on these sections to evaluate histopathological changes. Histopathological findings included tumor focus size, necrotic area, vascular proliferation, and cellular proliferation patterns. Tumor focus size was categorized as follows based on the diameter measured with a 4X objective on the microscope: >3500 µm was considered very severe, 3499-2000 µm severe, 1999-1000 µm moderate, 999-500 µm mild, and <500 µm very mild. Necrotic area size was classified based on the diameter of necrosis: >1500 µm as very severe, 1499-1000 µm severe, 999-500 µm moderate, 499-200 µm mild, and <199 µm very mild.

### Immunohistochemical Examinations

For immunohistochemical examinations, primary antibodies (Ki67 Cat no: ab15580 dilution ratio: 1/100; CMPK2 Cat no: PA5-34461, dilution ratio: 1/100, GFAP Cat no: ab6842 dilution ratio: 1/100) were used. 3-3' Diaminobenzidine (DAB) was used as chromogen. Mayer's hematoxylin was used for counterstaining. Sections were examined under a light microscope (Zeiss AXIO, Germany).

### Double Immunofluorescence Examination

In immunofluorescence examinations, primary antibody (NOP10 Cat no: ab134902 dilution ratio: 1/100) was applied to tissues and incubated according to the instructions. A fluorescent secondary antibody (FITC Cat No: ab6785 dilution Ratio: 1/1000 UK) was then applied and incubated in the dark for 45 minutes. Afterward, a second primary antibody (H2A.X Cat no: sc-517336 dilution ratio: 1/100) was applied and incubated according to the instructions. A different fluorescent secondary antibody (Texas Red Cat No: ab6719 dilution Ratio: 1/1000 UK) was applied and incubated in the dark for 45 minutes. Finally, DAPI (Cat no: D1306 dilution ratio: 1/200 UK) was applied to the sections for 5 minutes in the dark, followed by covering the tissues with coverslips. The stained tissues were examined under a fluorescence attachment-equipped microscope (Zeiss AXIO, Germany).

### Peripheral blood micronucleus assay

Blood smears were made on clean microscope slides, allowed to air-dry, treated with methanol for fixation, and then stained with Giemsa (10% in 0.1 mM of sodium phosphate buffer, pH 6.8) for 10 min. After washing with PBS, the slides were examined under a light microscope. The frequencies of micronuclei (MN) were calculated by evaluating 1000 cells per animal.

### Calculations and statistical analysis

Individual body weights were manually calculated. Group mean body weights; group mean body weight changes and organ weights were also manually calculated without statistical analysis. To determine the density of positive staining from immunohistochemical and immunofluorescence staining images, five randomly selected areas were evaluated using ZEISS Zen Imaging Software. Data were statistically defined as mean and standard deviation (mean ± SD) for the percentage of the area. One-way ANOVA followed by the Tukey test was performed to compare immunoreactive cells and immune-positive stained areas with healthy controls. GraphPad Prism software was used for analysis.

### Results

### In Vitro study

### Cell proliferation

U87-MG and U138-MG glioma cell lines were used to evaluate the effect of the treatment with the target compounds on glioblastoma *in vitro.* The effectiveness of the treatment with the target compounds was evaluated on cell proliferation, see Tables 12-14. Cilostazol and TMZ were used as reference drugs. Cilostazol is known to pass the blood-brain barrier and TMZ is a known drug for treatment of GBM.

**Table 12 shows the cell proliferation and inhibition of U87-MG and U138-MG cells when treated with reference drugs Cilostazol and TMZ.**

| | U87-MG | | U138-MG | |
|---|---|---|---|---|
| Drug | Proliferation (%) | Inhibition (%) | Proliferation (%) | Inhibition (%) |
| Cilostazol | 78 | 22 | 88 | 12 |
| TMZ | 80 | 20 | 78 | 22 |

**Table 13 shows the cell proliferation and inhibition of U87-MG cells when treated with the target compounds.**

| Target compound | Cell Proliferation (%) | Inhibition (%) | SD (±) (%) |
|---|---|---|---|
| TR-G001 | 78 | 22 | 2.8 |
| TR-G002 | 48 | 52 | 4.6 |
| TR-G003 | 79 | 21 | 1.3 |
| TR-G004 | 73 | 27 | 6 |
| TR-G005 | 77 | 23 | 2.5 |
| TR-G006 | 86 | 14 | 0.3 |
| TR-G007 | 91 | 9 | 5.2 |
| TR-G008 | 63 | 37 | 3.5 |
| TR-G009 | 72 | 28 | 3.0 |
| TR-G010 | 74 | 26 | 2.9 |
| TR-G011 | 76 | 24 | 2.5 |
| TR-G012 | 71 | 29 | 10.2 |
| TR-G013 | 71 | 29 | 3.5 |
| TR-G014 | 82 | 18 | 1.4 |
| TR-G014A | 82 | 19 | 4.2 |
| TR-G016 | 78 | 22 | 5.0 |
| TR-G017 | 94 | 6 | 3.5 |
| TR-G019 | 75 | 25 | 4.0 |
| TR-G020 | 72 | 29 | 2.1 |
| TR-G021 | 65 | 36 | 4.9 |
| TR-G022 | 98 | 2 | 2.5 |
| TR-G023 | 83 | 17 | 4.3 |
| TR-G024 | 80 | 20 | 4.5 |
| TR-G025 | 75 | 25 | 3.1 |
| TR-G026 | 92 | 8 | 1.4 |
| TR-G027 | 66 | 34 | 4.9 |
| TR-G028 | 64 | 36 | 5.2 |
| TR-G029 | 82 | 18 | 5.5 |
| TR-G030 | 53 | 47 | 4.6 |
| TR-G031 | 72 | 28 | 3.0 |
| TR-G033 | 82 | 19 | 4.0 |
| TR-G035 | 86 | 14 | 2.6 |
| TR-G036 | 68 | 32 | 3.9 |
| TR-G037 | 91 | 9 | 0.2 |
| TR-G038 | 71 | 30 | 2.1 |
| TR-G039 | 59 | 41 | 3.8 |
| TR-G040 | 91 | 9 | 3.1 |
| TR-G041 | 70 | 30 | 6.0 |
| TR-G042 | 70 | 31 | 1.8 |
| TR-G043 | 73 | 27 | 5.4 |
| TR-G045 | 71 | 29 | 7.1 |
| TR-G046 | 60 | 40 | 4.1 |
| TR-G047 | 77 | 23 | 7.0 |
| TR-G048 | 69 | 31 | 2.8 |
| TR-G049 | 76 | 24 | 2.8 |
| TR-G050 | 69 | 31 | 2.1 |
| TR-G051 | 79 | 21 | 7.5 |
| TR-G052 | 85 | 15 | 3.5 |
| TR-G053 | 42 | 58 | 1.4 |
| TR-G054 | 84 | 16 | 6.3 |
| TR-G055 | 85 | 15 | 1.2 |
| TR-G056 | 99 | 1 | 7.0 |
| TR-G057 | 85 | 15 | 5.0 |
| TR-G058 | 73 | 27 | 3.5 |
| TR-G059 | 79 | 21 | 1.4 |
| TR-G061 | 84 | 16 | 1.4 |
| TR-G062 | 87 | 13 | 2.3 |
| TR-G063 | 57 | 43 | 4.0 |
| TR-G064 | 70 | 30 | 1.6 |
| TR-G065 | 73 | 27 | 0.7 |
| TR-G066 | 67 | 33 | 4.9 |
| TR-G068 | 59 | 42 | 3.3 |
| TR-G069 | 64 | 36 | 3.5 |
| TR-G070 | 68 | 33 | 7.0 |
| TR-G071 | 97 | 3 | 4.2 |
| TR-G072 | 85 | 15 | 2.6 |
| TR-G073 | 87 | 13 | 0.9 |
| TR-G074 | 85 | 15 | 1.3 |
| TR-G075 | 68 | 32 | 3.1 |
| TR-G076 | 97 | 3 | 1.3 |
| TR-G077 | 88 | 12 | 1.5 |
| TR-G078 | 44 | 56 | 7.8 |
| TR-G079 | 94 | 6 | 5 |
| TR-G080 | 79 | 21 | 5.0 |
| TR-G081 | 65 | 35 | 3.0 |
| TR-G082 | 74 | 26 | 13.2 |
| TR-G084 | 65 | 35 | 5.3 |
| TR-G085 | 53 | 47 | 6.8 |
| TR-G086 | 79 | 21 | 1.7 |
| TR-G087 | 58 | 42 | 7.2 |
| TR-G089 | 67 | 33 | 2.1 |
| TR-G091 | 101 | -1 | 4.9 |
| TR-G092 | 61 | 39 | 1.4 |
| TR-G093 | 55 | 45 | 4.6 |
| TR-G094 | 78 | 22 | 2.8 |
| TR-G095 | 73 | 27 | 3.5 |
| TR-G096 | 75 | 25 | 7.1 |
| TR-G097 | 79 | 21 | 1.2 |
| TR-G099 | 71 | 29 | 2.2 |
| TR-G101 | 76 | 24 | 4.5 |
| TR-G102 | 66 | 35 | 5.4 |
| TR-G103 | 45 | 55 | 5.0 |
| TR-G104 | 49 | 51 | 2.1 |
| TR-G105 | 48 | 52 | 7.2 |
| TR-G106 | 73 | 27 | 2.1 |
| TR-G107 | 47 | 53 | 5.3 |
| TR-G110 | 58 | 42 | 2.2 |
| TR-G111 | 68 | 32 | 1.5 |
| TR-G112 | 60 | 41 | 4.0 |
| TR-G114 | 86 | 14 | 3.4 |
| TR-G115 | 82 | 18 | 4.7 |
| TR-G116 | 93 | 7 | 2.1 |
| TR-G117 | 96 | 4 | 5 |
| TR-G120 | 68 | 33 | 7.1 |
| TR-G121 | 69 | 32 | 2.1 |
| TR-G122 | 82 | 18 | 1.4 |
| TR-G123 | 92 | 8 | 3.9 |
| TR-G125 | 64 | 36 | 5.2 |
| TR-G126 | 70 | 30 | 2.1 |
| TR-G128 | 68 | 32 | 5.0 |
| TR-G129 | 81 | 19 | 5.5 |
| TR-G131 | 79 | 21 | 0.7 |
| TR-G132 | 78 | 22 | 4.9 |
| TR-G133 | 93 | 7 | 5.8 |
| TR-G134 | 70 | 30 | 5.6 |
| TR-G135 | 71 | 29 | 5.7 |
| TR-G136 | 89 | 11 | 1.2 |
| TR-G137 | 73 | 27 | 1.3 |
| TR-G138 | 50 | 50 | 3.2 |
| TR-G139 | 71 | 29 | 2.1 |
| TR-G13A | 46 | 54 | 4.7 |
| TR-G140 | 80 | 20 | 1.5 |
| TR-G141 | 53 | 47 | 6.4 |
| TR-G142 | 55 | 46 | 7.0 |
| TR-G143 | 63 | 37 | 6.5 |
| TR-G144 | 61 | 39 | 4.2 |
| TR-G145 | 49 | 51 | 5.5 |
| TR-G146 | 63 | 37 | 2.5 |
| TR-G147 | 89 | 11 | 3.7 |
| TR-G150 | 97 | 3 | 4.8 |

Treatment of U87-MG cells with 90 of the 130 synthesized target compounds, resulted in a reduction of cell proliferation with at least 20 % compared to non-treated cells. This means that treatment with 90 of the target compounds exhibited the same or lower cell proliferation than the known GBM drug TMZ, see Tables 12-13. Treatment with 50 of the target compounds resulted in a reduction of cell proliferation of at least 30 % compared to non-treated cells. Treatment of U87-MG cells with 80 of the target compounds exhibited the same or lower cell proliferation than the known drug Cilostazol. The top 5 target compounds whose treatment resulted in the lowest proliferation of U87-MG cells were TR-G053, TR-G078, TR-G103, TR-G1013A and TR-G107 wherein treatment with TR-G053 resulted in the lowest cell proliferation, 42 %.

**Table 14 shows the cell proliferation and inhibition of U138-MG cells when treated with the target compounds.**

| Target compound | Cell Proliferation (%) | Inhibition (%) | SD (±) (%) |
|---|---|---|---|
| TR-G001 | 88 | 12 | 3.0 |
| TR-G002 | 52 | 48 | 4.0 |
| TR-G003 | 84 | 16 | 5.2 |
| TR-G004 | 88 | 12 | 4 |
| TR-G005 | 77 | 23 | 10 |
| TR-G006 | 86 | 14 | 1.8 |
| TR-G007 | 82 | 18 | 2.6 |
| TR-G008 | 65 | 35 | 1.3 |
| TR-G009 | 87 | 13 | 2.5 |
| TR-G010 | 74 | 26 | 1.8 |
| TR-G011 | 60 | 40 | 5.3 |
| TR-G012 | 85 | 15 | 4.3 |
| TR-G013 | 78 | 22 | 1.0 |
| TR-G014 | 51 | 49 | 7.0 |
| TR-G014A | 68 | 32 | 5.3 |
| TR-G016 | 95 | 5 | 5.7 |
| TR-G017 | 82 | 18 | 4.7 |
| TR-G019 | 75 | 25 | 4.5 |
| TR-G020 | 80 | 20 | 2.5 |
| TR-G021 | 60 | 40 | 4.0 |
| TR-G022 | 75 | 25 | 3.0 |
| TR-G023 | 99 | 1 | 2.5 |
| TR-G024 | 70 | 30 | 5.0 |
| TR-G025 | 90 | 10 | 5.0 |
| TR-G026 | 90 | 10 | 2.6 |
| TR-G027 | 54 | 46 | 3.0 |
| TR-G028 | 106 | -6 | 11.5 |
| TR-G029 | 68 | 32 | 5.2 |
| TR-G030 | 63 | 38 | 1.1 |
| TR-G031 | 80 | 20 | 4.0 |
| TR-G033 | 71 | 29 | 5.7 |
| TR-G035 | 74 | 26 | 2.8 |
| TR-G036 | 81 | 19 | 0.7 |
| TR-G037 | 65 | 35 | 2.3 |
| TR-G038 | 95 | 5 | 5.0 |
| TR-G039 | 75 | 25 | 3.0 |
| TR-G040 | 98 | 2 | 1.3 |
| TR-G041 | 74 | 26 | 7.1 |
| TR-G042 | 83 | 17 | 3.5 |
| TR-G043 | 74 | 26 | 4.8 |
| TR-G045 | 71 | 30 | 6.0 |
| TR-G046 | 76 | 24 | 5.0 |
| TR-G047 | 62 | 38 | 3.6 |
| TR-G048 | 61 | 39 | 3.0 |
| TR-G049 | 63 | 37 | 2.1 |
| TR-G050 | 54 | 46 | 1.4 |
| TR-G051 | 67 | 33 | 7 |
| TR-G052 | 52 | 48 | 1.2 |
| TR-G053 | 53 | 47 | 2.4 |
| TR-G054 | 79 | 21 | 1.6 |
| TR-G055 | 92 | 8 | 5.2 |
| TR-G056 | 83 | 17 | 2.5 |
| TR-G057 | 99 | 1 | 4.4 |
| TR-G058 | 100 | 0 | 7.1 |
| TR-G059 | 81 | 20 | 4.9 |
| TR-G061 | 92 | 8 | 5.7 |
| TR-G062 | 87 | 13 | 2.2 |
| TR-G063 | 69 | 32 | 5.0 |
| TR-G064 | 78 | 22 | 4.8 |
| TR-G065 | 90 | 10 | 3.7 |
| TR-G066 | 39 | 62 | 3.0 |
| TR-G068 | 82 | 18 | 5.1 |
| TR-G069 | 96 | 4 | 1.3 |
| TR-G070 | 79 | 21 | 6.0 |
| TR-G071 | 100 | 0 | 0.0 |
| TR-G072 | 85 | 15 | 1.2 |
| TR-G073 | 94 | 6 | 1.5 |
| TR-G074 | 81 | 19 | 0.7 |
| TR-G075 | 85 | 15 | 0.6 |
| TR-G076 | 92 | 8 | 1.8 |
| TR-G077 | 88 | 12 | 4.3 |
| TR-G078 | 57 | 43 | 7 |
| TR-G079 | 112 | -12 | 3.5 |
| TR-G080 | 71 | 29 | 4.0 |
| TR-G081 | 108 | -8 | 6.0 |
| TR-G082 | 89 | 11 | 7.2 |
| TR-G084 | 71 | 30 | 4.0 |
| TR-G085 | 71 | 29 | 2.0 |
| TR-G086 | 79 | 21 | 0.6 |
| TR-G087 | 55 | 45 | 1.0 |
| TR-G089 | 67 | 33 | 0.3 |
| TR-G091 | 86 | 14 | 7.0 |
| TR-G092 | 65 | 35 | 4.1 |
| TR-G093 | 63 | 38 | 2.0 |
| TR-G094 | 80 | 20 | 3.2 |
| TR-G095 | 70 | 30 | 1.0 |
| TR-G096 | 70 | 30 | 1.3 |
| TR-G097 | 73 | 27 | 3.1 |
| TR-G099 | 79 | 21 | 0.2 |
| TR-G101 | 76 | 24 | 3.0 |
| TR-G102 | 49 | 51 | 3.0 |
| TR-G103 | 63 | 38 | 4.0 |
| TR-G104 | 83 | 17 | 2.0 |
| TR-G105 | 73 | 28 | 5.0 |
| TR-G106 | 66 | 35 | 6.0 |
| TR-G107 | 103 | -3 | 3.5 |
| TR-G110 | 69 | 31 | 1.1 |
| TR-G111 | 91 | 9 | 2.8 |
| TR-G112 | 43 | 58 | 2.0 |
| TR-G114 | 85 | 15 | 4.1 |
| TR-G115 | 52 | 48 | 7 |
| TR-G116 | 59 | 41 | 3.7 |
| TR-G117 | 106 | -6 | 5.8 |
| TR-G120 | 67 | 34 | 6.5 |
| TR-G121 | 97 | 4 | 5.9 |
| TR-G122 | 90 | 11 | 5.0 |
| TR-G123 | 109 | -9 | 1.8 |
| TR-G125 | 82 | 18 | 7.0 |
| TR-G126 | 97 | 3 | 3.2 |
| TR-G128 | 68 | 32 | 5.0 |
| TR-G129 | 74 | 26 | 4.6 |
| TR-G131 | 54 | 46 | 3.2 |
| TR-G132 | 73 | 27 | 1.2 |
| TR-G133 | 77 | 23 | 6.5 |
| TR-G134 | 73 | 27 | 7.0 |
| TR-G135 | 80 | 20 | 3.3 |
| TR-G136 | 60 | 40 | 2.0 |
| TR-G137 | 60 | 40 | 7.0 |
| TR-G138 | 65 | 35 | 3.5 |
| TR-G139 | 83 | 17 | 4.0 |
| TR-G13A | 53 | 47 | 7 |
| TR-G140 | 91 | 9 | 6.8 |
| TR-G141 | 65 | 35 | 5.5 |
| TR-G142 | 70 | 30 | 6.8 |
| TR-G143 | 49 | 51 | 5.2 |
| TR-G144 | 70 | 30 | 2.9 |
| TR-G145 | 59 | 41 | 7.0 |
| TR-G146 | 80 | 21 | 6.9 |
| TR-G147 | 122 | -22 | 7.5 |
| TR-G150 | 79 | 21 | 2.1 |

Treatment of U138-MG cells with 78 of the 130 synthesized target compounds, resulted in a reduction of cell proliferation with at least 20 % compared to non-treated cells. Treatment with 68 of the target compounds exhibited the same or lower cell proliferation as the known GBM drug TMZ, see Tables 12 and 14. Treatment with 44 of the target compounds resulted in a reduction of cell proliferation of at least 30 % compared to non-treated cells. Treatment of U138-MG with 101 of the target compounds exhibited the same or lower cell proliferation as the known drug Cilostazol. The top 5 target compounds whose treatment resulted in the lowest cell proliferation of U138-MG cells were TR-G066, TR-G112, TR-G143, TR-G102 and TR-G014 wherein treatment with TR-G066 resulted in the lowest cell proliferation, 39 %.

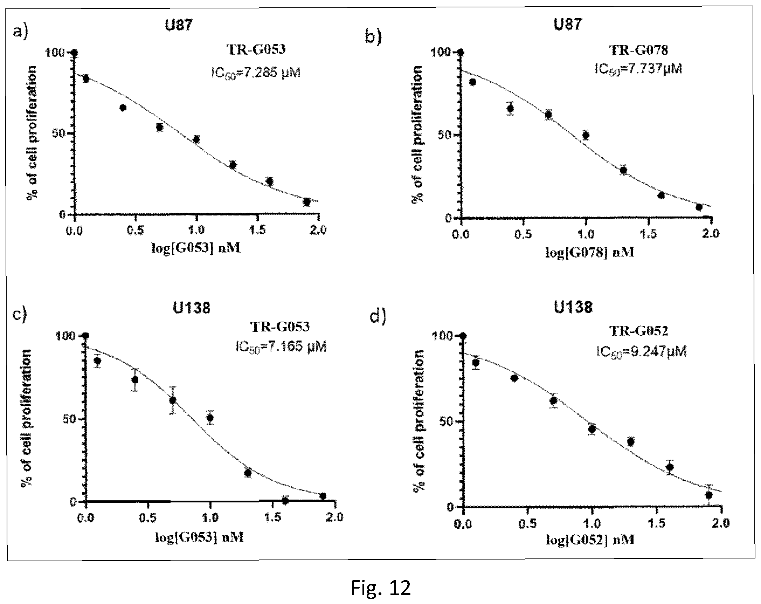

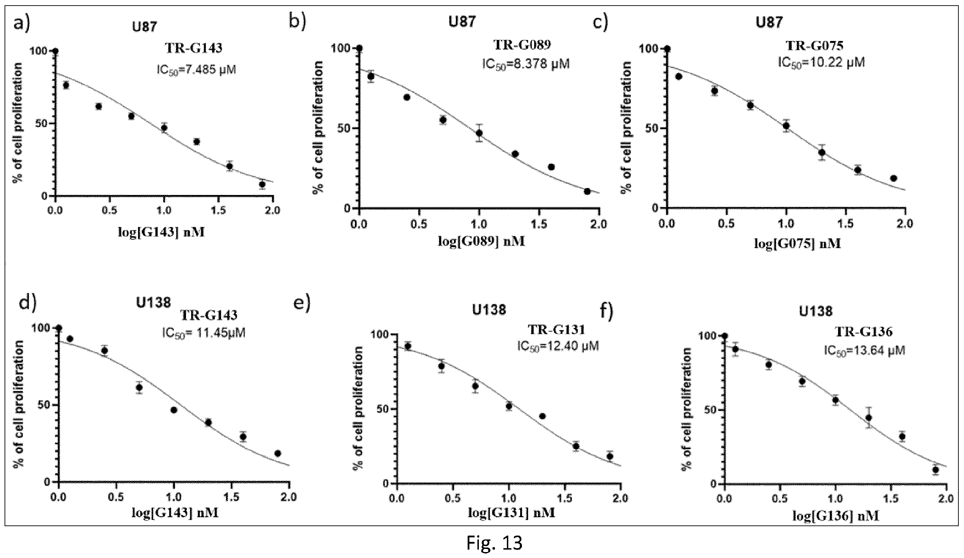

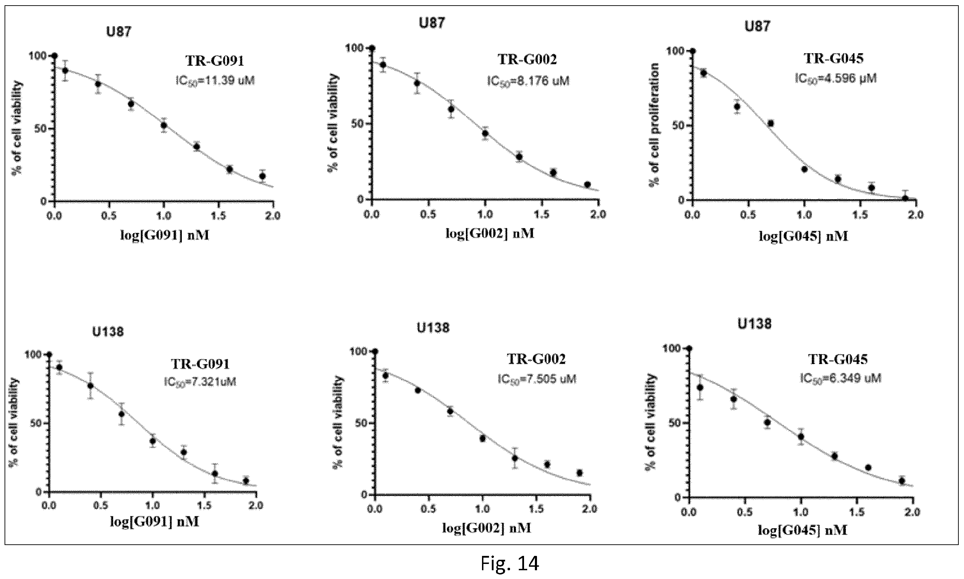

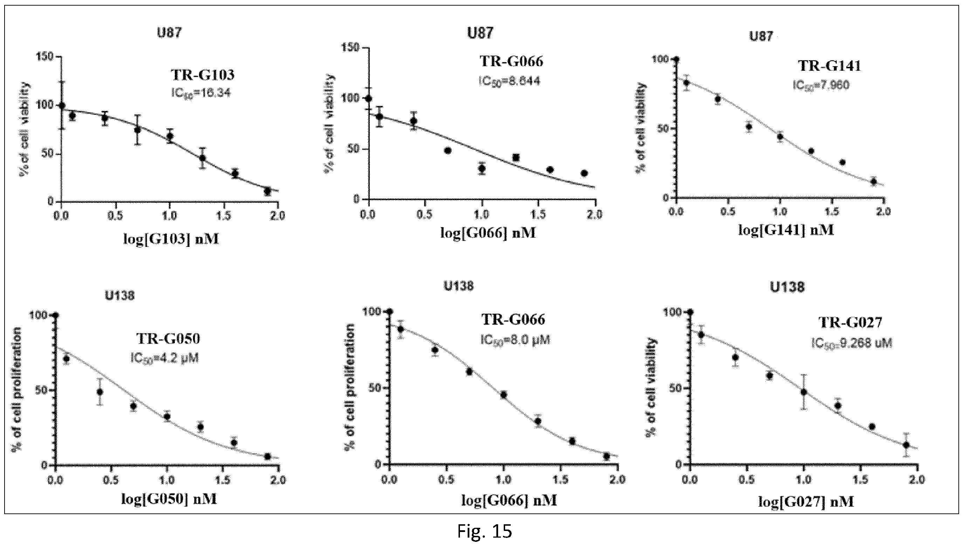

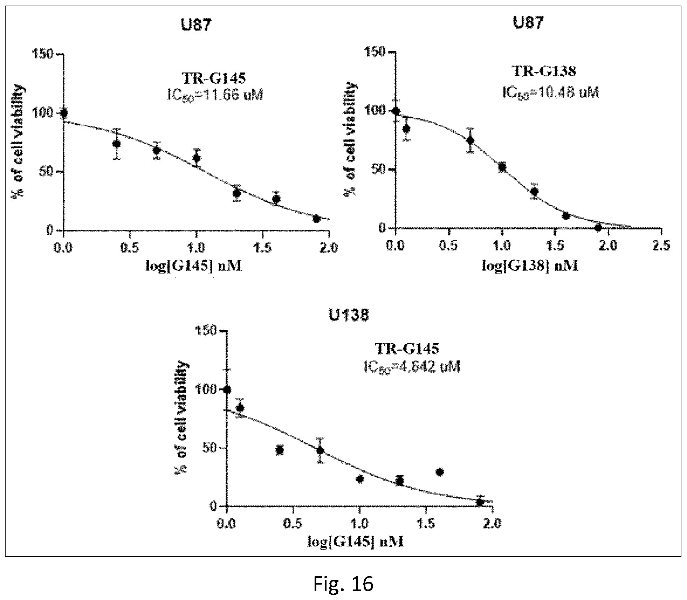

IC₅₀ values were determined for some of the target compounds, see Fig. 12-16. IC₅₀ values in U87-MG cells varied from 4.6-16.3 µM while the IC₅₀ values in U138-MG cells varied from 4.2-13.6 µM.

### Permeability study

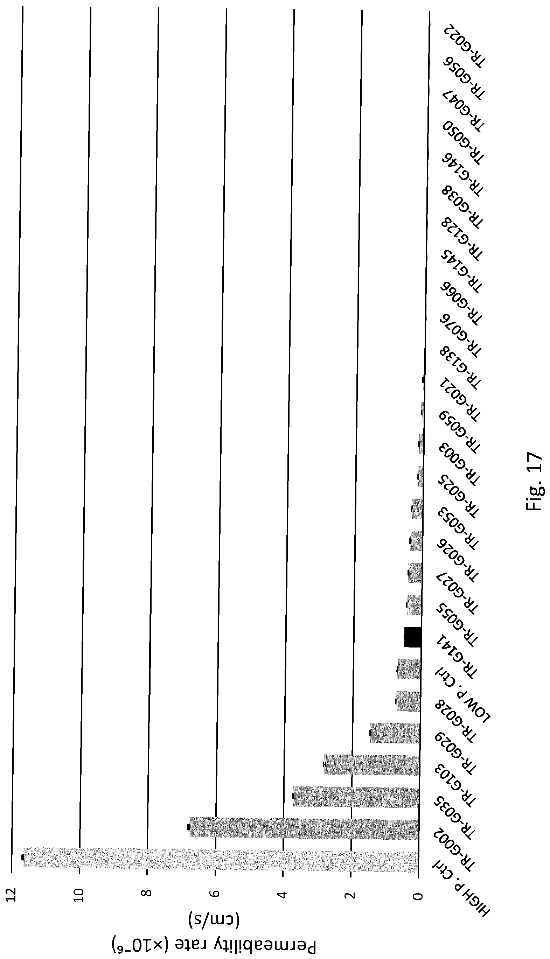

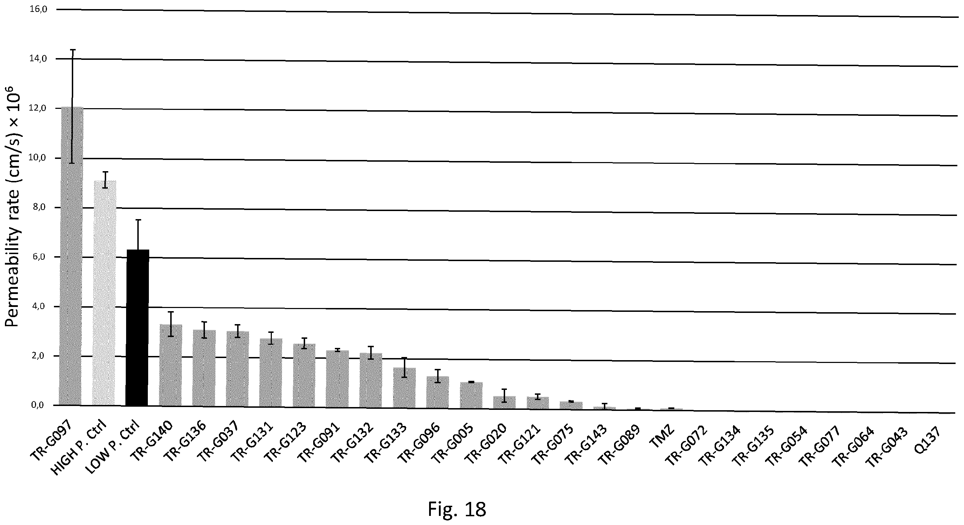

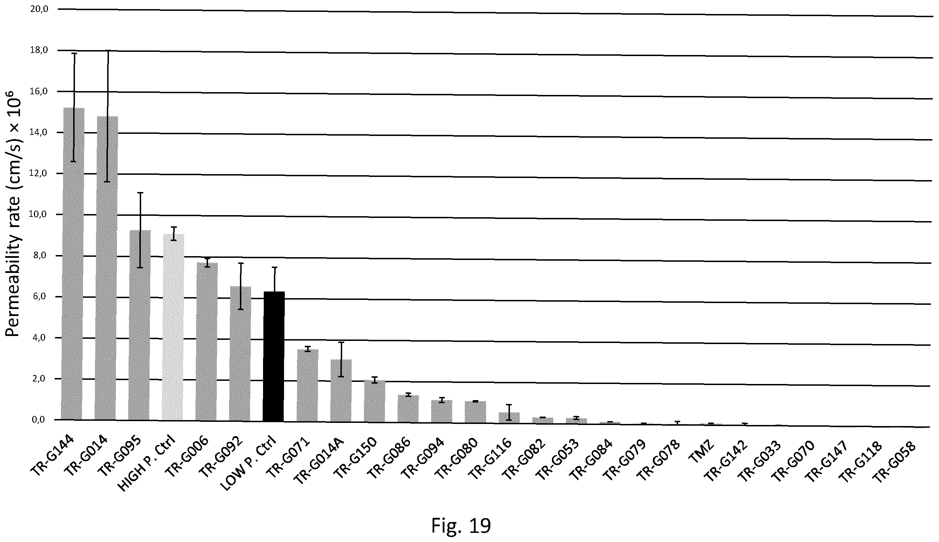

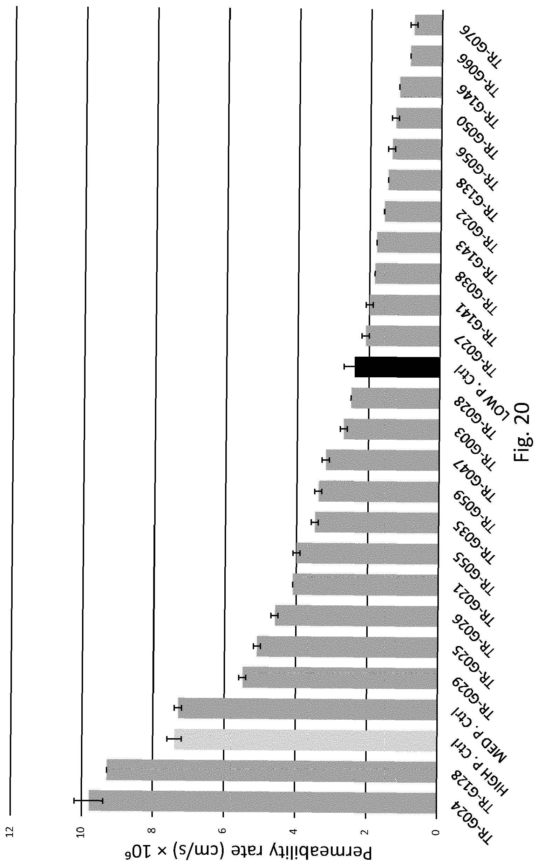

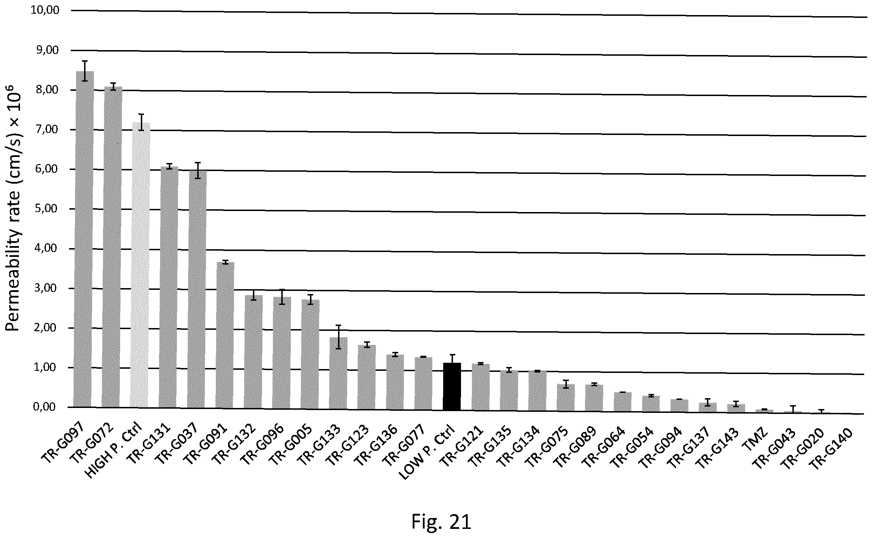

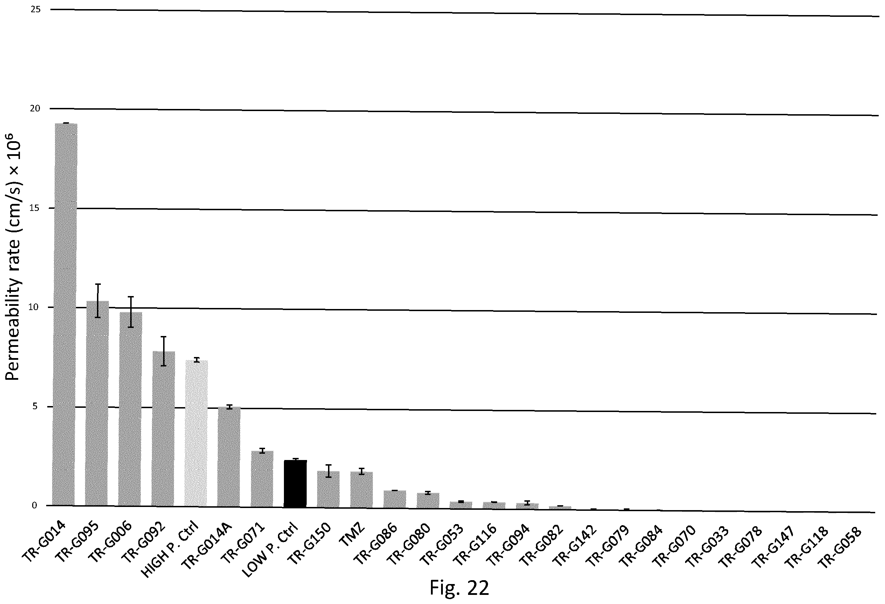

A permeability study using the PAMPA assay was conducted for some of the target compounds, see Fig. 17-22. The permeability rate of the target compounds through a blood-brain barrier and gastrointestinal mimicking membrane were determined.

The permeability results through the blood-brain barrier mimicking membrane showed that TR-G097, TR-G144, TR-G014 and TR-G095 are highly permeable while TR-G002, TR-G035, TR-G103, TR-G029 and TR-G025 have middle permeability and the rest of the tested compounds have lower permeability.

The permeability results through the gastrointestinal mimicking membrane showed that TR-G024, TR-G128, TR-G097, TR-G072, TR-G014, TR-G095, TR-G006 and TR-G092 are highly permeable, while the other tested compounds had a lower permeability.

### In Vivo study

### Body weight

The untreated GBM rats (induced GBM after 14 days) exhibited a decrease in body weight compared to the healthy control group. No significant change in body weight was observed in GBM rats treated with TMZ and the target compounds compared to the GBM rats.

### Evaluation of survival

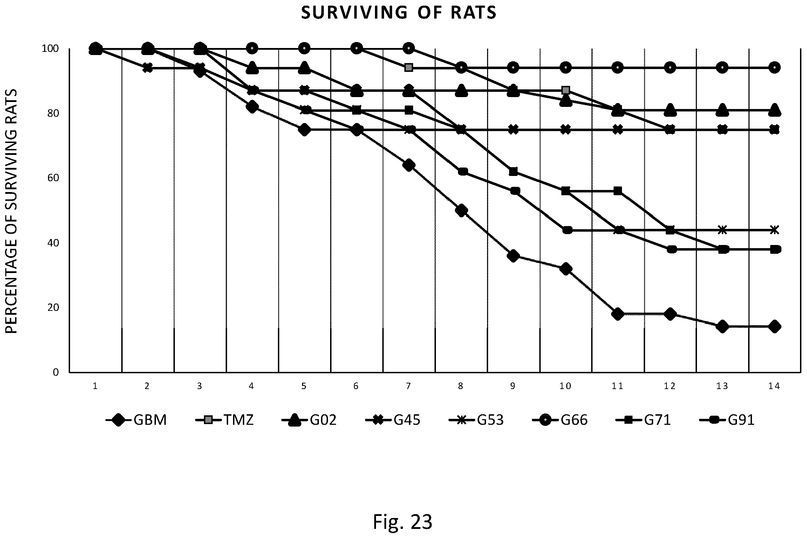

Overall survival was assessed from the end of tumor induction (14^{th} day) until death or end of experiment (28^{th} day) and the results can be seen in Fig. 23. Treatment with all tested target compounds and TMZ prolonged the survival of the treated rats compared to GBM rats. GBM rats treated with TR-G066, TR-G002, TR-G045 and TMZ exhibited significantly improved survival compared to untreated GBM rats, wherein the TR-G066 treated GBM rats exhibited the highest survival.

### MRI analysis of tumor volumes

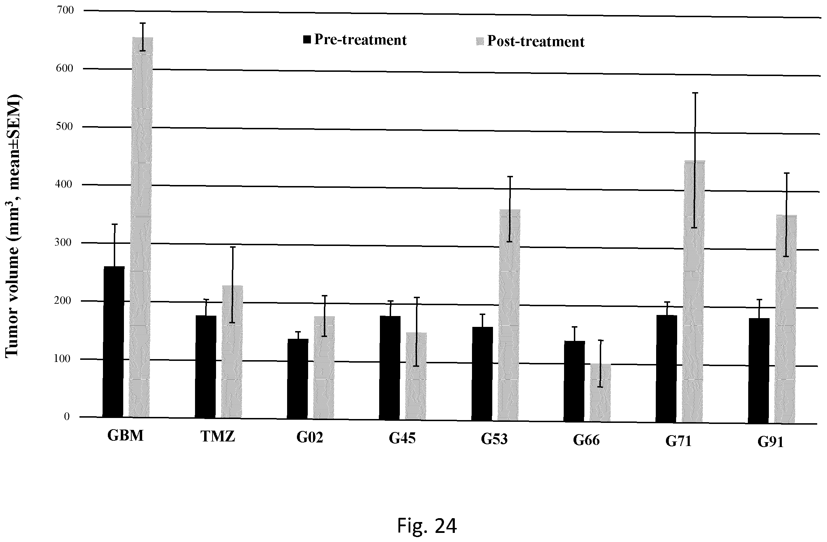

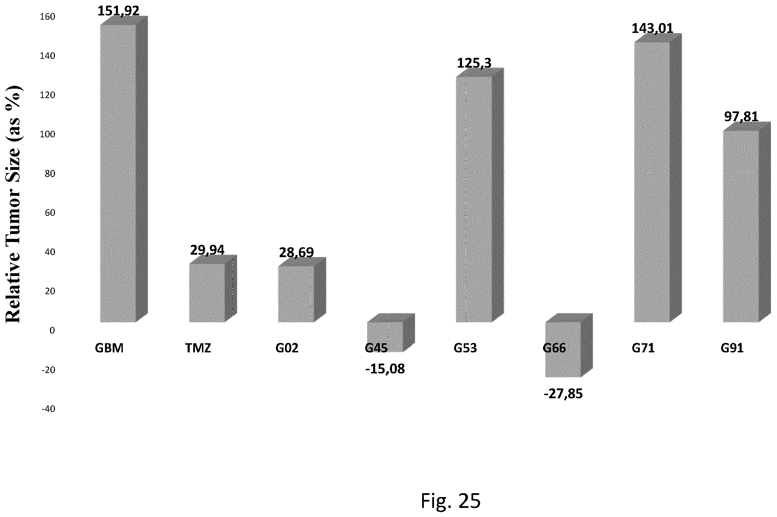

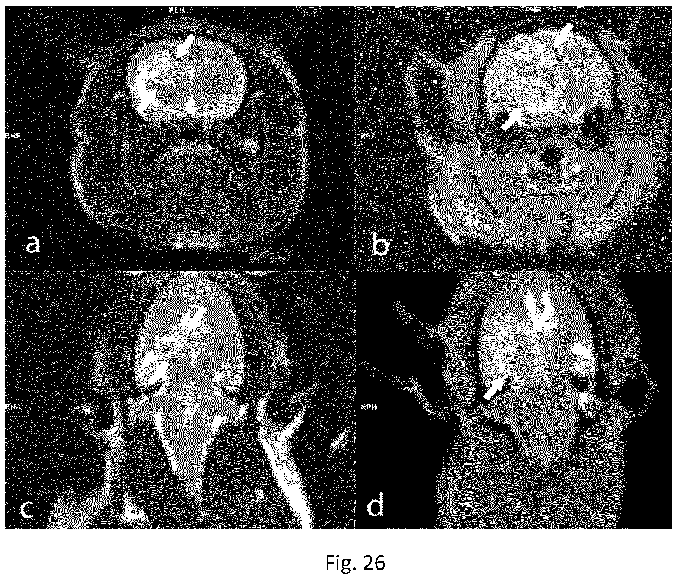

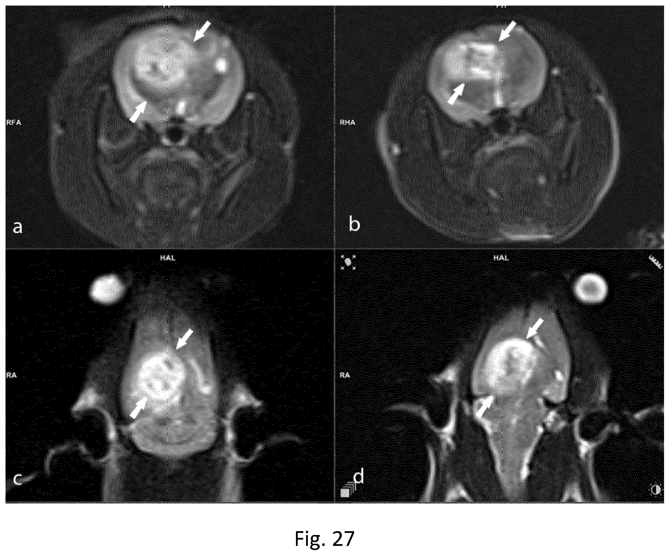

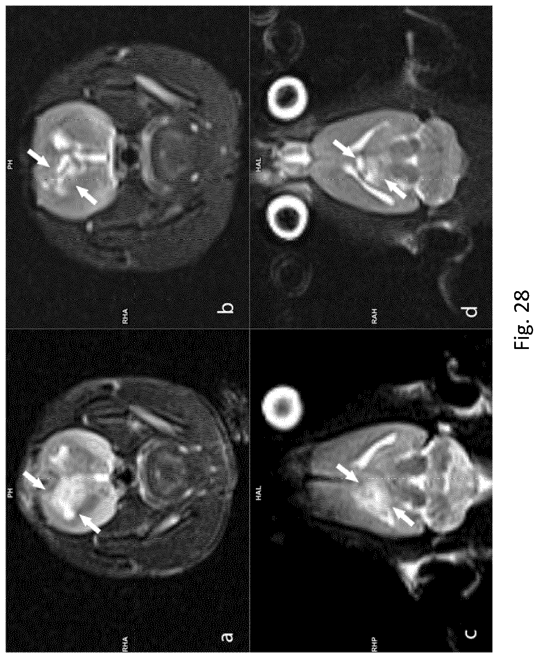

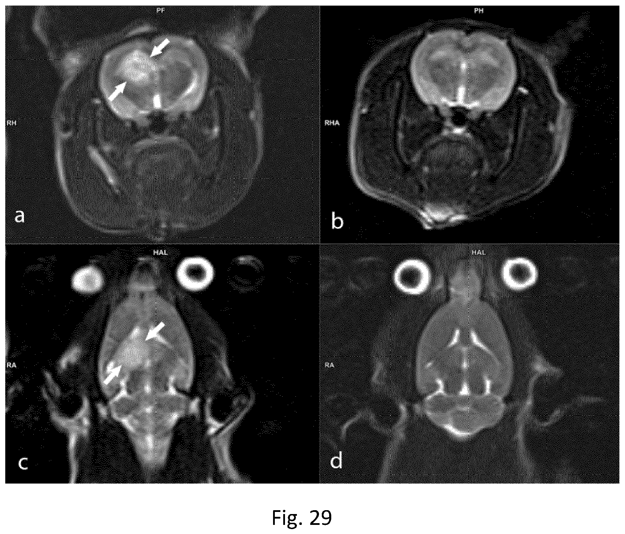

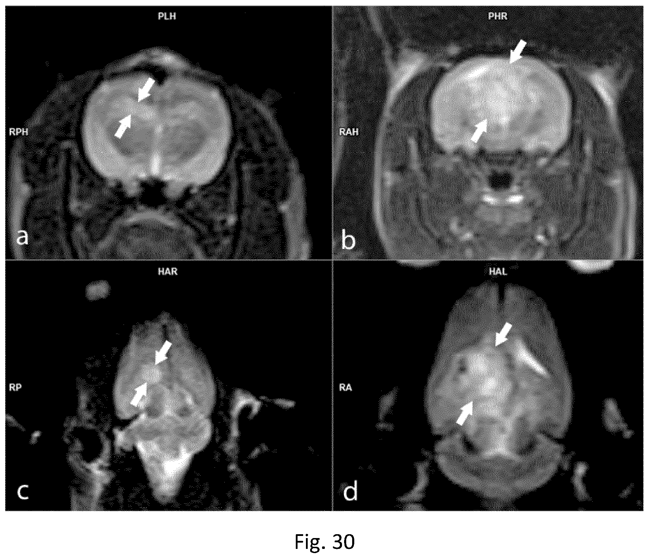

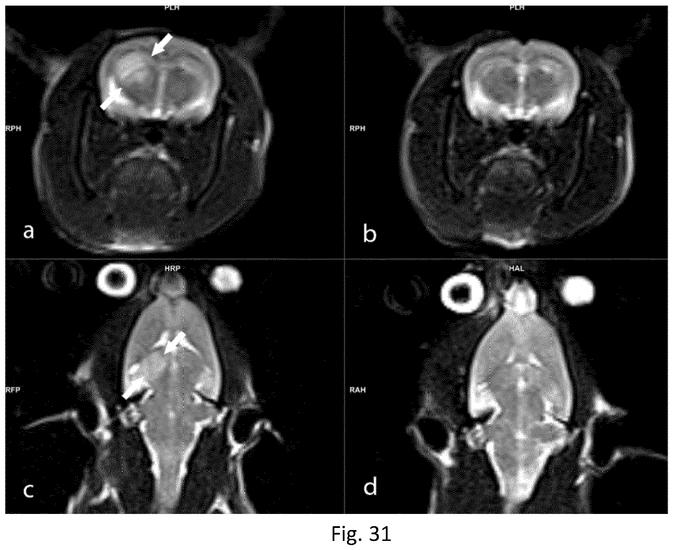

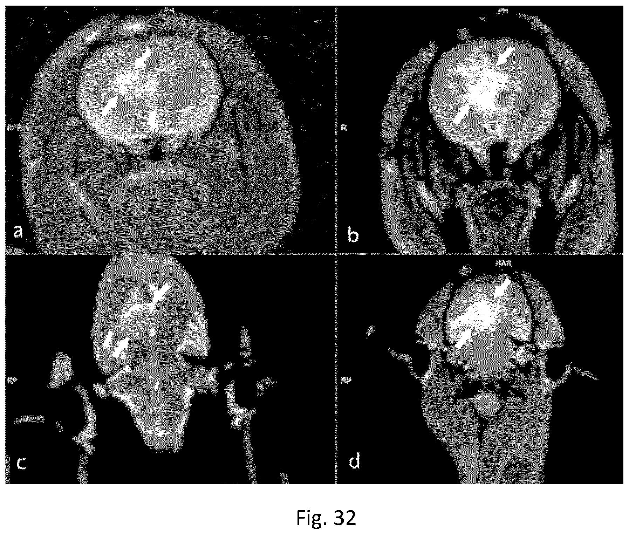

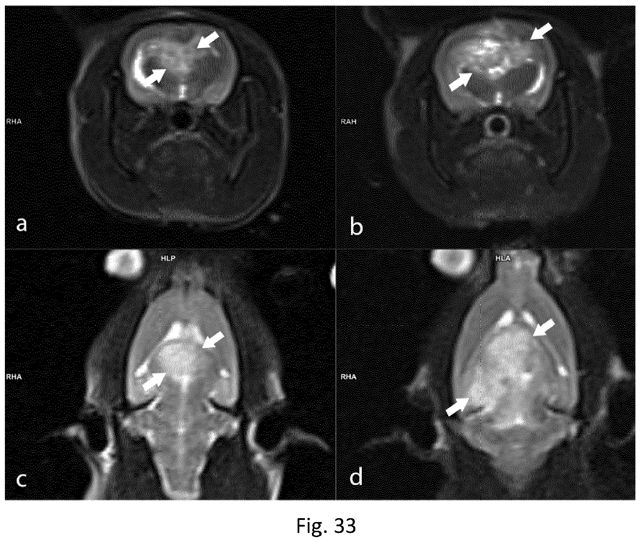

Tumor volumes were determined 14 days after tumor implantation in the animals using MRI, see Fig. 24-33. Rats having a tumor volume of 80 mm³ and above were included in the experiments. 6 different target compounds were administered along with TMZ as reference drug to animals with confirmed tumor formation.

Treatment with all the target compounds and TMZ resulted in a deceleration in tumor size compared to the control group, see Fig. 24-25. Treatment with TR-G002 resulted in a small increase in tumor size, similar to the increase obtained after treatment with TMZ. Treatment with TR-G045 and TR-G066 resulted in a decrease in tumor size.

The antitumor activities of tested compounds were in the following order: TR-G066> TR-G045> TR-G002>TMZ> TR-G091> TR-G053> TR-G071.

### Histopathological findings

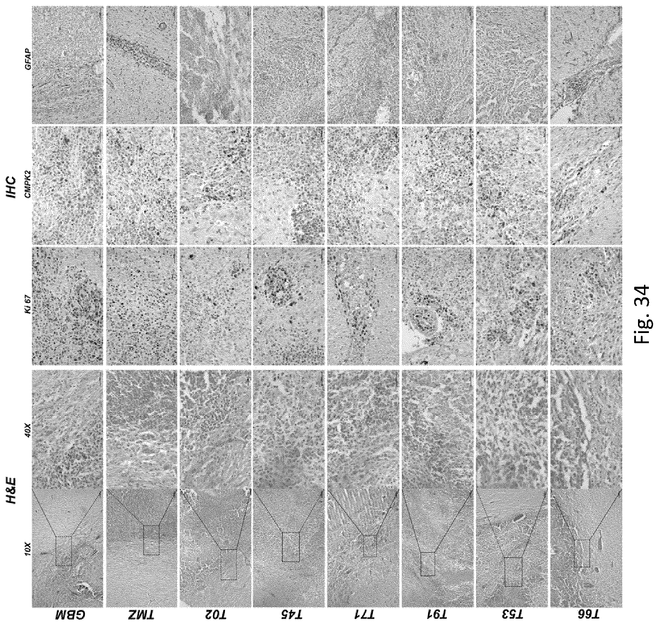

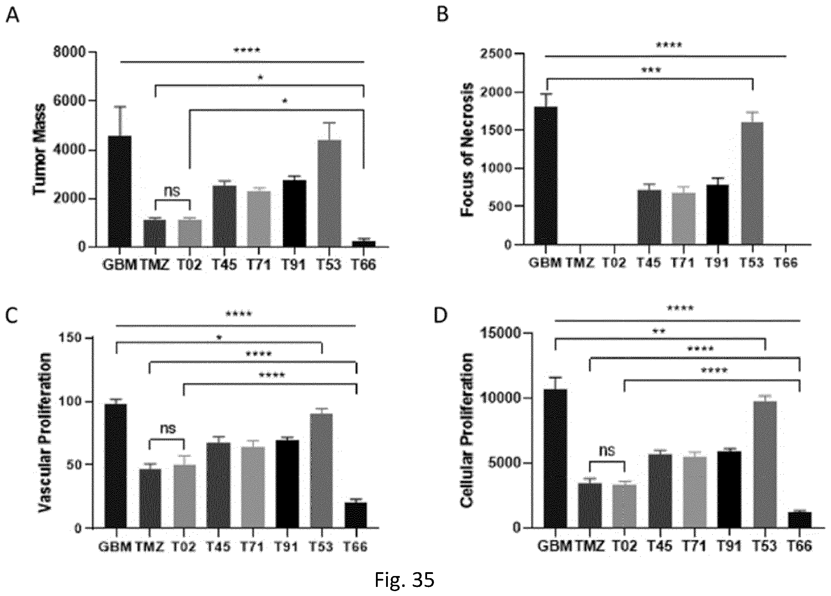

In the GBM rats, histopathological examination of brain tissues revealed extensive tumor foci containing grey and white matter, see Fig. 34-35. In the central portions of the tumor foci, large areas of necrosis, intense vascular proliferation, and cells with a high level of mitotic figures and hyperchromatic cells were identified, particularly around blood vessels, see Fig. 34-35.

In the TMZ treated GBM rats, histopathological examination of brain tissues showed a reduction in the diameter of the tumor focus, with very few cells exhibiting mitosis and no observed necrotic cells. Mild levels of vascular proliferation and cell proliferation were observed, see Fig. 34-35.

In the TR-G002 treated group, histopathological examination of brain tissues revealed a significantly reduced tumor focus with mild levels of vascular and cellular proliferation, see Fig. 34-35.

In the TR-G045 treated GBM rats, histopathological examination of brain tissues classified the tumor focus as moderate. Necrotic cells were present in the central regions, along with moderate mitosis, atypical hyperchromatic tumor cells, and vascular proliferation in the vicinity of healthy tissue, see Fig. 34-35.

In the TR-G053 treated GBM rats, histopathological examination of brain tissues showed a tumor focus including both grey and white matter. Severe necrosis in the inner parts of the tumor focus, intense mitosis around blood vessels, and hyperchromatic cells near healthy tissue were identified, see Fig. 34-35.

In the TR-G066 treated GBM rats, histopathological examination of brain tissues revealed few tumor cells, particularly around blood vessels, rather than a distinct tumor focus shape, see Fig. 34-35.

In the TR-G071 group, histopathological examination of brain tissues indicated an expanded tumor focus with moderate necrotic cells in the central part and moderate levels of mitosis and vascular proliferation, see Fig. 34-35.

In the TR-G091 treated group, histopathological examination of brain tissues revealed an extended tumor focus encompassing both grey and white matter. Moderate necrosis, mitotic figures, and atypical cells were observed in the central part of the tumor focus, see Fig. 24-35.

### Immunohistochemical and immunofluorescence findings

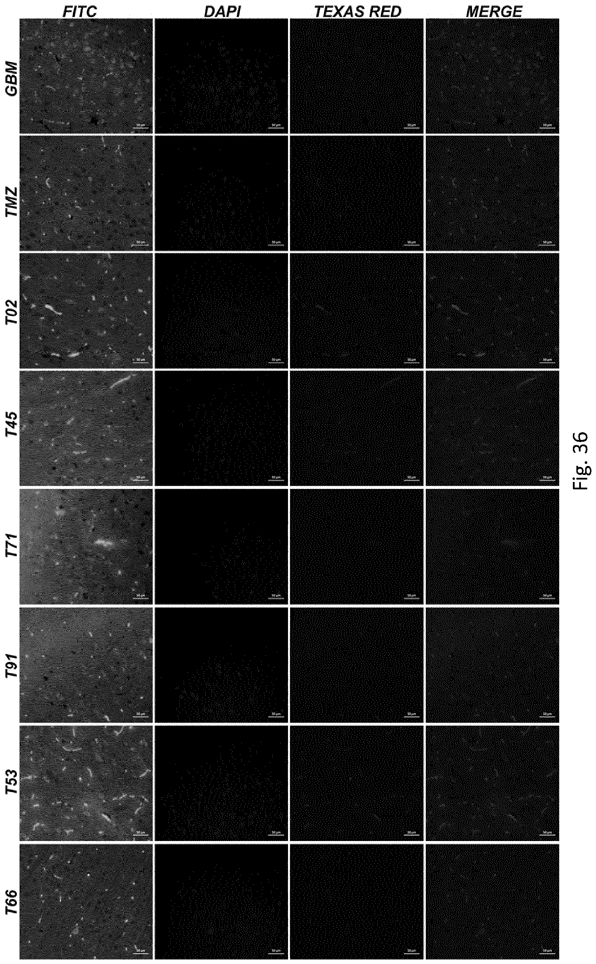

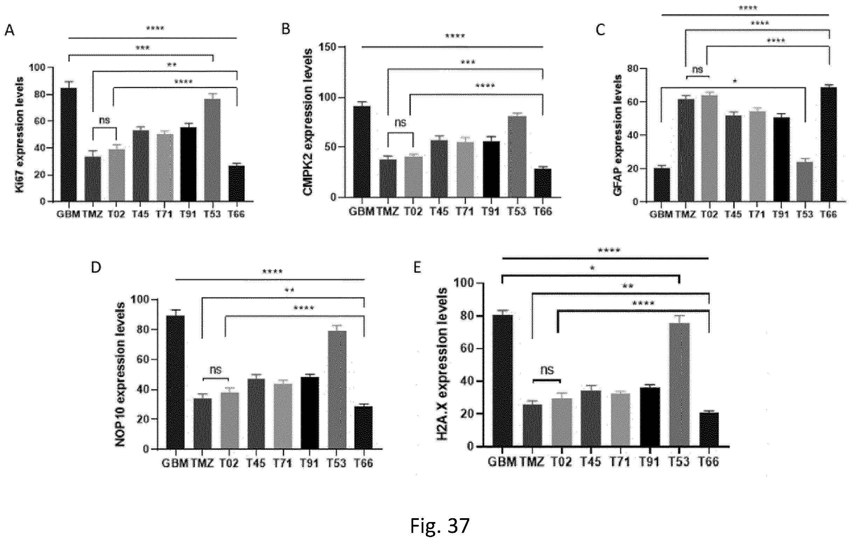

In the GBM rats, immunohistochemical and immunofluorescence staining of brain tissues revealed intense cytoplasmic expression of Ki67 in cells around blood vessels and near healthy tissue, severe cytoplasmic expression of CMPK2 in tumor cells, and mild GFAP expression in astrocytes in non-tumor regions, see Fig. 34. In brain tissues, severe cytoplasmic expressions of Nopio and H2AX were observed in the vicinity of tumor tissue, see Fig. 36-37.

In the TMZ treated GBM rats, immunohistochemical and immunofluorescence staining of brain tissues showed mild cytoplasmic expression of Ki67 and CMPK2 in tumor cells around blood vessels, and severe GFAP expression in astrocytes around the tumor, see Fig. 34. In these brain tissues, mild expressions of Nopio and H2AX were observed in neurons around tumor tissue, see Fig. 36-37.

In the TR-G002 treated GBM rats, immunohistochemical and immunofluorescence staining of brain tissues revealed mild cytoplasmic expression of Ki67 and CMPK2 in tumor cells around blood vessels, and severe GFAP expression in astrocytes around the tumor, see Fig. 34. In TR-G002 treated brain tissues, mild expressions of Nopio and H2AX were observed in neurons around tumor tissue, see Fig. 36-37.

In the TR-G045 treated GBM rats, immunohistochemical and immunofluorescence staining of brain tissues showed moderate cytoplasmic expression of Ki67 and CMPK2 in tumor cells, and moderate GFAP expression in astrocytes around the tumor, see Fig. 34. In the TR-G045 treated brain tissues, moderate cytoplasmic expressions of Nopio and H2AX were observed in neurons, see Fig. 36-37.

In the TR-G071 treated GBM rats, immunohistochemical and immunofluorescence staining of brain tissues revealed moderate Ki67 and CMPK2 expression in tumor cells, and moderate GFAP expression in astrocytes around the tumor, see Fig. 34. In the TR-G071 treated brain tissues, moderate expressions of Nopio and H2AX were detected in neurons, see Fig. 36-37.

In the TR-G091 treated group, immunohistochemical and immunofluorescence staining of brain tissues showed moderate cytoplasmic Ki67 expression, particularly around normal tissue and blood vessels, and moderate CMPK2 expression in tumor cells throughout the focus. Moderate GFAP expression in astrocytes around the tumor was observed, see Fig. 34. In the TR-G091 treated brain tissues, moderate cytoplasmic expressions of Nopio and H2AX were noted in neurons, especially around the tumor focus, see Fig. 36-37.

In the TR-G053 treated group, immunohistochemical and immunofluorescence staining of brain tissues revealed severe cytoplasmic expression of Ki67 and CMPK2 in tumor cells, and mild GFAP expression in astrocytes around the tumor mass, see Fig. 34. In TR-G053 treated brain tissues, severe cytoplasmic expressions of Nopio and H2AX were observed in neurons around the tumor tissue, see Fig. 36-37.

In the TR-G066 treated group, immunohistochemical and immunofluorescence staining of brain tissues showed very mild cytoplasmic expression of Ki67 and CMPK2 in tumor cells around blood vessels, and very severe GFAP expression in astrocytes around the tumor, see Fig. 34. In TR-G066 treated brain tissues, very mild cytoplasmic expressions of Nopio and H2AX were observed in neurons around tumor tissue, see Fig. 36-37.

### Haematological parameters

When haematological parameters were examined, no significant changes were observed in parameters other than WBC, HCT, and PLT values. As compared to the corresponding heathy group, slightly elevated levels WBC were noted in GBM rats. Treatment with TMZ and target compounds did not cause any significant changes in WBC level compared to the GBM rats. The PLT level was significantly increased in the GBM rats when compared to corresponding heathy group. In TMZ and target compounds treated GBM rats, no significant changes were found in PLT levels compared to GBM rats, see Table 15.

**Table 15 shows the haematological parameters.**

| **Group** | **WBC** | **RBC** | **HGB** | **HCT** | **MCV** | **MCH** | **MCHC** | **PLT** |
|---|---|---|---|---|---|---|---|---|
| | (10⁹/L) | (10¹²/L) | g/dL | % | fL | pg | g/dL | (10⁹ /L) |
| **Healthy control** | 2.34 | 7.88 | 14.66 | 47.68 | 59.44 | 17.62 | 31.42 | 621.20 |
| **GBM** | 3.15 | 8.62 | 15.30 | 46.80 | 56.60 | 17.70 | 31.40 | 876.00 |
| **TMZ** | 4.92 | 8.36 | 15.13 | 48.13 | 57.55 | 18.10 | 31.47 | 829.67 |
| **TR-G002** | 3.65 | 8.11 | 14.88 | 48.53 | 59.99 | 18.43 | 30.71 | 805.75 |
| **TR-G045** | 4.32 | 8.34 | 14.12 | 47.24 | 60.48 | 18.46 | 30.54 | 845.60 |
| **TR-G053** | 3.40 | 8.23 | 14.67 | 48.77 | 59.33 | 17.83 | 30.07 | 823.33 |
| **TR-G066** | 4.57 | 8.12 | 15.00 | 46.30 | 59.60 | 18.51 | 31.11 | 799.14 |
| **TR-G071** | 3.82 | 8.56 | 14.15 | 48.15 | 58.65 | 17.65 | 31.20 | 823.00 |
| **TR-G091** | 6.56 | 7.73 | 13.80 | 43.95 | 56.95 | 17.90 | 31.40 | 744.50 |

### Plasma chemistry parameters

Table 16 shows the levels of AST, ALT and LDH in the healthy and experimental rats. GBM rats showed significantly greater levels of all of these enzyme activities than healthy rats. Treating GBM rats with TR-G045, TR-G053, and TR-G066, respectively, significantly decreased these levels in comparison to the levels in untreated GBM rats.

**Table 16 shows plasma chemistry parameters.**

| Group | ALP | AST | ALT | LDH | TG | TCHOL | HDLC | LDL C | GLU | CREA. | UA |
|---|---|---|---|---|---|---|---|---|---|---|---|
| | IU/L | IU/L | IU/L | IU/L | mmol/l | mmol/l | mmol/l | mmol/l | mmol/l | mmol/l | mmol/l |
| Healthy control | 134.82 | 91.82 | 37.5 | 477.1 | 120 | 52.32 | 35.6 | 5.36 | 291.1 | 0.36 | 1.74 |
| GBM | 132.7 | 127.6 | 62.9 | 531.6 | 115 | 50.8 | 33.9 | 5.3 | 302.5 | 0.4 | 1.69 |
| TMZ | 128.7 | 114.63 | 42.4 | 499.85 | 110.82 | 58.75 | 37.58 | 5.12 | 310.68 | 0.43 | 1.67 |
| TR-G002 | 124.22 | 129.93 | 48.37 | 535.72 | 115.2 | 54.38 | 37.5 | 4.42 | 317.13 | 0.31 | 1.71 |
| TR-G045 | 138.47 | 104.7 | 54.5 | 506.78 | 119.82 | 56.52 | 37.58 | 5.28 | 315.97 | 0.35 | 1.6 |
| TR-G053 | 125.67 | 96.87 | 44.57 | 514 | 116.87 | 56.33 | 34.33 | 5.63 | 292.8 | 0.34 | 1.64 |
| TR-G066 | 137.08 | 96.48 | 38.94 | 476.16 | 122.32 | 58.56 | 34.24 | 4.94 | 294.9 | 0.38 | 1.7 |
| TR-G071 | 130.5 | 132.5 | 61.25 | 504.2 | 129.35 | 53.35 | 34.9 | 5.65 | 295.3 | 0.34 | 1.69 |
| TR-G091 | 122.87 | 127.9 | 46.97 | 546.17 | 121.4 | 54.43 | 36.57 | 5.03 | 298.57 | 0.29 | 1.7 |

### Genotoxicity evaluation by micronucleus assay

The micronuclei (MN) frequency was increased in GBM rats and the treated GBM rats compared to the healthy control, see Table 17. The highest observed MN frequency was obtained after treatment with TMZ while all the groups treated with the target compounds exhibited a lower MN frequency than both GBM rats and TMZ treated GBM rats. The lowest MN frequency was obtained for the TR-G053 treated GBM rats.

**Table 17 shows the MN frequencies of healthy rats, GBM rats before and after treatment with TMZ and the target compounds.**

| **Groups** | **MN frequency** |
|---|---|
| **Healthy control** | 3.50 ± 0.98 |
| **GBM** | 12.70 ± 2.21 |
| **TMZ** | 14.32 ± 3.10 |
| **TR-G002** | 11.62 ± 2.34 |
| **TR-G045** | 12.05 ± 1.78 |
| **TR-G053** | 9.67 ± 1.21 |
| **TR-G066** | 10.91 ± 1.89 |
| **TR-G071** | 12.43 ± 2.09 |
| **TR-G091** | 11.65 ± 2.30 |

The results obtained in the Examples section provide evidence that treatment with a compound according to formula (I) reduced the proliferation of glioblastoma cells. Furthermore, it was shown in an animal model that treating rats with induced GBM with compounds according to formula (I), specifically TR-G002, TR-G045, TR-G053, TR-G066, TR-G071 and TR-G091, resulted in a prolonged survival, decreased tumor volume, decelerated tumor growth and in some cases (treatment with TR-G046 and TR-G066) reduced the tumor size, and decreased the vascular and cellular proliferation. These results indicate that compounds according to formula (I) are well-suited for treatment of glioblastoma, such as GBM.

### References

Abad N, Al-Ostoot F.A, Ashraf S, Buhlak S, et al. (2023). "Synthesis, crystal structure, DFT, Hirshfeld surface analysis, energy frameworks and in-Silico drug-targeting PFKFB3 kinase of novel triazolequinoxalin derivative (TZQ) as a therapeutic Strategy against cancer." Heliyon 9, 11, e21312,
Abad N, et al. (2020). "A newly synthesized nitrogen-rich derivative of bicyclic quinoxaline Structural and conceptual DFT reactivity study." Journal of Physical Organic Chemistry 33.6 (2020): e4055.
Agnihotri S, Burrell KE, Wolf A, et al (2013). "Glioblastoma, a brief review of history, molecular genetics, animal models and novel therapeutic strategies". AITE, 61, 25-41.
Agnihotri, S., and Zadeh, G. (2016). "Metabolic reprogramming in glioblastoma: the influence of cancer metabolism on epigenetics and unanswered questions". Neuro Oncol. 18, 160-172.
Alcantara Llaguno, S. R., and Parada, L. F. (2016). "Cell of origin of glioma: biological and clinical implications". Br. J. Cancer 115, 1445-1450.
Anbarasi K, Sabitha KE, Devi CSS (2005) Lactate dehydrogenase isoenzyme patterns upon chronic exposure to cigarette smoke: Protective effect of bacoside A. Environ Toxicol Pharmacol 20:345-350. https://doi.org/10.1016/j.etap.2005.03.006
Angell RM, Atkinson FL, Brown MJ, Chuang TT, Christopher JA, Cichy-Knight M, Dunn AK, Hightower KE, Malkakorpi S, Musgrave JR, Neu M. N-(3-Cyano-4, 5, 6, 7-tetrahydro-1-benzothien-2-yl) amides as potent, selective, inhibitors of JNK2 and JNK3. Bioorganic & medicinal chemistry letters. 2007 Mar 1;17(5):1296-301.
Biasini, M.; et al. SWISS-MODEL: modelling protein tertiary and quaternary structure using evolutionary information. Nucleic Acids Res. 2014, 42, 252-258.
Bowie, J.; Lüthy, R.; Eisenberg, D. A method to identify protein sequences that fold into a known three-dimensional structure. Science 1991, 253, 164-70.
Chovanec, M., Abu Zaid, M., Hanna, N., El-Kouri, N., Einhorn, L. H., and Albany, C. (2017). Long-term toxicity ofcisplatin in germ-cell tumor survivors. Ann.Oncol. 28, 2670-2679.
Cobbs, C. S. (2013). Cytomegalovirus and brain tumor: epidemiology, biology and therapeutic aspects. Curr. Opin. Oncol. 25, 682-688. doi: 10-1097/CCO-0000000000000005.
Colovos C, Yeates TO. Verification of protein structures: Patterns of non-bonded atomic interactions. Protein Sci .1993;2:1511-1519.
Dallakyan, S., and Olson, A. J. (2015). "Small-Molecule Library Screening by Docking with PyRx," in Chemical Biology (Springer), 243-250. doi:10.1007/978-1-4939-2269-7_19.
Fang, C.; Wang, K.; Stephen, Z.R.; Mu, Q.; Kievit, F.M.; Chiu, D.T.; Press, O.W.; Zhang, M. Temozolomide nanoparticles for targeted glioblastoma therapy. ACS Appl. Mater. Interfaces 2015, 7, 6674-6682.
Fetro, C., and Scherman, D. (2020). Drug Repurposing in Rare Diseases: Myths and Reality. Therapie 75, 157-160.
Gittleman H, Lim D, Kattan MW, Chakravarti A, Gilbert MR, Lassman AB, Lo SS, Machtay M et al (2017) An independently validated nomogram for individualized estimation of survival among patients with newly diagnosed glioblastoma: NRG Oncology RTOG 0525 and 0825. Neuro-oncology. 19(5):669-677.
Grand View Research (GVR) Report: Glioblastoma Multiforme Treatment Market Size, Share & Trends Analysis Report By Treatment (Radiation Therapy, Immunotherapy), By Drug Class, By End Use, By Region, And Segment Forecasts, 2021-2028, Report ID: GVR-4-68039-527-3.
Halgren, Thomas A. 1996. "Merck Molecular Force Field. I. Basis, Form, Scope, Parameterization, and Performance of MMFF94" Journal of Computational Chemistry 17 (5-6): 490-519.
Healthcare Analyst, Inc.Report: Global Glioblastoma Multiforme Drugs Market $2.3 Billion by 2029, October 12, 2022
Heo, Hye Jin, et al. "Combination therapy with cilostazol, aripiprazole, and donepezil protects neuronal cells from β-amyloid neurotoxicity through synergistically enhanced SIRT1 expression." The Korean Journal of Physiology & Pharmacology: Official Journal of the Korean Physiological Society and the Korean Society of Pharmacology 24.4 (2020): 299. doi: 10.4196/kjpp.2020.24.4.299
Karrouchi, Khalid, et al. (2017) "New pyrazole-hydrazone derivatives: X-ray analysis, molecular structure investigation via density functional theory (DFT) and their high in-situ catecholase activity." International journal of molecular sciences 18.11: 2215.
Karrouchi, Khalid, et al. "Synthesis, antioxidant and analgesic activities of Schiff bases of 4-amino-1, 2, 4-triazole derivatives containing a pyrazole moiety." Annales Pharmaceutiques Franqaises. Vol. 74. No. 6. Elsevier Masson, 2016.
Larsson I, Uhlén M, Zhang C and Mardinoglu A (2020) Genome-Scale Metabolic Modeling of Glioblastoma Reveals Promising Targets for Drug Development. Front. Genet. 11:381.
Laskowski RA, MacArthur MW, Moss D, Thornton J M. PROCHECK: a program to check the stereochemical quality of protein structures. J App Cryst. 1993;26(2):283-291.
Liu, Chan-Chuan, et al. "Cilostazol eliminates radiation-resistant glioblastoma by re-evoking big conductance calcium-activated potassium channel activity." American journal of cancer research 11.4 (2021): 1148.
Missioui, Mohcine, et al. "Greener pastures in evaluating antidiabetic drug for a quinoxaline Derivative: Synthesis, Characterization, Molecular Docking, in vitro and HSA/DFT/XRD studies." Arabian Journal of Chemistry 15.6 (2022): 103851.
Molecular Operating Environment (MOE), 2019.01; Chemical Computing Group ULC, 1010 Sherbooke St. West, Suite #910, Montreal, QC, Canada, H3A 2R7, 2019." 2019.
Murata, Kohei, et al. "Phosphodiesterase type III inhibitor, cilostazol, inhibits colon cancer cell motility." Clinical & experimental metastasis 17 (1999): 525-530. DOI: 10.1023/a:1006626529536
Nielsen, J., and Hohmann, S. (2017). Systems Biology. Weinheim: Wiley-VCH.
Nuha, Demokrat, et al. "Design and synthesis of novel 2, 4, 5-thiazole derivatives as 6-APA mimics and antimicrobial activity evaluation." Phosphorus, Sulfur, and Silicon and the Related Elements 196.10 (2021): 954-960.
Oliva, C. R., Zhang, W., Langford, C., Suto, M. J., and Griguer, C. E. (2017). Repositioning chlorpromazine for treating chemoresistant glioma through the inhibition of cytochrome c oxidase bearing the COX4-1 regulatory subunit. Oncotarget 8, 37568-37583.
Park SH, Kim JH, Bae SS, Hong KW, Lee DS, Leem JY, Choi BT, Shin HK. Protective effect of the phosphodiesterase III inhibitor cilostazol on amyloid β-induced cognitive deficits associated with decreased amyloid β accumulation. Biochem Biophys Res Commun. 2011;408:602-608. doi: 10.1016/j.bbrc.2011.04.068.
Pinheiro, T., Otrocka, M., Seashore-Ludlow, B., Rraklli, V., Holmberg, J., Forsberg- Nilsson, K., et al. (2017). A chemical screen identifies trifluoperazine as an inhibitor of glioblastoma growth. Biochem. Biophys. Res. Commun. 494, 477-483.
Rock K, McArdle O, Forde P, et al (2014). A clinical review of treatment outcomes in glioblastoma multiforme the validation in a non-trial population of the results of a randomised Phase III clinical trial: has a more radical approach improved survival? Br J Radiol, 85, 729-33.
Stupp, R.; Mason,W.P.; van den Bent, M.J.; Weller, M.; Fisher, B.; Taphoorn, M.J.B.; Belanger, K.; Brandes, A.A.; Marosi, C.; Bogdahn, U.; et al. Radiotherapy plus concomitant and adjuvant temozolomide for glioblastoma. N. Engl. J. Med. 2005, 352, 987-996.4
Stuppner S, Waskiewicz J, Ruiu A (2020) Gross tumour volume comparison in oropharynx carcinomas using different intelligent imaging software. A retrospective analysis. Polish J Radiol 85:e287-e292. https://doi.org/10.5114/pjr.2020.96156
Suryawanshi, Yogesh, Prasad Sanap, and Vishal Wani. "Advances in the synthesis of non-isocyanate polyurethanes." Polymer Bulletin 76 (2019): 3233-3246
Tamimi AF, Juweid M. Epidemiology and Outcome of Glioblastoma. In: De Vleeschouwer S, editor. Glioblastoma [Internet]. Brisbane (AU): Codon Publications; 2017 Sep 27. Chapter 8. Available from: https://www.ncbi.nlm.nih.gov/books/NBK470003/ doi:
   10.15586/codon.glioblastoma.2017.ch8
Thari, Fatima Zahra, et al. (2022) "Synthesis, crystal structures, α-glucosidase and α-amylase inhibition, DFT and molecular docking investigations of two thiazolidine-2, 4-dione derivatives." Journal of Molecular Structure 1261,132960
UniProt Consortium, T. (2018). UniProt: the universal protein knowledgebase. Nucleic Acids Res. 46, 2699. doi: 10.1093/nar/gky092

## Claims

1. A compound according to formula (I) 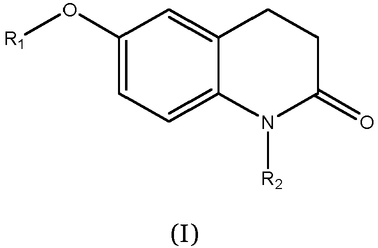 or a pharmaceutically acceptable salt or prodrug thereof;
wherein R1 is selected from optionally branched C3-C7 alkyl, optionally branched C2-C7 alkenyl, optionally substituted benzyl, 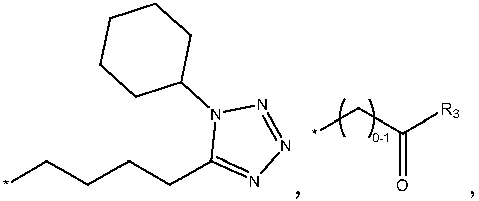 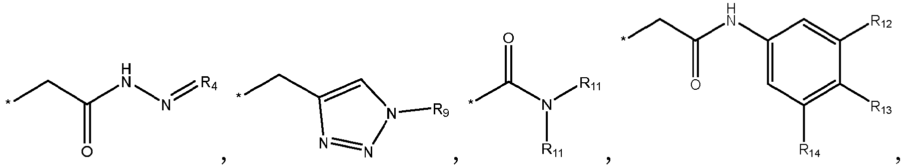 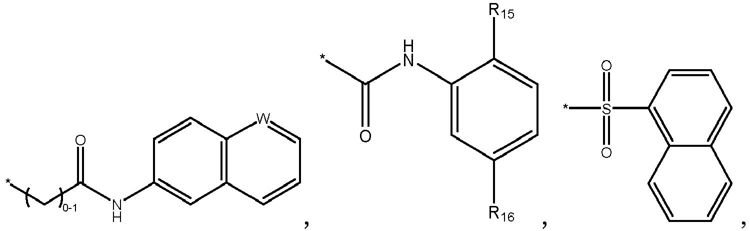 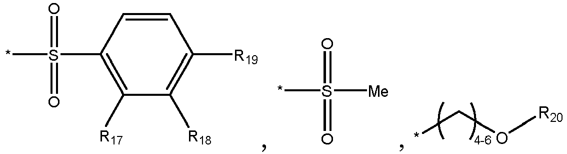 and 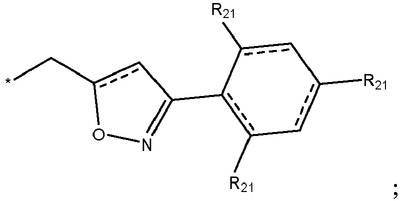
-̅ -̅ -̅ -̅ -̅ is independently a single bond or a double bond;
W is independently selected from carbon and nitrogen;
R3 is selected from optionally substituted naphthalene, 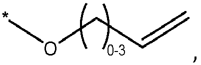 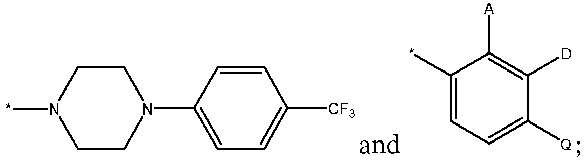
A is selected from -H, -X and -OMe ;
D is selected from -H, -X, -OMe and -CX3;
Q is selected from -H and -OMe;
X is a halogen;
R4 is selected from 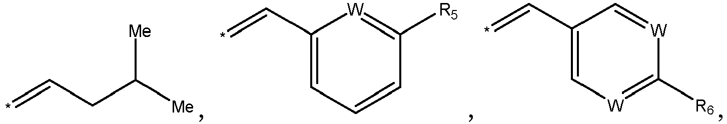 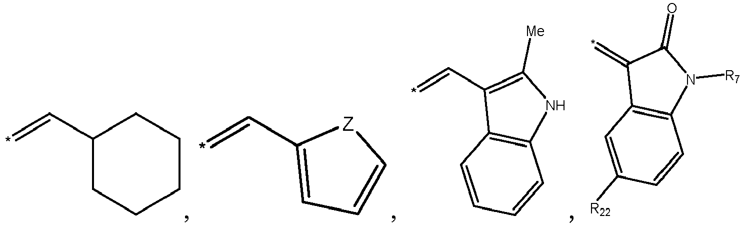 and 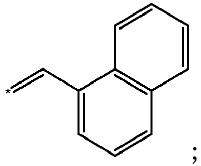
Z is selected from oxygen and sulphur;
R5 is selected from -H and 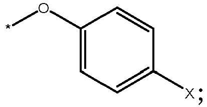
R6 is selected from -H, -X, C₃ alkyl, -NO₂ and 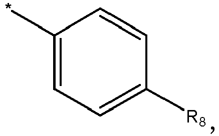
R7 is selected from -H, -Me;
R8 is selected from -H and -X;
R9 is selected from C3-C6 alkyl, 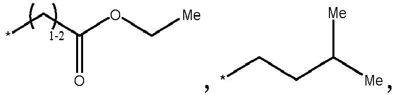 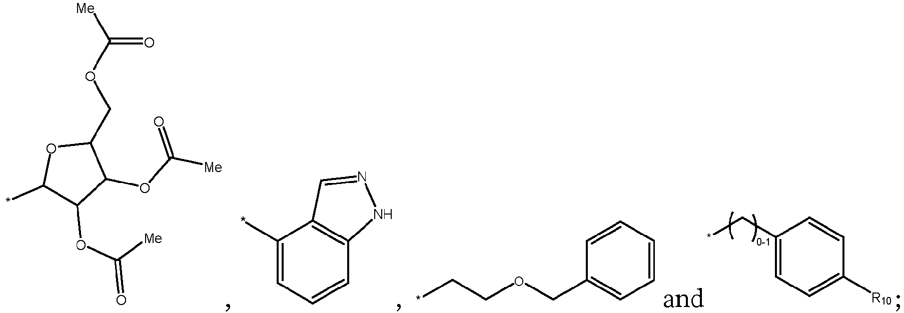
R10 is selected from -X and acetyl;
R11 is selected from a phenyl, C1-C3 alkyl and isopropyl;
R12 is selected from -H and -OMe;
R13 is selected from -OMe, phenyl, acetyl, morpholine and N-methyl piperazine;
R14 is selected from -H and -OMe provided that when R12 is -H, R13 and/or R14 is also -H;
R15 is selected from -H, -X, -Me, -OMe and -CF3;
R16 is selected from -H and -X;
R17 is selected from -H and -X;
R18 is selected from -H, -X and -OMe;
R19 is selected from -H, -NO₂, -OMe and-X;
R20 is selected from 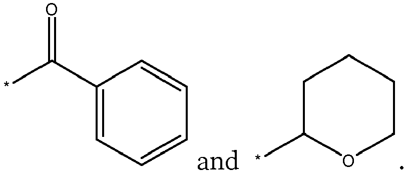
R21 is independently selected from -H and -X;
R22 is selected from -X and -NO₂;
R2 is selected from -H, optionally branched C3-C7 alkyl, C3-C5 alkenyl, 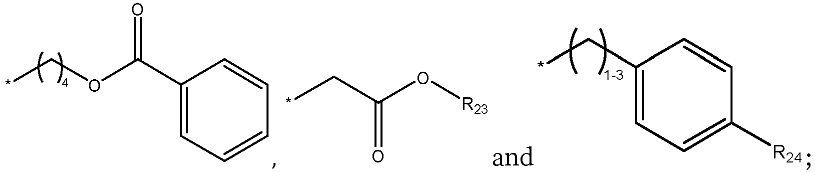
R23 is selected from C1-C4 alkyl and C3-C4 alkenyl; and
R24 is selected from -H, -F, phenyl and C2-C4 alkyl.

2. The compound according to claim 1, wherein R2 is -H.

3. The compound according to claim 1, wherein R2 is selected from 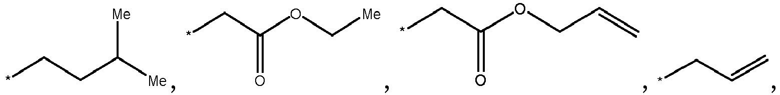 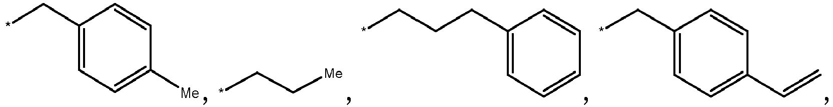 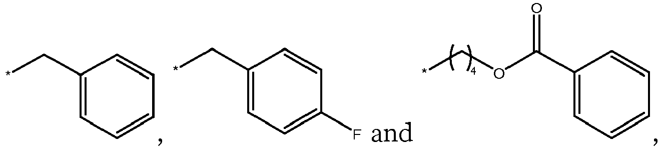 preferably

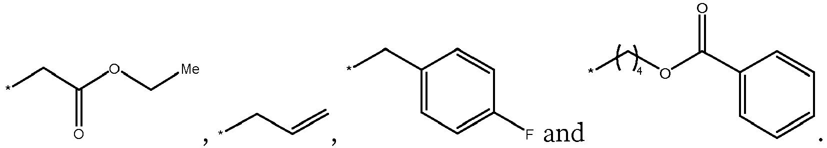

4. The compound according to claim 1 or 2, wherein R1 is

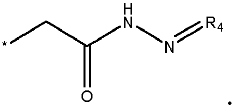

5. The compound according to claim 4, wherein R4 is selected from 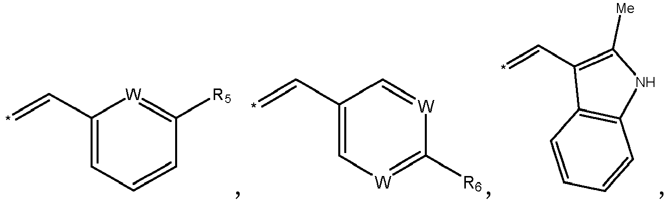 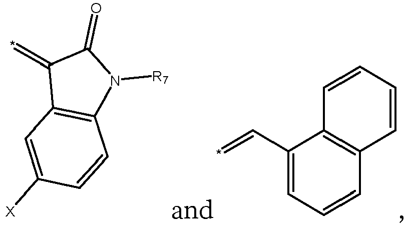 preferably 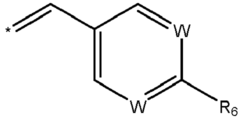 and 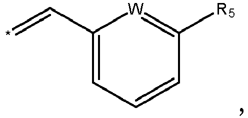 more preferably

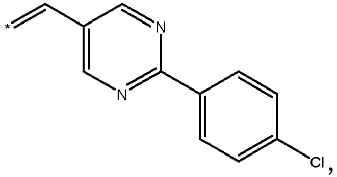

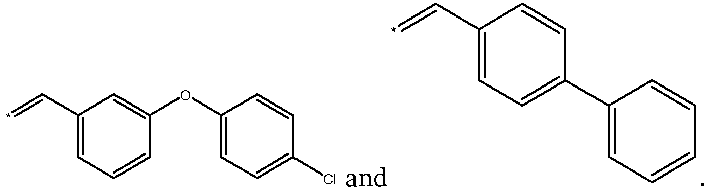

6. The compound according to claim 1 or 2, wherein R1 is a substituted benzyl, preferably 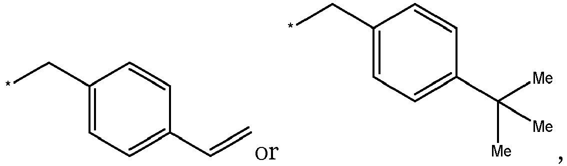 more preferably

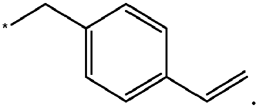

7. The compound according to claim 3, wherein R1 and R2 is

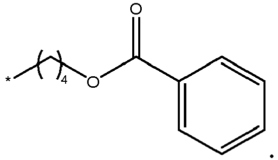

8. The compound according to claim 1 or 2, wherein R1 is a branched C3-C7 alkyl, preferably a branched C4-C7 alkyl, more preferably

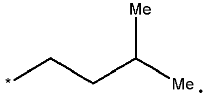

9. The compound according to claim 1 or 2, wherein R1 is 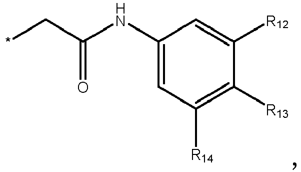 preferably 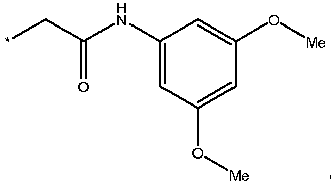 or

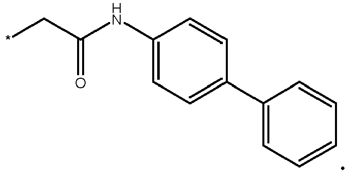

10. The compound according to claim 1 or 2, wherein R1 is 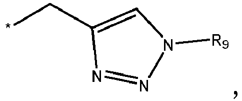 preferably

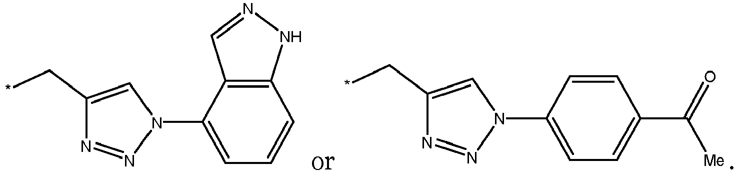

11. The compound according to claim 1 or 2, wherein R1 is 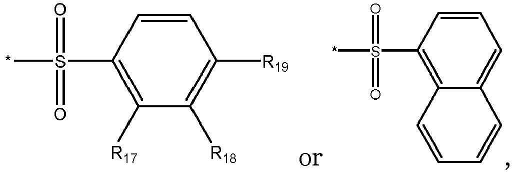 preferably 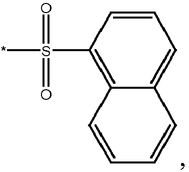 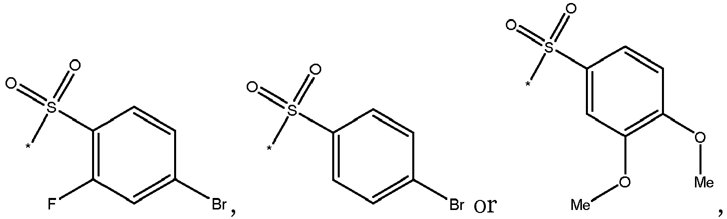 more preferably

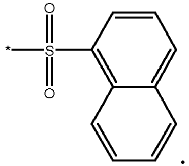

12. The compound according to claim 1 or 2, wherein R1 is 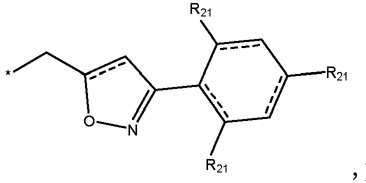 preferably 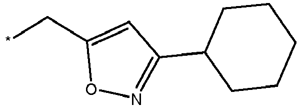 or

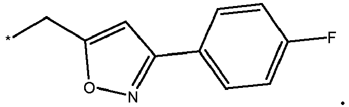

13. The compound according to claim 1 or 2, wherein R1 is 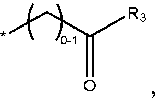 preferably

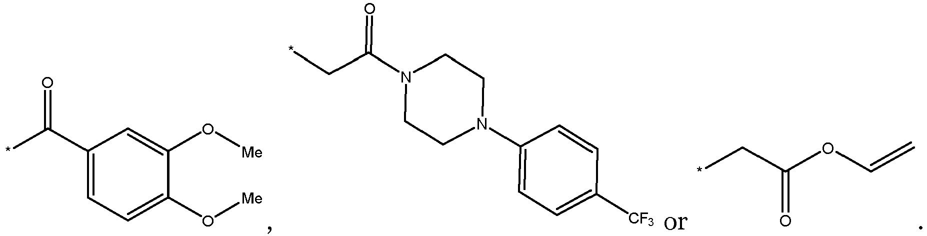

14. The compound according to claim 2 or 3, wherein R1 is

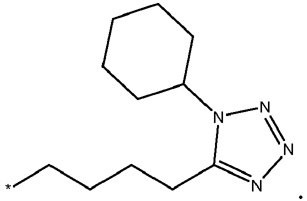

15. A pharmaceutical composition comprising a compound according to any one of claims 1-14.

16. A compound or a pharmaceutical composition according to any one of the previous claims, for use as a medicament.

17. A compound or a pharmaceutical composition according to any one of claims 1-15, for use in a method of treatment of glioma, such as glioblastoma, such as glioblastoma multiforme.
